(19) Europäisches Patentamt / European Patent Office / Office européen des brevets

(11) **EP 3 970 730 B1**

(12) **EUROPEAN PATENT SPECIFICATION**

(45) Date of publication and mention
of the grant of the patent:
**01.05.2024 Bulletin 2024/18**

(21) Application number: **21206569.2**

(22) Date of filing: **30.01.2017**

(51) International Patent Classification (IPC):
***A61K 33/00*** *(2006.01)* ***A61M 16/12*** *(2006.01)*
***A61B 5/026*** *(2006.01)*

(52) Cooperative Patent Classification (CPC):
**A61B 5/02007; A61B 5/0215; A61B 5/029;
A61B 5/4842; A61B 5/4848; A61B 5/686;
A61B 5/7275; A61B 8/0883; A61M 60/178;
A61M 60/20; A61M 60/515; A61M 60/538;
A61P 9/04; A61P 9/12; A61P 11/00;** (Cont.)

(54) **SYSTEM FOR USE AND MONITORING OF INHALED NITRIC OXIDE WITH LEFT VENTRICULAR ASSIST DEVICES**

SYSTEM ZUR VERWENDUNG UND ÜBERWACHUNG VON INHALIERTEM STICKOXID MIT LINKSVENTRIKULÄREN UNTERSTÜTZUNGSVORRICHTUNGEN

SYSTÈME POUR L'UTILISATION ET SURVEILLANCE DE L'OXYDE NITRIQUE INHALÉ AVEC DES DISPOSITIFS D'ASSISTANCE VENTRICULAIRE GAUCHE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2016 US 201662294711 P**

(43) Date of publication of application:
**23.03.2022 Bulletin 2022/12**

(62) Document number(s) of the earlier application(s) in accordance with Art. 76 EPC:
**17750567.4 / 3 413 899**

(73) Proprietor: **Mallinckrodt Pharmaceuticals Ireland Limited
Dublin 15 (IE)**

(72) Inventors:
- **POTENZIANO, Jim
  Binghampton, NY 13901 (US)**
- **GREENE, Douglas, Alan
  Basking Ridge, NJ 07920 (US)**
- **FLANAGAN, Craig
  Belmar, NJ 07719 (US)**

(74) Representative: **Jones, Nicholas Andrew
Withers & Rogers LLP
2 London Bridge
London SE1 9RA (GB)**

(56) References cited:
- **ENDO GEORGE J ET AL: "Nitric oxide inhalation prompts weaning from the right ventricular assist device: Evaluation under continuous-flow biventricular assistance", JOURNAL OF THORACIC AND CARDIOVASCULAR SURGERY, vol. 124, no. 4, 10 February 2002 (2002-02-10), pages 739-749, XP029482994, ISSN: 0022-5223, DOI: 10.1067/MTC.2002.124669**
- **ESTEP JERRY D ET AL: "The Role of Echocardiography and Other Imaging Modalities in Patients With Left Ventricular Assist Devices", JACC: CARDIOVASCULAR IMAGING, vol. 3, no. 10, 31 October 2010 (2010-10-31), pages 1049-1064, XP029642898, ISSN: 1936-878X, DOI: 10.1016/J.JCMG.2010.07.012**

**(Cont. next page)**

(52) Cooperative Patent Classification (CPC): (Cont.)
A61B 5/0816; G16H 20/40; G16H 50/30

**Description**

TECHNICAL FIELD

Embodiments of the present invention generally relate to the field of devices for delivering and monitoring inhaled nitric oxide (NO).

BACKGROUND

Modern semi-permanent continuous-flow left ventricular assist devices (LVADs) are cost-effective and durable surgically-implanted mechanical devices which augment or substitute for a poorly functioning or nonfunctioning diseased left ventricle of the heart to maintain blood circulation to the body. LVADs are now considered to be a reasonable alternative to orthotopic heart transplantation, especially given the severe shortage of suitable donor organs. Continuous-flow LVADs have replaced earlier pulsatile models because they are more durable, less cumbersome, and have been shown to increase survival, exercise capacity and quality of life. LVADs are used to sustain patients with advanced congestive heart failure (CHF) who cannot be managed medically either as a bridge-to-heart transplantation, as destination therapy or, in those patients whose CHF is deemed at least partially reversible, as a bridge-to-recovery. The frequency of sufficient recovery to permit LVAD explantation is estimated to be 10-20% in CHF of non-ischemic etiology and in <1% in ischemic CHF. LVAD implantation is generally indicated in CHF when cardiac index (CI) is <2 L/min/m$^2$, systemic systolic arterial pressure is <90 mm Hg, left atrial pressure is >20 mm Hg, or the systemic vascular resistance is >1.57 mm Hg/mL. Advances in the durability and miniaturization of LVADs, afforded by continuous-flow rather than pulsatile design, have enabled more extensive and longer-duration utilization.

Unfortunately, failure of the right ventricle has been reported in 15% of continuous-flow LVAD recipients within the first 30 days following implantation and in 20-50% of LVAD recipients overall. As such, right ventricular failure remains a major limitation of LVAD utility, and is associated with markedly poorer prognosis.

ENDO GEORGE J ET AL: "Nitric oxide inhalation prompts weaning from the right ventricular assist device: Evaluation under continuous-flow biventricular assistance",JOURNAL OF THORACIC AND CARDIOVASCULAR SURGERY, vol. 124, no. 4, 10 February 2002 discloses administering inhaled nitric oxide while measuring hemodynamic parameters such as left arterial pressure LAP during left ventricular assist device LVAD support.

Furthermore, continuous-flow LVADs generate reduced pulsatility of peripheral perfusion compared to pulsatile-flow LVAD devices and/or the normal circulation derived from a well-functioning human heart as measured by pulsatility index, pulse pressure and/or the frequency of opening of the aortic valve, and this reduced pulsatility has been implicated in a number of adverse events including reduced peripheral vascular compliance, gastrointestinal bleeding, arteriovenous malformations, hemolysis, pump thrombosis, aortic insufficiency and lower rate of recovery of left ventricular function.

Accordingly, there is a need for adjunctive therapies that enhance the use of LVADs and/or reduce the risk of right ventricular failure associated with LVADs and/or reduce the risk of other LVAD-related adverse events.

SUMMARY

A system according to the invention is defined in the independent claim. One or more aspects of the present disclosure that is not part of the claimed invention provide new adjunctive therapies that enhance the effectiveness of LVADs and/or reduce the risk of right ventricular failure associated with LVADs.

One aspect of the present disclosure that is not part of the claimed invention relates to a method of determining whether a patient with pulmonary hypertension will resolve the pulmonary hypertension with continued use of an LVAD. In various examples of this aspect, the method comprises measuring one or more pulmonary hemodynamic parameters of a patient with an LVAD to obtain a first pulmonary hemodynamic value; after obtaining the first pulmonary hemodynamic value, administering inhaled NO to the patient with the LVAD; and measuring one or more pulmonary hemodynamic parameters of the patient during or after the inhaled NO administration to obtain a second pulmonary hemodynamic value. A significant change in the pulmonary hemodynamic parameter from the first pulmonary hemodynamic value to the second pulmonary hemodynamic value can indicate that the patient is likely to resolve the pulmonary hypertension after continued use of the LVAD. For example, a significant change in the pulmonary hemodynamic parameter can be at least 10 mm Hg mPAP and/or at least 20% PVR, or equivalent changes as shown by echocardiography, MRI or other imaging technology.

In some examples of this aspect, the inhaled NO is administered at a concentration of 5 to 80 ppm for at least 10 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 10 minutes, about 15 minutes, about 20 minutes, about 25 minutes, about 30 minutes, about 35 minutes, about 40 minutes, about 45 minutes, about 50 minutes, about 55 minutes, and about 60 minutes.

Exemplary pulmonary hemodynamic parameters include mean pulmonary artery pressure (mPAP), diastolic pulmonary artery pressure (dPAP), pulmonary capillary wedge pressure (PCWP), transpulmonary gradient (TPG) and pulmonary vascular resistance (PVR). Other pulmonary hemodynamic parameters include combinations of and/or interrelations between these parameters, such as the difference between dPAP and PCWP. The one or more pulmonary hemodynamic parameters may be measured or estimated by any appropriate procedures, such as by performing a right heart catheterization, MRI or echocardiography.

In one or more examples, the method further comprises placing the patient on a heart transplant list if there is a significant change in the pulmonary hemodynamic parameter from the first pulmonary hemodynamic value to the second pulmonary hemodynamic value, such as a decrease in mPAP of at least 10 mm Hg and/or a decrease in PVR at least 20%. In some examples, the method further comprises explanting the LVAD and implanting a donor heart in the patient.

As an alternate to the above thresholds of 10 mm Hg mPAP and/or 20% PVR, other significant changes in the pulmonary hemodynamic parameter may be a decrease of 5 mm Hg (for pressure-related parameters such as mPAP or TPG) or a change in the parameter of at least 5% (for all parameters). Exemplary significant changes in the pulmonary hemodynamic parameter include a change of at least 5 mm Hg, at least 6 mm Hg, at least 7 mm Hg, at least 8 mm Hg, at least 9 mm Hg, at least 10 mm Hg, at least 15 mm Hg, at least 20 mm Hg, or at least 25 mm Hg, and/or at least 5%, at least 10%, at least 15%, at least 20%, at least 25%, at least 30%, at least 35%, at least 40% or at least 50%.

Another aspect of the present disclosure that is not part of the claimed invention relates to a method of optimizing the settings of an LVAD. In various examples of this aspect, the method comprises administering inhaled NO to a patient having an LVAD; performing an echocardiogram on the patient during the administration of inhaled NO; and adjusting or setting one or more parameters of the LVAD during the echocardiogram and during the administration of inhaled NO. Instead of performing an echocardiogram, other appropriate techniques may be used to set the LVAD parameters. In one or more examples adjusting or setting the LVAD parameters during administration of NO helps to optimize cardiac output.

In one or more examples of this aspect, adjusting or setting one or more parameters of the LVAD comprises one or more of (i) determining a low pump speed setting for the LVAD based on the minimal pump speed necessary for the patient's aortic valve to open with each heart beat or (ii) determining a high speed setting for the LVAD based on the pump speed at which the septum of the patient's heart flattens. In some examples, augmenting aortic valve opening and closing without flattening the septum could include setting a constant speed, or setting a range over which the speed could be modulated to accomplish this, such as in pulse modulation continuous flow.

In some examples of this aspect, the inhaled NO is administered at a concentration of 5 to 80 ppm for at least 10 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 1.5 hours, about 2 hours, about 2.5 hours and about 3 hours.

In some examples, the LVAD settings are changed over a series of incremental adjustments. For example, the LVAD pump speed may be adjusted upwards in two or more steps. One or more or all of these steps may be performed during the administration of inhaled NO as described herein.

Another aspect of the present disclosure that is not part of the claimed invention relates to a method of reducing the risk of right ventricular failure during LVAD use. In various examples of this aspect, the method comprises administering inhaled NO to a patient with an LVAD for at least 12 hours a day for at least 20 days.

Due to the fact that a patient with an LVAD had preexisting left ventricular dysfunction, it may be important to ensure that the LVAD is properly functioning prior to administering inhaled NO. Accordingly, in some examples, the method further comprises confirming that the LVAD is functioning properly before administering inhaled NO.

**[0019]** In one or more examples, the inhaled NO is administered after a patient has been weaned from cardiopulmonary bypass (CPB).

The inhaled NO may be administered for several days to many months or even longer. Exemplary treatment times include 10 days, 15 days, 20 days, 25 days, 30 days, 35 days, 40 days, 45 days, 2 months, 3 months, 4 months, 5 months, 6 months, 7 months, 8 months, 9 months, 10 months, 11 months, 1 year, 1.5 years, or 2 years. In some embodiments, the patient is administered inhaled NO indefinitely.

In some examples of this aspect, the inhaled NO is administered at a concentration of 5 to 80 ppm for at least 12 hours a day. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 12 hours a day, about 14 hours a day, about 16 hours a day, about 18 hours a day, about 20 hours a day, about 22 hours a day, or up to 24 hours a day.

Alternatively, the dose of NO may be prescribed based on the patient's ideal body weight (IBW). Exemplary NO doses may be in the range of about 25 to about 150 μg/kg IBW/hr, such as about 25, about 30, about 35, about 40, about 45, about 50, about 60, about 70, about 80, about 90, about 100, about 110, about 120, about 130, about 140 or about 150 μg/kg IBW/hr.

In one or more examples, the method further comprises monitoring one or more output parameters of the LVAD and/or one or more hemodynamic parameters of the patient, comparing the one or more output parameters and/or the one or more hemodynamic parameters to a predetermined range, and adjusting the dose of inhaled NO if the one or more outputs parameters and/or the one or more hemodynamic parameters are outside of the predetermined range. In some examples, the method further comprises providing an alert if the one or more output parameters and/or the one or more hemodynamic parameters are outside of the predetermined range. Such alerts can include an audible alert, a visual alert, a somatosensory alert (*e.g.* vibration) and/or a text alert. The inhaled NO dose may be adjusted automatically (*e.g.* by the NO delivery device or a control system in communication with the NO delivery device), or may be manually adjusted by a physician or other user.

Examples of LVAD parameters that may be monitored include, but are not limited to, pump speed (*e.g.* rpm), pump flow (*e.g.* L/min), pulsatility index, battery level, and LVAD status (*e.g.* operational, presence or absence of warnings).

Another aspect of the present disclosure that is not part of the claimed invention relates to a method of monitoring the left ventricle of a patient with an LVAD to determine whether the left ventricle of the patient is improving. In various examples of this aspect, the method comprises reducing the pump speed of the LVAD or turning off the LVAD; measuring one or more pulmonary hemodynamic parameters of the patient to obtain a first pulmonary hemodynamic value; preloading the left ventricle by administering inhaled NO to the patient; and measuring one or more pulmonary hemodynamic parameters after or during administration of inhaled NO to obtain a second pulmonary hemodynamic value. In some examples, the pulmonary hemodynamic parameter is selected from LAP, PCWP and CO, or may be any assessment of the left ventricular reserve to compensate for increased left ventricular preload that can be measured through right heart catheterization, echocardiographic, MRI or other techniques.

In some examples of this aspect, the inhaled NO is administered at a concentration of 5 to 80 ppm for at least 10 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 1.5 hours, about 2 hours, about 2.5 hours, about 3 hours, about 3.5 hours, about 4 hours, about 5 hours, about 6 hours, about 7 hours or about 8 hours.

According to one or more examples, an increase in LAP and/or PCWP from the first pulmonary hemodynamic value to the second pulmonary hemodynamic value of less than 5 mm Hg indicates that the left ventricle is improving. Other exemplary values that indicate an improvement in the left ventricle include an LAP and/or PCWP increase of less than 1 mm Hg, 2 mm Hg, 3 mm Hg, 4 mm Hg, 6 mm Hg, 7 mm Hg, 8 mm Hg, 9 mm Hg, 10 mm Hg, 11 mm Hg, 12 mm Hg, 13 mm Hg, 14 mm Hg or 15 mm Hg. In some examples, the method further comprises modifying treatment if the left ventricle is improving, such as explanting the LVAD from the patient. Other modifications in treatment can include changing the supportive medication (*e.g.* diuretics and/or inotropic medications) that the patient is given, such as reducing the supportive medication.

Yet another aspect of the present disclosure that is not part of the claimed invention relates to a method of exercising a heart of a patient having an LVAD. In various examples of this aspect, the method comprises reducing and/or modulating the pump speed of the LVAD or turning off the LVAD; preloading the left ventricle by administering inhaled NO to the patient for at least 5 minutes; discontinuing the inhaled NO administration; and repeating the preloading and discontinuation to exercise the left ventricle of the patient's heart.

In some examples of this aspect, the inhaled NO is administered at a concentration of 5 to 80 ppm for at least 5 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 5 minutes, about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 1.5 hours, about 2 hours, about 2.5 hours, about 3 hours, about 3.5 hours, about 4 hours, about 5 hours, about 6 hours, about 7 hours or about 8 hours.

Alternatively, the dose of NO may be prescribed based on the patient's ideal body weight (IBW). Exemplary NO doses may be in the range of about 25 to about 150 μg/kg IBW/hr, such as about 25, about 30, about 35, about 40, about 45, about 50, about 60, about 70, about 80, about 90, about 100, about 110, about 120, about 130, about 140 or about 150 μg/kg IBW/hr.

The preloading of the left ventricle may be performed multiple times per day, such as twice a day, three times a day, four times a day, five times a day, six times a day, seven times a day, eight times a day, nine times a day or ten times a day. Alternatively, the preloading may be performed once a day. If the preloading is performed multiple times per day, the preloading procedures may be clustered together (*e.g.* spaced apart by several minutes or a couple hours) or may be spread out throughout the day. The preloading of the left ventricle may also be performed once a week, two days a week, three days a week, four days a week, five days a week, six days a week, or seven days a week. In exemplary examples, the left ventricle is preloaded several times a day for several days a week, such as two to five times a day for two to four days a week or other combinations of the above daily and weekly preloading schedules.

Yet another aspect of the present disclosure that is not part of the claimed invention relates to a method of reducing the risk of adverse events during LVAD use. In various examples of this aspect, the adverse events are associated with reduced pulsatility caused by LVAD use and/or associated with impaired NO-mediated vascular function.

In various examples of this aspect, the method comprises administering inhaled NO to a patient with a continuous-flow or semi-pulsatile LVAD for at least 12 hours a day for at least 20 days.

Due to the fact that a patient with an LVAD had preexisting left ventricular dysfunction, it may be important to ensure that the LVAD is properly functioning prior to administering inhaled NO. Accordingly, in some examples, the method further comprises confirming that the LVAD is functioning properly before administering inhaled NO.

[0035] In one or more examples, the inhaled NO is administered after a patient has been weaned from cardiopulmonary bypass (CPB).

The inhaled NO may be administered for several days to many months or even longer. Exemplary treatment times include 10 days, 15 days, 20 days, 25 days, 30 days, 35 days, 40 days, 45 days, 2 months, 3 months, 4 months, 5 months, 6 months, 7 months, 8 months, 9 months, 10 months, 11 months, 1 year, 1.5 years, or 2 years. In some embodiments, the patient is administered inhaled NO indefinitely.

In some examples of this aspect, the inhaled NO is administered at a concentration of 5 to 80 ppm for at least 12 hours a day. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 12 hours a day, about 14 hours a day, about 16 hours a day, about 18 hours a day, about 20 hours a day, about 22 hours a day, or up to 24 hours a day.

Alternatively, the dose of NO may be prescribed based on the patient's ideal body weight (IBW). Exemplary NO doses may be in the range of about 25 to about 150 $\mu$g/kg IBW/hr, such as about 25, about 30, about 35, about 40, about 45, about 50, about 60, about 70, about 80, about 90, about 100, about 110, about 120, about 130, about 140 or about 150 $\mu$g/kg IBW/hr.

In one or more examples, the method further comprises monitoring one or more output parameters of the LVAD and/or one or more hemodynamic parameters of the patient, comparing the one or more output parameters and/or the one or more hemodynamic parameters to a predetermined range, and adjusting the dose of inhaled NO if the one or more outputs parameters and/or the one or more hemodynamic parameters are outside of the predetermined range. In some examples the method further comprises providing an alert if the one or more output parameters and/or the one or more hemodynamic parameters are outside of the predetermined range. Such alerts can include an audible alert, a visual alert, a somatosensory alert (*e.g.* vibration) and/or a text alert. The inhaled NO dose may be adjusted automatically (*e.g.* by the NO delivery device or a control system in communication with the NO delivery device), or may be manually adjusted by a physician or other user.

Examples of LVAD parameters that may be monitored include, but are not limited to, pump speed (*e.g.* rpm), pump flow (*e.g.* L/min), pulsatility index, battery level, and LVAD status (*e.g.* operational, presence or absence of warnings).

Yet another aspect that is not part of the claimed invention relates to a method of optimizing the inhaled NO dose to be used in conjunction with an LVAD, such as a continuous-flow or semi-pulsatile LVAD. In various examples of this aspect, the method comprises measuring endothelial function of a patient having a continuous-flow or semi-pulsatile LVAD, administering inhaled NO to the patient at a first dose, measuring the endothelial function of the patient during the administration of inhaled NO, and adjusting the inhaled NO dose to optimize endothelial function. Any appropriate techniques may be used to measure the endothelial function, including, but not limited to, flow-mediated dilation (FMD) and/or reactive hyperemic index (RHI). In one or more examples adjusting the NO dose helps to optimize the endothelial function and reduce the risk of adverse events associated with impaired NO-mediated vascular function.

In some examples the method further comprises measuring one or NO-related molecules and/or other biomarkers of endothelial function in the patient's blood and/or plasma. Exemplary NO-related molecules include whole blood and erythrocyte nitrite ($NO_2^-$), nitrate ($NO_3^-$) heme-nitrosylated hemoglobin [Hb(FeII)NO] and cysteine nitrosylated hemoglobin (also known as S-nitrosohemoglobin SNO-Hb), and nitrosylated plasma proteins. Other biomarkers of endothelial function include, but are not limited to, pulse amplitude tonometry (measuring post ischemic swelling of the fingertip) and peripheral arterial tonometry (using ultrasound to measure the size of the brachial artery after a blood pressure cuff is released)..

The present invention relates to a system for coordinating operation of the LVAD and the NO delivery device. Such a system may be used in any of the indications or methods described herein. In various embodiments of this aspect, the system comprises a control system in communication with the NO delivery device and/or the LVAD, wherein the control system monitors one or more parameters of the NO delivery device and/or one or more parameters of the LVAD and provides an alert if one or more parameters of the NO delivery device and/or LVAD are outside of a predetermined range. The system may also comprise the NO delivery device and/or the LVAD itself.

In one or more embodiments, the control system reduces a pump speed of the LVAD if there is a failure of the NO delivery device. The control system may also initiate a weaning procedure for the NO delivery device if there is a failure of the LVAD.

The control system may be integral to the NO delivery device, integral to the LVAD or a component of a stand-alone control module.

BRIEF DESCRIPTION OF THE DRAWINGS

The invention is set out in the appended claims.

FIG. 1 illustrates an exemplary NO delivery device that can be used in accordance with one or more embodiments of the invention

FIG. 2 illustrates an exemplary NO delivery device that can be used in accordance with one or more embodiments of the invention;

FIG. 3 illustrates an exemplary NO delivery device in communication with an LVAD that can be used in accordance with one or more embodiments of the invention;

FIG. 4 illustrates an exemplary hardware configuration that can be used in accordance with one or more embodiments of the invention;

FIG. 5 illustrates exemplary input and output parameters that can be used in accordance with one or more embodiments of the invention;

FIG. 6 illustrates an exemplary main menu with mode selection that can be used in accordance with one or more embodiments of the invention;

FIG. 7 illustrates an exemplary main menu with alarm settings that can be used in accordance with one or more embodiments of the invention;

FIG. 8 illustrates an exemplary main menu with configuration settings that can be used in accordance with one or more embodiments of the invention;

FIG. 9 illustrates an exemplary submenu for assessment of the likelihood of pulmonary hypertension resolution that can be used in accordance with one or more embodiments of the invention;

FIG. 10 illustrates an exemplary submenu for optimization of LVAD settings that can be used in accordance with one or more embodiments of the invention;

FIG. 11 illustrates an exemplary submenu for reducing the risk of right ventricular failure that can be used in accordance with one or more embodiments of the invention;

FIG. 12 illustrates an exemplary submenu for assessment of left ventricular function that can be used in accordance with one or more embodiments of the invention;

FIG. 13 illustrates an exemplary submenu for heart exercise that can be used in accordance with one or more embodiments of the invention; and

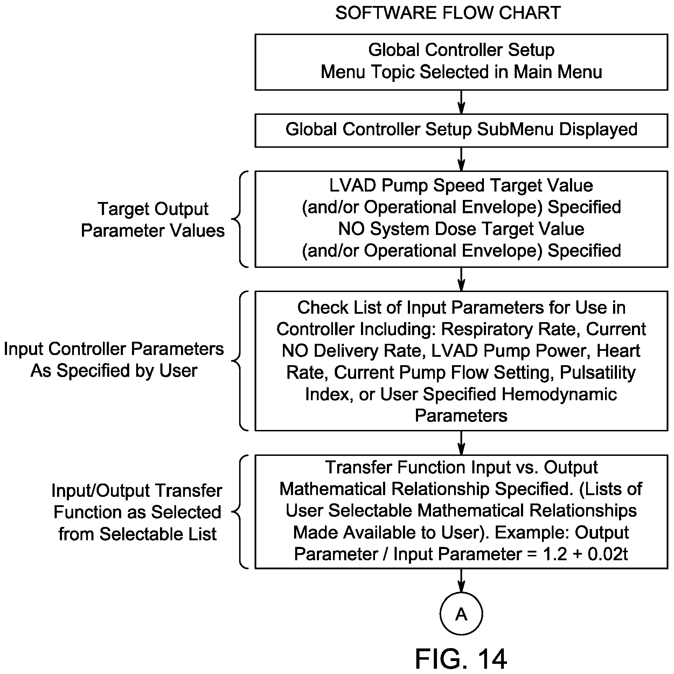

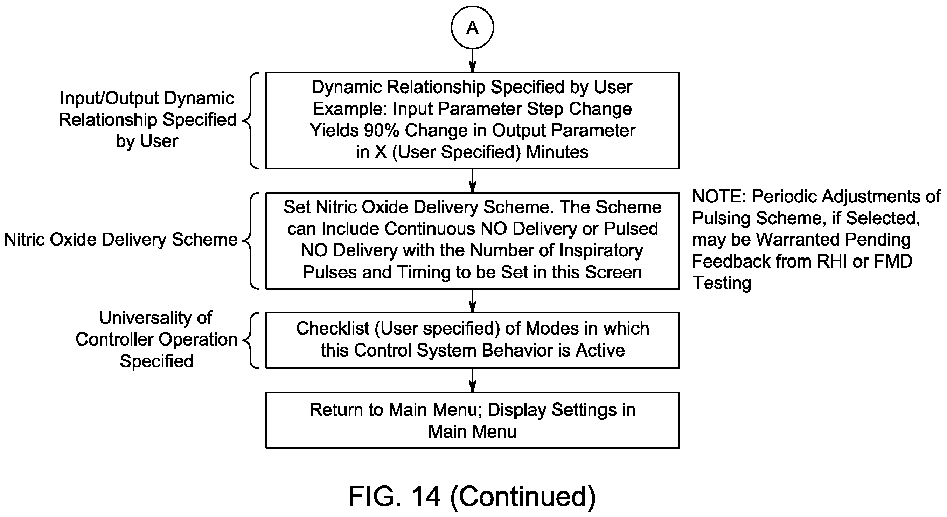

FIG. 14 illustrates an exemplary submenu for clinician setting of the control system that can be used in accordance with one or more embodiments of the invention.

DETAILED DESCRIPTION

**Nitric Oxide for Inhalation**

INOmax® (nitric oxide) for inhalation is an approved drug product. The FDA-approved prescribing information for INOmax® dated 2013 is attached as Appendix 1, and so forms part of the present disclosure. INOmax® is a selective pulmonary vasodilator, which, in conjunction with ventilatory support or other appropriate agents, is indicated for the treatment of term and near-term (>34 weeks gestation) neonates with hypoxic respiratory failure associated with clinical or echocardiographic evidence of pulmonary hypertension, where it improves oxygenation and reduces the need for extracorporeal membrane oxygenation. The recommended dose of INOmax® for the approved indication is 20 ppm, maintained for up to 14 days or until the underlying oxygen desaturation has resolved. Weaning should occur gradually. Adverse reactions per the label include methemoglobinemia and nitrogen dioxide levels, both which can be dose-dependent.

Inhaled NO may be administered via a NO delivery device such as the INOmax DSIR®, INOmax® DS or INOvent® delivery devices, each of which delivers operator-determined concentrations of NO in conjunction with a ventilator or breathing gas administration system after dilution with oxygen or an oxygen/air mixture. Other NO delivery devices and features of NO delivery devices are described below, including NO delivery devices having novel features not present in currently available NO delivery devices.

The source of NO used in any of the presently disclosed methods and devices can be a cylinder of compressed gas containing NO, typically as a mixture with an inert gas such as nitrogen or helium. The NO-containing gas can be generated by manufacturing the gases separately, mixing them in an appropriate ratio, and introducing them into an appropriate cylinder under pressure. The mixing can occur in two steps: first diluting bulk NO with nitrogen to a concentration of, *e.g.,* 5,000 ppm or 28,600 ppm in interim cylinders, and then diluting that mixture further by introducing the mixture into the final cylinders and filling them with more nitrogen to produce a concentration of, *e.g.,* 100 ppm or 800 ppm in the final cylinders. Care is taken not to introduce any water or oxygen into the cylinders. The cylinders can be equipped with an appropriate valve, shipped to the point of use, and attached to a NO delivery device to facilitate inhalation of the gas by the patient.

The source of NO can instead be a NO-generating device that generates NO from a suitable nitrogen source, such as air (see for reference U.S. Patent No. 5,396,882) or nitrogen dioxide (see for reference U.S. Patent No. 7,560,076). The source of nitrogen dioxide can be, for example, a canister of compressed nitrogen dioxide gas or a container of $N_2O_4$ (which, when treated under appropriate conditions, will give off nitrogen dioxide). Manufacturing a source of nitrogen dioxide can include the steps of compressing nitrogen dioxide gas into a suitable container or introducing $N_2O_4$ in liquid form into a suitable container. The container can be supplied in a device that includes a filter containing a reducing agent or antioxidant, such as ascorbic acid, which reduces the nitrogen dioxide to form NO at the patient's bedside. At the point of administration, such a NO-generating device is typically attached to a gas-delivery device (such as a ventilator) to facilitate inhalation of the newly formed NO gas by the patient.

**Definitions**

As used herein, the term "pulmonary hemodynamic parameter" refers to any parameter used to describe or evaluate the blood flow through the heart and pulmonary vasculature. Examples of pulmonary hemodynamic parameters include, but are not limited to, mean pulmonary artery pressure (mPAP), diastolic pulmonary artery pressure (dPAP) [also known as pulmonary artery diastolic pressure (PADP)], systolic pulmonary artery pressure (sPAP) [also known as pulmonary artery systolic pressure (PASP)], pulmonary capillary wedge pressure (PCWP) [also known as pulmonary artery wedge pressure (PAWP)], left atrial pressure (LAP), transpulmonary gradient (TPG), pulmonary vascular resistance (PVR) and cardiac output (CO).

Many of the pulmonary hemodynamic parameters described above are interrelated. For example, PCWP is often used as a more convenient, less invasive approximation of LAP. As another example, PVR is related to mPAP, PCWP and CO according to the following equation:

$$PVR \propto (mPAP - PCWP)/CO$$

As yet another example, TPG is the difference between mPAP and PCWP as shown by the following equation:

$$TPG = mPAP\text{-}PCWP$$

As a further example, mPAP is related to dPAP and sPAP according to the following equation:

$$mPAP = (2/3)dPAP + (1/3)sPAP$$

In some embodiments, the pulmonary hemodynamic parameters are measured directly, such as during a right heart catheterization. In other embodiments, the pulmonary hemodynamic parameters are estimated and/or evaluated through other techniques such as magnetic resonance imaging (MRI) or echocardiography.

The phrase "resolution of pulmonary hypertension (PH)" or variations thereof refers to a decrease in PH below a clinically relevant threshold. One example of resolution of PH is when the mPAP of a patient decreases below a threshold of 25 mmHg. However, in patients with severe right ventricular failure, the ventricle can be so weak that it cannot generate sufficient force to raise sPAP so that the mPAP is at least 25 mmHg. In such patients, PH and resolution thereof may be evaluated by analyzing the difference between dPAP and PCWP.

**Right Ventricular Failure in LVAD Implanted Patients**

As described above, right ventricular failure is a common problem after LVAD implantation. Post-LVAD right-sided heart failure is primarily related to the dynamic effects of the LVAD itself and/or the underlying right ventricular disease, as post-operative right heart failure occurs in only a small proportion of orthotopic heart transplants when performed in a similar population.

Post-LVAD right ventricular failure may be defined pathophysiologically as inability of the right ventricle to maintain adequate loading of the LVAD-assisted left ventricle despite adequate right ventricle preload, or to do so only at the expense of significantly elevated central venous pressure. Post-LVAD right heart failure is generally defined operationally as the need for implantation of a right ventricular assist device (RVAD), or the need for reinstitution of inhaled NO for greater than 48 hours, or the need for inotropic pharmacological therapy for greater than 14 days. Recent retrospective studies have each implicated various different pre-implantation clinical and hemodynamic parameters as being predictive of post-LVAD right heart failure with little consensus as to the most informative or most predictive factors; thus full understanding and accurate prediction of post-implantation right heart failure remains clinically problematic and mechanistically controversial. Putative predictive factors have ranged from non-specific demographic, clinical and laboratory measures of overall disease burden or patient "frailty", to conventional hemodynamic and echocardiographic measures of left ventricle, right ventricle and pulmonary vascular status, to very specific and specialized functional imaging parameters of the right ventricle. The pathophysiology of right ventricular failure after LVAD implantation appears to be multi-factorial, and includes pre-operative right ventricular dysfunction and pulmonary hypertension (PH), right ventricular ischemia, peri-operative fluctuations in pulmonary vascular resistance (PVR) in the setting of cardiopulmonary bypass (CPB), excessive right ventricular preload, and altered interventricular balance, although the relative importance of each of these factors is strongly debated. Superimposed perioperative procedures and/or complications thereof, such as intra-operative mechanical and/or ischemic damage to the right ventricle, and intra-operative hemorrhage requiring extensive fluid, colloid or blood product resuscitation, have also been invoked as acute predisposing or exacerbating factors for peri-operative right heart failure associated with LVAD implantation. Importantly, the direct and indirect effects of the LVAD itself on the anatomy and function of the left ventricle are also implicated as causing or contributing to post-implantation right ventricular dysfunction and right heart failure, despite optimization of LVAD adjustments and pharmacological support.

**Interactions Between the Left and Right Ventricles in Congestive Heart Failure**

In the healthy heart, the left ventricle is estimated to contribute 80% of the contractile flow and up to two-thirds of the contractile pressure generated by the right ventricle through a process termed mechanical systolic ventricular interaction (SVI). The (patho)physiological interactions between the left ventricle and the right ventricle in CHF are quite complex. The right ventricle may be directly damaged by the underlying disease process affecting the left ventricle in CHF. Most commonly, right ventricular failure in CHF results from increased right ventricular afterload due to chronic pulmonary vascular congestion and PH consequent to the primary left ventricular dysfunction. Additionally, dilation of the left ventricle in CHF realigns the anatomy of interventricular septal musculature to a less efficient transverse orientation, further impairing SVI and therefore overall right ventricular contractility. When combined with the increased right ventricle afterload, dysfunctional SVI unleashes a vicious cycle of progressive right ventricular dysfunction and right ventricular failure Reciprocally, as the failing right ventricle dilates, it intrudes and interferes with the relaxation (diastolic) filling of the left ventricle (termed diastolic ventricular interaction [DVI]), further exacerbating pulmonary vascular congestion and PH, creating a superimposed additional vicious cycle of progressive left ventricular failure and right ventricular failure. An additional form of remote interventricular interaction occurs at the level of the peripheral circulation, wherein the increased central systemic blood volume and central venous pressure in CHF increases right ventricular preload and right ventricular filling further increasing interventricular septal intrusion into the left ventricle and thereby further worsening DVI. Lastly, a further level of interventricular interaction occurs at the level of the pulmonary circulation, where chronic elevation of pulmonary venous and capillary pressures causes pulmonary vascular remodeling which, over time creates a relatively fixed, structurally-mediated increase in PVR, further worsening PH and right ventricular afterload (defined as World Health Organization [WHO] Group 2 Pulmonary Hypertension Secondary to Left-Sided Heart Disease).

**World Health Organization Group 2 PH Secondary to Left-Sided Heart Disease**

Up to three quarters of patients with end-stage CHF exhibit some degree of PH and right ventricular dysfunction, and one-third to one-half have moderate to severe or "fixed" PH unresponsive to vasodilator or inotroph challenge. Pharmacologic challenge has generally included some combination of inotropes (dobutamine, dopamine, milnirone), nonspecific vasodilators (nitroglycerin, sodium nitroprusside [SNP]) and/or partially or completely selective pulmonary vasodilators (prostacyclin, prostacyclin analogues, prostaglandin E1, sildenafil, inhaled NO) administered pre- or post-LVAD implantation or orthotopic heart transplant. The chronically failing left ventricle increases left atrial and pulmonary venous pressure which increases pulmonary artery pressure (PAP) and right ventricular afterload. With chronic stress, the pulmonary vasculature remodels, resulting in an increased PVR that further increases PAP out of proportion to the increased pulmonary venous pressure leading to an increase in trans-pulmonary pressure gradient (TPG) in approximately one-third or more of advanced CHF patients. However, the extent to which this increased PVR and TPG is mediated by tonic (and hence acutely reversible) compensatory pulmonary vasoconstriction versus relatively fixed structural vascular remodeling, and the degree to which each of these components is ultimately reversible in advanced CHF is often difficult to predict based on previously known methods. As the key intermediary between right ventricular output and left ventricular preload, the dynamic status of the pulmonary vasculature in patients with chronic CHF before, during and after LVAD placement is both highly complex and critically important to clinical outcome. PH unresponsive to pharmacological therapy has been associated with an increased risk of right heart failure and overall poor prognosis following orthotopic heart transplant, and is considered to be a contraindication for that procedure. In contrast, the predictive power of preoperative hemodynamic attributes of PH for the development of post-LVAD right heart failure is complex. Indeed, both low pre-implantation PAP (perhaps indicative of poor right ventricular contractility) and high pre-implantation PVR have been reported to be predictive of the subsequent development of post-LVAD right heart failure. The presence of fixed PH in end-stage CHF is now considered to be an indication for LVAD therapy versus a contraindication for heart transplant, based in part on the consistent observation that chronic unloading of the left ventricle by a well-functioning LVAD over time reverses "fixed" Group 2 PH in CHF patients in whom PH had been otherwise unresponsive to pharmacological intervention before LVAD implantation, thus rendering these patient eligible for heart transplant. Maximum improvement in PH status appears to be reached within the first 6 months of LVAD support, and remain stable thereafter. Thus, although the LVAD-treated end-stage CHF population will likely become preferentially enriched in patients with WHO Group 2 PH targeting bridge-to-transplant, destination therapy or even bridge-to-recovery, the importance of avoiding LVAD-related adverse events, which are associated with poorer post-heart transplant prognosis, has recently been emphasized .

**Interventricular Interactions and Right Heart Failure after LVAD Implantation**

Although the beneficial effects of continuous-flow LVADs on survival and quality-of-life as well as WHO Group 2 PH have been well documented, the direct and indirect effects of these devices on right ventricular function have only recently been evaluated in detail. The same SVI and DVI operative in non-LVAD-supported CHF may also play a clinically and therapeutically important role in post-LVAD right heart failure. The implanted LVAD actively increases left ventricular outflow which decompresses the left ventricle, thereby decreasing pulmonary vascular congestion and reducing right ventricular afterload; however the same augmented LVAD outflow increases right ventricular preload which may overwhelm the functional capacity of the previously stressed and/or damaged right ventricle. The decompression of the left ventricle also resets SVI and DVI. Current post-operative hemodynamic LVAD management primarily targets restoration of normal systemic peripheral end-organ (e.g. renal and hepatic) perfusion (as measured by CI) in order to permit gradual weaning of inotropic agents and diuretics. Fluid therapy is generally targeted to maintain initial pump speed >2 liters/min with a right atrial filling pressure < 20 mm Hg). After hospital discharge, attributes of pulmonary vascular congestion and PH and the need for inotropic pharmacological support generally decline as measures of peripheral end-organ perfusion progressively improve gradually over a period of days to weeks. Despite initial reductions in pulmonary vascular congestion, PH and excessive right ventricular afterload (which together should improve right ventricular function), right ventricular dysfunction as assessed by transthoracic echocardiogram may remain impaired for up to 3 months following successful LVAD implantation (although more rapid improvement has been reported in stable LVAD patients not requiring inotropic support). The still-weakened right ventricle may be unable to accommodate the increased forward flow generated by the LVAD-assisted left ventricular output, posing a continuing risk of right heart failure. The associated elevation of right arterial pressure one month post-LVAD implantation is linked with impaired exercise tolerance as reliably assessed by the distance walked in six minutes (6MWD) and is predictive of increased mortality risk. Persistent post-LVAD right ventricular dysfunction may reflect diminished intrinsic ability of the right ventricle to undergo self-repair and/or the fact that therapeutic hemodynamic adjustments prioritize the systemic circulation leaving the still-weakened right ventricle exposed to non-optimized hemodynamic stresses.

**Post-Operative Management and Adjustment of LVADs**

Both the intrinsic right ventricle and the implanted LVAD are preload-dependent and afterload-sensitive, and adequate but not excessive preload of the right ventricle is important to maintain adequate left ventricle/LVAD filling without excessive right ventricular volume overload in the immediate post-operative period. LVAD flows must be kept low enough to avoid right ventricular volume overload but high enough to sustain adequate end-organ perfusion. Inotropes, e.g. milrinone, dobutamine and epinephrine, used to wean from CPB are often continued for days after implantation. Nitroglycerin, SNP, nesiritide and sildenafil have been used to lower. Inhaled NO and prostacyclin have also been used to reduce PVR in order to do so without compromising systemic perfusion. Nevertheless, depending upon the setting of the continuous-flow LVAD rotational speed, right ventricular outflow through the pulmonary circulation may be inadequate to reliably fill the left ventricle, resulting in the development of negative pressure in the left ventricle. This negative left ventricular pressure not only compromises LVAD function, but also draws the interventricular septum leftward, disrupting SVI and essentially eliminating any septal contribution to right heart contractility, further reducing right ventricular outflow. This occurrence can precipitate severe right ventricular dysfunction and overt clinical right ventricular failure, which may decrease LVAD preload further impairing its function thereby causing worsening heart failure. In the intra-operative setting during LVAD implantation, trans-esophageal echocardiography (TEE) continuously monitors the position of the interventricular septum during LVAD adjustment and weaning from CPB; this is particularly important to monitor and manage the acute effects of CPB-withdrawal on the dynamic status of the pulmonary vasculature, which can produce severe acute intra-operative or peri-operative PH. Sub-acute post-operative right ventricular failure secondary to interventricular septal deviation and dysfunction is suspected when trans-thoracic echocardiography (TTE) reveals a dilated right ventricle accompanied by a small left ventricle and an aortic valve which remains closed due to negative left ventricular pressure. During the post-operative hospitalization, averaging 6 ICU and 20 total inpatient days, periodic TTE assessment of interventricular septal deviation predicts right ventricular failure and guides LVAD rotational speed adjustment to minimize leftward interventricular septal deviation and the consequent risk of right ventricular dysfunction and right ventricular failure. For example, a "ramped speed study" under TTE monitoring may be used to adjust the optimal pump speed taking into account changes in ventricular dimensions, displacement of the interatrial and interventricular septa, and the frequency of aortic valve opening as well as evidence of inadequate left ventricular preloading and right ventricular dysfunction. This TTE-directed optimization may be especially important in patients with poor 6MWD. Reduction of LVAD speed to optimize right ventricular function and/or manage post-LVAD right ventricular failure may require temporary reintroduction of inotropic pharmacological support and/or intravenous vasodilators to maintain adequate systemic end-organ perfusion. Despite these intensive measures, right ventricular failure remains a leading cause of early mortality after implantation of even the most modern continuous-flow LVADs. Furthermore, current approaches to optimize LVAD, right arterial and right ventricular hemodynamics with inotropic support and/or conventional intravenous vasodilators is limited by the fact that these agents may induce arrhythmias and/or systemic hypotension, increase oxygen demand, and worsen oxygenation due to pulmonary ventilation-perfusion mismatching, leaving ample room for new approaches that would optimize LVAD function and reduce the risk of right ventricular failure while avoiding these serious pharmacologic side effects. Furthermore, other therapeutic approaches may be needed to enable right ventricular outflow through the pulmonary circulation to maintain sufficient left ventricular preload to adequately fill the LVAD-assisted left ventricle at LVAD settings sufficient to maintain adequate end-organ perfusion during the critical post-implantation period.

**Pulmonary Vasodilators in the Management of Patients with Left Ventricular Assist Devices**

Acute pulmonary vasoreactivity testing (AVT) by right heart catheterization with selective pulmonary vasodilators such as inhaled NO is routinely performed in other forms of PH such as pulmonary arterial hypertension (PAH, or WHO Group 1 Pulmonary Hypertension). AVT with inhaled NO is only rarely and cautiously performed in non-LVAD-supported patients with CHF prior to heart transplantation, because acute highly-selective reduction in PVR and right ventricular afterload may overload the failing left ventricle thereby increasing right arterial and pulmonary venous pressure, potentially precipitating acute pulmonary edema. Instead, "fixed" versus "reversible" PH in advanced CHF is usually interrogated by the hemodynamic response or lack thereof to combinations of systemically-administered non-pulmonary-specific vasodilators such as SNP, nitroglycerin or adenosine and inotropic agents administered acutely, or in some studies, for over 72 hours. At times, acute or longer infusion of highest tolerated doses (*i.e.* free of systemic hypotension or other systemic side effects) of prostacyclin and prostaglandin $E_1$ in conjunction with inotropes or non-specific vasodilators have been used for PH-reversibility in this setting. Inhaled or intravenous prostanoids may be currently considered preferable to inhaled NO in non-LVAD-supported CHF patients in some but not all geographies despite significant decreases in systemic vascular resistance (SVR) and the clear demonstration that chronic intravenous prostacyclin therapy increases mortality in patients with end-stage CHF.

Because early extubation, removal of monitoring lines and ambulation are recommended, TTE becomes a primary tool to aid in the regulation of LVAD settings and hemodynamic fluid and pharmacological therapy in the post-acute post-implant setting. Management is complicated by the fact that right ventricular and both the pulmonary and systemic circulations are simultaneously undergoing complex dynamic interactions and adaptations to the newly functioning LVAD and the fact that most vasoactive drugs affect both circulatory systems simultaneously, *e.g.* SNP dilates both systemic and pulmonary resistance vessels. The high pulmonary selectivity and very short half-life of inhaled NO are ideal attributes to classify, manage and optimize the pulmonary vascular status in CHF patients on LVAD support, and to optimize LVAD performance in this setting. In the post-operative period, once the acute effects of CPB-induced PH have abated and the patient stabilized on a hemodynamic regimen, AVT with inhaled NO under echocardiographic and/or right heart catheterization guidance could be used to determine if inhaled NO should be continued in order to adjust left ventricular preload to optimize SVI and LVAD performance and reduce the risk of perioperative and postoperative acute right ventricular dysfunction and right ventricular failure. Specifically, doses of inhaled NO would be titrated against the right ventricular performance including measures of SVI and LVAD rotational speed, power and flow, until the correct combination is achieved to normalize/optimize right ventricular function, SVI, cardiac output, pulsatility and other hemodynamic parameters. In those patients in who CI or LVAD or right ventricular performance appears to benefit, inhaled NO would be continued during the taper of inotrope and/or intravenous systemic vasodilators. These parameters would then be re-monitored with echocardiographic assessment to maintain optimal settings during the post-acute recovery period. It would be anticipated that the provision of critical right ventricular afterload reduction and left ventricular preload enhancement during this period would permit increased CI with less LVAD power, improve pulse index, improve peripheral perfusion and exercise tolerance, and hasten and improve early cardiac rehabilitation, as well as reduce the risk of right heart failure. Additionally, the post-acute response to inhaled NO could be highly predictive of the likelihood of subsequent maximum resolution of residual PH over 6-months resulting from chronic CHF, particularly in those patients whose PH may have been characterized preoperatively as "fixed" by lack of response to the combination of inotropic agents and systemic vasodilators, as the effectiveness of these agents is often tolerability-limited. Thus, inhaled NO could represent a new paradigm in the optimal management of patients with functioning LVADs over the days, weeks and months following implantation.

## New Indications Relating to the Use of Inhaled NO with LVADs

In view of the above, aspects of the present disclosure provide for the utilization of inhaled NO as adjunctive therapy post-LVAD implantation. The inhaled NO therapy may be commenced pre-operatively pre-implantation or intra-operatively before, during or directly after implantation; such inhaled NO therapy may be continued beyond the time period in which it is clinically required in order successfully counteract the acute and temporary PH that occurs as a direct consequence of the CPB procedure itself. Alternatively, inhaled NO could be instituted (or re-instituted) after successful weaning from CPB and the direct consequence thereof.

In various aspects of the present disclosure inhaled NO would thusly be utilized for one or more novel applications to predict and prevent the development of right ventricular failure post successful weaning from CPB in LVAD recipients. As discussed above, right ventricular failure after institution of LVAD support may be consequent to the development or persistence of PH, further impairment of right ventricular contractility secondary to alterations in SVI and DVI or other ventricular interactions between the RV and the "unloaded" left ventricle, or further intrinsic impairment of right ventricular contractility given the known susceptibility of the right ventricle to myocardial preservation injury during CPB. Differentiation among these contributing causes is important since they would be managed differently.

One aspect of the present disclosure that is not part of the claimed invention provides a method to predict which post-implantation LVAD patients with PH are likely to resolve their PH with continued LV unloading by a functional LVAD. Those patients exhibiting a significant acute reduction in TPG and/or mPAP and/or PVR and/or other measures such as the difference between dPAP and PCWP with inhaled NO would be those more likely to resolve their increased TPG and/or mPAP and/or PVR following LVAD treatment, e.g. after several months of LVAD treatment.

When acute vasoreactivity testing (AVT) is performed pre-LVAD implantation, the PAP is a combination of increased pulmonary vascular resistance and elevated post-capillary pressure measured as either PCWP or right arterial pressure (RAP). The PCWP and RAP effect would be removed once the LVAD is in place, making the AVT results more clearly related to pulmonary vascular resistance, not confounded by increased PCWP and RAP. Accordingly, this aspect of the present invention provides an enhanced predictive tool by performing the AVT after LVAD implantation.

In various examples of this aspect, the method comprises measuring one or more pulmonary hemodynamic parameters of a patient with an LVAD to obtain a first pulmonary hemodynamic value; after obtaining the first pulmonary hemodynamic value, administering inhaled NO to the patient with the LVAD; and measuring one or more pulmonary hemodynamic parameters of the patient during or after the inhaled NO administration to obtain a second pulmonary hemodynamic value. A significant improvement in the pulmonary hemodynamic parameter from the first pulmonary hemodynamic value to the second pulmonary hemodynamic value, for example a decrease of at least 10 mm Hg and/or at least 20% can indicate that the patient is likely to resolve the pulmonary hypertension after continued use of the LVAD.

In some examples of this aspect, the inhaled NO is administered at a concentration of 5 to 80 ppm for at least 10 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 10 minutes, about 15 minutes, about 20 minutes, about 25 minutes, about 30 minutes, about 35 minutes, about 40 minutes, about 45 minutes, about 50 minutes, about 55 minutes, and about 60 minutes

Exemplary pulmonary hemodynamic parameters include mean pulmonary artery pressure (mPAP), transpulmonary gradient (TPG) and pulmonary vascular resistance (PVR). The one or more pulmonary hemodynamic parameters may be measured by any appropriate procedures, such as by performing a right heart catheterization.

Other known methods of performing acute vasoreactivity testing (AVT) with inhaled NO may also be used in addition to or as an alternative to the methods described above with respect to this aspect of the invention, provided that such AVT is performed after LVAD implantation.

In one or more examples of this aspect, the method further comprises placing the patient on a heart transplant list if the decrease in the pulmonary hemodynamic parameter from the first pulmonary hemodynamic value to the second pulmonary hemodynamic value is at least 10 mm Hg and/or at least 20%. In some examples the method further comprises explanting the LVAD and implanting a donor heart in the patient.

As an alternate to the above thresholds of 10 mm Hg and/or 20%, other significant decreases in the pulmonary hemodynamic parameter may be at least 5 mm Hg, at least 6 mm Hg, at least 7 mm Hg, at least 8 mm Hg, at least 9 mm Hg, at least 15 mm Hg, at least 20 mm Hg, or at least 25 mm Hg, and/or at least 5%, at least 10%, at least 15%, at least 25%, at least 30%, at least 35%, at least 40% or at least 50%.

Another aspect of the present disclosure that is not part of the claimed invention provides a method to use administration of inhaled NO during the adjustment and setting of the LVAD parameters and hemodynamic pharmacotherapy and fluid replacement therapy once the acute effects of prior CPB on pulmonary hemodynamics have elapsed.

One current methodology of setting an LVAD involves setting the revolutions/min (rpm) rate of an LVAD (such as a HeartMate II LVAD (HM II)) to provide adequate cardiac output and achieve optimal left ventricular decompression, while maintaining a pulsatility index (defined as the maximum LVAD flow rate minus the minimum LVAD flow rate divided by the average LVAD flow rate) of 3.5 to 4. Although modern "continuous-flow" LVAD devices do not themselves have valves that open and close to generate pulsatile flow, the flow rate through these devices at any device setting varies depending upon the pressure gradient between the left ventricle and the aorta such that the increased systolic pressure with each contraction of the left ventricle transiently increases flow through the LVAD creating some pulsatile variation in blood flow. In addition, the fixed-rate speed of a continuous-flow LVAD is usually adjusted to maximize left ventricular decompression and to improve cardiac output, while simultaneously allowing for a minimum aortic valve opening ratio of 1:3 (*i.e.* the left ventricular systolic pressure achieves a sufficiently high pressure relative to aortic pressure to permit opening of the aortic valve once out of every three systoles despite the continuing efflux of blood through the LVAD).

Another current methodology of setting an LVAD involves optimizing the rpm speed, both hemodynamically and echocardiographically, at the time of LVAD placement, before the patient is discharged from the hospital (*i.e.,* after admission for LVAD placement) and if clinical events such as new symptoms or suction events warranted further adjustment. However, these hemodynamic and echocardiographic assessments used to adjust LVAD settings are static in that they are performed at the left ventricular preload exhibited by the patient at the time and condition under which the test is performed (usually at rest).

The use of inhaled NO during part of the test procedure to maximally relax the pulmonary vessels and lower PVR would provide information on the maximal left ventricular preload that the right ventricle is able to generate unfettered by acutely-reversible pulmonary vasoconstriction. Such dynamic (rather than static) assessment would provide additional information to optimize any particular group of settings of the LVAD to produce the desired cardiac out and pulsatility parameters while avoiding the generation of left ventricular suction as determined by simultaneous TTE. The novel use of inhaled NO as an adjunct to adjusting LVAD parameters should improve the efficiency and safety of functioning LVADs, which should result in improved cardiac output, pulsatility indices and exercise tolerance, and reduce the risk of right heart failure.

As the above prior methods simply understand the extent to which LVAD and right ventricular settings and read-outs reflect the current level of PH without dissecting PH into fixed versus reversible by dynamic testing, these methods limit the range of options within which LVAD function would have to operate efficiently during the recovery period post-LVAD implantation. Accordingly, the more effective and accurate adjustment in LVAD parameters provided by this aspect of the invention can result in improved LVAD efficiency, cardiac output, end-organ perfusion, and exercise tolerance, and retesting with this paradigm and periodically re-setting LVAD parameters can hasten full recovery.

Accordingly, this aspect of the present disclosure that is not part of the claimed invention relates to a method of optimizing the settings of an LVAD by utilizing inhaled NO. In various examples of this aspect, the method comprises administering inhaled NO to a patient having an LVAD; performing an echocardiogram or similar functional hemodynamic or cardiac imaging assessment on the patient during the administration of inhaled NO; and adjusting or setting one or more parameters of the LVAD during the echocardiogram and during the administration of inhaled NO. In one or more examples adjusting or setting the LVAD parameters during administration of NO helps to optimize cardiac output, end-organ perfusion, LVAD efficiency and/or exercise tolerance.

In one or more examples of this aspect, adjusting or setting one or more parameters of the LVAD comprises one or more of (i) determining a low pump speed setting for the LVAD based on the minimal pump speed necessary for the patient's aortic valve to open with each heart beat or (ii) determining a high speed setting for the LVAD based for example on the pump speed at which the septum of the patient's heart flattens.

In some examples of this aspect, the inhaled NO is administered at a concentration of 5 to 80 ppm for at least 10 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 1.5 hours, about 2 hours, about 2.5 hours and about 3 hours.

In some examples, the LVAD settings are changed over a series of incremental adjustments. For example, the LVAD pump speed may be adjusted upwards in two or more steps. One or more or all of these steps may be performed during the administration of inhaled NO as described herein.

Another aspect of the present disclosure that is not part of the claimed invention provides for long-term use of inhaled NO after LVAD implantation. In one or more examples of this aspect, if AVT favorably predicts that PH will resolve after continued use of the LVAD, and/or if the settings and read-out and/or the TTE indicate more efficacious LVAD function and hemodynamic status under inhaled NO challenge, then the treating physician may wish to continue administering inhaled NO to the patient continuously for all or part of the convalescent period. In various examples inhaled NO would be administered to the patient during all or part of the day over a period of days, weeks or months to maintain the favorable LVAD function and/or hemodynamic status. Periodic testing may be performed as described above both on and off inhaled NO for a short period of time, such that when sufficient recovery had occurred so that inhaled NO was no longer producing and hemodynamic or TTE change, the patient may be carefully weaned from inhaled NO. This treatment would be expected to result in improved cardiac output and exercise tolerance more quickly, and reduce the risk of right heart failure.

In various examples of this aspect, the method comprises administering inhaled NO to a patient with an LVAD for at least 12 hours a day for at least 10 days. The inhaled NO may be administered for several days to many months or even longer. Exemplary treatment times include 10 days, 15 days, 20 days, 25 days, 30 days, 35 days, 40 days, 45 days, 2 months, 3 months, 4 months, 5 months, 6 months, 7 months, 8 months, 9 months, 10 months, 11 months, 1 year, 1.5 years, or 2 years. In some embodiments, the patient is administered inhaled NO indefinitely.

In some examples of this aspect, the inhaled NO is administered at a concentration of 5 to 80 ppm for at least 12 hours a day. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 12 hours a day, about 14 hours a day, about 16 hours a day, about 18 hours a day, about 20 hours a day, about 22 hours a day, or up to 24 hours a day.

Due to the fact that a patient with an LVAD had preexisting left ventricular dysfunction, it may be important to ensure that the LVAD is properly functioning prior to administering inhaled NO. Accordingly, in some examples, the method further comprises confirming that the LVAD is functioning properly before administering inhaled NO.

**[0087]** In one or more examples, the inhaled NO is administered after a patient has been weaned from cardiopulmonary bypass (CPB).

As an alternative to a constant concentration of NO, the dose of NO may be prescribed based on the patient's ideal body weight (IBW). Exemplary NO doses may be in the range of about 25 to about 150 $\mu$g/kg IBW/hr, such as about 25, about 30, about 35, about 40, about 45, about 50, about 60, about 70, about 80, about 90, about 100, about 110, about 120, about 130, about 140 or about 150 $\mu$g/kg IBW/hr.

In one or more examples, the method further comprises monitoring one or more output parameters of the LVAD and/or one or more hemodynamic parameters of the patient, comparing the one or more output parameters and/or the one or more hemodynamic parameters to a predetermined range, and adjusting the dose of inhaled NO if the one or more outputs parameters and/or the one or more hemodynamic parameters are outside of the predetermined range. In some examples, the method further comprises providing an alert if the one or more output parameters and/or the one or more hemodynamic parameters are outside of the predetermined range. The inhaled NO dose may be adjusted automatically (*e.g.* by the NO delivery device or a control system in communication with the NO delivery device), or may be manually adjusted by a physician or other user, such as in response to an alert.

Examples of LVAD parameters that may be monitored include, but are not limited to, pump speed (*e.g.* rpm), pump flow (*e.g.* L/min), pump power, pulsatility index, battery level, and LVAD status (*e.g.* operational, presence or absence of warnings).

In some examples, the LVAD has a minimum and/or maximum pump speed that is set by the physician, and can be specific for the individual patient. Alternatively or additionally, the LVAD may also have a minimum and/or maximum pump speed set by the manufacturer of the LVAD. Regardless of whether the minimum and/or maximum pump speed is set by a physician or the manufacturer, exemplary minimum pump speeds include 100 rpm, 200 rpm, 300 rpm, 400 rpm, 500 rpm, 6000 rpm, 700 rpm, 800 rpm, 900 rpm, 1,000 rpm, 1,500 rpm, 2,000 rpm, 2,500 rpm, 3,000 rpm, 4,000 rpm, 5,000 rpm, 6,000 rpm, 7,000 rpm, 8,000 rpm, 9,000 rpm, 10,000 rpm, 11,000 rpm, 12,000 rpm, 13,000 rpm, 14,000 rpm and 15,000 rpm, and exemplary maximum pump speeds include 1,000 rpm, 1,500 rpm, 2,000 rpm, 2,500 rpm, 3,000 rpm, 4,000 rpm, 5,000 rpm, 6,000 rpm, 7,000 rpm, 8,000 rpm, 9,000 rpm, 10,000 rpm, 11,000 rpm, 12,000 rpm, 13,000 rpm, 14,000 rpm, 15,000 rpm, 20,000 rpm and 30,000 rpm. The minimum and maximum pump speeds may depend on the design of the LVAD.

Similarly, in some examples the LVAD has a minimum and/or maximum pump flow that is set by the physician, and can be specific for the individual patient. Alternatively or additionally, the LVAD may also have a minimum and/or maximum pump flow set by the manufacturer of the LVAD. Regardless of whether the minimum and/or maximum pump flow is set by a physician or the manufacturer, exemplary minimum pump speeds include 1 L/min, 1.5 L/min, 2 L/min, 2.5 L/min, 3 L/min, 3.5 L/min, 4 L/min, 4.5 L/min, 5 L/min, 6 L/min, 7L/min, 8 L/min, 9 L/min and 10 L/min, and exemplary maximum pump flows include 3 L/min, 3.5 L/min, 4 L/min, 4.5 L/min, 5 L/min, 6 L/min, 7L/min, 8 L/min, 9 L/min, 10 L/min, 11 L/min, 12 L/min, 13 L/min, 14 L/min and 15 L/min. The minimum and maximum pump flows may depend on the design of the LVAD.

In some examples, the pulsatility index of the LVAD has a minimum and/or maximum threshold. As explained above, the pulsatility index is the maximum pump flow minus the minimum pump flow, divided by the average pump flow. As the pulsatility index is an indication of how much support the LVAD is providing to the heart (a higher pulsatility index indicates that the LVAD is providing more support), high pulsatility indices can be a cause of concern. Accordingly, exemplary maximum pulsatility indices include values of 5, 6, 7, 8, 9, 10, 11, 12, 13, 14, 15 or 20.

In some examples, the battery level of the LVAD is monitored. As a low battery can indicate a future or imminent shutdown of the LVAD, when the battery level of the LVAD drops below a certain threshold, the inhaled NO dose may be lowered, a weaning protocol may be initiated, and/or an alert is provided. Examples of minimum battery levels i.nclude 30%, 25%, 20%, 15%, 10%, 9%, 8%, 7%, 6%, 5%, 4%, 3%, 2% or 1% battery remaining or 6 hours, 5 hours, 4 hours, 3 hours. 2 hours, 1 hour, 30 minutes, 20 minutes, 15 minutes, 10 minutes, 5 minutes, 4 minutes, 3 minutes, 2 minutes or 1 minute of battery life remaining.

Similarly, other indicators of LVAD malfunction or complete LVAD failure may be monitored, and the inhaled NO dose may be adjusted, a weaning protocol may be initiated and/or an alert may be provided. Again, the inhaled NO dose may be adjusted automatically (*e.g.* by the NO delivery device or a control system in communication with the NO delivery device), or may be manually adjusted by a physician or other user.

Another aspect of the present disclosure that is not part of the claimed invention relates to a method of monitoring the left ventricle of a patient with an LVAD. In various examples of this aspect, the method comprises reducing the pump speed of the LVAD or turning off the LVAD; measuring one or more pulmonary hemodynamic parameters of the patient to obtain a first pulmonary hemodynamic value; preloading the left ventricle by administering inhaled NO to the patient; and measuring one or more pulmonary hemodynamic parameters of the patient after or during administration of inhaled NO to obtain a second pulmonary hemodynamic value. In some examples, the pulmonary hemodynamic parameter is selected from LAP, PCWP and CO, or may be any assessment of the left ventricular reserve to compensate for increased left ventricular preload that can be measured through right heart catheterization, echocardiographic, MRI or other techniques..

In some examples of this aspect, the inhaled NO is administered at a concentration of 5 to 80 ppm for at least 10 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 1.5 hours, about 2 hours, about 2.5 hours, about 3 hours, about 3.5 hours, about 4 hours, about 5 hours, about 6 hours, about 7 hours or about 8 hours.

According to one or more examples, an increase in LAP and/or PCWP from the first pulmonary hemodynamic value to the second pulmonary hemodynamic value of less than 5 mm Hg indicates that the left ventricle is improving. Other exemplary values that indicate an improvement in the left ventricle include an LAP and/or PCWP increase of less than 1 mm Hg, 2 mm Hg, 3 mm Hg, 4 mm Hg, 6 mm Hg, 7 mm Hg, 8 mm Hg, 9 mm Hg, 10 mm Hg, 11 mm Hg, 12 mm Hg, 13 mm Hg, 14 mm Hg or 15 mm Hg. In some embodiments, the method further comprises modifying treatment if the left ventricle is improving, such as explanting the LVAD from the patient. Other modifications in treatment can include changing the supportive medication (*e.g.* diuretics and/or inotropic medications) that the patient is given, such as reducing the supportive medication.

Another aspect of the present disclosure that is not part of the claimed invention relates to a method of exercising a heart of a patient having an LVAD. In various examples of this aspect, the method comprises reducing and/or modulating the pump speed of the LVAD or turning off the LVAD; preloading the left ventricle by administering inhaled NO to the patient for at least 5 minutes; discontinuing the inhaled NO administration; and repeating the preloading and discontinuation to exercise the left ventricle of the patient's heart.

In some examples of this aspect, the inhaled NO is administered at a concentration of 5 to 80 ppm for at least 5 minutes. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 5 minutes, about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 1.5 hours, about 2 hours, about 2.5 hours, about 3 hours, about 3.5 hours, about 4 hours, about 5 hours, about 6 hours, about 7 hours or about 8 hours.

The preloading of the left ventricle may be performed multiple times per day, such as twice a day, three times a day, four times a day, five times a day, six times a day, seven times a day, eight times a day, nine times a day or ten times a day. Alternatively, the preloading may be performed once a day. If the preloading is performed multiple times per day, the preloading procedures may be clustered together (*e.g.* spaced apart by several minutes or a couple hours) or may be spread out throughout the day. The preloading of the left ventricle may also be performed once a week, two days a week, three days a week, four days a week, five days a week, six days a week, or seven days a week. In exemplary embodiments, the left ventricle is preloaded several times a day for several days a week, such as two to five times a day for two to four days a week or other combinations of the above daily and weekly preloading schedules.

In some examples, the first instance or first few instances of the exercising of the patient's left ventricle is performed under the direct supervision of a physician, but once an appropriate exercise protocol has been determined for a patient, then the exercise protocol may be performed under patient control or may be automated by the NO delivery device and/or control system.

Another aspect of the present disclosure that is not part of the claimed invention relates to a method of reducing the risk of adverse events during LVAD use. In particular, it is believed that inhaled NO can improve cardiovascular function of LVAD recipients with functioning LVADs, reduce the risk of adverse events associated with continuous-flow or semi-pulsatile LVADs (e.g. thrombosis, gastrointestinal bleeding) and improve the function of transplanted hearts by making the cardiovascular system more compliant.

A specific deficiency in continuous-flow LVADs is that the reduced pulsatility of peripheral perfusion generated by these devices compared to pulsatile-flow LVAD devices and/or the normal circulation derived from a well-functioning human heart as measured by pulsatility index, pulse pressure and/or the frequency of opening of the aortic valve has been implicated in a number of adverse events including reduced peripheral vascular compliance, gastrointestinal bleeding, arteriovenous malformations, hemolysis, pump thrombosis, aortic insufficiency and lower rate of recovery of left ventricular function. These adverse events can also be associated with semi-pulsatile LVADs.

Evidence of deranged microvascular function following continuous-flow LVAD support has been documented by several sensitive techniques evaluating post-ischemic autoregulation of blood flow, including flow-mediated dilation (FMD) of the brachial artery and the reactive hyperemic index (RHI), both of which has been shown to be predominantly mediated by locally released NO.

**[0106]** While not wishing to be bound by any particular theory, it is believed that diminished LVAD-generated pulsatility is linked to derangement of NO-mediated vascular autoregulatory function because pulsatile flow is considered the key generator of microvascular directional shear stress at the level of the microvascular endothelial cell that is strongly implicated in the autoregulation of NO biology in the vascular system. Thus, it is believed that at least some of the adverse events associated with continuous-flow LVAD's and their reduced pulsatility could be secondary to impaired NO-mediated autoregulatory vascular function.

Shear stress, and hence pulsatility, is a key factor in adapting organ perfusion to changes in cardiac output presumably in part through regulation of NO. Deranged NO-mediated autoregulatory vascular function is reflected not only in peripheral vessels but also in coronary vessels as well, including an association with a higher rate of in-stent thrombosis following percutaneous coronary intervention. It is believed that impaired NO-mediated coronary vascular autoregulation could provide a potential mechanism that would contribute to reduced LV recovery during continuous-LVAD support. NO not only mediates autoregulation of vascular tone, but also modulates platelet activation and adhesion, such that deranged NO regulation could contribute to the increased thrombosis associated with continuous-flow LVAD support. Attributes of NO-mediated vascular dysfunction are considered to be significant overall risk factors for adverse cardiovascular outcomes in the general population. Indeed, some authors have suggested that impaired endothelial dilation is the ultimate cause of cardiovascular diseases such as coronary artery disease and peripheral artery disease. These widespread effects of NO on vascular health are thought to be mediated in large part by modulating oxidative stress through the control of nitrosylation of a wide range of regulatory proteins.

Inhalational NO could therefore improve endothelial function and vascular health, and thereby important clinical outcomes, in patients on long-term continuous-flow LVAD support, through two related and potentially complementary or synergistic mechanisms. Firstly, as discussed above, inhaled NO, by dilating pulmonary vessels, would increase left ventricular preload and improve left ventricular filling, thereby increasing the frequency of aortic valve opening and closing, and hence pulsatility for any given LVAD speed setting. This effect might be more dominant during periods of rest or sleep, or during periods of increased cardiac output demand such as exercise, depending upon the characteristics of the LVAD device and setting.

Secondly, inhaled NO also has the potential to improve NO-mediated vascular autoregulatory function and health independent of its effect of circulatory pulsatility, by improving NO bioavailability throughout the peripheral and coronary circulation. NO can be generated by NO synthase (NOS) or by the breakdown of nitrite or other compounds to NO. Inhalation of NO for even short periods of time (e.g. two hours) increases the circulating levels of a variety of molecules that have the potential to regenerate NO within the microcirculation including nitrite ($NO_2^-$), nitrate ($NO_3^-$) and S-nitrosohemoglobin. These and other NO-derive molecules have been invoked in the beneficial so-called "distal effects" of inhaled NO. For example, inorganic nitrite is an endogenous substance produced by the oxidation of NO• under aerobic conditions (such as in the lung) but conversely, in acidic conditions such as might be found in ischemic tissues, $NO_2^-$ can be chemically and enzymatically reduced back to NO• where it has favorable effects alleviating some of the consequences of local ischemia including the amelioration of ischemia-reperfusion injury. Thus, inhaled NO is expected to improve and/or sustain the tissue integrity and autoregulation of the peripheral vasculature and the tissues which it supplies, including the myocardium, through a complex cascade of NO-mediated biochemical effects in the setting of endothelial dysfunction that accompanies prolonged continuous-flow LVAD support.

Accordingly, embodiments of the present invention provide for the administration by inhalation of NO at doses and for durations that are expected to (1) improve pulsatility through the NO's direct hemodynamic action on the pulmonary vasculature thereby promoting left ventricular filling, aortic valve opening and closure, and thereby maximize pulsatile flow and/or (2) increase peripheral NO bioavailability through provision NO precursors in the form of NO metabolites such as $NO_2^-$, $NO_3^-$, nitrosohemoglobin and other that are formed in the lung as a result of NO inhalation and transported by the circulation to peripheral tissues where NO can be reformed further reducing endothelial dysfunction.

In various examples of this aspect, the method comprises administering inhaled NO to a patient with an LVAD for at least 12 hours a day for at least 10 days. The inhaled NO may be administered for several days to many months or even longer. Exemplary treatment times include 10 days, 15 days, 20 days, 25 days, 30 days, 35 days, 40 days, 45 days, 2 months, 3 months, 4 months, 5 months, 6 months, 7 months, 8 months, 9 months, 10 months, 11 months, 1 year, 1.5 years, or 2 years. In some embodiments, the patient is administered inhaled NO indefinitely.

In some examples of this aspect, the inhaled NO is administered at a concentration of 5 to 80 ppm for at least 12 hours a day. Exemplary inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm. Exemplary NO administration times include about 12 hours a day, about 14 hours a day, about 16 hours a day, about 18 hours a day, about 20 hours a day, about 22 hours a day, or up to 24 hours a day.

Due to the fact that a patient with an LVAD had preexisting left ventricular dysfunction, it may be important to ensure that the LVAD is properly functioning prior to administering inhaled NO. Accordingly, in some embodiments, the method further comprises confirming that the LVAD is functioning properly before administering inhaled NO.

[0114] In one or more embodiments, the inhaled NO is administered after a patient has been weaned from cardiopulmonary bypass (CPB).

As an alternative to a constant concentration of NO, the dose of NO may be prescribed based on the patient's ideal body weight (IBW). Exemplary NO doses may be in the range of about 25 to about 150 μg/kg IBW/hr, such as about 25, about 30, about 35, about 40, about 45, about 50, about 60, about 70, about 80, about 90, about 100, about 110, about 120, about 130, about 140 or about 150 μg/kg IBW/hr.

In one or more examples, the method further comprises monitoring one or more output parameters of the LVAD and/or one or more hemodynamic parameters of the patient, comparing the one or more output parameters and/or the one or more hemodynamic parameters to a predetermined range, and adjusting the dose of inhaled NO if the one or more outputs parameters and/or the one or more hemodynamic parameters are outside of the predetermined range. In some examples, the method further comprises providing an alert if the one or more output parameters and/or the one or more hemodynamic parameters are outside of the predetermined range. The inhaled NO dose may be adjusted automatically (*e.g.* by the NO delivery device or a control system in communication with the NO delivery device), or may be manually adjusted by a physician or other user, such as in response to an alert.

Examples of LVAD parameters that may be monitored include, but are not limited to, pump speed (*e.g.* rpm), pump flow (*e.g.* L/min), pump power, pulsatility index, battery level, and LVAD status (*e.g.* operational, presence or absence of warnings).

In some examples, the LVAD has a minimum and/or maximum pump speed that is set by the physician, and can be specific for the individual patient. Alternatively or additionally, the LVAD may also have a minimum and/or maximum pump speed set by the manufacturer of the LVAD. Regardless of whether the minimum and/or maximum pump speed is set by a physician or the manufacturer, exemplary minimum pump speeds include 100 rpm, 200 rpm, 300 rpm, 400 rpm, 500 rpm, 6000 rpm, 700 rpm, 800 rpm, 900 rpm, 1,000 rpm, 1,500 rpm, 2,000 rpm, 2,500 rpm, 3,000 rpm, 4,000 rpm, 5,000 rpm, 6,000 rpm, 7,000 rpm, 8,000 rpm, 9,000 rpm, 10,000 rpm, 11,000 rpm, 12,000 rpm, 13,000 rpm, 14,000 rpm and 15,000 rpm, and exemplary maximum pump speeds include 1,000 rpm, 1,500 rpm, 2,000 rpm, 2,500 rpm, 3,000 rpm, 4,000 rpm, 5,000 rpm, 6,000 rpm, 7,000 rpm, 8,000 rpm, 9,000 rpm, 10,000 rpm, 11,000 rpm, 12,000 rpm, 13,000 rpm, 14,000 rpm, 15,000 rpm, 20,000 rpm and 30,000 rpm. The minimum and maximum pump speeds may depend on the design of the LVAD.

Similarly, in some examples the LVAD has a minimum and/or maximum pump flow that is set by the physician, and can be specific for the individual patient. Alternatively or additionally, the LVAD may also have a minimum and/or maximum pump flow set by the manufacturer of the LVAD. Regardless of whether the minimum and/or maximum pump flow is set by a physician or the manufacturer, exemplary minimum pump speeds include 1 L/min, 1.5 L/min, 2 L/min, 2.5 L/min, 3 L/min, 3.5 L/min, 4 L/min, 4.5 L/min, 5 L/min, 6 L/min, 7L/min, 8 L/min, 9 L/min and 10 L/min, and exemplary maximum pump flows include 3 L/min, 3.5 L/min, 4 L/min, 4.5 L/min, 5 L/min, 6 L/min, 7L/min, 8 L/min, 9 L/min, 10 L/min, 11 L/min, 12 L/min, 13 L/min, 14 L/min and 15 L/min. The minimum and maximum pump flows may depend on the design of the LVAD.

In some examples, the pulsatility index of the LVAD has a minimum and/or maximum threshold. As explained above, the pulsatility index is the maximum pump flow minus the minimum pump flow, divided by the average pump flow. As the pulsatility index is an indication of how much support the LVAD is providing to the heart (a higher pulsatility index indicates that the LVAD is providing more support), high pulsatility indices can be a cause of concern. Accordingly, exemplary maximum pulsatility indices include values of 5, 6, 7, 8, 9, 10, 11, 12, 13, 14, 15 or 20.

In some examples, the battery level of the LVAD is monitored. As a low battery can indicate a future or imminent shutdown of the LVAD, when the battery level of the LVAD drops below a certain threshold, the inhaled NO dose may be lowered, a weaning protocol may be initiated, and/or an alert is provided. Examples of minimum battery levels include 30%, 25%, 20%, 15%, 10%, 9%, 8%, 7%, 6%, 5%, 4%, 3%, 2% or 1% battery remaining or 6 hours, 5 hours, 4 hours, 3 hours. 2 hours, 1 hour, 30 minutes, 20 minutes, 15 minutes, 10 minutes, 5 minutes, 4 minutes, 3 minutes, 2 minutes or 1 minute of battery life remaining.

Similarly, other indicators of LVAD malfunction or complete LVAD failure may be monitored, and the inhaled NO dose may be adjusted, a weaning protocol may be initiated and/or an alert may be provided. Again, the inhaled NO dose may be adjusted automatically (e.g. by the NO delivery device or a control system in communication with the NO delivery device), or may be manually adjusted by a physician or other user.

Another aspect of the present disclosure that is not part of the claimed invention provides a method for optimizing the inhaled NO dose for a continuous-flow or semi-pulsatile LVAD to reduce the risk of adverse events during LVAD use and/or optimize endothelial function.

In some examples of this aspect, dosing would be optimized by first optimizing the continuous-flow LVAD settings by conventional means and/or by the additional means described above. Next, endothelial function may be measured, such as by FMD and/or RHI. This adjustment could include the introduction of pulse modulation into the regulation of continuous-flow LVAD use and/or the use of Enhanced External Counter Pulsation Therapy (EECP Therapy) to which could be used in concert with inhaled NO. Inhaled NO would be commenced at one or more doses, for various periods of time, following which endothelial function would be re-measured using the same technique. Inhaled NO dosing would be modified in various stepwise fashions to identify an optimal dosing paradigm to maximize the improvement in endothelial function. Additionally, various NO-related molecules and other biomarkers of endothelial function would be measured in blood or plasma and correlated with the RHI and/or FMD measurement, to provide optimal monitoring of endothelial function to facilitate ongoing dose adjustments. Such correlation may detect and/or determine the optimal parameter to measure to ensure most ideal management of dosing of inhaled NO for the purpose of optimizing and/or individualizing inhaled NO dosing to maximize endothelial function in patients on long-term continuous-flow LVAD support.

In some examples of this aspect, the inhaled NO is administered at an initial concentration of 5 to 80 ppm. Exemplary initial inhaled NO concentrations include about 5 ppm, about 10 ppm, about 15 ppm, about 20 ppm, about 25 ppm, about 30 ppm, about 35 ppm, about 40 ppm, about 45 ppm, about 50 ppm, about 55 ppm, about 60 ppm, about 65 ppm, about 70 ppm, and about 80 ppm.

As an alternative to a constant concentration of NO, the dose of NO may be prescribed based on the patient's ideal body weight (IBW). Exemplary initial inhaled NO doses may be in the range of about 25 to about 150 $\mu$g/kg IBW/hr, such as about 25, about 30, about 35, about 40, about 45, about 50, about 60, about 70, about 80, about 90, about 100, about 110, about 120, about 130, about 140 or about 150 $\mu$g/kg IBW/hr.

Exemplary increments for optimizing the inhaled NO dose include adjusting the NO concentration by 0.1 ppm, 0.2 ppm, 0.5 ppm, 1 ppm, 2 ppm, 3 ppm, 4 ppm, 5 ppm, 6 ppm, 7 ppm, 8 ppm, 9 ppm, 10 ppm, 15 ppm and 20 ppm, or 0.1, 0.2, 0.5, 1, 2, 3, 4, 5, 6, 7, 8, 9, 10, 15, 20, 25, 30, 35, 40 or 50 $\mu$g NO/kg IBW/hr.

Once the inhaled NO dose is optimized, the NO may be continued to be administered during long-term LVAD use, such as described above.

## CONTROL SYSTEM

The methods described above may be implemented in one or more devices or systems utilizing a combination of hardware and software. It is understood that a control system as described herein may be a standalone or discrete hardware and software based device which communicates with the LVAD and the NO delivery device, or the control system may be a predominantly software-only implementation resident on the existing LVAD hardware or, the control system may be a predominantly software-only implementation resident on the existing NO delivery device hardware.

In various embodiments, the control system includes appropriate components for transmitting and/or communicating information and/or data between the NO delivery device, the LVAD and the control system. Communication to and from the control system may be over a communication path, where the communication path may be wired or wireless, and wherein suitable hardware, firmware, and/or software may be configured to interconnect components and/or provide electrical communications over the communication path(s). For example, the communication path may be a wireless optical line-of-sight signal (e.g. infrared signal) from one transceiver to another transceiver or from a transmitter to a receiver.

### Control System Hardware Architecture:

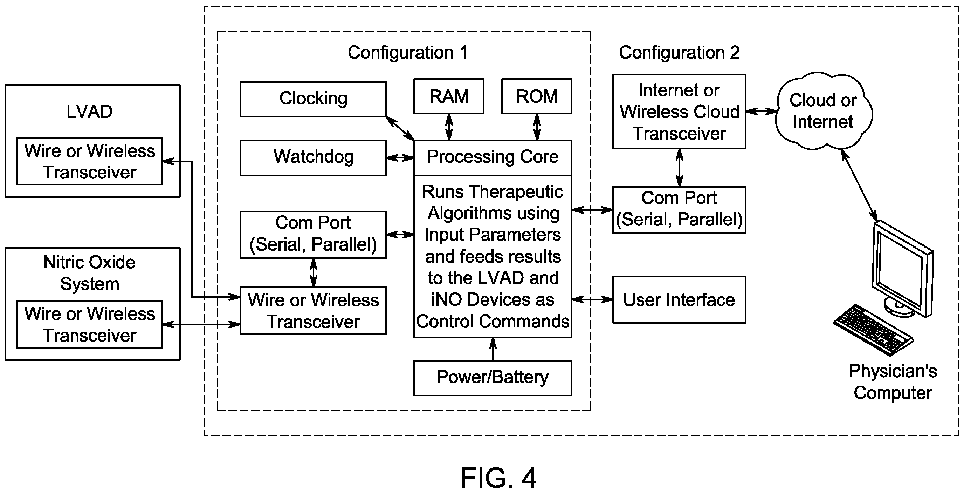

Regardless of the integration technique (standalone or integral to the LVAD or NO delivery device), the control system may comprise certain hardware elements as are shown in FIG. 4. Configuration 1 of FIG. 4 includes an exemplary set of hardware elements for the control system. These elements can include a processing core which can be implemented using a microprocessor, a microcontroller, a field-programmable gate array (FPGA) or other digital logic processing device. The control system can also comprise volatile and/or non-volatile memory with the volatile memory allowing for efficient data processing, and the latter being resident to allow for programming of clinical scenarios or algorithms (code) into the invention. Also resident are the processor support functions of clocking (oscillator) as well as a safety watchdog. Two way data communications is provided with both the LVAD and NO delivery devices as noted earlier or simply with the other system if the invention is implemented resident on the LVAD or NO device hardware. A battery or wall power supply is provided to power the electronics and this battery or wall power may be shared with the LVAD or nitric oxide delivery devices.

Configuration 2 of FIG. 4 also provides an exemplary set of hardware elements for the control system, which is a more expansive configuration than Configuration 1. In Configuration 2, data is also exchanged with the internet or cloud in order that a physician can remotely view patient data and remotely make adjustments to the LVAD or NO delivery device. It is understood that analytics specific to embodiments of the invention and/or optimization of treatment protocols may also be provided to the physician remotely (*e.g.* in the form of a cloud or internet based application, or as a program resident on the physicians laptop, tablet, smart phone or other device) for patient status tracking or adjustment of treatment regimens. Configuration 2 may also include a user interface for two way information exchange with the patient (or caregiver).

If the control system is implemented integral to the NO delivery device or the LVAD, then many if not all of the hardware elements described above may already be resident on the NO delivery device or on the LVAD.

### Control System Input/Output (IO) Hardware:

Regardless of whether the control system is standalone or resident on the LVAD or NO delivery device, many of the processing tasks are unaffected and therefore may be equivalent. However, certain tasks such as input/output routines and hardware-level code-interaction-dependent tasks (e.g. addressing of memory) may vary depending on whether the control system is standalone or resident on the LVAD or NO delivery device.

The I/O hardware can include at least one bidirectional data port in the case of the LVAD and NO device resident designs and at least two bidirectional data ports in the case of the standalone implementation. It is understood that this bidirectional data port is preferably an opto-isolated serial port; however, other bidirectional data communications schemes including parallel ports, LANs, USB, etc. are also possible. Such bidirectional communications schemes are abundantly described in the art. Further, it is possible that bidirectional communications can be implemented via wireless techniques such as by optical means such as infrared (IR) or by radio frequency (RF) means such as by Bluetooth. Regardless of the communication technique, the bidirectional data transfer preferably occurs in a timely fashion.

**Control System User Interface Hardware:**

For a control system that is resident on the NO delivery device or resident on the LVAD, the user interface can be integrated into the existing, yet extended, user interfaces of those devices. For instance, if the control system is implemented on a NO delivery device, the control system may require additional settings, alarm and modes not currently resident on such a NO delivery device, and therefore the user interface of the NO delivery device can be extended to incorporate such interaction. Further, in the case of a control system that is resident on an LVAD system, the LVAD system user interface can be modified to extend its functionality consistent with new modes, alarms and settings. In addition, many LVAD systems provide for data display on a device worn on the patient and also provide for clinician mediated settings on an ancillary system monitor. It is understood that the functionality of such ancillary system monitor user interfaces can be extended to accommodate the control system.

Finally, in some embodiments of a control system implemented on a standalone device, the user interface can have one of at least two configurations. The first configuration can include an ancillary system monitor which is used by the clinician to set up the system and then removed from the system after setup and initiation of the system. Such a strategy is consistent with the Configuration 1 of FIG. 4. Alternately, a user interface can be integrated into the control system and worn by the user in the form of a module, preferably with a graphical display. Such a strategy is consistent with the more elaborate architecture of Configuration 2 of FIG. 4.

**Control System Remote Connectivity Hardware:**

As shown in FIG. 4, additional functionality may be added to the control system in the form of remote connectivity capability. Hardware for implementing such a strategy may consist of a wired or wireless link to either a computer with an internet link or to a transceiver capable of remote information exchange such as GSM or other remote wireless techniques which can be tied into an internet or cloud infrastructure. It is further understood that this remote information exchange can be implemented using secure/encrypted data links. Other similar techniques are known to those skilled in the art and include, for instance, current bidirectional internet/cloud based secure data exchange schemes such as those based on *e.g,* Intel's Internet of Things (IoT) architecture and similar emerging architectures. Remote connectivity capability can include a remote application programmed on a computer, a cell phone, smart phone, tablet or other device which receives information from either the internet or from a cloud and which displays this information and allows for bi-directional communication with the control system for many purposes, *e.g.* settings adjustments.

**Control System Software Architecture:**

The control system can include a software component which asserts itself as the master over the functionality of the resident NO delivery device and/or LVAD controllers. This software component can be responsible for controlling the NO delivery device and LVAD in an integrated fashion consistent with the clinical scenarios outlined above and/or consistent with the user-specified controller functionality specified below.

Master Slave Configuration

Regardless of whether the invention is resident on the NO delivery device, on the LVAD or is standalone, the control system can be capable of operating with the NO delivery device and LVAD in a master/slave configuration. Specifically, the control system can assert a master role when modes of operation are engaged in which the control of the LVAD and NO delivery device are controlled by the control system. This may include removing the option to make settings adjustments on the LVAD or NO delivery device without overriding the master/slave configuration. Such a master/slave configuration can obviate potential control ambiguity resulting from adjustments to two or even three user interfaces. Such a master/slave configuration can be made possible by integrating additional code into the LVAD or NO delivery device allowing for a master/slave configuration, or the master/slave configuration can be implemented by allowing the control system to immediately override any settings changes made to the LVAD or NO delivery device. Finally, the master/slave configuration can be set and overridden by a clinician through the user interface screen of the control system or remotely via a cloud/internet based application or via a resident application on a tablet, smart phone, laptop or other device.

Input/Output Parameters

The control system can utilize certain input parameters from the LVAD and/or the NO delivery device, and the control system can include software functionality which is implemented in such a fashion as to command control of aspects of the LVAD and NO delivery device functionality. Further, a set of output parameters from the control system can be used so that the LVAD and NO delivery device can be commanded to perform certain tasks.

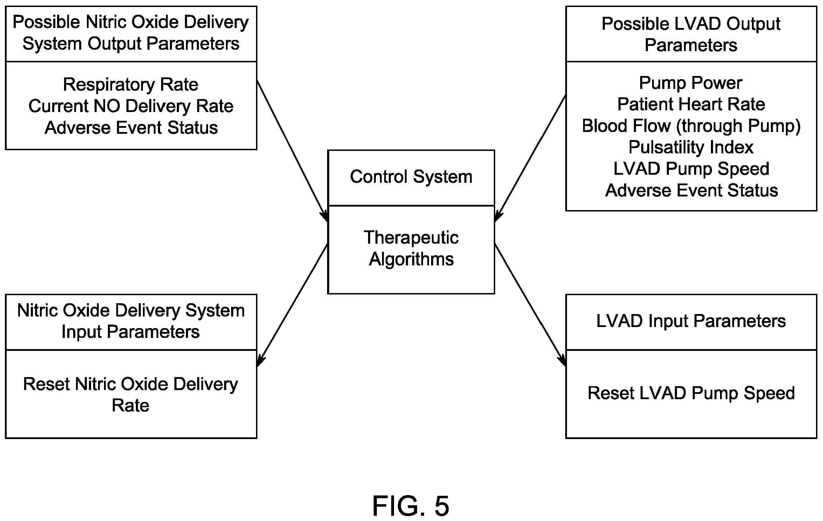

An exemplary set of input and output parameters is shown in FIG. 5. For example, the input parameters from the NO delivery device can include, but are not limited to, the patient's respiratory rate, the current NO delivery rate (*e.g.* ppm, mg/kg IBW/hr, etc.), and/or the adverse event status of the NO delivery device. Exemplary input parameters from the LVAD include, but are not limited to, pump power, patient heart rate, blood flow through the pump, pulsatility index, LVAD pump speed, and/or the adverse event status of the LVAD. Exemplary output parameters from the control system to the NO delivery device include, but are not limited to, resetting/adjusting the NO delivery rate. Exemplary output parameters from the control system to the LVAD include, but are not limited to, resetting/adjusting the LVAD pump speed. Other relevant input/output parameters may also be used.

As set forth above, a control system coordinating operation of the LVAD and the NO delivery device may be used in any of the indications or methods described herein. In various embodiments of this aspect, the system comprises a control system in communication with the NO delivery device and/or the LVAD, wherein the control system monitors one or more parameters of the NO delivery device and/or one or more parameters of the LVAD. If one or more parameters of the NO delivery device and/or LVAD and/or of the patient are outside of a predetermined range, the control system may adjust the LVAD pump settings, the inhaled NO dose, initiate a weaning protocol and/or provide an alert. The system may also comprise the NO delivery device and/or the LVAD itself.

In one or more embodiments, the control system reduces a pump speed of the LVAD if there is a failure of the NO delivery device that discontinues NO administration. Failure of the NO delivery device can either be relatively minor or may be major. The control system may also initiate a weaning procedure for the NO delivery device if there is a failure of the LVAD and/or if the NO delivery device is going to be shutoff or if there is a failure of the NO delivery device. The control system may also adjust the inhaled NO dose and/or provide an alert if any of the monitored parameters are outside of the relevant range and/or if there is a failure of the LVAD and/or NO delivery device.

The control system may provide for autonomous control of LVAD and/or NO delivery device functionality once the control systems' "rules set" has been input by the clinician and once the devices have been initiated by the clinician. The rules set may consist of initial LVAD and NO system settings, input vs. output parameter relationships, alarm settings, communications configuration etc. It is understood that the rules set can be entered interactively (*e.g.* pump speed can be adjusted during patient assessment) or the rules set can be entered preemptively prior to patient use of the device(s). The rules set can be accessible through a menu-driven software program which allows the clinician to input all aspects of required system performance. Some of the rules the clinician enters may be statically set, *i.e.* the setting does not change once set. In some embodiments, a high pump speed alarm is an example of a static rule. The rules may define a variable input vs. output relationship over time; for instance, the pump speed might be commanded to be reduced over time or further the rules set may allow the controller to control parameter variation within predefined limits or control parameter variation through linear or nonlinear control techniques in the which input/output parameter relationship can be controlled dynamically.

Menu-Driven Modes of Control

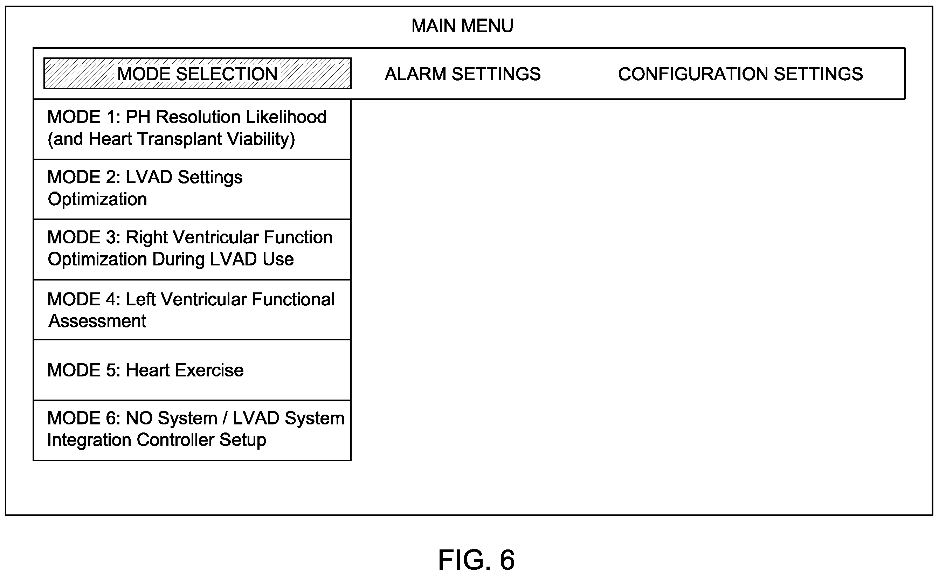

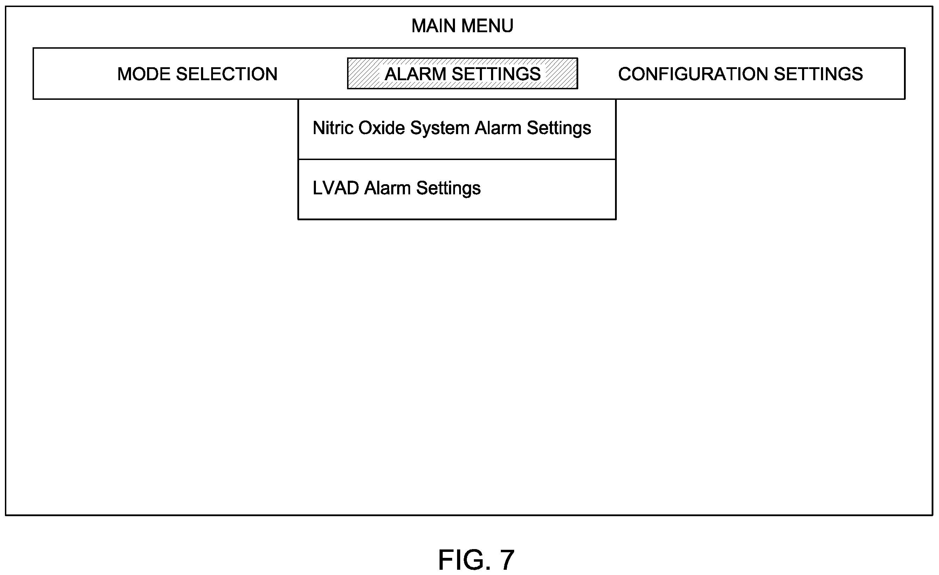

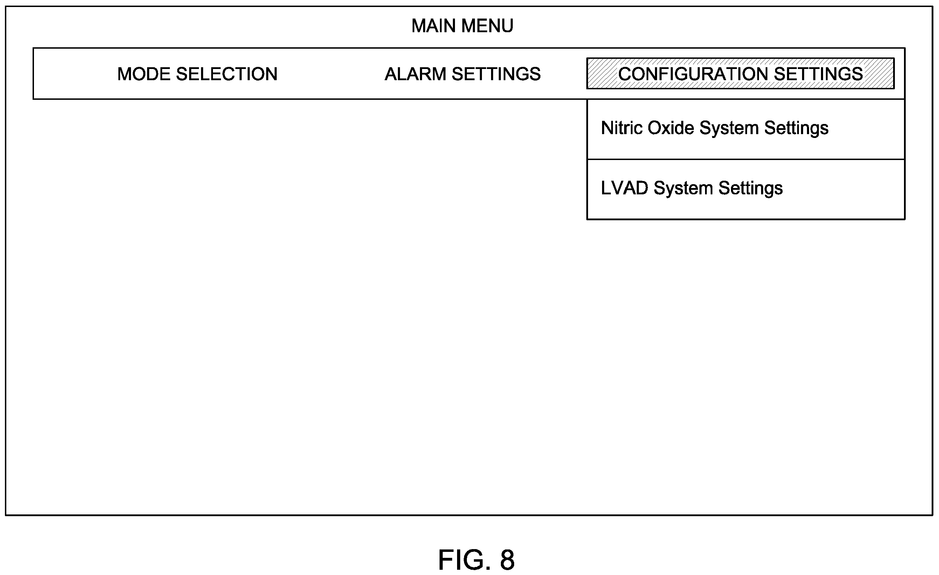

In one or more embodiments, the control system user interface allows for selecting a number of menu-driven control schemes in which the operation of the NO delivery device and/or LVAD is controlled consistent with the clinical needs of the patient or clinician. An example of such a main user interface menu is shown in FIGS. 6-8. For example, FIG. 6 shows a menu that allows for the user to select a "mode" or control scheme, which can be any of the indications or methods described above. In some embodiments, when one particular mode is enabled, the other modes are disabled, *i.e.* the modes are user accessible with exclusivity. The menu can also include other user settable parameters such as alarm limits, NO delivery device and LVAD system configuration settings for therapy startup, weaning parameters, or for the case when an active mode is disabled (*e.g.* when a mode is disabled, the configuration settings would be used as default settings). FIG. 7 shows an exemplary alarm settings menu and FIG. 8 shows an exemplary menu for setting the NO delivery device and/or LVAD system configuration settings. These menus can include submenus for setting the alarms and/or configuration settings. In some embodiments, the alarms, NO delivery device settings and/or LVAD settings may have some options disabled based on the mode selection. Further, the settings in these configuration submenus can allow for default settings to be locked which are returned to when none of the mode selection options are active.

Exemplary modes are described in further detail below. These exemplary modes are not intended to be limiting, and fewer than all of these modes or additional modes can be provided in a menu-driven user interface of the control system.

MODE 1: PH Resolution Likelihood (and Heart Transplant Viability)

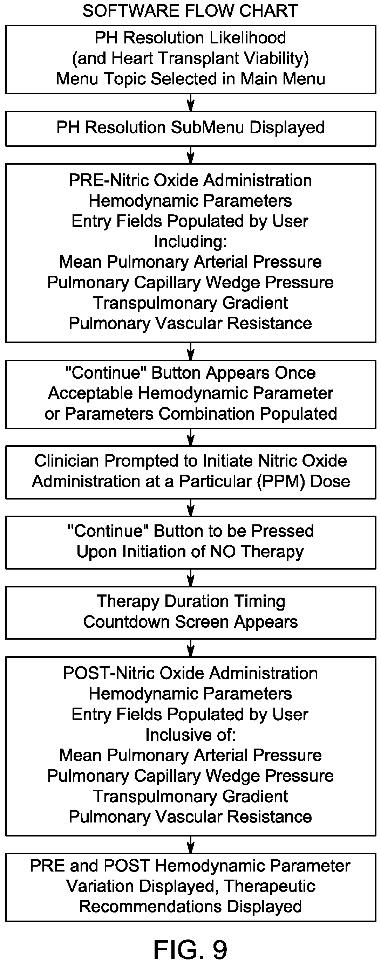

The Mode 1 submenu can include settings and operational flow aimed at determining the patient's hemodynamic response to inhaled NO administration for the purpose of determining if the patient might resolve pulmonary hypertension after continued use of the LVAD. In some embodiments, this mode can also be used to assess a patient's heart transplant viability. The mode can allow the user to input germane hemodynamic values before and/or after a NO delivery settings change. The results can be subject to clinical opinion or the results can be compared to acquired clinical study data and predictions for patient outcome provided by the software. FIG. 9 shows one embodiment of the program flow for the submenu relating to this mode.

MODE 2: LVAD Setting Optimization

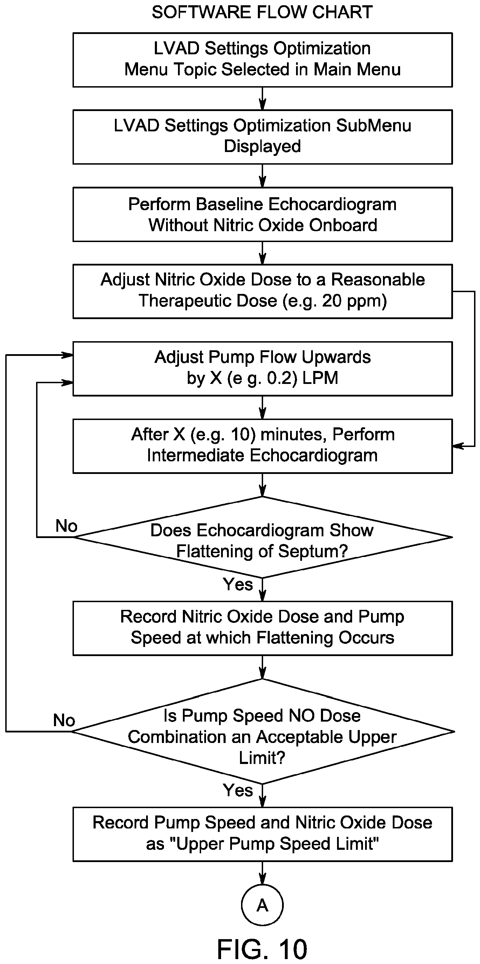

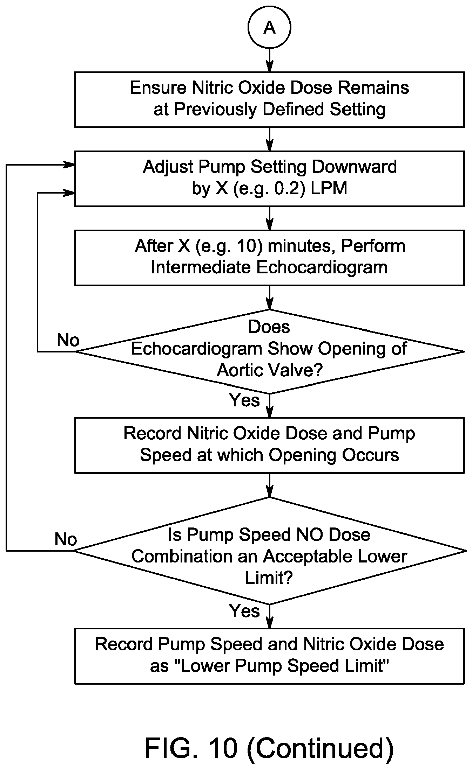

Mode 2 can include a method for determining the upper and lower limits of LVAD pump speed operation concurrent with inhaled NO delivery. FIG. 10 shows one embodiment of the program flow for the submenu relating to this mode. In this mode, the pump speed (or potentially other LVAD parameters) are adjusted during administration of NO in order that cardiac output can be optimized. Specifically, an echocardiogram is utilized in this mode to determine the low pump speed setting corresponding to the minimal pump speed necessary for the aortic valve to open with each heart beat and also to determine the high pump speed at which the septum of the heart flattens. In this mode, the user is prompted to make an initial nitric oxide setting and subsequent pump speed adjustments in the software. The software ultimately records the target pump speeds for utilization.

MODE 3: Right Ventricular Function Optimization During LVAD Use

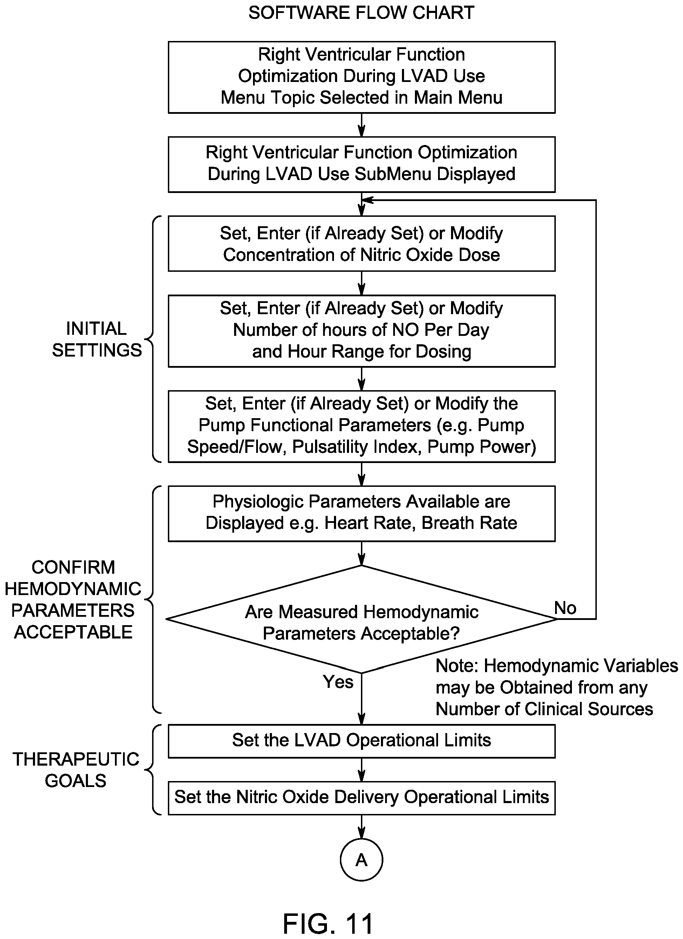

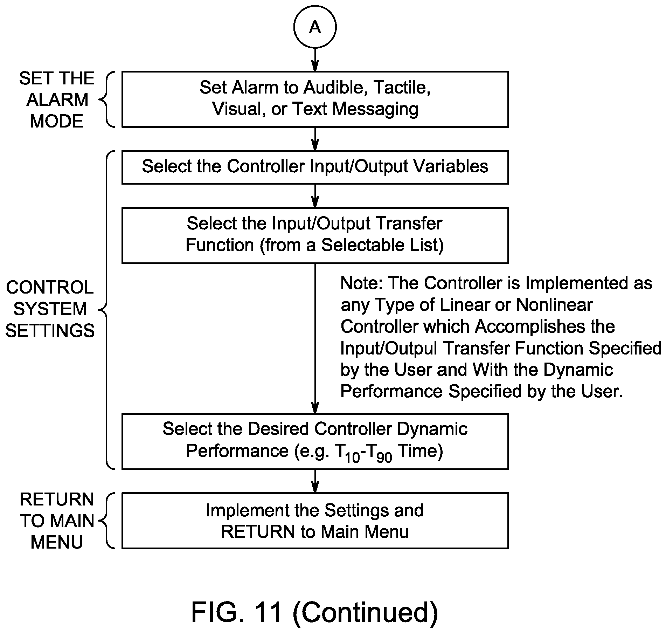

Mode 3 can provide a method for reducing the risk of right ventricular failure during LVAD use based on concurrent delivery of nitric oxide with LVAD use by making settings adjustments to both the NO dose and LVAD settings. FIG. 11 shows one embodiment of the program flow for the submenu relating to this mode. These settings can be adjusted manually (as shown in the first portion of the flow diagram in FIG. 11) or can be adjusted by a user defined control system and user defined transfer function and dynamic performance characteristics (see latter portion of flow diagram in FIG. 11). Monitoring of the patient can be performed by any number of clinical techniques including hemodynamic assessment. Finally, alarm limits for measured LVAD and NO delivery device parameters can be set.

MODE 4: Left Ventricular Functional Assessment

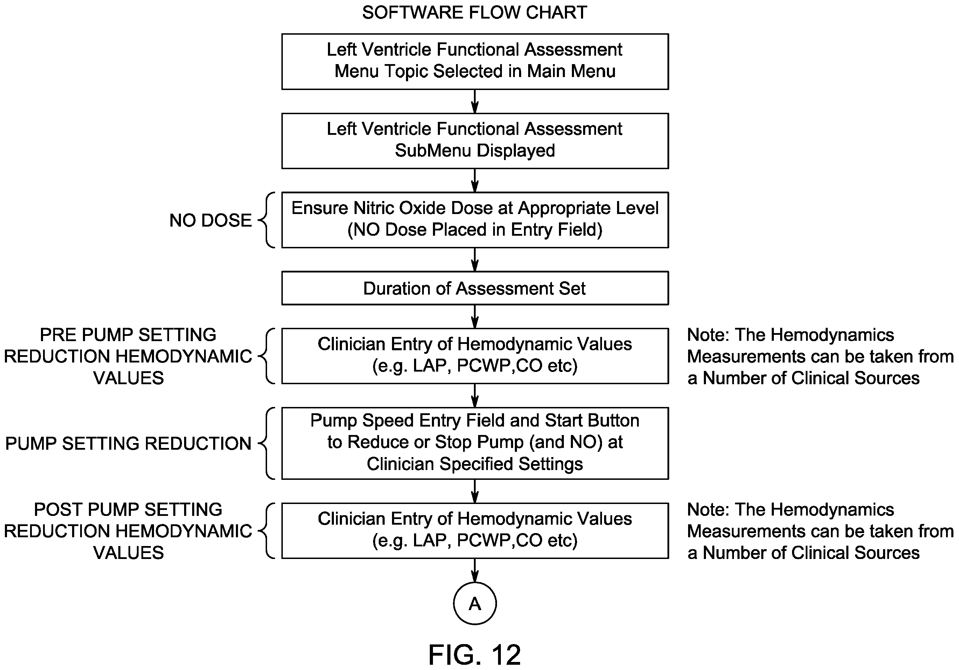

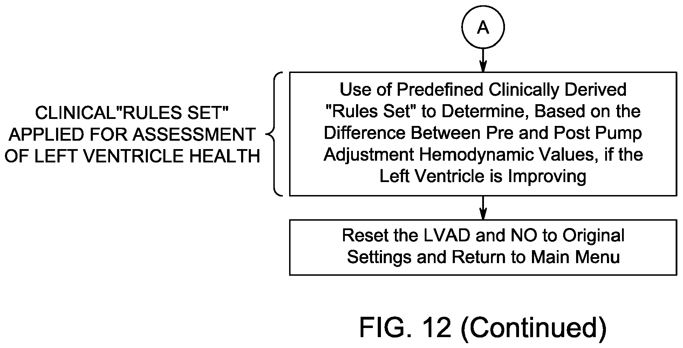

Mode 4 can be used to assess the status of left ventricular function following either a reduction in setting or complete cessation of the LVAD pump. FIG. 12 shows one embodiment of the program flow for the submenu relating to this mode. Ventricular function can be assessed by means of measured hemodynamic parameters such as LAP, PCWP or CO. Finally, pre- and post-pump speed adjustment hemodynamic parameter changes may be optionally assessed by a smart algorithm (see second to last box in FIG. 12) which provides clinical feedback based on these hemodynamic changes about the status of the left ventricle.

MODE 5: Heart Exercise Facility

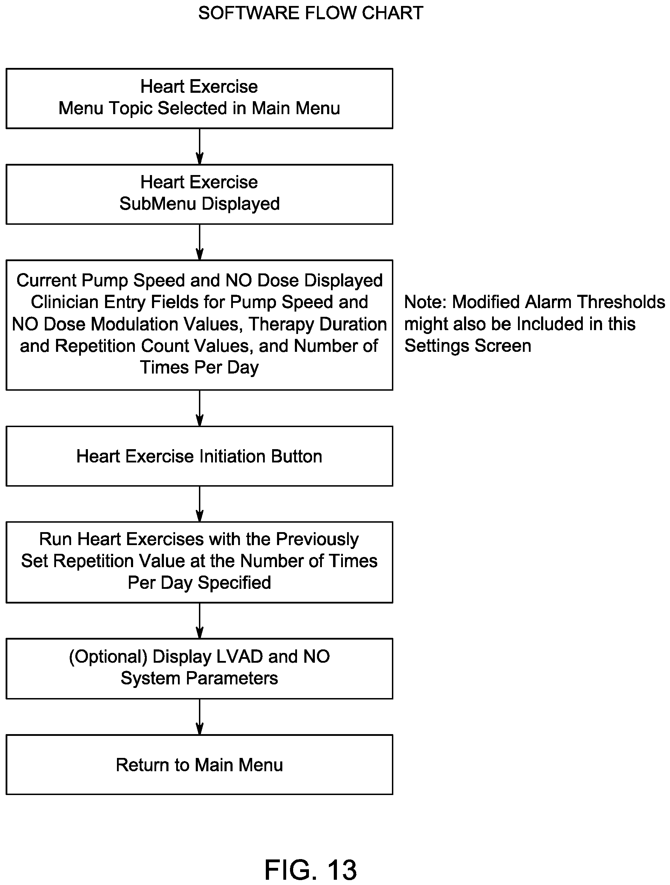

Mode 5 can be used to exercise the heart by first reducing the speed of or stopping the LVAD pump, then preloading the left ventricle by short term administration of NO and then subsequently cycling off and on the dose of NO to exercise the (left ventricle) heart. An exemplary submenu for implementing this mode is shown in FIG. 13.

MODE 6: Global Control System Settings

Mode 6 can be used to set the control system, which can oversee the functionality of the NO delivery device and/or the LVAD. This control system setting can be set to be the master control system used at all times during the operation of the invention or alternately it may be disabled in particular modes previously described. The control system settings can provide methods for adjustment of nitric oxide dose or LVAD pump speed based on the input parameters specified in FIG. 5 or alternately based on measured hemodynamic parameters input at timely intervals by the clinician. In this mode, the clinician can be allowed to specify the input parameters and also the transfer functions between single or multiple input parameters and the output parameters (*e.g.* pump speed and NO dose). The dynamic performance of this relationship can also be user specified. Finally, timed, respiratory cycle synchronized pulsed delivery of NO can also be specified. This may include single or multiple pulses of NO of fixed or time-varying concentration profiles. The ability of the clinician to turn on the controller (enabling output parameter variation in time) or to disable the controller in favor of making static (output) settings adjustments can also be specified. These settings can be displayed along with measured parameters and alarm settings (as specified in another main menu item) in the main menu screen in order that rapid assessment of patient status can be obtained by the clinician at a glance.

## LVAD

The LVAD may be any appropriate LVAD prescribed by a physician, including, but not limited to, pulsatile, semi-pulsatile (such as those that perform pulsatile pump speed modulation), or continuous-flow LVADs. The LVAD can have any appropriate mechanism of providing blood flow, including, but not limited to, valves, centrifugal pumps, turbines, etc. The LVAD can be internal or external to the patient. In some embodiments, the LVAD is an internal LVAD that is implanted in the patient.

## NO DELIVERY DEVICE

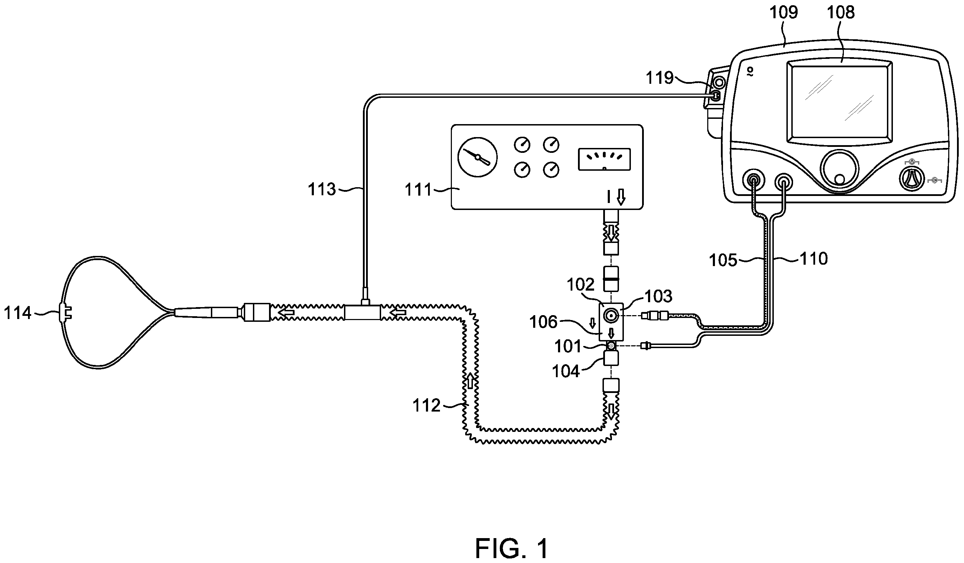

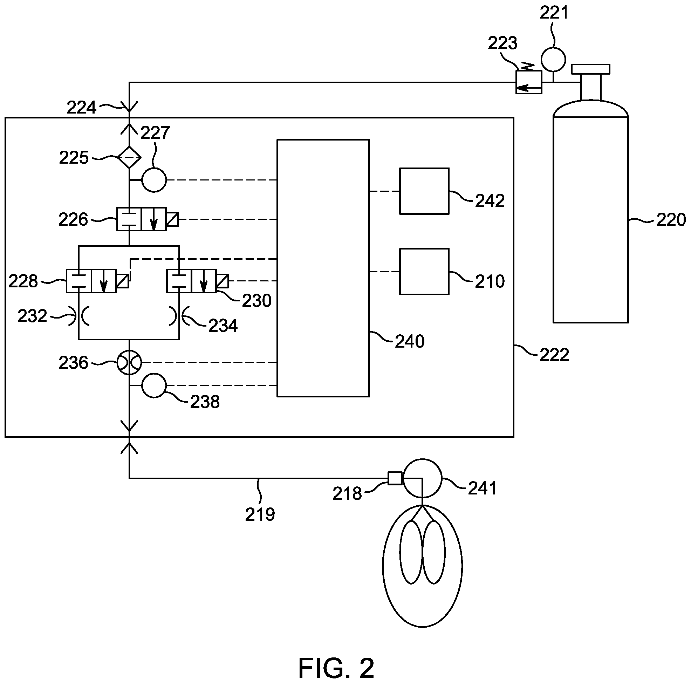

The NO delivery device may include any appropriate components for administering inhaled NO to the patient, including flow sensors, valves, flow controllers, processors, safety shut-off valves, purge valves, tubing etc. The NO delivery device may administer a constant concentration of inhaled NO, such as described by U.S. Patent No. 5,558,083. An example of such a NO delivery device is shown in FIG. 1. The NO delivery device may administer a plurality of pulses of inhaled NO to provide a dose of inhaled NO that is independent of a patient's breathing pattern, such as described by U.S. Patent No. 7,523,752. An example of such a NO delivery device is shown in FIG. 2. The NO delivery device may also have the features described in any of the following U.S. Patents and published U.S. Patent Applications: U.S. Patent No. 8,573,209; U.S. Patent No. 8,573,210; U.S. Patent No. 8,770,199; and U.S. Patent App. Pub. No. 2014/0283828. Other appropriate NO delivery devices are known in the art, such as the INOmax DSIR®, INOmax® DS and/or INOvent®.

In the exemplary NO delivery device shown in FIG. 1, a therapeutic injector module 103 is in fluid communication with a first inlet 101 and a second inlet 102. First inlet 101 is in fluid communication with therapeutic gas injector tube 110, which is in fluid communication with a therapeutic gas supply comprising NO. Second inlet 102 is in fluid communication with breathing gas delivery system 111, which is illustrated as a ventilator. The arrows in FIG. 1 indicate the direction of flow for the breathing gas and the combined gas mixture of therapeutic gas and breathing gas. Flow sensor 106 is in fluid communication and downstream of second inlet 102, and monitors the flow of breathing gas through therapeutic injector module 103. The top view of therapeutic injector module 103 is shown. The therapeutic gas and breathing gas mix in therapeutic injector module 103 to provide a gas mixture. Injector module cable 105 connects therapeutic injector module 103 with control module 109. Control module 109 comprises display 108, which can display information about NO delivery and/or any of the parameters described herein, and can provide any alerts as described herein. Inspiratory breathing hose 112 is in fluid communication with outlet 104 and nasal cannula 114 or other patient interface. The inspiratory breathing hose provides the gas mixture of breathing gas and therapeutic gas to nasal cannula 114, which delivers the gas mixture to the patient. Patient gas sample line 113 diverts some of the flow of the gas mixture from inspiratory breathing hose 112 and brings it to sample block 119 for measuring NO, $O_2$ and/or $NO_2$ concentrations in the gas delivered to the patient.

In the exemplary NO delivery device shown in FIG. 2, a supply tank 220 can be in fluid communication with a tank pressure gauge 221 and a regulator 223 to bring the tank pressure down to the working pressure of gas delivery device 222. The pharmaceutical gas can enter gas delivery device 222 through an inlet 224 that can provide a ready connection between delivery device 222 and supply tank 220 via a conduit. Gas delivery device 222 can have a filter 225 to ensure no contaminants can interfere with the safe operation of the device and/or a pressure sensor 227 to detect if the supply pressure is adequate and can thereafter include a gas shut off valve 226 as a control of the pharmaceutical gas entering deliver device 222 and to provide safety control in the event delivery device 222 is over delivering the pharmaceutical gas to the patient. In the event of such over delivery, shut off valve 226 can be immediately closed and an alarm 242 can be sounded to alert the user that the gas delivery device has been disabled. As such, shut off valve 226 can be a solenoid operated valve that can be operated from signals directed from a central processing unit including a microprocessor.

Downstream from shut off valve 226 can be a flow control system that controls the flow of the pharmaceutical gas to the patient through the gas conduit 219 to the patient device 218 to the patient 241. In exemplary embodiments, the flow control system can comprise a first flow control valve that can be a high flow control valve 228 and a second flow control valve that can be a low control valve 230 and there can be a first flow orifice that can be a high flow orifice 232 and a second flow orifice that can be a low flow orifice 234. The purpose and use of the flow valves 228, 230 and flow orifices 232, 234 will be later explained. A gas flow sensor 236 can also be located in the flow of pharmaceutical gas to patient device 218 and, as shown, can be downstream from the flow control system, however, gas flow sensor 236 may alternatively be located upstream of the flow control system.

Next, there can be a patient trigger sensor 238. When the patient breathes in during inspiration it can create a small sub atmospheric pressure in the nose or other area where patient device 218 is located, and hence in patient device 218 itself. Patient trigger sensor 238 can detect this pressure drop and can provide a signal indicative of the start of inspiration of the patient. Similarly, when the patient breathes out there can be a positive pressure in patient device 218 and patient trigger sensor 238 can detect that positive pressure and can provide a signal indicative of the beginning of expiration. This can allow patient trigger sensor 238 to determine not only the respiratory rate of the patient but also the inspiratory times and/or expiratory times. It will be understood that other techniques can be used to determine the respiratory rate of the patient, inspiratory times, and expiratory times, and/or other aspects of patient breathing.

There can also be a central processing unit (CPU) 240 that can communicate with patient trigger sensor 238, flow valves 228, 230, gas shut off valve 226, and other components in order to carry out various exemplary embodiments of the present disclosure. CPU 240 can include a processing component such as a microprocessor to carry out all of the solutions to the equations that can be used by the gas delivery device 222 to deliver the predetermined quantity of the pharmaceutical gas to a patient. The CPU 420 can be connected to the front panel 210 where the user can enter settings and monitor therapy.

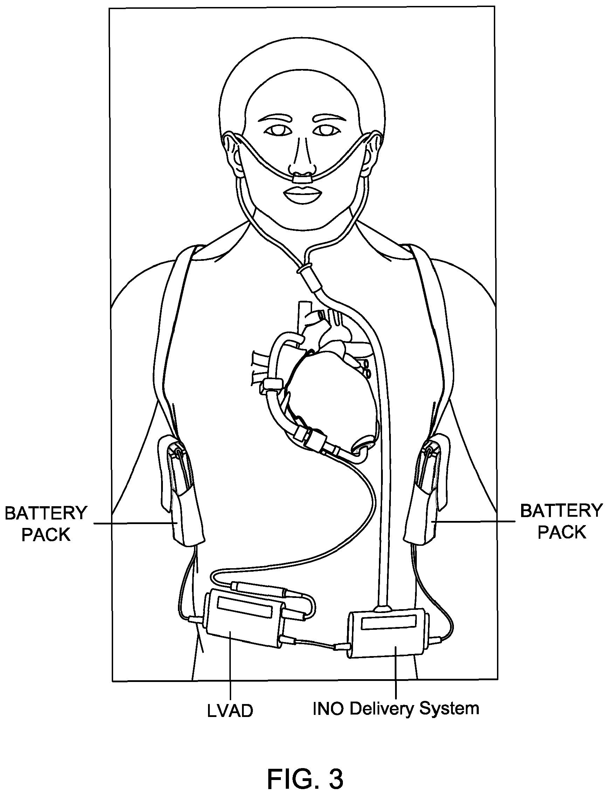

As shown in FIG. 3, the NO delivery device may be in communication with the LVAD. In the embodiment shown in FIG. 3, the control system is integral to the NO delivery device. However, as described above, the control system can also be integral to the LVAD or can be a separate, stand-alone control module.

The NO delivery device may also include components for monitoring the gas that is administered to the patient, such as gas concentration sensors (e.g. $O_2$, NO and/or $NO_2$ sensors), sampling pumps, etc. The NO delivery device may also include redundant sensors and/or valves and have an automatic backup delivery system in case of failure of the primary NO delivery system. The NO delivery device may also include one or more sensors for feedback control of the NO delivery and/or for independent safety monitoring of NO delivery. The NO delivery device can also provide alarms if any of the monitored parameters meet or exceed a certain level or if other safety issues are present.

The NO delivery device may be portable and light (*e.g.,* less than 10 lbs) so that it does not hinder the patient's mobility. The NO delivery device may run on a battery and have a battery life that meets a certain minimum criteria, such as having a battery life of at least 16 hours. The NO delivery device may also include a backup battery or other power source.

The NO source may include two or more gas cylinders such that continuous NO administration is not interrupted when one of the gas cylinders is replaced. Also, instead of a cylinder of NO-containing gas, the NO may be generated bedside, such as by an appropriate chemical reaction, *e.g.* the reaction of a NO-releasing agent and a reductant such as ascorbic acid. Other methods for generating nitric oxide bedside are also known in the art.

The NO delivery device may also include an automated pre-use checkout procedure with automatic purge to clear $NO_2$, and on-screen setup instructions. The system may also have on-screen alarm help, and wireless connectivity to communicate with an electronic medical record (EMR) system or a tech support desk for remote troubleshooting. Another safety feature may be the incorporation of sensors and mechanisms to automatically detect fluid or gas leaks.

The NO delivery device may have additional safety features such as a weaning protocol that slowly reduced the inhaled NO dose to avoid a sudden increase in PAP. Such a weaning protocol may be initiated by a "wean button" on the NO delivery device, or may initiated automatically by the control system in communication with the LVAD and/or the NO delivery device. The weaning protocol may include small changes in the NO dose, such as reducing the dose by 5 ppm for several minutes, followed by reducing the dose by a further 5 ppm for several minutes, until the patient is completely weaned off of the inhaled NO. Other increments for a weaning protocol include 0.1 ppm, 0.2 ppm, 0.5 ppm, 1 ppm, 2 ppm, 3 ppm, 4 ppm, 5 ppm, 6 ppm, 7 ppm, 8 ppm, 9 ppm, 10 ppm, 15 ppm and 20 ppm, or 0.1, 0.2, 0.5, 1, 2, 3, 4, 5, 6, 7, 8, 9, 10, 15, 20, 25, 30, 35, 40 or 50 µg/kg IBW/hr. Multiple weaning protocols may be used based on the failure detected and whether device shutoff is eminent. The weaning protocol may also be individualized for a particular patient, such as having the weaning protocol be dependent on the degree of the patient's current and/or prior PH.

As described above, in some embodiments the inhaled NO dose is adjusted in response to the monitoring of various parameters of the LVAD and/or the patient. Such adjustments can include an increase or a decrease in the inhaled NO dose (either in ppm or µg/kg IBW/hr). Increments for an adjustment include 0.1 ppm, 0.2 ppm, 0.5 ppm, 1 ppm, 2 ppm, 3 ppm, 4 ppm, 5 ppm, 6 ppm, 7 ppm, 8 ppm, 9 ppm, 10 ppm, 15 ppm and 20 ppm, or 0.1, 0.2, 0.5, 1, 2, 3, 4, 5, 6, 7, 8, 9, 10, 15, 20, 25, 30, 35, 40 or 50 µg/kg IBW/hr.

In accordance with the disclosure that is not part of the claimed invention, a method of determining whether a patient with pulmonary hypertension will resolve the pulmonary hypertension with continued use of a left ventricular assist device (LVAD) is provided. The method may include measuring one or more pulmonary hemodynamic parameters of a patient with an LVAD to obtain a first pulmonary hemodynamic value; after obtaining the first pulmonary hemodynamic value, administering inhaled nitric oxide to the patient with the LVAD; and measuring one or more pulmonary hemodynamic parameters of the patient during or after the inhaled nitric oxide administration to obtain a second pulmonary hemodynamic value. The decrease in the pulmonary hemodynamic parameter from the first pulmonary hemodynamic value to the second pulmonary hemodynamic value of at least 10 mm Hg and/or at least 20% indicates that the patient is likely to resolve the pulmonary hypertension after continued use of the LVAD.

The method may further include selecting the pulmonary hemodynamic parameter from mean pulmonary artery pressure (mPAP), transpulmonary gradient (TPG) and pulmonary vascular resistance (PVR). The method may further include administering the nitric oxide at a concentration of 5 to 80 ppm for at least 10 minutes.

In accordance with the invention, the method may include measuring one or more pulmonary hemodynamic parameters by performing a right heart catheterization. The patient may be placed on a heart transplant list if the decrease in the pulmonary hemodynamic parameter from the first pulmonary hemodynamic value to the second pulmonary hemodynamic value is at least 10 mm Hg and/or at least 20%. The LVAD may be explanted and a donor heart is implanted in the patient.

In accordance with the disclosure that is not part of the claimed invention, a method of optimizing the settings of a left ventricular assist device (LVAD) is provided. The method may include administering inhaled nitric oxide to a patient having an LVAD; performing an echocardiogram on the patient during the administration of inhaled nitric oxide; and adjusting or setting one or more parameters of the LVAD during the echocardiogram and during the administration of inhaled nitric oxide to optimize cardiac output. The adjusting or setting of one or more parameters of the LVAD may include one or more of (i) determining a low pump speed setting for the LVAD based on the minimal pump speed necessary for the patient's aortic valve to open with each heart beat or (ii) determining a high speed setting for the LVAD based on the pump speed at which the septum of the patient's heart flattens. The inhaled nitric oxide may be administered at a concentration of 5 to 80 ppm for at least 10 minutes.

In accordance with the disclosure that is not part of the claimed invention, a method of reducing the risk of right ventricular failure during left ventricular assist device (LVAD) use is provided. The method may include administering inhaled nitric oxide to a patient with an LVAD for at least 12 hours a day for at least 20 days to reduce the risk of right ventricular failure, and confirming that the LVAD is functioning before administering inhaled nitric oxide. The inhaled nitric oxide may be administered after a patient has been weaned from cardiopulmonary bypass (CPB), and may be administered for at least 30 days, or at least 3 months. The inhaled nitric oxide may be administered at a concentration of 5 to 80 ppm or at a dose of 25 to 150 $\mu$g/kg IBW/hr.

The method may further include monitoring one or more output parameters of the LVAD and/or one or more hemodynamic parameters of the patient, comparing the one or more output parameters and/or the one or more hemodynamic parameters to a predetermined range, adjusting the dose of inhaled nitric oxide if the one or more outputs parameters and/or the one or more hemodynamic parameters are outside of the predetermined range, and providing an alert if the one or more output parameters and/or the one or more hemodynamic parameters are outside of the predetermined range.

In accordance with the disclosure that is not part of the claimed invention, a method of monitoring the left ventricle of a patient with a left ventricular assist device (LVAD) is provided. The method may include reducing the pump speed of the LVAD or turning off the LVAD; measuring one or more pulmonary hemodynamic parameters of the patient to obtain a first pulmonary hemodynamic value; preloading the left ventricle by administering inhaled nitric oxide to the patient; and measuring one or more pulmonary hemodynamic parameters of the patient after or during administration of inhaled nitric oxide to obtain a second pulmonary hemodynamic value. The pulmonary hemodynamic parameter may be selected from left atrial pressure (LAP), pulmonary capillary wedge pressure (PCWP) and cardiac output (CO). The inhaled nitric oxide may be administered at a concentration of 5 to 80 ppm for at least 10 minutes. Treatment may be modified by explanting the LVAD from the patient if the left ventricle is improving. An increase in LAP and/or PCWP from the first pulmonary hemodynamic value to the second pulmonary hemodynamic value of less than 5 mm Hg may indicate that the left ventricle is improving.

In accordance with the disclosure that is not part of the claimed invention, a method of exercising a heart of a patient having a left ventricular assist device (LVAD) is provided. The method may include reducing the pump speed of the LVAD or turning off the LVAD; preloading the left ventricle by administering inhaled nitric oxide to the patient for at least 5 minutes; discontinuing the inhaled nitric oxide administration; and repeating the preloading and discontinuation to exercise the left ventricle of the patient's heart. The preloading of the left ventricle may include administering inhaled nitric oxide at a concentration of 5 to 80 ppm for a time period in the range from 5 to 30 minutes, and may be performed one to five times a day.

In accordance with the disclosure that is not part of the claimed invention, a method of reducing the risk adverse events during use of a continuous-flow or semi-pulsatile left ventricular assist device (LVAD) is provided. The method may include administering inhaled nitric oxide to a patient with a continuous-flow or semi-pulsatile LVAD for at least 12 hours a day for at least 20 days. The adverse events may be associated with reduced pulsatility and/or associated with impaired NO-mediated vascular function. Before administering inhaled nitric oxide, function of the LVAD may be confirmed. The inhaled nitric oxide may be administered after a patient has been weaned from cardiopulmonary bypass (CPB), and may be administered for at least 30 days or 30 months. The inhaled nitric oxide may be administered at a concentration of 5 to 80 ppm or at a dose of 25 to 150 $\mu$g/kg IBW/hr.

The method may further include monitoring one or more output parameters of the LVAD and/or one or more hemodynamic parameters of the patient, comparing the one or more output parameters and/or the one or more hemodynamic parameters to a predetermined range, and adjusting the dose of inhaled nitric oxide if the one or more outputs parameters and/or the one or more hemodynamic parameters are outside of the predetermined range. An alert may be provided if the one or more output parameters and/or the one or more hemodynamic parameters are outside of the predetermined range.

In accordance with the disclosure that is not part of the claimed invention, a method of optimizing the dose of inhaled nitric oxide for use with a continuous-flow or semi-pulsatile left ventricular assist device (LVAD) is provided. The method may include measuring endothelial function of a patient having a continuous-flow or semi-pulsatile LVAD; administering inhaled nitric oxide to the patient at a first dose; measuring the endothelial function of the patient during the administration of inhaled nitric oxide; and adjusting the dose of inhaled nitric oxide to optimize endothelial function. The measuring the endothelial function of the patient comprises measuring one or more of (i) flow-mediated dilation (FMD) or (ii) reactive hyperemic index (RHI).

The method may include measuring one or NO-related molecules and/or other biomarkers of endothelial function in the patient's blood and/or plasma. The NO-related molecules may be selected from the group consisting of nitrite ($NO_2^-$), nitrate ($NO_3^-$) and nitrosohemoglobin.

In accordance with the invention, a system is provided. The system may include a control system in communication with a nitric oxide delivery device and/or a left ventricular assist device (LVAD), wherein the control system monitors one or more parameters of the nitric oxide delivery device and/or one or more parameters of the LVAD and provides an alert if one or more parameters of the nitric oxide delivery device and/or the LVAD are outside of a predetermined range. The control system may reduce a pump speed of the LVAD if there is a failure of the nitric oxide delivery device. The control system may initiate a weaning procedure for the nitric oxide delivery device if there is a failure of the LVAD. The control system may be integral to the nitric oxide delivery device, integral to the LVAD or a component of a stand-alone control module. The system may include the nitric oxide delivery device and the LVAD.

Although the invention herein has been described with reference to particular embodiments, it is to be understood that these embodiments are merely illustrative of the principles and applications of the present invention.

**Preventive or treatment use of INOmax® in the premature neonate is not approved by the U.S. Food and Drug Administration (FDA). Therefore, safety and efficacy are not considered to have been established. For this reason, Mallinckrodt cannot make recommendations for use of the drug in such applications and must defer all diagnostic, prophylactic, and treatment decisions in this population solely to the clinician.**

**INOmax is not approved for use in premature newborns, children, or adults.**

## INTRODUCTION

It is estimated that 12.18% of all births resulted in premature delivery (<37 weeks gestation) in the United States in 2009 leaving these infants highly vulnerable to serious complications and death.[1] Preterm neonates are at particular risk for long-term neurocognitive and medical impairment, including chronic lung disease (CLD) such as bronchopulmonary dysplasia (BPD).[2] Prior to the routine use of exogenous surfactant and antenatal steroid therapy, the fibrosis and inflammation associated with this diagnosis were primarily attributed to barotrauma and oxygen toxicity.[2,3] More recently, lungs from infants dying with BPD have demonstrated less fibrosis than historically found.[3] Common findings also now include abnormal alveolarization with decreases in pulmonary microvascular development as well as vascular endothelial growth factor (VEGF) and its receptors, signaling additional pathogenic factors requiring study.[3]

In multiple studies, the use of inhaled nitric oxide (NO) in animals treated with VEGF receptor antagonists appeared to promote pulmonary vascular and alveolar growth.[4-7] Protecting the premature lung from injury and promoting normal lung maturation therefore now represents two potential methods by which outcomes for neonates at risk of BPD might be improved. To date, several primary clinical studies have evaluated the safety and effectiveness of inhaled NO for BPD prophylaxis in preterm infants with mixed results.[8-15] Doses ranged from 5 to 20 ppm, however, it remains unclear what dose should be considered optimal in this population. Overall, subjects with less severe respiratory distress seemed to demonstrate some benefit while those with the greatest degree of prematurity had less successful outcomes. These data have broadened understanding of the complex pathogenesis contributing to the development of BPD and prompted researchers to ask if changes in treatment-related variables such as timing, dosing, and duration of NO treatment might be beneficial in optimizing therapy outcomes.

## TABLE OF CONTENTS

| | |
|---|---|
| ATS/AHA GUIDELINE | 72 |
| Abman et al 2015 | 72 |
| NICHD EVALUATION | 73 |
| Truog et al 2014 | 73 |

(continued)

| | |
|---|---|
| COMMITTEE ON FETUS AND NEWBORN; AMERICAN ACADEMY OF PEDIATRICS CLINICAL REPORT* | 73 |
| Kumar P and Committee on Fetus and Newborn 2013 | 73 |
| NIH CONSENSUS STATEMENT | 74 |
| Cole et al, 2011 | 74 |
| META-ANALYSES | 75 |
| Askie et al, 2011 | 75 |
| Donohue et al, 2011 | 75 |
| Barrington & Finer, 2010 | 76 |
| Hoehn et al, 2006 | 76 |
| Hoehn et al, 2000 | 77 |
| EUNO (MERCIER, 2010) | 77 |
| Durrmeyer X, 2013 | 78 |
| NO CLD (BALLARD RA, 2006) | 80 |
| Banks, 1999 | 81 |
| Kaplan, 2006 | 82 |
| Ballard PL, 2007 | 82 |
| Di Fiore, 2007 | 83 |
| Truog, 2007 | 83 |
| Ballard PL, 2008 | 84 |
| Hibbs, 2008 | 84 |
| Keller, 2009a | 85 |
| Keller, 2009b | 85 |
| Posencheg, 2010 | 86 |
| Ballard PL, 2010 | 86 |
| Walsh, 2010 | 87 |
| EARLY INO (KINSELLA, 2006) | 88 |
| Kinsella, 2009 | 88 |
| Watson, 2009 | 90 |
| *Gadhia, 2014* | 91 |
| INNOVO (FIELD, 2005 | 92 |
| Ahluwalia, 2006 | 93 |
| Hoo, 2009 | 93 |
| Huddy, 2008 | 93 |
| HASCOET, 2005 | 95 |
| | 95 |
| Desandes, 2004 | 96 |
| Hamon, 2009a | 97 |
| Hamon, 2009b | 97 |
| Hascoet, 2009 | 97 |
| NICHD-PINO (VAN MEURS, 2005) | 99 |
| Van Meurs, 2007 | 100 |
| Van Meurs, 2005 | 100 |
| Chock, 2008 | 100 |
| Ambalavanan, 2008 | 101 |
| Gordon, 2006 | 102 |
| Hintz, 2006 | 102 |
| Sokol, 2006 | 103 |
| Hintz, 2007 | 103 |
| SCHREIBER, 2003 | 105 |
| Srisuparp, 2002 | 106 |
| Izhar, 2001 | 106 |

(continued)

| | |
|---|---|
| Izhar, 2000 | 107 |
| Mestan, 2005 | 107 |
| White, 2010 | 109 |
| Cooper, 2011 | 109 |
| Patrianakos-Hoobler, 2011 | 111 |
| FRANCO-BELGIAN COLLABORATIVE NO TRIAL GROUP (MERCIER, 1999) | 112 |
| Truffert, 2003 | 113 |
| SVEDENKRANS ET AL, 2015 | 123 |
| AIKIO ET AL, 2012 | 124 |
| SHARMA ET AL, 2011 | 125 |
| DEWHURST ET AL, 2010 | 126 |
| O'DONNELL, FRANTA, & MILETIN, 2010 | 127 |
| HOPPER ET AL, 2009 | 128 |
| SU & CHEN, 2008 | 128 |
| KHEMANI ET AL, 2007 | 129 |
| KUMAR ET AL, 2007 | 130 |
| TANAKA ET AL, 2007 | 131 |
| DANI ET AL, 2006 | 132 |
| LINDWALL ET AL, 2005 | 133 |
| ATHAVALE ET AL, 2004 | 134 |
| UGA ET AL, 2004 | 135 |
| AIKIO ET AL, 2003 | 136 |
| CLARK PL ET AL, 2002 | 137 |
| TRUOG ET AL, 2002 | 138 |
| DU ET AL, 2002 | 139 |
| KINSELLA ETAL, 1999 | 140 |
| YADAV & EMMERSON, 1999 | 141 |
| CHEUNG ET AL, 1998 | 142 |
| LAUBSCHER ET AL, 1997 | 143 |
| SKIMMING ETAL, 1997 | 144 |
| SUBHEDAR, RYAN, SHAW, 1997 | 145 |
| SUBHEDAR & SHAW, 1997 | 146 |
| BENNETT ET AL, 2001 | 146 |
| VAN MEURS ET AL, 1997 | 147 |
| PELIOWSKI ET AL, 1995 | 148 |

## SUMMARY - INDIVIDUAL STUDIES

A search of the medical literature was conducted for the period 1995 to present in PubMed, EMBASE, the Cochrane Databases, and the abstract files of the Pediatric Academic Societies using the search terms: "nitric oxide", neonate or infant or newborn, and "preterm or premature or preemie". The search yielded the following clinical studies and meta-analyses, which have been indexed by study characteristics as follows:

| Studies by Characteristics | | |
|---|---|---|
| **By Study Design** | | |
| **Primary (main phase)** | | |
| **>100 subjects:** EUNO (Mercier, 2010); NO CLD (Ballard, 2006)[13,14]; Early iNO (Kinsella, 2006)[16]; IN NOVO (Field, 2005)[17]; Hascoet, 2005[18]; NICHD-PiNO (Van Meurs, 2005)[11]; Schreiber, 2003[8]; Franco-Belgian Collaborative NO Trial Group (Mercier, 1999)[19] | | |
| | **Meta-analyses** | JHU EPC (Donohue, 2011)[20]; MAPPiNO (Askie, 2011)[21]; Cochrane Review (Barrington & Finer, 2010)[22]; Hoehn, Krause & Bührer, 2006[23]; Hoehn, Krause & Bührer, 2000[24] |
| | **Multicenter**, **randomized**, **controlled** | EUNO (Mercier, 2010; NO CLD (Ballard, 2006); Early iNO (Kinsella, 2006); INNOVO (Field, 2005); Hascoet, 2005; NICHD-PiNO (Van Meurs, 2005); Franco-Belgian Collaborative NO Trial Group (Mercier, 1999) |
| | **Single site, randomized, controlled** | Schreiber, 2003 |
| | **Pilot** | Van Meurs, 2007 (NICHD-PiNO); Srisuparp, 2002[25] (Schreiber, 2003) |
| | **1-yr follow-up** | Watson, 2009[26] (Kinsella, 2006); Hibbs, 2008[27] (pulmonary morbidity - NO CLD); Hoo, 2009[28] (pulmonary morbidity-INNOVO) |
| | **2-yr follow-up (neurodevelopment)** | Walsh, 2010[29] (NO CLD); Keller, 2009b[30] (NO CLD); Kinsella, 2009[31] (Kinsella, 2006); Hintz, 2007[32] (NICHD-PiNO); Mestan, 2005[33] (Schreiber), Durrmeyer 2013[34] |
| | **4-5 yr follow-up** | Huddy, 2008[35] (cognitive/behavioral - INNOVO); Patrianakos-Hoobler, 2011[36] (school readiness - Schreiber) |
| | **7+ yr follow-up** | Hamon, 2009a[37] (clinical outcomes - Hascoet); Hamon, 2009b[38] (neurodevelopmental - Hascoet); Hascoet 2009[39], (pulmonary morbidity - Hascoet) |
| | **Subanalyses** | |

| Studies by Characteristics | | |
|---|---|---|
| **By Study Design** | | |
| | **Biomarkers of oxidative stress** | Ballard PL, 2008[40] (NO CLD); Hamon, 2005 (Hascoet); |
| | **Birth weight distribution of severe IVHIPVL and death** | Cooper, 2011[41] |
| | **BPD predictive value** | Ambalavanan, 2008[42] (NICHD-PiNO) |
| | **Cranial ultrasound interobserverreliability and accuracy** | Hintz, 2006[43] (NICHD-PiNO) |
| | **Evolution of brain injury** | White, 2010[44] (Schreiber); Kaplan, 2006[45] (NO CLD) |
| | **Hemodynamic indices** | Desandes, 2004[46] (Hascoet) |
| | **Inflammatory markers and mediators** | Truog, 2007[47] (NO CLD); Izhar, 2001[48] (Schreiber) |
| | **NO metabolites** | Posencheg, 2010[49] (NO CLD); Ballard P, 2010[50] (NO CLD) |
| | **NO response** | Keller, 2009a[51] (NO CLD); Ahluwalia, 2006[52] (INNOVO) |
| | **NO toxicity - related to dose** | Sokol, 2006[53] (NICHD-PiNO) |
| | **OI correlation with other variables** | Van Meurs, 2005 (NICHD-PiNO) |

EP 3 970 730 B1

EP 3 970 730 B1

| Studies by Characteristics | | |
|---|---|---|
| **By Study Design** | | |
| | PPROM, oligohydramnios, and suspected pulmonary hypoplasia | Chock, 2009[54] (NICHD-PiNO) |
| | **Pulmonary function** | Di Fiore, 2007[55] (NO CLD); Izhar, 2000[56] (Schreiber) |
| | **RDS subcohort** | Truffert, 2003[57] (Franco-Belgian) |
| | **Spontaneous intestinal perforation incidence** | Gordon, 2006[58] (NICHD-PiNO) |
| | **Surfactant composition and function (TAF)** | Ballard PL, 2007[59] (NO CLD) |
| | **<100 subjects**: Aikio 2012[60];Sharma 2011[61] ;O'Donnell 2010[62];Dewhurst 2010[63]; Hopper 2009[64];Kumar 2007[65]; Su & Chen, 2008[66]; Khemani, 2007[66]; Tanaka, 2007[68]; Dani, 2006[69,70]; Lindwall, 2005[71]; Uga 2004[72]; Athavale, 2004[73]; Aikio, 2003[74]; Clark, 2002[75]; Truog, 2002[76]; Du, 2002[77]; Banks, 1999[78]; Kinsella, 1999[12]; Cheung, 1998[79]; Laubscher, 1997[80]; Skimming, 1997[81]; Subhedar, 1997[82]; Van Meurs, 1997[9]; Peliowski, 1995[83], | |
| | **Randomized, controlled** | Su & Chen, 2008; Dani, 2006; Lindwall, 2005; Kinsella, 1999; Subhedar, 1997; Skimming, 1997 |
| | **Nonrandomized, prospective** | Aikio, 2012; Athavale, 2004; Aikio, 2003; Clark, 2002; Laubscher, 1997 |
| | **Historical cohort** | Tanaka, 2007 |
| | **Observational** | Peliowski, 1995 |
| | **Retrospective** | Sharma 2011, O'Donnell 2010; Kumar 2007; Khemani, 2007; Uga 2004; Du, 2002 |
| | **Pilot** | Lindwall, 2005; Banks, 1999 (Phase II, open-label, noncontrolled) |

(continued)

| Studies by Characteristics | | |
|---|---|---|
| **By Study Design** | | |
| | **Other/Not provided** | O'Donnell 2010; Dewhurst 2010; Truog, 2002; Van Meurs, 1997 |
| | **2+ yr follow - up** | Bennett, 2001[84] |
| | **Subanalysis** | Subhedar & Shaw, 1997[85] (changes in oxygenation, respiratory support, and pulmonary hemodynamics) |
| **By Combined Study Criteria** (Early noted) routine = entry at <3 days in intubated neon ates; Early rescue = entry at <3 days, based on oxygenation status; Later treatment = entry at >3 days, basis as | | |
| | **Early routine treatment** | EUNO (Mercier, 2010); Early iNO (Kinsella, 2006); Schreiber, 2003 |
| | **Early rescue treatment** | Aikio, 2012; INNOVO (Field, 2005); Hascoet, 2005; NICHD-PiNO (Van Meurs, 2005); Franco-Belgian Collaborative NO Trial Group (Mercier, 1999); Su & Chen, 2008; Dani, 2006; Aikio, 2003; Kinsella, 1999; Srisuparp, 2002 (Pilot for Schreiber, 2003) |
| | **Later treatment, BPD-based risk** | NO CLD (Ballard, 2006); Subhedar, 1997; Subhedar & Shaw, 1997 |
| | **Later treatment, established or evolving BPD** | Athavale, 2004; Clark, 2002; Truog, 2002; Banks, 1999 |

| By Combined Study Criteria | |
| --- | --- |
| (Early noted) routine = entry at <3 days in intubated neon ates; Early rescue = entry at <3 days, based on oxygenation status; Later treatment = entry at >3 days, basis as | |
| **Other** | Khemani, 2007; Tanaka, 2007; Lindwall, 2005; Uga, 2004 (pulmonary hypoplasia due to oligohydramnios); Du, 2002 (term and preterm - HRF or PPHN subjects); Yadav, 1999; Laubscher, 1997 (rescue in term and preterm); Van Meurs, 1997; Skimming, 1997 (preterms with RDS); Peliowski, 1995 |
| | |
| **Subjects had existing BPDICLD** | Khemani, 2007; Athavale, 2004; Clark, 2002; Truog, 2002 |

| By Birth Weight as Entry Criteria (g) | |
| --- | --- |
| **401 to 1,500** | Van Meurs, 2005 |
| **>500** | EUNO (Mercier, 2010) |
| **426 to 1,453** | Uga, 2004 |
| **448 to 1,790** | Banks, 1999 |
| **500 to 1,250** | NO CLD (Ballard, 2006); Kinsella, 2006 |
| **500 to 1,500** | Athavale, 2004 |
| **<750 to 1,500** | Hascoet, 2005 |
| **<750 to 2,000** | Schreiber, 2003 |
| **750 to 2,500** | Sharma 2011, Van Meurs, 1997 |
| **Mean of 1,000** | Yadav, 1999 |
| **>1,000** | Lindwall, 2005, |
| **<1,250** | Clark, 2002; Truog, 2002; Kaplan 2006 |

EP 3 970 730 B1

<div align="center">(continued)</div>

| By Birth Weight as Entry Criteria (g) | | |
|---|---|---|
| **<1,500** | | Aikio, 2012; O'Donnell, 2010; Aikio, 2003, Sokol 2006 |
| **Studies by Characteristics** | | |
| ≤1,500 | Su & Chen, 2008; Cheung, 1998 | |
| **By Endpoints** | | |
| **BPD/CLD** | EUNO (Mercier, 2010); Chock, 2008; NO CLD (Ballard, 2006); Kinsella, 2006; Sokol, 2006; INNOVO (Field, 2005); NICHD-PiNO (Van Meurs,2005); Van Meurs , 2005 (Pilot); Uga, 2004; Schreiber, 2003; Franco-Belgian Collaborative NO Trial Group (Mercier, 1999-was one of several secondary outcomes); Dani, 2006; Subhedar, Ryan, & Shaw, 1997; Subhedar & Shaw, 1997; Kinsella, 1999 (BPD one of several secondary outcomes); Bennett, 2001 BPDwas | |
| **Severe IVH/PVL or Death** | Cooper, 2011 | |
| **Oxygenation** | Aikio, 2012; Sharma 2011; Dewhurst, 2010; O'Donnell, 2010 (in former BPD subjects); Su & Chen, 2008 (secondary outcome inc. CLD); Khemani, 2007 (existing BPD subjects - PAH); Lindwall, 2005; Athavale, 2004; Aikio, 2003; Clark, 2002; Banks, 1999; Yadav, 1999; Cheung, 1998; Laubscher, 1997; Skimming 1997 ('RDS); Peliowski, 1995 | |

34

(continued)

| **By Endpoints** | | |
|---|---|---|
| | **Pulmonary HTN** | Sharma 2011; Kumar, 2007 (ID risk factors for PH); Tanaka, 2007 (neurodevelopmental outcomes for PPHN subjects); Truog, 2002 interaction with endothelin-1 in PPHN and CLD subjects); Du, 2002 (NO as rescue in term or preterm with HRF or PPHN) (NO |
| **By NO Dose (ppm)** | | |
| | **1-20** | Van Meurs, 1997 |
| | **5** | EUNO (Mercier, 2010); Kinsella, 2006; Kinsella, 1999 |
| | **5-10** | Hascoet, 2005; NICHD-PiNO (Van Meurs, 2005); Schreiber, 2003 |
| | **5-20** | Su & Chen, 2008; Du, 2002, Skimming 1997 |
| | **5-30** | Tanaka, 2007 |
| | **5-40** | INNOVO (Field, 2005) |
| | **10** | Dani, 2006; Athavale, 2004; Lindwall, 2005; Franco-Belgian Collaborative NO Trial Group (Mercier, 1999) |
| | **10-20** | Sharma 2011; Srisuparp, 2002; Svedenkrans 2015 |
| | **10-40** | Laubscher, 1997 |
| | **20** | Aikio, 2012; Ballard, 2006; Clark, 2002; Truog, 2002; Aikio, 2003; Banks, 1999; Subhedar, 1997; Peliowski, 1995 |
| **By NO Treatment Duration** | | |
| | **15 min** | Skimming, 1997; Svendenkrans 2015 |
| | **30 min** | Lindwall. 2005 |

(continued)

| By NO Treatment Duration | | |
|---|---|---|
| | **1 hr** | Yadav, 1999 |
| | **2 hrs** | Athavale, 2004 |
| | **12 hrs** | Tanaka, 2007 |
| | **3-4 days** | Aikio, 2012; Aikio, 2003; Banks, 1999 (initial study period: 72 hours, responders received prolonged therapy from 8 to 90 days) |
| | **Max: 137 hrs** | Peliowski, 1995 |
| | **Mean: 98.5±21.4 hrs** | Dani, 2006 |
| | **Mean: 4.9±2.3 days** | Su & Chen, 2008 |
| | **63 hrs (22-240 hrs)** | Sharma 2011 |
| | **7 days** | Schreiber, 2003; Srisupar, 2002; Truog, 2002; Van Meurs, 1997 |
| | **7-21 days** | EUNO (Mercier, 2010) |
| | **Max: 13 days** | Clark, 2002 |
| | **14 days** | Kinsella, 2006; NICHD-PiNO (Van Meurs, 2005) |
| | **18 days** | Laubscher, 1997 |
| **Studies by Characteristics** | | |
| | **21 days or extubation** | Kinsella, 2006 |
| | **Min: 24 days** | NO CLD (Ballard, 2006) |
| | **Unclear** | INNOVO (Field, 2005); Hascoet, 2005 |

| By Treatment Delivery Mode | | |
|---|---|---|
| | **Nasal CPAP** | EUNO (Mercier, 2010); Ballard, 2006; Lindwall, 2005 |
| | **Mechanical ventilation - type not specified** | Sharma 2011, Khemani, 2007; Schreiber, 2003 |
| | **Conventional ventilation only** | Su & Chen, 2008 |
| | **Conventional or high-frequency ventilation** | Ballard, 2006; All other studies |
| *Studies that included Neurological injury or Neurological outcomes as inclusion or part of Adverse event monitoring* | | |
| | **Incidence of PVL, IVH, or ROP as part of primary or secondary outcomes** | Mercier 2010 (EUNO); Kaplan 2006; Kinsella 2006; Hascoet 2005 (main phase); Van Meurs 2005 (main phase NICHD PiNO study); Van Meurs 2007; Van Meurs 2005; Hintz 2006; Sokol 2006; Srisuparp 2002; Schreiber 2003 (main phase); White 2010; Franco-Belgium Collaborative NO trial Group 1999 (main phase); Durrmeyer 2013 |
| | **Neurodevelopmental outcomes- Mental developmental index (MDI), Psychomotor Development Index (PDI), Palisano gross motor score, Bayley II SID, or blindness/ deafness** | Walsh 2010; Kinsella 2009; Hintz 2007; Mestan 2005; Durrmeyer 2013 |
| | **Neurodevelopmental impairment as primary or secondary outcome** | Keller 2009; Kinsella 2009; INNOVO (Field 2005); Hintz 2007; Durrmeyer 2013 |
| | **Neurological safety monitoring** | Ballard 2006 (main phase) |
| | **Cognitive** or **Behavioral development** | Huddy 2008; Patrianakos-Hoobler 2010[36] (school readiness); Hamon 2009; Durrmeyer 2013 |

**AHA/ATS PEDIATRIC PULMONARY HYPERTENSION GUIDELINE**

A guideline was developed to address the question of how to evaluate and treat pediatric pulmonary hypertension (PH), which is defined as a resting mean pulmonary artery pressure (mPAP) >25 mmHg after the first few months of life. Prior to this publication, there have only been studies in adult PH and related pulmonary vascular disease (PVD) studies describing treatment guidelines, very few studies describe the safety and efficacy of therapies for pediatrics and no treatment guidelines have been published. A working group of clinicians and clinician-scientists was established to create a guidelines document for the care of children with childhood disorders of PH, including PH related to cardiac, lung, and systemic diseases as well as idiopathic pulmonary artery hypertension (IPAH) that resulted from PVD. IPAH is considered a diagnosis of exclusion and is a pulmonary vasculopathy indicating the absence of diseases of the left side of the heart or valves, lung parenchyma, thromboembolism, or other miscellaneous causes. See the following table for the definitions of PH as described by these guidelines:

| Disorder | Definition |
|---|---|
| Pulmonary Hypertension (PH) | mPAP $\geq$ 25 mmHg in children >3 months of age at sea level |
| Pulmonary arterial hypertension (PAH) | mPAP $\geq$ 25 mmHg, PAWP <15 mmHg, PVRI >2 WU/m$^2$ |
| Idiopathic pulmonary arterial hypertension (IPAH) or isolated PAH | PAH with no underlying disease known to be associated with PAH, referred to as HPAH with positive family or genetic evaluation |
| PHVD | Broad category that includes forms of PAH but includes subjects with elevated TPG (mPAP-left atrial pressure or PAWP >6 mmHg) or high PVRI as observed in patients with cavopulmonary anastomoses without high mPAP |
| mPAP = mean pulmonary artery pressure; PVRI = pulmonary vascular resistance index; PAWP = pulmonary artery wedge pressure; HPAH = heritable pulmonary artery | |
| hypertension; PHVD = pulmonary hypertensive vascular disease | |

The specific recommendations that were stated in these guidelines are described in the table below. The levels of evidence was based on clinical trial data, magnitude of treatment effect, a risk versus benefit assessment, whether the given treatment was useful or effective, and an estimate of the certainty or precision of the treatment effect. Expert consensus and clinical experience were also used in the recommendations where adequate data was not available.

**NICHD EVALUATION**

Since the NIH Consensus Development Conference (described below), the use of NO in preterm infants still remains controversial. A study by the NICHD NRN (National Institute of Child Health and Human Development Neonatal Research Network) was conducted with two objectives: 1) to examine the range of NO use in extremely low gestational age (GA) infants within the NRN sites both before and after the NIH statement was published; and 2) to evaluate NO use with a propensity score modeling approach that looked at the outcomes of severe BPD or death that were treated at NRN centers. This approach adjusted for any systematic differences that were seen in babies that were treated vs not treated with NO before comparing the outcomes of severe BPD or death. This study used the Generic Database Registry (GDB) of the Eunice Kennedy Shriver NICHD NRN for subject selection. Details of the study are described in the table below.

**NIH CONSENSUS STATEMENT and META-ANALYSES**

Several meta-analyses/systematic reviews appear in the medical literature and are briefly described in the table below. Members of the National Institute of Health (NIH) Consensus Development Conference panel (Cole et al, 2011[86]) conducted a review of these data as well as input from study investigators and conference attendees. Details of the consensus statement and meta-analyses/systematic reviews are provided below in tabular form.

| Reference | Guideline/Studies Included | Results |
|---|---|---|
| **ATS/AHA GUIDELINE** | | |
| Abman et al 2015[87] | Members of the committee were selected by the American Heart Association (AHA) and the American Thoracic Society (ATS) to ensure that the members fit the criteria of broad pediatric pulmonary hypertension experience in both clinical and research areas. | The following recommendations were made in these guidelines with respect to the use of inhaled nitric oxide for pediatric pulmonary hypertension. The classification of the levels of evidence is described at the end of the recommendations<br><br>**Persistent PH of the Newborn**<br><br>"iNO can be beneficial for pretem infants with severe hypoxemia that is due primarily to PPHN physiology rather than parenchymal lung disease, particularly if associated with prolonged rupture of membranes and oligohydramnios (Class IIa; Level of Evidence B)."<br><br>**Congenital Diaphragmatic Hernia**<br><br>"iNO therapy can be used to improve oxygenation in infants with CDH and severe PH but should be used cautiously in subjects with suspected LV dysfunction (Class IIa; Level of Evidence B)."<br><br>**Bronchopulmonary Dysplasia**<br><br>"Treatment with iNO can be effective for infants with established BPD and symptomatic PH (Class IIa; Level of Evidence C)."<br><br>**Pediatric Heart Disease**<br><br>"In addition to conventional postoperative care, iNO and/or inhaled prostacyclins should be used as the initial therapy for pulmonary hypertensive crises and failure of the right side of the heart (Class I; Level of Evidence B)."<br><br>Class IIa; Level of Evidence B was classified as having the following:<br><br>1) Recommendation in favor of treatment or procedure being useful/effective<br>2) Some conflicting evidence from multiple randomized trials or meta-analysis<br><br>Class IIa; Level of Evidence C was classified as having the following:<br><br>1) Recommendations in favor of treatment or procedure being useful/effective<br>2) Only diverging expert opinion, case studies, or standard of care<br><br>Class I; Level of Evidence B was classified as having the following: |

| Reference | Guideline/Studies Included | Results |
|---|---|---|
| | | 1) Recommendations that procedure or treatment is useful/effective<br>2) Evidence from single randomized trial or nonrandomized studies |
| **NICHD EVALUATION** | | |
| Truog et al 2014[88] | The study was conducted to provide additional information on the use of NO in the extremely low gestational age (GA) infants across and within NRN sites, both before and after the NIH consensus development conference statement. There were two goals that were described for this study: 1) to examine the range of NO use in extremely low gestational age (GA) infants within the NRN sites both before and after the NIH statement was published; and 2) to evaluate NO use with a propensity score modeling approach that looked at the outcomes of severe BPD or death that were treated at NRN centers. | There were a total of 13 sites 4757 subjects born between 2008 and 2011 were available for analysis.<br><br>1) The changes in NO use were categorized by NO initiation day: <7 days (n=128); 7-28 days (n=140); or >28 days (n=47). Overall there was a decrease in NO use on after day 7 among all 13 sites from 4.6% before NIH conference (Epoch 1) to after the publication of the conference to 1.6% (Epoch 2), (*P*<0.0001). In 2011 only 13 subjects between day 7 and 28, and 5 subjects after 28 days started NO.<br>2) The propensity score modeling was conducted in subjects that initiated NO at >7 days (these subjects were associated with lower gestational ages and with significant respiratory support by 7 days of age) or who never started NO. With the propensity adjusted analysis approach, the use of NO was associated with an increased risk of severe BPD or death (OR = 2.24, 95% CI 1.23 to 4.07).<br><br>The overall conclusion made by the authors stated that these results demonstrated a wide variability in the use of NO for extremely preterm infants during 2008 through 2010, however there was a dramatic drop in NO use in 2011. In addition the use of NO in extremely preterm infants was not associated with an improvement in death or incidence of severe BPD in infants <29 weeks GA, especially in those infants that were already severely ill at day 7 of life. |
| **COMMITTEE ON FETUS AND NEWBORN; AMERICAN ACADEMY OF PEDIATRICS CLINICAL REPORT\*** | | |
| Kumar P and Committee on Fetus and Newborn 2013[89] | A guidance document published by the American Academy of Pediatrics summarizes the existing evidence regarding inhaled nitric oxide in preterm infants and provides a guidance for use in this population | The panel conducted a formal literature evaluation and graded the quality of evidence for specific areas.<br><br>*Evidence quality A; Grade of recommendation, strong*: based on the results of randomized controlled trials, traditional meta-analyses, and individualized patient data, the panel stated that neither rescue nor routine use of NO improves survival in preterm infants with respiratory failure. In addition they stated that the evidence does not support treating preterm infants who have respiratory failure with NO for preventing or improving bronchopulmonary dysplasia (BPD), severe intraventricular hemorrhage, or other neonatal morbidities.<br><br>*Evidence quality, A; no recommendation provided*: For the incidence of cognitive impairment, cerebral palsy, or neurodevelopmental impairment in preterm infants |

| Reference | Guideline/Studies Included | Results |
|---|---|---|
| | | treated with NO, this was found to be similar to that of control |
| | | Neither an evaluation of the quality of the evidence or a recommendation was made with regards to high dose treatment of NO (20 ppm) beginning in the second postnatal week, as providing a reduction in the rate of BPD, additional trials to confirm this was recommended. |
| | | A meta-analysis of individual-patient data including gestational age, race, oxygenation index, postnatal age at enrollment, evidence of pulmonary hypertension, and mode of ventilation found no statistically significant differences in NO effect. The effects of NO on pulmonary outcomes of preterm infants in early childhood were found in only limited data with inconsistent results. |
| | | The safety of NO use in this population suggested that NO was safe and does not increase lung inflammation or oxidative stress.[47,90,40,49] |
| | | *A response to the AAP clinical report was made by several neonatologists identifying areas that they disagreed with from the AAP clinical report.[91] The response consisted mainly of two main points that the authors wanted to discuss: The authors asked the Committee to revise its statement on the use of NO for premature infants by removing the "no NO" recommendations for premature infants with pulmonary hypertension such that neonatologists use their clinical judgment to make their own decisions in this critical area. In addition, they requested that the Committee emphasize the importance of additional studies to be conducted as to whether NO may benefit specific populations of premature infants such as African-Americans or others, instead of implying that research regarding NO in this population is complete.* |

### NIH CONSENSUS STATEMENT

| Reference | Guideline/Studies Included | Results |
|---|---|---|
| Cole et al, 2011[86] | The consensus statement was derived from panel discussions, data from the Johns Hopkins University Evidence-based Practice Center (JHU EPC) systematic review; presentations by study investigators during the 2-day public session; and input from the conference attendees.<br><br>Six questions were posed considering the effect and safety of NO regarding: 1) survival rates and/or the incidence or severity of BPD; 2) short-term risks; 3) long-term pulmonary and/or neurodevelopmental outcomes; 4) variance of BPD and/or death as well as neurodevelopmental | 1) Overall, current evidence did not demonstrate that NO therapy increases survival rates in premature neonates requiring respiratory support and that, in those subjects surviving at 36 weeks postmenstrual age, evidence did not support the hypothesis that NO reduces the incidence of BPD.<br>2) No evidence was found that NO either decreases or increases the risk of late-onset sepsis, patent ductus arteriosus, severe retinopathy of prematurity (ROP), and pulmonary complications such as air leaks or pulmonary hemorrhage.<br>3) There was evidence in 1 study of an advantage in long-term pulmonary outcome, but the overall evidence does not justify widespread use of NO in the prevention of long-term pulmonary disease. Variance in measures and timing of assessments used to evaluate neurodevelopmental status made analysis difficult. No significant between-group differences were found in motor delay, visual, or hearing impairment. In two studies that found an association between NO therapy and the incidence of cerebral |

| Reference | Guideline/Studies Included | Results |
|---|---|---|
| | impairment across subpopulations; 5) impact of timing of dose initiation, mode of delivery, dose, duration, and concurrent therapies; and 6) what future studies are needed to evaluate the risks, benefits, and alternatives to NO therapies.<br><br>The final statement is available at:<br><br>http://consensus.nih.gov/2010/inofinalstatement.htm | palsy, the results contradicted each other. A small number of the individual studies and none of the meta-analyses found significant associations between NO therapy and any type of neurodevelopmental outcome up to the age of 5 years. It was suggested that future studies use newer assessment tools such as neuroimaging and standardized behavioral testing at age-appropriate time points.<br>4) The JHU EPC yielded insufficient evidence of improved neurodevelopmental outcomes in any subpopulation. The consensus panel recommended special caution in studies of early rescue use in premature subjects weighing <1,000 g and who are <34 weeks' gestation. The panel also noted that there are rare clinical situations that have not been adequately studied in premature neonates (e.g. pulmonary hypertension, hypoplasia) and suggest that clinicians discuss the current understood risk/benefit profile and remaining uncertainties with the families.<br>5) There is insufficient evidence that variations in timing of initiation, dose, duration, mode of ventilation, mode of administration, or concurrent treatment are harmful in terms of BPD, death, or neurodevelopmental outcome. The panel noted that although the evidence suggests some treatment regimens may provide greater benefits, further study is warranted to confirm those findings.<br>6) Future research is needed to evaluate: effect on lung development and function; to confirm the potential benefit of later timing, higher dose, and longer duration; pharmacology and toxicology specific to the premature neonate; effect of treatment-related variables on subpopulations by randomly assigning them separate as opposed to post hoc analyses; alternative therapies; long-term safety and efficacy up to a minimum of school-age; and use of newer assessment tools with potentially better predictive accuracy. |

### META-ANALYSES

| Reference | Guideline/Studies Included | Results |
|---|---|---|
| Askie et al, 2011<br><br>(MAPPiNO) | 12 studies were included. Authors stated that use of individual patient data (IPD) compared with aggregate data enabled a more robust assessment of the relationship between patient-level and trial-level characteristics and treatment effect.<br><br>Total n = 3,298<br><br>Primary studies included:<br>EUNO-Mercier, 2010; Van Meurs, 2007; Ballard, 2006; Kinsella, 2006; Dani, 2006; Hascoet, 2005; INNOVO-Field, 2005; Van Meurs, 2005; Schreiber, 2003; Srisuparp, 2002; Kinsella, 1999; Subhedar, 1997; Tanaka, 2007. | Included ventilated, preterm neonates <37 weeks gestational age that were randomized to either NO or placebo. BPD or death composite was not significantly different between the groups (RR: 0.96, 95% CI 0.92 to 1.01; P=0.11). Trending toward significance, 25% of the NO subjects vs 23% of the controls had severe neurological events on imaging (RR: 1.12, 95% CI 0.98 to 1.28, P=0.09). No significant differences in NO effect by any patient-level characteristics tested were noted. Evidence of improved outcome (interaction test P<0.001) was noted in studies where initial NO dose was >5 ppm vs ≤5 ppm, but this effect was not observed using other dosing or exposure levels. The authors concluded that routine NO use in preterm neonates with respiratory failure could not be recommended and that a larger starting dose may be associated with improved outcome. Further studies were suggested to confirm these findings as included trials varied in design. |
| Donohue et al, | 14 RCTs were analyzed for NICU mortality, BPD, | Systematic review done by the Johns Hopkins University Evidence-based Practice Center (JHU |

| Reference | Guideline/Studies Included | Results |
|---|---|---|
| 2011[20]<br><br>(JHU EPC) | and short-term risks. 7 follow-up studies and 1 observational study were considered for other research questions including post-discharge mortality and longer-term outcomes such as neurodevelopmental impairment.<br><br>Primary (follow-up) studies included: EUNO-Mercier, 2010; Su & Chen 2008 Van Meurs, 2007; Ballard, 2006 (Hibbs, 2007; Walsh, 2010); Kinsella, 2006 (Watson, 2009); Dani, 2006; Hascoet, 2005 (Hamon, 2005); INNOVO-Field, 2005 (Huddy, 2008); Van Meurs, 2005 (Hintz, 2007); Schreiber, 2003 (Mestan, 2005); Srisuparp, 2002; Kinsella, 1999; Franco-Belgian Collaborative-Mercier, 1999; Subhedar, 1997; Tanaka, 2007.<br><br>Total n = 3,461 | EPC). The full evidence report is available at: http://www.ahrq.gov/downloads/pub/evidence/pdf/inoinfants/inoinfants.pdf)<br><br>NO dose varied from 5 to 40 ppm and subjects ≤34 weeks were enrolled from birth to 27 days. Of the 14 RCT and their 5 follow-up studies, 6 were judged to have low risk of bias. 3 of 14 RCT had unclear risk of bias, and 5 of 14 RCT had high risk of bias. Tanaka's observational study had a low risk of bias. Each short-term and long-term outcome was considered as having low-to-moderate Grade of evidence. Mortality data varied in the time of measurement while in the NICU (e.g. 7 days, 28 days, 36 weeks, or 1-year corrected age) but no significant between-group differences were noted in any of the 14 RCT (RR: 0.93, 95% CI 0.82 to 1.15). A small difference favoring NO (7% reduction) in the risk of the composite outcome of BPD or death at 36 weeks was noted (RR: 0.93, 95% CI 0.87 to 0.99). The risk of BPD alone at 36 weeks did not differ between NO and controls (RR: 0.93, 95% CI 0.86 to 1.003). No significant between-group differences were noted in the incidence of neurodevelopmental impairment (RR: 0.91, 95% CI 0.77 to 1.21), cerebral palsy (RR: 1.36, 95% CI 0.88 to 2.10), or cognitive impairment (RR: 0.72, 95% CI 0.35 to 1.45). The authors concluded that the data provided no evidence supporting NO therapy in premature neonates with respiratory failure other than within the context of rigorously conducted randomized clinical studies. |
| Barrington & Finer, 2010[22] (Cochrane Review) | 14 studies were included and grouped post hoc as noted below:<br><br>**Early rescue (total n=1,006)**: Su & Chen, 2007; Van Meurs, 2007; Dani, 2006; Hascoet, 2005; INNOVO (Field), 2005; Van Meurs, 2005; Srisuparp, 2002; Franco-Belgian Collaborative (Mercier, 1999); and Kinsella, 1999.<br><br>**Early routine (total n=1,800):** EUNO (Mercier), 2010; Kinsella, 2006; and Schreiber, 2003.<br><br>**Later rescue (total n=624):** Ballard, 2006; and Subhedar, 1997. | No overall analyses of the 14 trials were performed due to heterogeneity in study protocols. No significant effect was found on mortality or BPD in early rescue or later use groups, though the effect approached significance in routine use (typical RR 0.93 [95% CI 0.86 to 1.01). No clear effect was demonstrated on the frequency or severity of any Grade of IVH, though a nonsignificant 20% increase in severe IVH was observed in the early rescue group. No significant effect on the incidence of neurodevelopmental impairment was noted. The authors concluded that early rescue use did not appear to be effective, though later use to prevent BPD may be. Early routine use did not affect serious brain injury or improve the chance of survival without BPD. |
| Hoehn et al, 2006[23] | 4 published studies (Schreiber, 2003; Franco-Belgian Collaborative [Mercier], 1999; Kinsella, 1999; Subhedar, 1997) and 1 preliminary report published in abstract form (Van Meurs,2004)<br><br>Total n = 808 | Treatment failure defined as the combined endpoint of mortality or BPD and data on ICH were not available for the Van Meurs study (n=415). The risk of treatment failure was significantly reduced in NO group (126 of 208) vs controls (139 of 185) ($P$=0.0025; RR 0.81 [95% CI 0.70 to 0.93]; number needed to treat = 7). The incidence of BPD was also reduced significantly in the NO group (188 of 415) vs controls (215 of 393) ($P$=0.0092; RR 0.83 [95% CI 0.72 to 0.95]); number needed to treat = 11. Of the 415 NO subjects, 169 died compared with 155 of 393 in the control group ($P$=0.72; RR 1.03 [95% CI 0.87 to 1.22]). Similarly, no significant between-group differences were observed in the incidence of major ICH. The authors concluded that although it appeared that NO may decrease BPD and treatment failure, results must be interpreted with caution as the largest study (Van Meurs) was terminated early due to an increase in severe ICH. Further studies were recommended to evaluate if reduction in BPD |

| Reference | Guideline/Studies Included | Results |
|---|---|---|
| | | would be associated with changes in neurodevelopmental outcomes. |
| Hoehn et al, 2000[24] | 3 published studies (Franco-Belgian Collaborative [Mercier], 1999; Kinsella, 1999; and Subhedar, 1997)<br><br>Total n = 210 | Though oxygenation did greatly improve within 1 hour of NO therapy, this did not translate into reduction in mortality (the primary outcomes of these trials). No significant difference in number of deaths occurred between the NO subjects (44 of 111) and the control subjects (40 of 99) ($P$=0.91) with the odds ratio (OR) in favor of NO at 0.97, 95% CI 0.54 to 1.75. The incidence of death or BPD was also nonsignificantly different between the NO (32 of 111) and the control subjects (34 of 99) ($P$=0.39; OR 0.77, [95% CI 0.41 to 1.45]). Though nonsignificant, the rate of IVH trends toward a slight increase in the NO (35 of 111) vs the control subjects (25 of 99) ($P$=0.33; OR 1.37; 95% CI 0.69 to 2.74). The authors state the future studies are required to determine whether or not this is correlated with NO or comorbidity of this population. |

NIH = National Institute of Health; NO= inhaled nitric oxide; n = number of subjects; BPD = bronchopulmonary dysplasia; RCT = randomized controlled trial; ppm = parts per million; RR = relative risk; CI = confidence interval; IVH = intraventricular hemorrhage; ICH = intracranial hemorrhage; OR = odds ratio

## INDIVIDUAL CLINICAL STUDIES

Primary clinical studies with ≥100 neonatal subjects are presented below in both tabular and narrative form. The primary studies are ordered in descending chronology, then alphabetically if two or more studies were published in the same year. Narratives are only provided for the main phase portion of multi-phase trials. Following the narrative section, data from all smaller trials are presented in tabular format alone.

| Study | Description | Gestational Age | Birth Weight | Dose | Primary & Secondary Outcomes | Results | Summary |
|---|---|---|---|---|---|---|---|
| *EUNO (MERCIER, 2010)[2,15]* Main Phase | Randomized, multicenter, double-blinded, placebo-controlled, of inborn, preterm neonates  Early routine treatment study  n=800 | From 24+0 to 28+6 weeks  Mean NO, 26.4±1.3 weeks  Mean placebo, 26.6±1.3 weeks  Entered within 12-24 hrs of birth | >500 g  Mean NO: 851±207 g  Mean placebo: 864±192 g | 5 ppm x 7-21 days initiated via nasal CPAP, CMV, or HFOV within 2-26 hours after study entry | P: Survival at 36 weeks GA without BPD (stratified by age and birth weight)  S: Survival without brain injury; days on mechanical ventilation; duration of hospitalization, incidence of ICH, treated ductus arteriosus, intestinal perforations/sepsis, pneumothorax, or pulmonary hemorrhage | Rate of success (Alive without BPD) was nonsignificantly (*P*=0.73) lower in the NO group (65%) vs the placebo group (66%). Success rate was highest in both groups in the 26-28 GA wk stratification vs the 24-25 GA wk group. [ By birth weight, the highest rate of success was in the largest neonates (1,000-1,250 g) compared to the smaller babies (500-999 g).]  No significant differences were noted in survival without brain injury, duration of ventilation or hospitalization, or | After a thorough review of the data and consultation with multiple clinical experts, the sponsor's assessment is that the data from this single study does not represent a safety signal and that the observed differences in death rates do not have any apparent likelihood of association with NO therapy. Investigators concluded that 5 ppm NO, started with the 1st 24 hours after birth and continued for a median of 3 weeks did not improve survival without BPD in very preterm neonates with mild to moderate respiratory failure. |

| Study | Description | Gestational Age | Birth Weight | Dose | Primary & Secondary Outcomes | Results | Summary |
|---|---|---|---|---|---|---|---|
| | | | | | | incidence of adverse events though there the percentage of serious ICH was higher with NO (29%) vs controls (23%), as was PDA (NO,15%; controls,11%), and intestinal perforation (NO, 4%; controls, 3%). The NO group had a lower incidence of pulmonary hemorrhage (3% vs 4% in controls). | |
| **Durrmeyer X, 2013[34]** (2-yr follow-up of EUNO study) | 2 yr neurodevelopmental assessment.  At 1 yr, n=661 (NO=322, Placebo=339) ; at 2 yr, n=630 (NO=306, Placebo=324). | 1 yr and then at 2 yr from the original age of 24-28 + 6 weeks.  1 yr and 2yr: Mean NO 26.5 (1.3); Mean Placebo 26.6 (1.3 | 1 yr mean NO: 860.2(213.8); 1 yr mean placebo: 878(193.0); nonsignificant  2 yr mean NO: 858.0(211.2); 2 yr mean placebo: 883.9(188.3); *P*=(0.0 | 5 ppm x 7-21 days initiated via nasal CPAP, CMV, or HFOV within 2-26 hours after study entry | P:Survival at 1 and 2 years  Neurologic: the Gross Motor Function Classification System (GMFCS) and the Manual Ability Classification System for identifying infants with cerebral palsy and function. Cognitive scale evaluated by Bayley Scales of Infant and Toddler Development, third edition (Bayley-III).  Developmental: disabilities classified as severe, moderate, or minimal/no | Total 11 infants died between 36 weeks' and age 2 years (corrected for prematurity): 4 of 395 (1.0%) in the NO ; 7 of 397 (1.8%) in placebo. Of these, 9 deaths were before hospital discharge. Subsequently, 1 died of pneumonia (NO arm) and 1 died of sudden infant death syndrome (placebo arm).  No significant differences were noted in mean cognitive composite scores, frequency of cerebral palsy, seizure disorder, hearing communication, or vision deficits in the 2 groups.  No significant differences with respect to proportion of ROP between groups. | The authors concluded that NO therapy started within the first 24 hours after birth with an initial dose of 5 ppm resulted in no effect on growth, neurologic development, or respiratory outcome up to 2 years of age. An additional neurologic assessment is planned at 7 years. |

| Study | | Description | Gestational Age | Birth Weight | Dose | Primary & Secondary Outcomes | Results | Summary |
|---|---|---|---|---|---|---|---|---|
| | | | | | 4). | disability.<br><br>Retrospective assessment of retinopathy of prematurity (ROP).<br><br>S: Survival without neurodevelopmental disability, hospitalization, growth and other outcomes, use of concomitant medications and therapies. | Majority of subjects in either group had minimal or no neurodisabilities. Similar frequencies of moderate and severe neurodisabilities were seen between both groups.<br><br>There were no significant differences between groups in survival without neurodevelopmental disability or hospitalization. There was a statistically smaller mean head circumference at 1 year ($P = 0.03$) in the NO group; a smaller number of subjects taking respiratory medications considered relievers during the first year, 28 of 322 (8.7%) in the NO group vs 37 of 339 (10.9%) in placebo group, $P = 0.38$ ; and 34 of 322 (10.6%) in the NO group vs 45 of 339 (13.3%) in the placebo group, $P = 0.28$ taking medications classified as preventers. | |
| | Planned 7-yr follow-up | 7-yr neurodevelopmental assessment + pulmonary outcomes | - | - | - | - | - | - |

| Study | | Description | Gestational Age | Birth Weight | Dose | Primary & Secondary Outcomes | Results | Summary |
|---|---|---|---|---|---|---|---|---|
| NO CLD (BALLARD RA, 2006)[13,14]<br><br>Main Phase | | Multicenter, blinded, randomized, stratified according to birth weight. (500-799g and 800-1250g)<br><br>Used a median respiratory severity score.(not directly convertible to OI because it did not use PaO$_2$) Mean was 3.5 for both groups = approx. OI of 7<br><br>Later treatment study, based on BPD risk[92]<br><br>n=582 | <32 weeks GA<br><br>Mean GA: 26 wks<br><br>Entry at 7-21 days old<br><br>Mean age at entry: 16 days | 500-1250 g<br><br>Mean BW: NO 766g, placebo 759g | 20 ppm X 24 days. 20 ppm for 48-96 hrs, then down to 10ppm, 5 ppm and 2 ppm at weekly intervals. Conventional and high-frequency ventilation were used as was nasal CPAP<br><br>Minimum treatment duration was 24 days. | P: Survival without BPD at 36 WGA<br><br>S: Duration of oxygen therapy and days in hospital | The rate of survival without BPD (unable to maintain sat >88% on RA) was significantly higher in the NO group. (43.9% vs 36.8%). This was true in both weight strata. Neonates treated with NO were also discharged sooner and received supplemental oxygen for a shorter time | Prolonged NO that is initiated between 7 and 21 days of age in preterm infants undergoing mechanical ventilation significantly improved survival without BPD without short term adverse effects.<br><br>Major drop in death/BPD<br><br>In a post hoc analysis, it was observed that NO significantly improved survival without BPD for infants enrolled at 7-14 days compared to placebo but not for infants enrolled at 15-21 days. |

| Study | Description | Gestational Age | Birth Weight | Dose | Primary & Secondary Outcomes | Results | Summary |
|---|---|---|---|---|---|---|---|
| Banks, 1999[78]<br><br>Pilot | A phase II, open-label, noncontrolled pilot study of subjects with severe, ventilator-dependent BPD to assess safety and obtain preliminary data on possible efficacy and dosing regimen for future trial (Ballard, 2006).<br><br>n=16 | 23-29 weeks GA Median GA: 25.5 weeks<br><br>Age at entry: 1-7 months Median age at entry: 2.5 months | 448-1,790 g<br><br>Median BW: 787 g | 20 ppm x 72 hours. Responders (n=11) received prolonged therapy from 8 to 90 days | P: Change from baseline in $PaO_2$ at 1 hour and change from baseline in $FiO_2$ at 72 hours.<br><br>S: Change in $PaCO_2$ at 1 hour and change in mean airway pressure and ventilator efficiency index at 72 hours<br><br>(Effects of prolonged use in responders was described but no statistical testing done) | 11 of 16 subjects had significant increase in $PaO_2$ at 1 hour (median change: 24 mm Hg; range: −15 to 59 mm Hg; $P<0.01$). At 72 hours, 11 subjects had ≥15% decrease in $FiO_2$ and 7 of these subjects had reductions of ≥35% ($P<0.01$). No significant change in mean airway pressure at 72 hours (median change: −1 cm $H_2O$; range: −10 to +4 cm $H_2O$). At 72 hours, three subjects who required high-frequency oscillator ventilation + 100% oxygen at entry tolerated a ≥5 cm $H_2O$ reduction in mean airway pressure. No significant improvement in ventilator efficiency index at 72 hours (median ratio: 1.0; range: 0.7 to 2.0).<br><br>Prolonged: 11 of 16 subjects maintained improved oxygenation after 6 days with a median $FiO_2$ decrease of 38% (range: 17-60%). 10 of 11 subjects continued their improvement throughout NO therapy with median decrease in $FiO_2$ of 52% (range: 0-72%). At 6 days, some subjects were able to tolerate lower mean airway pressures (median change: −2 cm H2O; range: +1 to −11). By therapy end, median change in mean airway pressure: −5 cm H2O (range: +1 to −16). No improvement in ventilator efficiency ratio. | Met-Hb levels remained at <3% throughout NO therapy. No subject developed IVH or showed extension of existing IVH and no subject had clinical evidence of bleeding during therapy. Plasma 3-nitrotyrosine measured in 9 of 16 subjects and was detected or elevated in those with severe BPD at entry, prior to NO therapy (median: 0.33 ng/mg of protein; range: 0.16 to 1.22). Levels increased after 72 hours of NO in those subjects with decreased Fio2, while prolonged NO therapy produced significant reductions (mean: 55 ± 18%; $P<0.01$)<br><br>Long-term outcome (3 to 24 months):<br>4 of 11 with prolonged therapy were weaned; two remained ventilated with oxygen <30%; one received a lung transplant; and 4 of 11 died 11 days to 5 months after enrollment (Mortality rate: 36%). 3 of 5 nonresponders died (60%) and none were weaned. |

| Study | Description | Gestational Age | Birth Weight | Dose | Primary & Secondary Outcomes | Results | Summary |
|---|---|---|---|---|---|---|---|
| Kaplan, 2006[45]<br><br>Subanalysis | A nested, matched case-control was conducted to evaluate the possible contribution of NO to evolution of brain injury. At the time of this analysis, enrollment was 67% completed. Each subject with change from baseline in their head ultrasound (HUSs) was matched by birth weight group and study site to 2 control subjects with stable HUSs<br><br>n=393 available cases | N/A | <1,250 g. | N/A | Change in HUS from baseline either from normal HUS or worsening severity of existing IVH, hydrocephalus, porencephalic cyst, PVL. Associations between perinatal and neonatal risk factors, including NO administration were evaluated with univariable and multivariable analyses. | 53% of the cases received NO as compared to 56% of the control subjects. 19 subjects (4.8%) had evolution of HUS results. No variable was found to be significantly associated with evolution, though the predictive value maternal tocolytic use approached significance (OR 0.21, 95% CI 0.04-1.03). Use of NO found not to be significantly (OR 0.90, 95% CI 0.30-2.70) associated with evolution of brain injury. | NO use did not increase the odds of brain lesion evolution in this cohort of ELBW subjects. |
| Ballard PL, 2007[90]<br><br>Subanalysis | Concurrent subpopulation study of surfactant function and composition via tracheal aspirate (TAF).<br><br>n=99 | Mean: 25.5 weeks | Range: 725-755 g | N/A | Measurement of surfactant function and composition | After 4 days of NO therapy, minimum surface tension decreased vs increases in the placebo group ($P=0.02$). No significant differences were noted at any other time point and no significant differences noted in composition. | Due to the results at 4 days, the relationship with main phase primary outcome (survival) was evaluated. In the NO group with ↓ surface tension, survival without BPD was 60% compared to the 25% in the placebo group that had increased values. Authors commented that the study was not sufficiently powered to fully assess these Day 4 findings, but that the overall safety of NO was supported. |

| Study | Description | Gestational Age | Birth Weight | Dose | Primary & Secondary Outcomes | Results | Summary |
|---|---|---|---|---|---|---|---|
| Di Fiore, 2007[55] Subanalysis | Concurrent subcohort, blinded study of pulmonary function performed at 6 of the 21 multicenter sites to assess airway compliance and resistance. | NO: 25.5±1.2 weeks GA; mean age at entry: 15.2±3.7 days Placebo: 25.5±1.1 weeks GA; mean age at entry: 15.6±4.0 days | NO: 752±178 g Placebo: 748±143 g | 20 ppm | Expiratory resistance and dynamic compliance (normalized by weight) | Baseline resistance and compliance were similar between the groups. There was no significant effect of NO on either variable at 1 hr, 1 week, or 2 weeks of study gas. | When administered NO at 20 ppm, no short- or medium-term benefits on respiratory mechanics were seen in these mechanically ventilated preterm neonates contradicting data from earlier studies in baboon and ovine models of BPD. The authors speculate that NO may improve respiratory outcomes by augmenting lung maturation and decreasing lung inflammation negating the need for effects on respiratory mechanics. |
| Truog, 2007[47] Subanalysis | Concurrent subpopulation study of inflammatory markers and mediators in tracheal aspirate. n=99 | Age at lavage: Mean NO: 15 days Mean placebo: 16 days | N/A | N/A | Objectives were to 1) characterize relationship between inflammatory markers/mediators and disease severity; 2) identify whether NO response can be predicted from baseline TAF; and to assess potential mechanisms of NO in lungs | No correlation between disease severity and markers/mediators noted. NO use did not alter markers or total cell counts significantly vs placebo at any point. No differences were noted between early (7-14 days) and later (15-21) study entry and markers/mediators. | Post hoc analysis of the 47 NO subjects demonstrated that 21 survived without BPD at 36 weeks while 26 had unfavorable outcomes at ≥36 weeks. Subjects with positive outcomes had a trend toward higher 8-epi-PGF$_{2\alpha}$ ($P$=0.017) vs those with unfavorable outcomes, but this difference disappeared by Day 4. Authors concluded there was no evidence to support NO-associated increases in lung injury or inflammation. |

| Study | Description | Gestational Age | Birth Weight | Dose | Primary & Secondary Outcomes | Results | Summary |
|---|---|---|---|---|---|---|---|
| Ballard PL, 2008[40] Subanalysis | Concurrent subpopulation study of plasma biomarkers of oxidative stress: relationship to lung disease and NO therapy n=100 | N/A | N/A | N/A | Relationship between NO therapy and lung disease via measurement of plasma biomarkers of oxidative stress | At baseline, total protein correlated inversely (r=-0.25, $P$=0.03) with respiratory severity score (RSS), while carbonyl content was positively correlated with RSS (r=0.49, $P$<0.001). No significant correlation was noted between plasma 3-nitrotyrosine and RSS or carbonyl levels and values were similar between positive and negative outcomes groups. No biomarker significantly correlated with gestational age. Subjects without BPD had 30% lower carbonyl levels ($P$=0.03) and lower RSS ($P$=0.001) at baseline than those with adverse outcomes. No significant differences were noted in total plasma protein, carbonylation, or 3-nitrotyrosine at any time. | Authors concluded that NO therapy does not alter plasma biomarkers of oxidative stress, supporting the safety of NO. Further studies were suggested as study limitations included 4-yr storage period of samples that potentially could have impacted assays and insufficient study power to detect small differences that might have potential physiologic significance. |
| Hibbs, 2008[27] 1-yr follow-up | 1-year follow-up at 12 ± 3 months of age, adjusted for prematurity. Original treatment allocation randomized by site to account for regional differences in care post-discharge. Measured markers of pulmonary | Mean NO: 25.8±1.4 weeks Mean placebo: 25.7±1.5 weeks | Mean NO: 769±163 g Mean placebo: 762±150 g | N/A | Pulmonary outcomes: (bronchodilator use, chest "wheezing or whistling", steroid use, diuretic use, oxygen use, hospitalizations) Bronchodilator use relationship to race, gender, birth weight, and age at study entry | Significantly less bronchodilator, steroid (inhaled and systemic), and oxygen use in NO group vs placebo. No significant differences were noted between the groups in chest "wheezing or whistling" or hospitalizations. Significantly fewer subjects (white and non-white; male and female) | Significantly less pulmonary comorbidities were noted in the NO group. The authors suggested that clinicians considering adoption of the Ballard protocol should review this study's findings in conjunction with neurodevelopmental outcomes to be reported (see Walsh, 2010). |

| Study | | Description | Gestational Age | Birth Weight | Dose | Primary & Secondary Outcomes | Results | Summary |
|---|---|---|---|---|---|---|---|---|
| | | morbidity (chest wheezing; bronchodilator, steroid, diuretic, or oxygen use; re-hospitalization). n=455 | | | | | subjects in the NO group used bronchodilators. The same pattern was seen for all birth weights and ages at study entry. | |
| Keller, 2009a[51] 2-yr subanalysis | | Subanalysis of differential responses to NO at 36 weeks vs 40 weeks based on age at entry, race, gender, and other parameters. | N/A | N/A | N/A | Overall and differential effects of NO therapy at 40 PMA related to: age, various demographic variables, and RSS score at baseline as well as extubation prior to study entry. Relationship between bronchodilator use at 1-yr and NO response at original 36-wk endpoint vs 40-wk PMA also assessed. | No significant between-group differences were noted for any variables tested ($P \geq 0.12$). NO improved outcomes at 40 weeks for all subgroups and positive outcome at 40 weeks more strongly predicted reduced bronchodilator use during first year (RR 1.57; 1.27 to 1.94) than did survival without BPD (RR 1.26; 1.06 to 1.51). | Authors concluded that all subgroups may respond to NO by 40 weeks even if no response is apparent at 36 weeks PMA, showing potential for improved respiratory status during year one. |
| Keller, 2009b[30] 2-yr follow-up | | 24-mth follow-up to evaluate associate neonate characteristics with neurodevelopmental impairment (NDI) at 2 years of age | N/A | N/A | N/A | Overall and differential effects of NO therapy at 2 years of age on NDI related to birth weight, age at study entry, race, gender, dexamethasone exposure>3 days, BPD at 36 or 40 weeks, neurological injury, and maternal education level. | Multiple perinatal predictors were found to be significantly associated with NDI: female gender ($P=0.02$); steroid exposure >3 days ($P=0.007$); BPD at 36 wks ($P<0.0005$); BPD at 40 wks ($P<0.0005$); and neurologic injury ($P=0.0001$). No variable had a significant modification of treatment effect | Characteristics commonly associated with NDI showed significant relationships as expected, but none was significantly modified by NO therapy. |

| Study | | Description | Gestational Age | Birth Weight | Dose | Primary & Secondary Outcomes | Results | Summary |
|---|---|---|---|---|---|---|---|---|
| Posencheg, 2010[49] Subanalysis | | Concurrent subpopulation study of relationship between NO metabolites in tracheal aspirate fluid (TAF) and plasma at different doses n=102 | N/A | N/A | 2-20 ppm | Dose-related effects of S-nitroso, N-nitroso, nitrite, nitrate, and metal-NO complexes at 2, 5, 10, and 20 ppm | NO metabolites were increased by 1.7-2.3 fold at 10 and 20 ppm vs placebo in TAF and by 1.6-2.3 fold in plasma at the same doses. | Post hoc analysis showed improved clinical outcomes in subjects with lower metabolite levels at study entry. Authors concluded that total metabolites levels after NO treatment indicated efficient and quantitative delivery as well as potential for increased NO delivery to non-pulmonary tissues. Further comment was made that because no risk of ↑ comorbidities related to prematurity were noted, that extra-pulmonary delivery may confer neuroprotective effects in these subjects. |
| Ballard PL, 2010[50] Subanalysis | | Randomized, controlled evaluation of urine NO metabolites (NOx) in a subset of the NO CLD subjects n=50 | Median: 26.1 ± 1.5 weeks | Median: 764 ± 142 g | 20 ppm for 3-4 days, 10 ppm for 7 days, 5 ppm for 7 days and 2 ppm for 7 days | Urine levels of NOx using a chemiluminescent NO analyzer; creatinine levels using a colorimetric assay (modified Jaffe reaction) compared with NO dose and clinical outcome | Subjects randomized between 7 and 21 days to either NO (n=25) or placebo (n=25). In controls, NOx did not change over time (median: 206 pmol/mg; range: 37-1,269). In the 11 subjects without BPD at 36 wks, median NOx was 485 vs 189 pmol/g when compared with the 14 subjects with BPD ($P=0.13$). In NO-treated subjects, there was a3-fold increase over the study period. Median NOx 214 at study entry; 628 between Days 1-11 ($P<0.0001$); 464 between Days 12-25 ($P<0.0001$); and 401 | The authors observed a 3-fold increase over the study period in the NO-treated subjects. They concluded this reflected the dose of NO given and that pulmonary outcome may not only be affected by the endogenous amount produced, but also by the levels achieved during NO therapy. Investigators also noted the 3-fold increase seen in this study was similar to that seen in plasma and tracheal aspirate sample analysis of NO CLD subjects by Posencheg et al, 2009) |

| Study | Description | Gestational Age | Birth Weight | Dose | Primary & Secondary Outcomes | Results | Summary |
|---|---|---|---|---|---|---|---|
| | | | | | | between Weeks 4-8 ($P$=0.03). | |
| Walsh, 2010[29]<br><br>2-yr follow-up | 24-month follow-up to compare neurodevelopmental outcomes between NO and placebo survivors.<br><br>n=496 | Mean NO: 25.8 weeks<br><br>Mean placebo: 25.7 weeks | Mean NO: 767 g<br><br>Mean placebo: 762 g | N/A | Neurodevelopmental impairment (NDI) defined as one or more: 1) MDI or PDI score <70; 2) Palisano gross motor score ≥2 "unable to walk or crawl"; 3) bilateral deafness with aids; or 4) bilateral blindness.<br><br>Outcomes evaluated stratified by multiple demographic and clinical characteristics and subanalysis stratified on 3 post-randomization variables: BPD at 36 and 40 weeks and exposure >3 days to postnatal dexamethasone | No significant between-group differences noted based on age at study entry, birth weight, gestational age or other demographic variables. Subanalysis of treatment effect was also nonsignificant for BPD at 36 weeks ($P$=0.48), BPD at 40 weeks ($P$=0.61), and postnatal steroid use ($P$=0.19). Multivariate modeling for NDI showed significant ($P$<0.0005) association with BPD at 40 weeks though this did not modify treatment effect. | Authors concluded that 20 ppm NO over 24 days in ventilated preterm neonates aged 7-21 days was both safe and effective and provided improved survival without BPD or apparent adverse effects on growth or neurodevelopment. |

| Study | Description | Gestational Age | Birth Weight | Dose | Primary & Secondary Outcomes | Results | Summary |
|---|---|---|---|---|---|---|---|
| EARLY INO (KINSELLA, 2006)[16]<br><br>Main Phase | Multicenter, blinded, randomized, with stratification according to birth weight. (500-749g, 750-999g, 1000-1250g) NO administered within 48 hours after birth.<br><br>No criteria regarding severity of illness, other than being intubated. mean OI: 5.4 (NO group) vs 5.8 (placebo)<br><br>Early routine treatment study[92]<br><br>n=793 | <34 wks GA<br><br>Mean GA: 25.6 wks,<br><br>Entry at <48 hrs old; mean of 30hrs old | 500-1250g<br><br>Mean BW: NO 796 g, placebo 788g | 5 ppm x 21 days or extubation. Conventional and high-frequency ventilation used. | P: Death or BPD<br><br>S: Severe IVH, PVL and ventriculomegaly | Overall, the incidence of combined end-points of death and BPD didn't differ significantly between NO and placebo EXCEPT among infants in the 1,000-1,025g group, where there was a significant reduction in the combined end points (38.5 % vs 64.1%) and BPD alone (29.8 % vs 59.6%)<br><br>NO reduced the overall risk of brain injury as seen by a decrease incidence of PVL, IVH and ventriculomegaly<br><br>No significant between-group differences noted in duration of mechanical ventilation.<br><br>No significant between-group differences noted in any secondary outcomes (air leak, pulmonary hemorrhage, symptomatic ductus arteriosus, necrotizing entercolitis, retinopathy of prematurity requiring treatment, sepsis, medication use at 36 weeks, or medication use among survivors). | Early, low dose NO did not reduce the risk of BPD in premature infants with RF and a birth weight of 500-1,250 g, but it did reduce the risk among infants with a birth weight of 1,000g or more, and the risk of brain injury in the overall population. |
| Kinsella, 2009[31]<br><br>1,2-yr follow-up | 1 year (n=468) and 2 year (n=458) follow-up study of the 611 preterm neonates who | N/A | N/A | N/A | P: neurodevelopmental status (BSID, PDI, and MDI) | No significant ($P$>0.12) between-group differences were seen in BSID, PDI, or MDI scores at 1 or 2 years of age | Though NO therapy did reduce early brain injury in this study population, it did not contribute to improved neurodevelopmental |

| Study | Description | Gestational Age | Birth Weight | Dose | Primary & Secondary Outcomes | Results | Summary |
|---|---|---|---|---|---|---|---|
| | survived to discharge. | | | | | | outcomes at ages 1 and 2 years. |

| Study | Description | Gestational Age | Birth Weight | Dose | Primary & Secondary Outcomes | Results | Summary |
|---|---|---|---|---|---|---|---|
| Watson, 2009[26]<br><br>1-yr follow-up | Neurodevelopmental evaluation (n=652 or 82.2%)<br><br>Survival data (n=631)<br><br>Course of Care (n=503) | N/A | N/A | N/A | Clinical outcomes assessed via in-person neurodevelopmental evaluation at 1-yr corrected age and by telephone interviews once every 3 months. Neurodevelopmental impairment included any of the following: blindness, cerebral palsy, deafness, MDI or PDI<70. Unimpaired included MDI and PDI >85 without other deficits | Neurodevelopmental Impairment (NDI): Subjects completing testing were more often in middle birth weight stratum (42% vs 30%, P=0.04); Caucasian (66% vs 50%, P=0.003); and inborn (82% vs 65%, P<0.0001).<br><br>Survival free from NDI was greater in NO group (67.9% vs 55.6%, P=0.04).<br><br>Overall survival: Not different between NO group (79.2%) vs placebo (74.5%) (P=0.12). Six subjects died after hospital discharge (NO, n=2 vs placebo, n=4).<br><br>Among subgroups, NO subjects in 750-999 g birth weight range had lower rates of death or NDI (32.1%) vs placebo (44.4%) (P=0.04) and an increased trend toward survival (89.2% vs 81.9%, P=0.09).<br><br>Supplemental oxygen use was common in survivors (72% for a median of 3 months). No differences by treatment arm or in the 1000-1250 g birth weight stratum in which NO had reduced BPD at 36 wks postmenstrual age. The NO subjects in 500-749 g birth weight range had greater oxygen dependency (11.7%) vs placebo (4.0%) (P=0.04). No significant differences in outpatient medication or hospital use. | Both treatment groups commonly experienced neurodevelopmental impairment of some sort, as well as pulmonary morbidity. Substantial healthcare resources were consumed by both groups. |

| Study | Description | Gestational Age | Birth Weight | Dose | Primary & Secondary Outcomes | Results | Summary |
|---|---|---|---|---|---|---|---|
| Gadhia, 2014[26]<br><br>Retrospective analysis | NO with vitamin A (n=118) vs NO alone (n=253) on incidence of BPD and/or Death; | <34 weeks | <1250 g | 5 ppm | Primary: composite of death or BPD; infant development assessment by Baley Scales at 1 and 2 years.<br><br>Secondary: incidence of intracranial hemorrhage, retinopathy of prematuriety, pulmonary hemorrhage, pneumothorax and necrotizing enterocolitis. | Significant reductions in the primary outcomes for the 750-999 grams birth weight infants who received NO plus vitamin A compared with the NO alone group (BPD: P=0.01; Death plus BPD: P=0.01). With regard to the neurodevelopment assessments at 1 and 2 years of age, there were 89 of the 232 (38%) subjects at 1 year that received NO plus vitamin A; 84 of 225 (37%) that received NO plus vitamin A at 2 years. Significant differences were only seen at the 1 year follow up and primarily within the 500-749 gram birth weight group (P=0.01). Overall Bailey scores of mental developmental index scores were also significantly higher in the combined therapy group (P=0.02)<br><br>No significant differences were seen in the incidence between groups for any of the secondary outcomes. | Combination therapy within the 750-999 grams birth weight group compared to the NO alone group had a significantly lower incidence of BPD and/or Death; combination therapy within the 500-749 grams birth weight group at 1 year had a significantly higher Bailey score of mental developmental index compared to the NO alone group. Since the original study was not powered to detect these differences a prospective and properly powered study is recommended to to explore this effect further. |

48

| Study | Description | Gestational Age | Birth Weight | Dose | Primary & Secondary Outcomes | Results | Summary |
|---|---|---|---|---|---|---|---|
| INNOVO (FIELD, 2005)[17]<br><br>Main Phase | Two parallel, multicenter, randomized, non-blinded studies of term/near-term and preterm neonates with severe HRF (preterm, n=108)<br><br>Median OI: 32<br><br>Early rescue treatment[92]<br><br>n=108 | <34 weeks GA and <28 days of age with severe respiratory failure.<br><br>Mean GA: 27 wks | 1,066 g NO group and 890 g control group | Initiated at 5ppm and could be increased to maximum of 40 ppm to achieve a satisfactory response (PaO$_2$ > 22.5 mm after 15 min)<br><br>Maximum treatment duration unclear<br><br>Received surfactant when appropriate. Conventional or high-frequency ventilation used. | P: Death or severe disability at 1 year corrected age and death before discharge or CLD.<br><br>S: Duration in hospital, on O$_2$, time on mechanical ventilation, pneumothorax, pulmonary hemorrhage, PDA, NEC, ROP, cerebral abnormalities, and infection,<br><br>At 1 year corrected age: neuromotor disabilities, vision, hearing, respiratory problems, seizures, hospital admissions. | There was some evidence of prolongation of ICU and increased costs of such care in the NO group.<br><br>59% of the 108 subjects died and 84% of the survivors had some type of disability (20% of them classified as severe). NO had no significant treatment effect on: 1) the primary outcome at 1 yr corrected age (RR 0.99, 95% CI 0.76-1.29, P=0.94); 2) death or supplemental oxygen at 36 weeks PMA (RR 0.98, 95% CI 0.87-1.12, P=0.80); 3) death alone (RR 0.85, 95% CI 0.62-1.16, P=0.30).<br><br>Trend noted toward more mechanical ventilation days in NO-treated group (log rank: 3.6; P=0.06), but no significant between-group differences.<br><br>Out of 55 NO subjects, 28 had established oral feeding by 1 yr corrected age vs controls (n=53) where 20 subjects had done so. By 1-hr corrected age, 5 subjects in each group had been discharged home on supplemental oxygen. | Initially planned for 200 subjects<br><br>A number of limitations were noted:<br>1. planned sample size not reached<br>2. 30% of infants in NO group were given other pulmonary vasodilators<br>3. infants entered already very sick and hence NO may have been administered too late<br><br>Authors concluded NO did not improve outcomes in severely ill neonates, but may be of some benefit in decreasing the risk of CLD in less-ill subjects. |

| Study | Description | Gestational Age | Birth Weight | Dose | Primary & Secondary Outcomes | Results | Summary |
|---|---|---|---|---|---|---|---|
| Ahluwalia, 2006[52]<br><br>Subanalysis | Nested, crossover design, four-period, four-dose response study in preterm neonates receiving NO concentrations of 5, 10, 20, and 40 ppm in randomized sequences x 15 minutes with 5-minute washout in between. Categorized as responders or non-responders based on post-ductal PaO$_2$ increase of 23 mm Hg.<br><br>Median OI: 27.1<br><br>n=13 | Median GA: 27 weeks; range: 25-29 weeks | Median: 983 g; range: 765-1,120 g | 5-40 ppm | Change in post-ductal PaO$_2$ to determine the effect of baseline disease severity prior to each dose on the absolute change | 11 of 13 subjects completed the 4-dose regimen. One subject receiving a single 5 ppm NO dose was withdrawn due to coagulopathy but was included in the final analysis. 46% of the 13 subjects had a clinically significant response to NO but no dose-related effect was identified. | The authors advised that the lowest effective dose should be used in clinical applications in this population and suggested further studies. |
| Hoo, 2009[28]<br><br>1-yr follow-up | A follow-up study of pulmonary function at 1 year of age, corrected for prematurity. Technically acceptable results were obtained in 30 subjects, 19 of which were randomized to NO (preterm, n=10) and 11 to control (preterm, n=6).<br><br>n=30 | N/A | N/A | N/A | Pulmonary function testing via measurement of maximal expiratory flow at residual capacity; incidence of respiratory illness; number of hospitalizations | No significant differences were noted between NO and placebo subjects in pulmonary function (-2.0 and -2.6 Z-scores for NO and non-NO, respectively, 95% CI of difference: -0.3, 1.6, P=0.2). 63% of the preterm subjects and 73% of subjects >34 weeks gestational age reported at least one respiratory-associated hospital admission (P=NS). | Hoo and colleagues noted that small sample size warrants caution when interpreting results of the study, but that considerable respiratory morbidity appeared to occur in neonates with severe respiratory failure at birth irrespective of gestational age or use of NO. Further studies were recommended. |
| Huddy, 2008[35]<br><br>4-5 yr follow- | A 4-5 year follow-up study in 38 of the 43 surviving INNOVO | Mean GA NO: 28.5 weeks; | Mean NO: 1,191 | N/A | 7 domains (cognitive, neuromotor, | The proportion of subjects in each disability category did not significantly differ | Huddy and colleagues concluded that short-term use of NO in this population |

| Study | Description | Gestational Age | Birth Weight | Dose | Primary & Secondary Outcomes | Results | Summary |
|---|---|---|---|---|---|---|---|
| up | preterm subjects to evaluate cognitive and behavioral outcomes. n=38 | postnatal age at study entry: 17 days Mean GA Placebo: 28.2 weeks; postnatal age at study entry: 13 days | ±403 g Mean Placebo: 1,142±440 g | | respiratory, general health, behavior, vision, hearing, and communication). Test scores outside the normal range indicated "impaired"; some functional loss but needing little support were "mild disability"; use of aids and requiring moderate assistance was "moderate disability"; and needing constant supervision and special education was "severely disabled". | between the 1-yr time point and this 4-5-yr follow-up study. 34 of the 55 (62%) original NO subjects had died or were severely disabled vs 37 of the 53 (70%) placebo subjects (RR 0.89, 95% CI 0.67-1.16). Stratification by postnatal age, diagnosis, and disease severity produced similar results. No significant between-group differences was noted in any of the domains tested or in cost utilization. | conveyed neither benefit nor harm in terms of longer-term clinical outcomes. Further studies were suggested as the authors posited that the high degree of illness in the INNOVO subjects at study entry may have limited the benefit of therapy. |

| Study | Description | Gestational Age | Birth Weight | Dose | Primary & Secondary Outcomes | Results | Summary |
|---|---|---|---|---|---|---|---|
| HASCOET, 2005[18] Main Phase | Randomized, multicenter, non-blinded study to evaluate early, low-dose NO vs placebo in infants <32 weeks gestation with HRF ($FIO_2$ >40%, $aAO_2$ <0.22). Randomization was blind until 6 hrs of age. After 48 hours of age, if the infant had HRF the allocation to NO or control was disclosed. Early rescue treatment[92] Total N=860; HRF N=145 | <32 weeks GA Mean GA: 25.7 weeks | From <750g to >1,500 g | Initiated at 5 ppm increased to 10 ppm if no response, weaned after 2 hrs If refractory hypoxemia developed ($PO_2$ <50, $PCO_2$ <50,$FiO_2$ of 1.0 NO was provided. Maximum treatment duration unclear Weaning of NO was based on response status. Conventional ventilation used though rescue HFOV was permitted. | P: Survival to 28 days without death, need for $O_2$, IVH or refractory hypoxemia S: The incidence of and severity of IVH, PVL, within the first 28 days of life, the prevalence of BPD or steroid treatment, the incidence of pulmonary hemorrhage, PDA, NEC, and nosocomial infections. | No difference between primary outcome between the NO and control group, however this refers to a larger cohort of subjects (N=860), many of whom were not eligible to receive the randomized intervention. There were no significant differences in any of the secondary outcomes - NO did not increase the risk of IVH, BPD, NEC, PDA or nosocomial infections. Low dose NO was not associated with any sign related to oxidant stress. | NO appears to be safe in premature infants but did not lead to a significant improvement in intact survival of day 28. Neonates in the NO group were initially sicker than the control group which may have blunted the benefit of NO. Sample size may have been too low to demonstrate an improvement in overall outcome Recommend that the use of NO should be considered when surfactant and optimal ventilation strategies have failed to achieve sufficient oxygenation soon after birth. In an accompanying editorial, Finer commented that the interpretation of these findings is further limited by a relatively high rate of open-label NO use during the study. |
| Hamon, 2005[93] Subanalysis | Randomized subanalysis of oxidative balance in very premature neonates | Mean NO GA: 27.3±0.4 weeks Mean | Mean NO: 1,083±58 g Mean | Same as main phase | Plasma malondialdehyde (MDA) as oxidative stress marker and total plasmatic glutathione (GSH), | After 24 hours, MDA was blunted in NO subjects vs placebo and was similar to reference subjects. GSH was more stable in NO subjects when no | Authors concluded that early, low-dose (5 ppm) NO improved oxidative balance and provided clinical benefit in these preterm subjects with moderate HRF up to |

| Study | Description | Gestational Age | Birth Weight | Dose | Primary & Secondary Outcomes | Results | Summary |
|---|---|---|---|---|---|---|---|
| | NO, n=37 HRF Placebo, n=39 HRF Reference, n=164 non-HRF | placebo GA: 27.9±0.4 weeks; Mean reference Group GA: 28.9±0.1 weeks | Placebo: 1,102±54 g; Mean Reference Group: 1,206±26 g | | intraerythrocyte GSH peroxidase, and rise in GSH reductase activities as antioxidant defenses | difference was noted in GSH reductase and peroxidase activity. By Day 28, an association was noted between oxygen dependence and: 1) higher MDA increases; 2) risk of death. Higher initial drop in GSH was associated with IVH. | Day 28 of life. Hamon et al posited that results support that the deleterious cascade of inflammatory and oxidative injuries was reversible at this postnatal time point (28 days) |
| Desandes, 2004[46] Subanalysis | Randomized subanalysis of hemodynamic indices in very premature neonates <32 weeks GA with HRF and aAO2<0.22. N=35 | Mean NO GA: 27.5±2.3 weeks; mean NO age at entry: 16.5±10.8 hrs; Mean placebo GA: 27.8±2.1 weeks; mean placebo age at entry: 19.8±15.4 hrs. | N/A | 5 ppm | Mean pulmonary blood flow velocity (MPBFV). Low blood flow (LPBF) defined as MPBFV<0.2 m/s. Also evaluated effect on ductus arteriosus | 28 subjects diagnosed with LPBF (NO, n=11; placebo, n=17). 30 minutes after NO, only 8 subjects had LPBF. The aAO2 ratio increased significantly in the NO group from 0.14±0.05 to 0.24±0.08 ($P$=0.027), but this was only apparent in those with LPBF. In NO subjects initially without LPBF, 2 neonates developed it and had decreases in aAO2 ratio (from 0.259 to 0.151; and from 0.217 to 0.198). No significant between-group differences in PDA at baseline or at final post-weaning echocardiograph. NO use did result in a moderate but nonsignificant increase in exclusive left-to-right shunting (22 of 35 after NO vs 15 of 35 at baseline). Bi-directional shunting tended to decrease ($P$=0.10) with | Severe, but not refractory hypoxemia was not predictive of LPBF. Of the 2 neonates who developed LPBF after NO, one had moderate left ventricular dysfunction and both had severe lung disease requiring HFOV. The authors posited that this suggests some influence of ventricular function on response to NO and also that improvement in ventilation is a necessary precursor to treatment. Desandes et al concluded that echocardiographic evaluation of LPBF appeared able to determine which neonates <32 weeks GA with HRF were most likely to benefit from NO on systemic oxygenation. |

| Study | Description | Gestational Age | Birth Weight | Dose | Primary & Secondary Outcomes | Results | Summary |
|---|---|---|---|---|---|---|---|
| | | | | | | treatment (2 of 4 NO subjects had increased bi-directional shunting after weaning) and no subject had exclusive right-to-left shunting. | |
| Hamon, 2009a[37] 7-yr follow-up | A 7-year follow-up study of clinical outcomes. Total N=108 NO, n=20 with HRF Placebo, n=22 with HRF Reference, n=66 non-HRF | Median GA: 29 week; range (28-30 weeks) | N/A | N/A | Height, weight, head circumference, intact neurodevelopment, and re-hospitalization for pulmonary illness | No significant differences between any of the groups for any of the outcomes. | Authors concluded that early, low-dose NO use in sick, premature neonates had no adverse impact on anthropometric variables, neurodevelopment, or general outcomes compared with control and reference groups. |
| Hamon, 2009b[38] 7-yr follow-up | A 7-year follow-up study of neurodevelopmental and cognitive outcomes. Total N=108 NO, n=20 with HRF Placebo, n=22 with HRF Reference, n=66 non-HRF | Median NO: 28.5 [28-30] weeks; Median placebo: 28 [27-30] weeks; Median reference: 29 [28-30] | N/A | N/A | Intact neurodevelopment, need for special medical support, global IQ, normal school Grade, and need for special education | Results are given as either percentage or median and [interquartile range] in order of NO, placebo, then reference group. Intact neurodevelopment: 80%, 77.3%, 80% Special medical support: 65%, 54.5%, 59.4% Global IQ: 87 [76-96]; 97 [83-108]; 91 [82-104] Normal school Grade: 84.2%, 90.5%, 78.5% Needed special education: 73.7%, 54.4%, 58.5% | Authors concluded that early, low-dose NO use in sick, premature neonates had no adverse impact on neurodevelopmental or cognitive outcomes compared with control and reference groups. |
| Hascoet, 2009[39] | A 7-year follow-up study of respiratory | Median: 29 [28-30] | N/A | N/A | Forced expiratory volume before and | No significant between-group differences in any | NO therapy appeared to results in either similar or |

| Study | | Description | Gestational Age | Birth Weight | Dose | Primary & Secondary Outcomes | Results | Summary |
|---|---|---|---|---|---|---|---|---|
| | 7-yr follow-up | outcomes.<br><br>Total N=101<br><br>NO, n=18 with HRF<br>Placebo, n=19 with HRF<br>Reference, n=64 non-HRF | | | | after betamimetics; maximal Mid-Expiratory Flow (MMEF); total lung capacity; residual volume; and incidence of re-hospitalizations for pulmonary illness | variable except for MMEF (*P*=0.005) though a trend toward significant increase (*P*=0.066) was noted in total lung capacity in the NO group.<br><u>MMEF results:</u><br>NO: 1.900 [1.380-2.380]<br>Placebo: 1.480 [1.053-1.584]<br>Ref: 1.338 [1.120-1.742] | improved pulmonary test results in this preterm population. |

| Study | Description | Gestational Age | Birth Weight | Dose | Primary & Secondary Outcomes | Results | Summary |
|---|---|---|---|---|---|---|---|
| *NICHD-PINO (VAN MEURS, 2005)*[11,94]<br><br>Main Phase | Randomized, multicenter, placebo-controlled study to evaluate NO in preterm neonates with severe RF (OI>10 after surfactant therapy)<br><br>Early rescue treatment[92]<br><br>critically ill neonates<br><br>n=420 | <34 WGA<br><br>Mean age hr: 26 hrs NO, 28hrs placebo<br><br>Mean GA:26 wks | 401-1500g<br><br>Mean:8 40 g in NO group and 837g in placebo group | 5-10 ppm generally up to 14 hours.<br><br>Maximum treatment duration was 336 hours.<br><br>Conventional or high-frequency ventilation used. | P: Death or BPD<br><br>S: Decrease the incidence of Grade III or IV IVH or PVL, and the # days of assisted ventilation, O2 use, length of hospitalization and ROP | There was no difference between the incidence of the primary outcome between the two groups.(NO vs placebo). The mode of ventilation also had no significant effect on the incidence of death or BPD.<br><br>As for the secondary outcomes, the frequency of severe IVH or PVL was not significantly different between the two groups<br><br>These neonates were already critically ill and the incidence of IVH or PVL was NOT assessed prior to receiving NO.<br><br>No significant between-group differences were noted in duration of mechanical ventilation | The use of NO in critically ill preterm neonates weighing <1500g with severe respiratory failure does not decrease the rate of BPD or death.<br><br>Mean OI: 24.6 first stratum and 20.4 for the second (modified criteria after first interim analysis)<br><br>Post hoc analysis suggested that infants >1000g seemed to benefit from NO, with a decrease in the incidence of BPD or death without any increase in the rate of IVH. Conventional ventilation was significantly associated with death (*P*=0.01).<br><br>Conversely, infants with a BW of <1000g who were treated with NO had an apparent increase in mortality and a higher rate of IVH.<br><br>The trial was terminated after the second planned interim analysis. At that time, the incidence of Grade III or IV IVH or PVL was significantly higher in the NO group.<br><br>Recruitment ceased with the enrollment of 420 patients instead of the planned 440. |

52

| Study | Description | Gestational Age | Birth Weight | Dose | Primary & Secondary Outcomes | Results | Summary |
|---|---|---|---|---|---|---|---|
| Van Meurs, 2007[10]<br><br>Pilot | Concurrent, randomized, controlled, pilot study of larger preterm neonates (<34 GA weeks, >1500 g) with respiratory distress. Primary outcomes: death and/or BPD.<br><br>n=29 | Mean NO: 31.1±1.2 weeks<br><br>Mean Placebo: 31.4±1.11 weeks | Mean NO: 1,970± 391 g<br><br>Mean Placebo: 2,168± 441 g | 5-10 ppm up to 14 hours | P: Death and/or BPD<br><br>S: Incidence of IVH (Grade 3-4) or PVL; days on oxygen, duration of hospitalization, days on mechanical ventilation, incidence of air leak, incidence of ROP. | No significant between-group differences were reported in either primary or secondary outcomes (before or after adjustment of OI entry strata). Though nonsignificant ($P$=0.08), there was a trend toward less days on ventilation in the NO group (8.7±5.4 days) compared to placebo (16.8±13.9 days). Methemoglobin levels remained ≤4% and $NO_2$ levels remained at ≤3 ppm for all subjects combined. | The authors concluded that NO use in this population did not result in significant decrease in death and/or BPD and suggested further studies with larger sample sizes were needed to confirm the results |
| Van Meurs, 2005[95]<br><br>Subanalysis | Concurrent subanalysis of NICHD PiNO cohort using logistic regression to determine the relationship between OI and various outcome variables.<br><br>n=420 | N/A | N/A | N/A | Correlation between OI and: 1) death; 2) BPD( oxygen use at 36 weeks PMA; 3) physiologic BPD (oxygen saturation <90% in room air) ; and 4) severe (Grade 3-4) IVH or PVL | Mean OI was significantly correlated with death ($P$<0.0001); showed a trend toward significance (P=0.0810) in correlation with physiologic BPD, but had no significant relationship with the other outcomes. | Mean OI was significantly correlated with mortality in this population. |
| Chock, 2008[10,54]<br><br>Subanalysis | Retrospective, subset analysis of preterm neonates with PPROM, oligohydramnios, and suspected pulmonary hypoplasia from the NICHD-PiNO and larger preemie pilot studies. Primary outcomes: death | Mean NO: 27±2 weeks<br><br>Mean Placebo: 29±3 weeks | Mean NO: 1,039± 355<br><br>Mean Placebo: 1,179± 369 g | N/A | P: Death and/or BPD<br><br>S: Severe IVH or PVL, days on mechanical ventilation, days on oxygen, change in $PaO_2$ from baseline. | No significant between-group differences for death and/or BPD ($P$=0.18), death ($P$=0.57), or for severe IVH or PVL ($P$=0.46). The relative risk for BPD at 36 weeks PMA was more favorable in the NO group but did not reach statistical significance (RR 0.50, 95% CI 0.22-1.11, | Authors concluded that the data suggests that NO use resulted in acute improvement in oxygenation, shorter duration of supplemental oxygen and mechanical ventilation without increase in severe IVH or PVL in this population, but recommended that larger, randomized studies were |

| Study | Description | Gestational Age | Birth Weight | Dose | Primary & Secondary Outcomes | Results | Summary |
|---|---|---|---|---|---|---|---|
| | and/or BPD.<br><br>n=12 | | | | | $P$=0.43). Change in $PaO_2$ was greater in the NO group compared to placebo but was nonsignificant (39±50 vs -11±15, $P$=0.09). Days on oxygen (61±38 days vs 90±6 days) and ventilator (19±15 days vs 39±30 days) were also lesser in NO group vs placebo, however, no $P$-value was reported for either. | needed to confirm these results. |
| Ambalavanan, 2008[42]<br><br>Subanalysis | Retrospective analysis of NICHD PiNO study to characterize variables that are predictive of death and/or BPD in preterm neonates with severe respiratory failure. Used regression and CART models.<br><br>n=420 | Mean: 26±2 weeks | Mean: 840 g | N/A | Predictive value of 1) magnitude of initial improvement in $PaO_2$; 2) severity of illness; 3) demographics; 4) response to NO; 5) disease status prior to study entry; and 6) treatment prior to study entry. | Neither magnitude of NO response nor randomization were predictive of death and/or BPD. Lower birth weight, male gender, additional surfactant doses, higher $FiO_2$ at study entry, higher OI, and outborn status were associated with death and/or BPD. Stepwise regression showed a significant association between higher birth weight (variable: per 100 g) and lower death and/or BPD rates only in the NO group (OR, 95% CI 0.65 [0.56-0.76], c statistic for model = 0.84). CART model results were similar with birth weight >1,072 g associated with less likelihood of death/BPD (53% vs 83% for birth weight ≤1,072 g). $FiO_2$ ≥90% alone and $FiO_2$ | Variables predictive of death and/or BPD were identified. Further studies were suggested to determine if short-term indicators of NO response, if any, are associated with long-term benefit or if they may be of use in adjusting NO dosing. |

53

| Study | Description | Gestational Age | Birth Weight | Dose | Primary & Secondary Outcomes | Results | Summary |
|---|---|---|---|---|---|---|---|
| | | | | | | ≥90% in subjects with ≥2 surfactant doses both predicted negative outcomes. | |
| Gordon, 2006[58] Subanalysis | Retrospective analysis cross referencing NICHD PiNO data sets and generic NICHD data sets to identify and characterize the incidence of SIP in the PiNO subjects and determine if NO therapy was protective. n=232 available matched records, 13 with SIP | N/A | N/A | N/A | Incidence of SIP; day of life that SIP was diagnosed; postnatal steroid and indomethacin exposure | 13 subjects of 232 (5.6%) available matched records had SIP. Of the 13, seven were treated with no and six were controls. In the NO group, 47% had postnatal steroid and indomethicin exposure compared with 49% in the control subjects. 12 of the 13 SIP subjects were diagnosed within the first 15 days of life. Though not significant, there was a trend toward later diagnosis in the NO (10.3±2.75 days) vs controls (8.0±3.2 days) ($P$=0.1) | The overall incidence of SIP was similar between the groups with a trend toward later diagnosis in the NO group. Authors concluded that this was consistent with their hypothesis that the pharmacologic steroid-indomethacin synergy depletes NO in the ileum increasing SIP vulnerability. Further studies suggested as this analysis was limited by retrospective design, small sample size and variable NO exposure time (1.4 to 173 hours) |
| Hintz, 2006[43] Subanalysis | Retrospective analysis of interobserver reliability and accuracy of interpretation of cranial ultrasound (CUS) findings comparing local to two central readers (n=1219 CUS) | N/A | N/A | N/A | Interobserver reliability of central readers and accuracy of local readers to central readers at various Grades of IVH, PVL, and ventricular size | 1219 CUS (2438 hemispheres) were interpreted by both blinded central readers, while 538 CUS read by local readers were also reviewed by the central readers. Reliability was excellent between the central readers for severe IVH, PVL, and ventricular size, but only fair when | The authors concluded that results demonstrated consistency between local and central readers for unfavorable CUS findings, but suggested caution in the interpretation of less severe abnormalities. |

| Study | Description | Gestational Age | Birth Weight | Dose | Primary & Secondary Outcomes | Results | Summary |
|---|---|---|---|---|---|---|---|
| | | | | | | abnormalities were less severe. Sensitivity and specificity were similarly high for local reading of severe abnormalities but sensitivity was less so for Grade 1 and 2 IVH. | |
| Sokol, 2006[53] Subanalysis | Previously completed subgroup analysis suggested there was a differential NO response based on BW <1,000 g. These subjects had higher degrees of mortality and IVH. The authors retrospectively used bivariate analysis to evaluate the potential for dose-related toxicities. If outcomes were significantly unfavorable, birth weight strata (401-750 g; 751-1,000 g; and 1,001-1,500 g) were evaluated. (n=420) | N/A | VLBW as in main phase | Analyzed data from initial doses of 0, 5, or 10 ppm and assessed 3 hours after gas initiation | NO dose effect on BPD/death, BPD, and severe IVH/PVL. | Several significant differences were noted at baseline in the three NO dosage groups (0, 5, and 10 ppm: 1) percentage of pulmonary hemorrhage (8.1%, 5.0%, 17.3%; $P$=0.0275); 2) age at randomization (28.9, 22.6, 31.6 hours; $P$=0.0161); 3) percentage of high-frequency ventilation (59.7%, 50.0%, 71.2%; ($P$=0.0285); 4) mortality (43.4%, 45.8%, 65.4%; $P$=0.0162). When mortality was stratified by birth weight, increasing doses correlated overall with increasing mortality for subgroups (P=0.0064). Significant results were noted in the lowest BW group (401-750g) where the rate increased from 52.3% at baseline to 60.4% at 10 ppm and 88.0% at 10 ppm (P=00046) | The authors concluded that a definite correlation was demonstrated between increasing the initial dose and increasing mortality across all BW suggesting a concern over possible dose-related toxicity in these subjects. |
| Hintz, 2007[32] 2-yr follow-up | 18-22 corrected age, follow-up study of NICHD-PiNO | Mean NO: 26.8±2.3 weeks | Mean NO: 958±27 | N/A | Death, neurodevelopmental impairment (NDI) | No significant differences were noted in death or NDI between the NO | Post hoc analysis of the primary and secondary outcomes according to birth |

| Study | | Description | Gestational Age | Birth Weight | Dose | Primary & Secondary Outcomes | Results | Summary |
|---|---|---|---|---|---|---|---|---|
| | | study to evaluate neurodevelopmental outcomes (CP, MDI, PDI, blindness, and deafness) in preterm neonates (<34 GA weeks, <1500 g)<br><br>n=193 | Mean Placebo: 26.2±2.2 weeks | 6 g<br><br>Mean Placebo: 864±271 g | | defined as moderate-to-severe cerebral palsy [CP]; BSID-II Mental Developmental Index [MDI] or Psychomotor Developmental Index [PDI] <70 with or without CP; bilateral blindness or deafness. Unimpaired status was defined as MDI and PDI ≥85 without CP, blindness or deafness. Head circumference and weight were also measured. | group (78%) and placebo subjects (73%) (RR 1.07, 95% CI 0.95-1.19, P=0.30). Moderate-to-severe CP was significantly higher in the NO group (20%) vs placebo (11%) (RR 2.01, 95% CI 1.01-3.98, P=0.0453). No significant between-group differences were noted in the other variables and too few subjects were bilaterally blind or deaf to model the adjusted RR. | weight ≤ or >1,000 g and ventilation model (high-frequency vs conventional). When results for all original subjects who had a primary outcome determined (152 in each group) were analyzed, the risk for "death" was significantly higher (P=0.04) in the NO group (64%) vs placebo (52%) as was the risk for "death or CP" (NO, 74%; controls, 59%, P=0.01) in subjects weighing ≤1,000 g. When using conventional ventilation, NO subjects >1,000 g vs controls had a significantly higher risk for "death" (NO, 64%; controls, 44%, P=0.02) and "death or CP" (NO, 71%; controls, 53%, P=0.02). When analysis was isolated to the follow-up cohort, a trend (P=0.055) toward higher incidence of "CP" in NO subjects (20%) vs controls (7%). Hintz and colleagues concluded that these findings demonstrate no benefit on death, NDI, or neurodevelopmental outcomes when premature neonates receive NO therapy and recommended that such treatment be reserved for research settings until further studies provide conclusive results. |

| Study | Description | Gestational Age | Birth Weight | Dose | Primary & Secondary Outcomes | Results | Summary |
|---|---|---|---|---|---|---|---|
| *SCHREIBER, 2003*[8]<br><br>Main Phase | A randomized, single-center, double-blind, placebo-controlled, 2x2 factorial design study in preterm neonates <34 weeks GA, <72 hours old, and BW <2,000 g.<br><br>Infants randomized to receive either IMV or HFOV.<br><br>Mean OI:6.94<br><br>Early routine treatment in intubated preterm neonate study[82]<br><br>n=207<br><br>NO, n=105<br>Placebo, n=102 | <34 WGA,<72 hours old (Mean 12.9hrs NO,14 hrs placebo), with RDS requiring mechanical ventilation and surfactant therapy Mean GA:27 wks | <2000g<br><br>Mean NO: 1017g and placebo: 949g | 10 ppm on day 1 (12-24 hrs), followed by 5 ppm x 6 days | P: Death or CLD<br><br>S: Incidence of pulmonary hemorrhage and IVH<br><br>Post hoc analysis, stratified according to the severity of the disease, was done to evaluate whether NO differentially benefited infants with mild, as compared with severe initial resp. disease. | The incidence of the primary outcome (death or CLD) was significantly lower in the NO group (48.4%) compared to placebo (63.7%), (RR 0.76, 95% CI 0.60 to 0.97; P=0.03) and did not vary significantly in either group based on birth weight (P=0.36).<br><br>In *post hoc* analysis, NO significantly decreased the risk of the primary outcome vs placebo among infants whose baseline OI was below the median (RR 0.53, 95% CI 0.35 to 0.81) but the influence of ventilation strategy was found to be nonsignificantly associated (P=0.11). The incidence of pulmonary hemorrhage. did not differ significantly between groups; however, treatment with NO was associated with a significant reduction in the incidence of severe IVH and PVL compared to placebo (RR 0.53, 95% CI 0.28 to 0.98; P=0.04).<br><br>Among the survivors, the median duration of mechanical ventilation was 16 days in the NO group compared to 28.5 days in the placebo group (P=0.19). Median hospital stay duration was 65 days in the NO group vs 76 days for those subjects receiving placebo (P=0.22). | In contrast to other trials, this study studied all premature infants with RDS who required mechanical ventilation.<br><br>Schreiber and colleagues concluded that low-dose NO initiated early reduces the incidence of CLD and death in these subjects and may also reduce the risk of two complications often associated with prematurity, severe IVH and PVL.<br><br>Further studies, sufficiently powered to detect a significant interaction, were suggested to reevaluate the impact of IMV compared to HFOV on NO therapy in this population. |

| Study | Description | Gestational Age | Birth Weight | Dose | Primary & Secondary Outcomes | Results | Summary |
|---|---|---|---|---|---|---|---|
| **Srisuparp, 2002**[25]<br><br>Pilot | A prospective, randomized, non-crossover, open-label pilot study in mechanically ventilated preterm neonates <2,000 g BW who initially presented with less severe RDS. Primary outcome was severe IVH (Grades 3-4).<br><br>Baseline OI (NO=10.8±1.5; placebo: 11.9±2.2)<br><br>n=34 | Mean NO: 26.8±0.5 weeks<br><br>Mean placebo: 27.2±0.5 weeks | Mean NO: 874±70 g<br><br>Mean placebo: 901±73 g | 20 ppm x 30 minutes, then to 10 ppm within 6-12 hrs, then weaned from 5 to 1 ppm.<br><br>Treatment up to 7 days.<br><br>Conventional or high-frequency ventilation used. | P: Incidence of Grade 3-4 IVH<br><br>S: Actual and percentage change in $PaO_2$ and OI | .Baseline characteristics were similar. The NO group had less exposure to antenatal steroids than controls, but the difference was not significant. Compared to baseline, the NO group had significant decreases in OI ($P$=0.02) and increases in $PaO_2$ after 30 minutes. Though %Δ in improvement in $PaO_2$ ($P$=0.07) and $PaCO_2$ ($P$=0.02) was noted, the between-group differences did not reach statistical significance. No significant between-group differences were noted in primary outcomes and reported adverse events. | The authors concluded that NO acutely improves oxygenation in neonates with mild-to-moderate respiratory failure without an associated increase in IVH. Further studies were suggested to evaluate long-term benefits such as the incidence of BPD and mortality in this patient population. |
| **Izhar, 2001**[48]<br><br>Subanalysis | A randomized subanalysis, concurrent with the Schreiber study, to investigate the potential anti-inflammatory effects of NO in premature neonates with respiratory distress by measuring interleukin-8 (IL-8) concentration in the tracheal aspirate fluids (TAF). Serial samples were collected on days 1, 7, 14, and 21 or until extubation. | Mean NO: 25.8±0.4 weeks<br><br>Mean placebo: 25.2±0.4 weeks | Mean NO: 795±41 g<br><br>Mean placebo: 728±57 g | 10-5 ppm or placebo x 7 days. | IL-8 protein concentrations | No significant between-group differences were noted in IL-8 concentrations. The control subjects did, however, exhibit a significant ($P$<0.05) time-dependent increase in TAF IL-8 concentrations while the NO group did not. | TAF IL-8 concentration did not increase in NO-treated ventilated preterm neonates with respiratory distress syndrome. The authors concluded that these data suggest that NO has an anti-inflammatory effect in this patient population. |

| Study | Description | Gestational Age | Birth Weight | Dose | Primary & Secondary Outcomes | Results | Summary |
|---|---|---|---|---|---|---|---|
| | N=21 (NO, n=11; controls, n=10) | | | | | | |
| **Izhar, 2000**[56]<br><br>Subanalysis | Concurrent, randomized subanalysis to measure pulmonary toxicity by determining protein nitrosylation, and to evaluate inflammatory effects of NO vs placebo by measuring interleukin-8 in a subset cohort of the Schreiber study. Serial samples of tracheal aspirate fluid (TAF) taken.<br><br>N=6 (NO, n=3; controls, n=3) | N/A | N/A | 10-5 ppm or placebo x 7 days. | TAF protein nitrosylation or IL-8 concentration | No significant difference was noted on Days 1-7 of treatment in protein nitrosylation between the NO group (85±37 pg/ml, n=4) or controls (87±25 pg/ml, n=7). Post-treatment (Days 14-42), the NO group tended to have lower IL-8 concentrations (83±12 pg/ml, n=9) than did the control subjects (127±30pg/ml, n=7). | The authors concluded that NO therapy neither increased pulmonary toxicity as evidenced by lack of increase in protein nitrosylation, |
| **Mestan, 2005**[33]<br><br>2-yr follow-up | 2-year follow-up for neurodevelopmental outcomes.<br><br>n=138 (NO, n=70, controls, n=68) | Mean NO: 27.5±2.4 weeks<br><br>Mean placebo: 27.2±2.6 weeks | Mean NO: 1,026± 366 g<br><br>Mean placebo: 958±356 g | N/A | Developmental outcome levels: 1) *disability*: cerebral palsy, bilateral blindness or deafness; 2) *delay*: MDI or PDI <70 without disability; or 3) *normal*: absence of both disability and delay.<br><br>Additionally, | There were 168 survivors at age two (NO, n=89; controls, n=79), of which 89 subjects (53%) had received NO similar to 51% in the original cohort. Twenty-nine of these subjects were lost to follow-up and were reported to have had greater BW [1,255 ± 453 vs 994 ± 360 g, $P$<0.001] and older gestational ages | The authors suggested that other factors may contribute to abnormal developmental outcome and therefore evaluated the extent to which they accounted for the significant effect of NO, but found that after adjustment, the differences in the risk of abnormal neurodevelopment remained essentially unchanged between the NO and control groups. A *post* |

| Study | Description | Gestational Age | Birth Weight | Dose | Primary & Secondary Outcomes | Results | Summary |
|---|---|---|---|---|---|---|---|
| | | | | | anthropometric measurements were reported and infant development was assessed using the Bayley Scales of Infant Development-II (BSID-II), Mental Development Index (MDI), and Psychomotor Development Index (PDI). | [28.6 ± 3.0 vs 27.4 ± 2.5 weeks, P=0.03] than those included in the follow-up study. Of the 139 remaining subjects available for follow-up evaluation, data from one NO subject was excluded from analysis because consensus could not be reached on neurodevelopmental level being normal or delayed. Baseline characteristics of subjects and mothers (NO, n=70; controls, n=68) were similar except for a significantly decreased incidence of severe IVH or periventricular leukomalacia (PVL) of 9% in the NO group compared to 24% in the control subjects (P=0.02). Forty-eight of the 138 subjects had abnormal primary outcomes and four significant risk factors were found to be associated: 1) BW per 100 g increment (RR, 95% CI 0.91 [0.85 to 0.99], P=0.02); 2) male gender (RR, 95% CI 2.02 [1.21 to 3.36], P=0.007); 3) presence of chronic lung disease (RR, 95% CI 2.11 [1.29 to 3.43], P=0.002); and 4) presence of severe IVH/PVL (RR, CI 95% | hoc analysis was performed to compare BSID-II score regardless of disability diagnosis. When compared to controls, the NO group had half the risk of cognitive impairment (either MDI or PDI scores <70, or both; RR, 95% CI 0.53 [0.29 to 0.94], P=0.03) but no significant difference was noted in psychomotor impairment (RR, 95% CI 0.73 [0.33 to 1.61], P=0.48). The NO group had a significantly higher median weight vs controls (P=0.04) and higher age- and sex-adjusted z score for weight (P=0.02). Mestan and colleagues concluded that the use of NO throughout the first week of life in these preterm neonates provided better neurodevelopmental outcomes at two years of age than did placebo. They additionally posited that because NO use increased the likelihood of survival without neurodevelopmental impairment, it may also significantly improve quality of life for these subjects and their families as well as decrease society burdens of caring for these high-risk children. |

| Study | Description | Gestational Age | Birth Weight | Dose | Primary & Secondary Outcomes | Results | Summary |
|---|---|---|---|---|---|---|---|
| | | | | | | 1.76 [1.10 to 2.81], P=0.05). | |
| White, 2010[44] Subanalysis | Using a subset of the Schreiber subjects, investigators retrospectively reviewed the data to identify the incidence of IVH within the first 2 days of life in preterm neonates with moderate RDS and also to evaluate the effect of NO on IVH extension in those with existing IVH. Radiologists reading all cranial ultrasounds were blinded to the study group assignment. n = 115 | Mean GA: 26.6 ± 2.4 wks | Mean BW: 875 ± 269 g | 10 ppm on Day 1, followed by 5 ppm on Days 2-7 | P: Incidence of IVH within 1st two days of life. Effect of NO on extension of existing IVH | Of the 155 subjects, 73 were treated with NO and 42 controls received placebo. There was a significant between-group difference in the timing of the pre-study cranial ultrasounds. They were done earlier in the placebo group (13.3 ± 6.5 hrs) than in the NO group (26.7 ± 14.4 hrs)(P<0.05). One of the 8 NO subjects with existing IVH progressed to Grade 3-4 vs 3 of 7 subjects in the placebo group (P=0.28). | The authors concluded that the incidence of IVH in the 1st two days of life in preterm neonates with moderate RDS is uncommon and NO did not appear to increase the severity of pre-existing IVH. |
| Cooper, 2011[41] Subanalysis | Subanalysis of the randomized, single-center, double-blind, placebo-controlled main phase study of preterm neonates undergoing mechanical ventilation for RDS n=207 NO, n=105 Placebo, n=102 | <34 weeks | Range: ≤750 to 2,000 g | Inititated at 10 ppm on Day 1, then reduced to 5 ppm for Days 2-7 | Birth weight distribution of severe IVH/PVL and death P-value for birth weight interaction = 0.45 | All weight combined: unadjusted analysis (OR 0.46, 95% CI 0.25, 0.84; P=0.012). Multivariate analysis (adjusted for ventilator type and BW; OR 0.46, 95% CI 0.26, 0.91; P=0.024) (adjusted for ventilator type, BW, GA, and sepsis; OR 0.48, 95% CI 0.25, 0.91; P=0.025). BW ≤750 g: | NO group had significantly less severe IVH/PVL than placebo and was most marked in the ≤750 g subgroup, though this interaction was not significant. In this subgroup, the incidence of death (NO: 28.1%; placebo: 40%) and of IVH/PVL (NO: 15.6%; placebo: 35%) was decreased in comparison to placebo subjects. The authors stated that the data |

| Study | Description | Gestational Age | Birth Weight | Dose | Primary & Secondary Outcomes | Results | Summary |
|---|---|---|---|---|---|---|---|
| | | | | | | Unadjusted analysis (OR 0.27, 95% CI 0.10, 0.73; $P$=0.01).<br><br>BW 751-1,000 g: Unadjusted analysis (OR 0.61, 95% CI 0.18, 2.01, $P$=0.55).<br><br>BW 1,001-1,500 g: Unadjusted analysis (OR 0.97, 95% CI 0.26, 3.62, $P$=1.00).<br><br>BW 1,500-2,000 g: Unadjusted analysis (OR 0.79, 95% CI 0.04, 14.0, $P$=1.00). | suggests birth weight alone is not solely responsible for NO effect on death or severe IVH/PVL and recommended that further studies should not exclude extremely small premature subjects based on BW alone – index of disease severity should also be considered. |

| Study | Description | Gestational Age | Birth Weight | Dose | Primary & Secondary Outcomes | Results | Summary |
|---|---|---|---|---|---|---|---|
| Patrianakos-Hoobler, 2011[36]<br><br>5-yr follow-up | 5 yr and 6 mth follow-up predicting school age readiness based on neurodevelopmental assessments at age 2 years.<br><br>n=121 | Mean: 27.3±2.6 weeks | Mean 987±374 g | N/A | School readiness was defined as ability to function at age-appropriate levels in multiple domains (cognitive, sensory, motor, and social) via standardized scores from the Pediatric Functional Independence Measure (PFIM); the Bracken School Readiness Assessment (BSRA); the Peabody Picture Vocabulary Test (PPVT); the Test of Visual-Motor Integration (TVMI); and measurements of sensory impairments or autism. Subjects were categorized into one of four school readiness levels (Levels 3 and 4 were school-ready and levels 1 and 2 were not) | Children lost to follow-up between the 2-year and 5-year study points did not differ significantly in mean birth weight and gestational age. At 5 years of age, 32% of the subjects required some degree of special educational support compared with those categorized as disabled (11%) or delayed (23%) at 2 years of age. The 2-year subjects classified as "normal" were similar in proportion to 5-year subjects classified as school-ready, but the levels of outcomes were not all the same. For the subjects classified as "not school-ready", "normal" neurodevelopment at age 2 years was 15% (12 of 80) predictive, a status of "delayed" was 50% (14 of 28) predictive, and categorization as "disabled" (12 of 13) was 92% predictive of being non-school-ready. Using the Hollingshead Index of Social Position (ISP) to measure the contribution made by socioeconomic status to the outcomes, the authors noted that 75% of subjects with severe socioeconomic adversity (ISP level 5) and "delay" at age two were classified as not school-ready at 5 years and 6 months of age. In contrast, of the children with developmental "delay", but higher socioeconomic status (ISP levels 1-3), 64% were classified as school-ready at the 5 year-6 month follow-up. | Patrianakos-Hoobler and colleagues concluded that 2-year neurodevelopmental assessments of these preterm subjects are good predictors for school readiness and also highlighted the importance of addressing socioeconomic status and access to good-quality preschool education in children with the highest biomedical and social risks so they can be ready to learn. The authors recommended longitudinal follow-up studies beyond two years to determine the resource needs and impact of early interventional programs in this population. |

| Study | Description | Gestational Age | Birth Weight | Dose | Primary & Secondary Outcomes | Results | Summary |
|---|---|---|---|---|---|---|---|
| FRANCO-BELGIAN COLLABORATIVE NO TRIAL GROUP (MERCIER, 1999)[19]<br><br>Main Phase | A prospective, randomized, controlled, multicenter study of 85 preterm (OI 12.5 to 30) and 107 near-term neonates (OI 15 to 40) with RDS, PPHN, or MAS. NO 10 ppm or control ventilation used (some crossover occurred). Primary endpoint was the OI at 2 hours.<br><br>Median (IQR) OI at baseline: NO, 20.2 (8.3); placebo, 18.0<br><br>Early rescue treatment study[92]<br><br>Total N=204; preterm, n=85 | Of the 85 preterm neonates:<br><br>Median (IQR) NO: 29.6 (2.6) weeks<br><br>Median (IQR) placebo: 29.0 (3.1) weeks | Of the 85 preterm neonates:<br><br>Median (IQR) NO: 1,200 (570) g<br><br>Median (IQR) placebo: 1,150 (520) g | Subjects were randomized to either 10 ppm NO (n=40) or ventilation treatment (conventional mandatory or high-frequency oscillatory) without NO (n=45).<br><br>If OI reached the upper limits based on GA, 20 ppm NO was administered to neonates in the control group and exogenous surfactant and/or switch of ventilation type was carried out in the NO group (per French Drug Agency recommendations). | P: Decrease in OI at 2 hours.<br><br>S: Duration of mechanical ventilation, oxygen use, and time in NICU, as well as incidence of CLD, ICH, PVL, and death. | Reductions in OI were greater in the NO group compared to controls (median -5.4 vs -3.6); however, this difference did not reach statistical significance (P=0.11). No significant differences were observed in between preterm NO and control subjects who were alive and without neurological complication (NO, 40%; control, 38%); had ICH and PVL (NO, 32%; control, 27%); or those who died (NO,27%, control, 35%). Preterm neonates who received NO spent fewer days on mechanical ventilation (12 days vs 16 days), oxygen supplementation (14 days vs 23 days), and in the NICU (26 days vs 33 days); however, none of these differences were statistically significant (P≥0.29). Trial limitations, as reported by the authors, included: 1) substantial variability by trial site in ventilator strategies, extubation and oxygen supplementation criteria; 2) higher baseline OI in the NO group suggesting higher disease severity; 3) lacking of blinding; 4) crossover from placebo to NO for ethical reasons; and 5) less than expected preterm enrollment rate. | The authors concluded that even though early, low-dose NO shortened duration of ventilation and length of NICU stay in the near-term neonates, it was not significantly beneficial in the preterm cohort of this study. |

| Study | Description | Gestational Age | Birth Weight | Dose | Primary & Secondary Outcomes | Results | Summary |
|---|---|---|---|---|---|---|---|
| Truffert, 2003[57]<br><br>Subanalysis | A subanalysis of the RDS cohort (NO, n=62; controls, n=68). Results not broken stratified by preterm and near-term. Same primary and secondary outcomes as main phase trial.<br><br>Median (IQR) baseline OI NO: 21.3 (9.6)<br><br>Median (IQR) baseline OI placebo: 18.9 (8.0)<br><br>Total N=130 | N/A | N/A | N/A | P: Treatment success defined as OI reduction of ≥33% of at least 10 points. | There was a greater trend for treatment success (defined as an OI reduction >33% or >10 points) in 28 subjects treated with NO compared to 19 controls (OR 2.25, 95% CI 1.06 to 4.75). No significant between-group differences were noted in noted between the incidence of hemorrhage, IVH, or PVL; use of oxygen, days in hospital, or duration of mechanical ventilation. Mortality (NO, n=13; controls, n=17; OR 0.89 [0.37, 2.14]) was also not significantly different between the groups. | Truffert and colleagues concluded that early use of NO in RDS significantly reduced the rate of deterioration of hypoxemia 2 hours without excessive neurological abnormalities or modification of mortality or pulmonary morbidity |

NO = inhaled nitric oxide; hrs = hours; g = grams; ppm = parts per million; GA = gestational age; BPD = bronchopulmonary dysplasia; P: = primary endpoint; S: = secondary endpoint; CPAP = continuous positive airway pressure; CMV = conventional mechanical ventilation; HFOV = high-frequency mechanical ventilation; ICH = intracranial hemorrhage; PVL = periventricular leukomalacia; IVH = intraventricular hemorrhage; ROP = retinopathy of prematurity; NEC = necrotizing enterocolitis; wk = week, PDA = patent ductus arteriosus, vs = vs; CLD = chronic lung disease; OI = oxygenation index; PaO₂ = partial pressure of arterial oxygen; BW = birth weight, WGA = weeks gestational age; RA = room air; HUS = head ultrasound; OR = odds ratio; CI = confidence interval; ELBW = extra-low birth weight; HFOV = high-frequency oscillatory ventilation; TAF = tracheal aspirate fluid; RSS = Respiratory Severity Score; NDI = neurodevelopmental impairment; MDI = Mental Developmental Index; PDI = Psychomotor Developmental Index; PMA = post-menstrual age; BSID = Bayley Scales of Infant Development; RR = relative risk; RF = respiratory failure; HRF = hypoxic respiratory failure; PCO₂ = partial pressure of arterial carbon dioxide; FiO₂ = fraction of inspired oxygen; MDA = plasma malondialdehyde; GSH = total plasmatic glutathione; MPBFV = mean pulmonary blood flow velocity; LPBF = low pulmonary blood flow; aAO₂ = alveolar-arterial oxygen gradient; MMEF = maximal mid-expiratory flow; CART = Classification and Regression Tree model; SIP = spontaneous intestinal perforation; CUS = cranial ultrasound; IL-8 = interleukin-8 protein cytokine; PFIM = Pediatric Functional Independence Measure; BSRA = Bracken School Readiness Assessment; PPVT = Peabody Picture Vocabulary Test; TVMI = Test of Visual-Motor Integration; ISP = Hollingshead Index of Social Position; IQR = interquartile range; ICH = intracranial hemorrhage; RDS = respiratory distress syndrome; PPHN = persistent pulmonary hypertension of the newborn; MAS = meconium aspiration syndrome; NICU = neonatal intensive care unit.

## EUNO (European Union Nitric Oxide): INOT-27 Study

### Mercier et al, 2010 (Main phase)

A prospective, randomized, multicenter, double-blinded, placebo-controlled study was conducted by Mercier et al to evaluate the safety and efficacy of inhaled nitric oxide (NO) in reducing the risk of BPD in 800 preterm infants with respiratory distress who ranged in gestational age from $24^{+0}$ to $28^{+6}$ weeks.[15] Gestational age was defined by first trimester ultrasound or by the last menstrual period if no ultrasound was available. Mild-to-moderate respiratory failure subjects were included if surfactant was required or continuous positive airway pressure (CPAP) was necessary ($FiO_2 \geq 0.30$ on a mean airway pressure $\geq 4$ cm $H_2O$ to maintain $SpO_2 \geq 85\%$) within 24 hours of birth. Outborn infants, those $\geq 29$ weeks gestational age or with birth weights <500 gm were excluded as were infants requiring $FiO_2 > 0.5$ to maintain $SpO_2 >85\%$ on sufficient airway pressure to achieve adequate chest inflation two hours after exogenous surfactant was given. Additional exclusion criteria included: substantial congenital heart disease (other than patent ductus arteriosus), lung hypoplasia; severe bleeding or coagulopathy with high-risk of diathesis; decision made to withhold further treatment due to severe birth abnormalities, or use of investigational drugs or device before or during the study.

Of this intent-to-treat (ITT) population, 399 subjects were randomized to the NO group while 401 subjects were included in the placebo group. Subjects received either 5 ppm NO or placebo (nitrogen gas) for 7-21 days with early initiation of treatment (2-26 hours after birth). If subjects required mechanical ventilation for <7 days, study gas was administered via either nasal cannula (NC) or nasal continuous positive airway pressure (nCPAP). Stratified by age and birth weight, the primary efficacy outcome was determined as survival at 36 weeks postmenstrual age without BPD. Secondary outcomes included: survival without substantial brain injury; days on mechanical ventilation; duration of hospitalization; and safety endpoints including the incidence of periventricular leukomalacia (PVL), intraventricular hemorrhage (IVH), treated ductus arteriosus, perforations/sepsis, pneumothorax, pulmonary hemorrhage, retinopathy of prematurity (ROP), or necrotizing enterocolitis (NEC).

No statistically significant between-group differences were found in the baseline characteristics as shown below in Table 1.

**Table 1:** Baseline Characteristics

| Variable | NO (n=399) | Placebo (n=401) |
|---|---|---|
| Gestational Age, weeks | 26.4 ± 1.3 | 26.6 ± 1.3 |
| Birth Weight in grams, mean (SD) | 851 (207) | 864 (192) |
| Gender (M/F) | 207/192 | 220/181 |
| Multiple Births | 29% | 30% |
| Chorioamnionitis | 28% | 23% |
| Antenatal Corticosteroid Use | 89% | 90% |
| Postnatal Surfactant Use | 89% | 88% |
| CPAP (% at study entry) | 10% | 10% |
| Intubated (% at study entry) | 90% | 90% |
| Oxygenation Index, mean(SD) | 8.0 (10.7) | 8.6 (12.7) |
| SD = standard deviation; CPAP = continuous positive airway pressure | | |

No significant between-group difference (P=0.73) was demonstrated in the primary efficacy outcome (overall treatment success) as measured by survival at 36 weeks postmenstrual age (PMA) without the occurrence of BPD. Complete primary outcome data is reported below in Table 2.

Table 2: Primary Efficacy Outcomes

| Outcome at 36 Weeks PMA | NO n (%) | n | Placebo n (%) | n | RR (95% CI) | P Value |
|---|---|---|---|---|---|---|
| Alive at 36 weeks | 343 (86%) | 399 | 359 (90%) | 401 | 0.74 (0.48-1.15) | 0.21 |
| BPD at 36 weeks | 81 (24%) | 339 | 96 (27%) | 358 | 0.83 (0.58-1.17) | 0.29 |
| Success (Alive without BPD) | | | | | | |
| Overall | 258 (65%) | 395 | 262 (66%) | 400 | 1.05 (0.78-1.43) | 0.73 |

(continued)

| By Gestational Age | | | | | | | |
|---|---|---|---|---|---|---|---|
| | $24^{+0}$ to $25^{+6}$ Weeks | 75 (53%) | 141 | 67 (50%) | 134 | 1.14 (0.71-1.82) | 0.60 |
| | $26^{+0}$ to $28^{+6}$ Weeks | 183 (72%) | 254 | 195 (73%) | 266 | 0.94 (0.64-1.38) | 0.75 |
| By Race | | | | | | | |
| | Black | 25 (68%) | 37 | 28 (58%) | 48 | 1.49 (0.61-3.65) | 0.38 |
| | Non-Black | 233 (65%) | 358 | 234 (66%) | 352 | 0.94 (0.69-1.28) | 0.70 |
| Outcome at 36 Weeks PMA | | NO n (%) | n | Placebo n (%) | n | RR (95% CI) | P Value |
| By Birth Weight (Mercier et al, 2009)[2] | | | | | | | |
| | 500 to 749 g | 68 (49%) | 138 | 64 (49%) | 131 | N/A | N/A |
| | 750 to 999 g | 117 (68%) | 171 | 112 (67%) | 168 | N/A | N/A |
| | 1,000 to 1,250 g | 73 (85%) | 86 | 84 (84%) | 99 | N/A | N/A |

PMA = postmenstrual age; n = number; RR = relative risk; CI = confidence interval; BPD = bronchopulmonary dysplasia; g = grams; N/A = not provided by the author

Though the success rate was higher in females (NO: 133 of 189 [70%]; placebo: 121 of 181 [67%]) than for males (NO: 125 of 206 [61%]; placebo: 141 of 219 [64%]), no relative risk related to treatment was seen (females: 1.18, 95% CI 0.76-1.83 compared with males: 0.85, 95% CI 0.58-1.26). Older subjects ($\geq$26 weeks gestational age) were more likely to survive without BPD than younger subjects, but neither showed benefit from NO. A higher survival rate without BPD was observed in black subjects treated with NO compared with non-blacks, although the difference was not significant. Secondary outcomes related to survival without substantial brain injury are noted below in Table 3 (Substantial brain injury was defined as Grade 3 or 4 intraventricular hemorrhage, periventricular hemorrhage, or periventricular leukomalacia on the basis of head ultrasound.)

Table 3: Secondary Outcomes Related to Survival without Substantial brain injury

| Outcome at 36 Weeks PMA | NO n (%) | n | Placebo n (%) | n | RR (95% CI) | P Value |
|---|---|---|---|---|---|---|
| Alive without brain injury | 181 (69%) | 261 | 188 (76%) | 249 | 0.78 (0.53-1.17) | 0.23 |
| By Gestational Age | | | | | | |
| $24^{+0}$ to $25^{+6}$ Weeks | 66 (61%) | 109 | 62 (68%) | 91 | 0.72 (0.40-1.29) | 0.27 |
| $26^{+0}$ to $28^{+6}$ Weeks | 115 (76%) | 152 | 126 (80%) | 158 | 0.79 (0.46-1.35) | 0.39 |

NO = inhaled nitric oxide; RR = relative risk; CI = confidence interval

Of the 800 subjects, 792 (99%) received treatment. The mean $\pm$ SD duration of therapy in the NO group was 16.3 $\pm$ 6.5 days and 16.4 $\pm$ 6.6 days in the placebo group. When subjects who died during therapy were excluded, the mean duration of treatment in the NO group was 17.3 $\pm$ 5.9 days and 17.4 $\pm$ 5.9 days in the placebo group. Other secondary outcomes are reported in Table 4.

Table 4: Other Secondary Outcomes

| Outcome | NO n (%) | All Assessed n | Placebo n (%) | All Assessed n | P Value |
|---|---|---|---|---|---|
| No Adverse Event (AE) | 380 (96%) | 395 | 365 (92%) | 397 | 0.02 |
| AE Suspected Related to Study Drug | 133 (34%) | 395 | 138 (35%) | 397 | 0.76 |
| AE at <26 Weeks Gestational Age | 137 (98%) | 140 | 131 (98%) | 133 | 1.00 |
| AE at $\leq$26 Weeks Gestational Age | 243 (95%) | 255 | 234 (89%) | 264 | 0.006 |

(continued)

| Outcome | NO n (%) | All Assessed n | Placebo n (%) | All Assessed n | P Value |
|---|---|---|---|---|---|
| Serious AE | 158 (40%) | 395 | 164 (41%) | 397 | 0.72 |
| Fatal AE | 39 (10%) | 395 | 32 (8%) | 397 | 0.39 |
| Days of Assisted Ventilation | | | | | |
| Mean (SD) | 44 (26) | 350 | 45 (29) | 338 | 0.72 |
| Days of Hospitalization | | | | | |
| Mean (SD) | 93 (35) | 348 | 94 (36) | 337 | 0.68 |

No significant between-group differences were noted in the incidence of Grade 1 IVH (both: 5%), Grade 2 IVH (both: 11%), Grade 3 IVH (NO: 12%; placebo: 9%); or Grade 4 IVH (both: 0%). Low rates of PVL were also reported in the NO (2%) and placebo (<1%) groups. By the 36-Week time point, a total of 56 of the 399 (14%) subjects in the NO group expired as compared to 42 deaths within the 401 (10%) placebo subjects (P=0.21). In the safety population, the most common causes of death were: intracranial hemorrhage, sepsis, and pulmonary hemorrhage, with incidences not significantly different between the groups. Specifics of the serious adverse events reported are shown below in Table 5.

**Table 5:** Secondary Outcomes: Serious Adverse Events (Safety Population)

| Serious Adverse Events | Study Drug | |
|---|---|---|
| | NO (n=395) | Placebo (n=397) |
| Intracranial Hemorrhage | 114 (29%) | 91 (23%) |
| Patent Ductus Arteriosus (PDA) | 59 (15%) | 45 (11%) |
| Sepsis | 32 (8%) | 31 (8%) |
| Intestinal Perforation | 16 (4%) | 13 (3%) |
| Pulmonary Hemorrhage | 12 (3%) | 14 (4%) |
| Pneumothorax | 12 (3%) | 13 (3%) |

Overall, survival without chronic lung disease did not significantly differ between groups and neither gestational age nor birth weight appeared to influence treatment outcomes. Similarly, early, low-dose inhaled nitric oxide did not change the rate of intraventricular hemorrhage or periventricular leukomalacia. After a thorough review of the data and consultation with multiple clinical experts in the therapeutic area, the sponsor's assessment is that the data from this single study does not represent a safety signal and that the observed differences in death rates do not have any reasonable likelihood of association with NO treatment. While the actual cause, if any, of the apparent imbalance is not yet known, other confounding factors including low birth weight, gestational age, and site-related factors provide alternative explanations. The investigators concluded that the use of 5 ppm NO, started within the first 24 hours after birth and continued for a median of three weeks, did not improve survival without BPD in very preterm neonates with mild to moderate respiratory failure. A long-term follow-up phase is scheduled for the current study to include neurological assessment of subjects at two and seven years of age, while pulmonary function testing is slated for subjects at age seven years.

**NO CLD (Nitric Oxide to Prevent Chronic Lung Disease)**

***Ballard, RA* et *al, 2006 (Main phase)***

Ballard and colleagues conducted a randomized, stratified, double-blind, multicenter, placebo-controlled trial to assess the effects of inhaled nitric oxide (NO) in improving survival without bronchopulmonary dysplasia (BPD; also known as chronic lung disease) in 582 premature infants (≤1250 g, mean gestational age [GA] 26 weeks) requiring ventilatory support at 7 to 21 days of age,[13.14] Subjects were randomized (median age at entry 16 days) to receive either NO or placebo; randomization of infants was stratified according to birth weight (500-799 g and 800-1250 g). In the case of multiple births, the first infant was randomized and all siblings were non-randomly assigned to that same treatment arm. The dose of NO was initiated at 20 ppm for 48 to 96 hours; the dose was subsequently reduced to 10, 5, and 2 ppm at weekly intervals for a minimum treatment duration of 24 days. The primary endpoint was survival without BPD (requirement for ventilatory support or unable to maintain oxygen saturation >88% while breathing room air) at 36 weeks post-menstrual age.

There were no significant differences in gestational age, birth weight, sex, race or ethnicity, or preexisting conditions between the study groups at baseline. The mean birth weight of NO- and placebo-treated infants was 766 g and 759 g, respectively; the median respiratory severity score was 3.5 for both groups (equivalent to an oxygenation index [OI] between 5 and 9). No significant between-group differences were noted between subjects receiving mechanical ventilation (high-frequency and conventional) and those receiving nasal continuous airway pressure (nCPAP). Outcome data is shown below in Table 6.

**Table 6:** Outcome Results

| Outcome | | NO (%) | Placebo (%) | Relative Benefit (95% CI) | P Value |
|---|---|---|---|---|---|
| **Total population** | | | | 1.26 (1.02 to 1.55) | 0.03 |
| | Survived without BPD | 43.9 | 36.5 | | |
| | Death | 5.4 | 6.3 | | |
| | BPD | 50.7 | 56.9 | | |
| **Birth weiqht: 500-799 g** | | | | 1.26 (0.98 to 1.62) | 0.07 |
| | Survived without BPD | 43.1 | 37.6 | | |
| | Death | 6.6 | 7.6 | 1.01 (0.96 to 1.07) | |
| | BPD | 50.3 | 54.8 | | |
| **Birth weiqht: 800-1250 g** | | | | 1.30 (0.91 to 1.87) | 0.14 |
| | Survived without BPD | 45.4 | 35.2 | | |
| | Death | 3.1 | 3.3 | 1.00 (0.95 to 1.06) | |
| | BPD | 51.5 | 61.5 | | |
| NO = inhaled nitric oxide; CI = confidence interval; BPD (bronchopulmonary dysplasia | | | | | |

Among subjects with a birth weight of 500-799 g, neonates who were treated with NO were discharged sooner (P=0.04) and received supplemental oxygen therapy for a shorter period (P=0.006). The primary outcome remained significant when nonrandomized siblings were excluded (relative benefit, 95% CI, 1.01 to 1.53, P=0.035). At 40 and 44 weeks postmenstrual age, significantly less NO subjects remained in the hospital or required respiratory support (mechanical ventilation, nCPAP, or supplemental oxygen) than did control subjects (40 weeks: P=0.01; 44 weeks: P=0.03). If BPD occurred, it was less severe in the NO subjects compared to controls and by 52 weeks PMA, the majority of all subjects had either been discharged or no longer required respiratory support. In a post hoc analysis, it was observed that NO significantly improved survival without BPD for infants enrolled at 7 to 14 days compared to placebo (49.1% vs 27.8%; P=0.005), but not for infants enrolled at 15 to 21 days (40.7% vs 42.8%). Ballard and colleagues concluded that prolonged NO therapy started between 7 and 21 days of age in this preterm population significantly improved survival without short-term adverse events or bronchopulmonary dysplasia.

**Early Inhaled Nitric Oxide Therapy in Premature Newborns with Respiratory Failure**

***Kinsella* et *al, 2006 (5 ppm; 21-day treatment)***

Kinsella and colleagues conducted a randomized, multicenter trial to assess the effects of low-dose NO in premature newborns (n=793; birth weight from 500 to 1250 g and mean GA 25.6 weeks) with respiratory failure (RF) and requiring mechanical ventilation (conventional or high-frequency).[16] Subjects were randomized (mean age at randomization ~30 hours) to receive either NO 5 ppm or placebo for 21 days (or until extubation); randomization was stratified according to birth weight (500-749 g, 750-999 g, and 1000-1250 g). The primary outcome measure was the combined endpoint of death or BPD (need for supplemental oxygen or mechanical ventilation at 36 weeks post-menstrual age plus abnormal chest radiograph findings). Other outcomes included severe (Grade 3 or 4) intracranial hemorrhage (ICH), periventricular leukomalacia (PVL), and ventriculomegaly. The mean birth weight of NO- and placebo-treated infants was 796 g and 788 g, respectively; the baseline OI was 5.4 and 5.8, respectively.

Overall, the incidence of the combined endpoint of death or BPD did not differ significantly between NO and placebo (71.6% vs 75.3%; RR 0.95, 95% CI 0.87 to 1.03; $P$=0.24); however, in the cohort of infants with a birth weight of 1000 to 1250 g, NO was associated with significant reductions in the combined primary endpoint (38.5% vs 64.1%; RR 0.60, 95% CI 0.42 to 0.86; $P$=0.004) and in BPD alone (29.8% vs 59.6%; RR 0.50, 95% CI 0.32 to 0.79; $P$=0.001). There were no significant differences in the incidence of the primary endpoint or BPD between groups in the other birth weight strata. No significant differences were noted between NO and placebo subjects, respectively, in any secondary outcome including incidence of air leak (6.3% vs 6.1%, $P$=0.94); pulmonary hemorrhage (6.0% vs 6.6%, $P$=0.75); patent ductus arteriosus (PDA) requiring medical treatment (54.0% vs 53.7%, $P$=0.92); PDA requiring surgical ligation (21.6% vs 21.8%, $P$=0.96); necrotizing enterocolitis (14.0% vs 12.5%, $P$=0.54); retinopathy of prematurity requiring intervention (16.6% vs 15.2%, $P$=0.59); use of postnatal corticosteroids (60.2% vs 55.9%, $P$=0.24); use of medications at 36 weeks ($P$>0.30); or medication use among survivors ($P$>0.25). In the overall cohort, NO was also associated with a significant reduction in PVL compared to placebo (5.2% vs 9.0%; RR 0.58, 95% CI 0.33 to 1.00; $P$=0.048), as well as for the combined endpoints of severe ICH, PVL, and ventriculomegaly (17.5% vs 23.9%; RR 0.73, 95% CI 0.55 to 0.98; $P$=0.03).

Kinsella and colleagues concluded that NO did not reduce the overall incidence of BPD in premature neonates with respiratory failure and a birth weight of 500 to 1,250 g. However, the risk of BPD was reduced in the subgroup of neonates weighing at least 1,000 g at birth and also resulted in a reduction of risk for brain injury in the entire population as a whole.

**INNOVO: Neonatal Ventilation With Inhaled Nitric Oxide Vs Ventilatory Support Without Inhaled Nitric Oxide for Preterm Infants with Severe Respiratory Failure**

***Field et al, 2005 (5-40 ppm, study period up to 12 hours, NO therapy available beyond that point)***

The effects of NO in preterm infants (n=108; GA <34 weeks and <28 days of age) with severe RF requiring conventional or high-frequency ventilatory support (and having received surfactant when appropriate) were evaluated by Field and colleagues in a randomized, multicenter, controlled, non-blinded trial (a parallel study was conducted in term neonates, n=60, but is not reported here).[17] Preterm subjects were randomized to either NO 5 ppm (n=55) or ventilatory support without NO (n=53). The dose of NO could be increased up to a maximum of 40 ppm to achieve a satisfactory response (increase in $PaO_2$ >22.5 mm Hg after 15 minutes). Though this was not a crossover trial, 3 subjects in the NO group died before receiving NO and 4 subjects in the No-NO group were administered NO secondary to clinician judgment. The primary outcomes were death or severe disability at one year corrected age and death before discharge or CLD (supplemental oxygen at 36 weeks post-menstrual age and/or on the expected date of delivery). Secondary outcomes included: duration of hospital stay; time on ventilation and oxygen; adverse events; age when full oral feeding was established; 1-year corrected neurodevelopmental and psychomotor outcomes, respiratory status, and hospitalizations. Results are provided below in Tables 7 and 8.

**Table 7:** Results Related to NO Administration

| Parameters | NO (n=55) | No NO (n=53) |
|---|---|---|
| **Administration of NO** | | |
| Time from randomization to administration, hrs, median (IQR) | 1.23 (0.83-2.25) | - |
| **Duration of NO treatment** | | |
| <48 hours | 25 | 1 |
| >48 hours <3 days | 3 | 1 |
| >3 days | 24 | 2 |
| **Treatment response at 1 hour[a]** | | |
| improved | 30 | 2 |
| Deteriorated | 2 | 0 |
| No change | 17 | 2 |
| Other | 1 | 0 |
| Missing data | 2 | 0 |

(continued)

| Treatment response at 12 hours[a] | | |
|---|---|---|
| improved | 28 | 1 |
| Deteriorated | 7 | 1 |
| No change | 13 | 2 |
| Other | 1[b] | 0 |
| Not applicable: died | 1 | 0 |
| Missing data | 2 | 0 |
| **Days on ventilator, median (IQR)** | 7.0 (2.0 - 26.0) | 4.0 (1.0 - 9.0) |
| **Days on supplemental oxygen, median (IQR)** | 15.0 (2.0 - 71.0) (n=50)[c] | 6.0 (1.0 - 17.0) (n=49)[c] |
| **Days in hospital, median (IQR)** | 43.0 (2.0 - 104.0) | 7.0 (1.0 - 86.0) |
| **Adverse events potentially related to NO** | | |
| Methemoglobin >2% | 8 | 0 |
| NO$_2$ >2 ppm for >30 minutes | 1 | 0 |
| Other | 6[d] | 0 |

a = Rise in PaO$_2$ <3 kPa but 27% rise from baseline; b = NO stopped: coagulopathy; c = Data missing for 5 NO subjects and 4 non-NO subjects discharged home on oxygen; d = intraventricular hemorrhage with parenchymal involvement (n=2); intraventricular hemorrhage with associated platelet aggregation problems; coagulopathy; subcutaneous bleeding; and airway secretions.

Adverse events reported in NO subjects compared with no-NO subjects included: pneumothorax (20 in each group); pulmonary hemorrhage (4 vs 5, respectively); patent ductus arteriosus requiring treatment (9 vs 13, respectively); confirmed infection (23 vs 21, respectively); suspected infection (12 vs 17, respectively); retinopathy of prematurity (8 vs 4, respectively); and major cerebral abnormality on Day 7 and/or 36-week ultrasound scan (6 of 27 vs 10 of 21, respectively).

**Table 8:** Status at 1-Year Follow-Up

| Parameters | | NO (n=55) | No NO (n=53) |
|---|---|---|---|
| **Cause of death** | | | |
| | Immaturity | 14 | 20 |
| | Immaturity & cerebral event | 2 | 6 |
| | Immaturity & other event (e.g. infection) | 6 | 4 |
| | Bronchopulmonary dysplasia | 6 | 3 |
| | Congenital anomaly | 2 | 1 |
| **Age at death (days)** | | | |
| | ≤1 | 9 | 7 |
| | 2-6 | 3 | 10 |
| | 7-27 | 10 | 15 |
| | ≥28 | 8 | 2 |
| **Impairment or Disability at 1-year corrected age** | | | |
| | Yes, severe | 7 | 2 |
| | Yes, not severe | 16 | 12 |

(continued)

| Impairment or Disability at 1-year corrected age | | |
|---|---|---|
| On supplemental oxygen on expected date of delivery | 16 | 12 |
| On supplemental oxygen at 36-week postmenstrual age | 26 | 15 |
| Oral feeding established | 28 | 20 |
| Discharged home on supplemental oxygen | 5 | 5 |

At baseline, infants had a median OI of 32 and a mean GA of 27 weeks; birth weights in the NO and no NO groups were 1066 g and 890 g, respectively. Of the 108 subjects, 64 (59%) died and 37 of the 44 survivors (84%) had some type of impairment with 9 (20%) of them classified as severely disabled. Treatment with NO was not associated with a significant effect of on any of the pre-specified primary outcomes: death or severe disability at one year corrected age (RR 0.99, 95% CI 0.76 to 1.29; $P$=0.94); death or supplemental oxygen on expected date of delivery (RR 0.84, 95% CI 0.68 to 1.02; $P$=0.08); or death or supplemental oxygen at 36 weeks post-menstrual age (RR 0.98, 95% CI 0.87 to 1.12; $P$=0.80). For death, the RR for NO was 0.85 (95% CI 0.62 to 1.16; $P$=0.30). A trend was noted in NO subjects spending more time on ventilation (log rank: 1.4; $P$=0.06); on supplemental oxygen (log rank: 1.4; $P$=0.24); and in hospital (log rank: 3.5; $P$=0.06); however, this primarily reflected subjects who died ($P$=0.05; log rank test). On the contrary, these durations tended to be shorter in survivors who were treated with NO. Field and colleagues concluded that the evidence from this trial did not support the hypothesis that NO improves the outcome for preterm neonates with severe respiratory failure though they felt it was possible that NO use in less-ill preterm subjects may be beneficial in decreasing the risk of chronic lung disease. Further trials were suggested to confirm these findings.

**Hascoet et al, The Safety and Efficacy of Nitric Oxide Therapy in Premature Infants (10 tertiary perinatal center in France and Belgium, July 1999 to February, 2001)**

*Hascoet* et *al, 2005 (Editorial Comment: Finer* et *al, 2005)*

A randomized, multicenter trial to assess the effects of low-dose NO (5 ppm) in preterm infants (GA <32 weeks) was conducted by Hascoet and colleagues.[18] Gestational age <32 weeks was the single entry criterion, while subjects were excluded if they had refractory hypoxemia ($PO_2$<50 and $PCO_2$<50 mm Hg for $FiO_2$=1.0), thrombocytopenia <50,000/mm$^3$, or a major fetal abnormality. Of the total 860 premature neonatal subjects (NO, n=415; controls, n=445), 61 NO subjects (40% were <28 gestational weeks) and 84 control subjects (45% were <28 gestational weeks) also had HRF. NO was initiated at 5 ppm and maintained for the first hour, with subsequent dosage adjustments determined by the $aAO_2$ response (positive response: $aAO_2$ increase >22; intermediate response: $aAO_2$ <22 but decreasing $\geq$25%). If the response was positive, NO was decreased to 2 ppm and weaned after two hours. The dose remained at 5 ppm and re-evaluated every two hours if the response was intermediate. If no response was noted, NO was increased to 10 ppm for two hours and re-evaluated. In the event of treatment failure, subjects were weaned after four hours. The primary outcome was defined as intact survival at 28 days of age (i.e., without respiratory support, oxygen supplementation, or IVH >Grade I, and also no refractory hypoxemia).

There was no difference in the primary outcome between the NO and control groups (61.4% [23% with HRF] vs 61.1% [21.4% with HRF], $P$=0.943); however, this finding refers to a larger cohort of subjects (n=860), many of whom were not eligible to receive the randomized intervention. No significant between group differences were noted in intraventricular hemorrhage (6% vs 7%); patent ductus arteriosus (34% vs 37%), or necrotizing entercolitis (8% vs 6%). The risk of BPD increased significantly in HRF control subjects (OR = 3.264 [CI 1.461 to 7.292]) vs HRF subjects treated with NO (OR = 1.626 [CI 0.633 to 4.178]) in comparison with nonhypoxemic neonates. Hascoet and colleagues concluded that NO therapy appeared to be safe in preterm neonates but did not result in significant survival benefit at 28 days, though subjects with response to NO had much better outcomes than subjects with intermediate or no response. The authors noted that a larger sample size may be needed to demonstrate an improvement in overall outcome. In an accompanying editorial, Finer commented that the interpretation of these findings was further limited by (among other factors) a relatively high rate of open-label NO use during the study.[96]

**NICHD PiNO** *(National Institute of Child Health and Human Development Inhaled Nitric Oxide for Premature Infants with Severe Respiratory Failure Trial)* **& Concurrent Pilot in Larger Preterm Neonates**

*Van Meurs* **et** *al, 2005 (main phase, NICHD PiNO study)*

Van Meurs et al conducted a randomized, multicenter, blinded, placebo-controlled trial in preterm neonates (n=420; GA <34 weeks and birth weight of 401 to 1,500 g) with severe RF (OI ≥10 × 2 after surfactant) to determine the safety and efficacy of treatment with NO vs placebo.[11,91] (2004 abstract LB12 presented at Pediatric Academic Societies' Annual Meeting ). (NOTE: Initial OI requirement of OI ≥10 × 2 between 30 minutes and 12 hours apart was adjusted to OI ≥5 followed by a second OI ≥7.5 between 30 minutes and 24 hours apart after the Data Safety and Monitoring Committee noted higher mortality than expected in both treatment groups at interim analysis. Analysis was then stratified based on the OI criterion). Eligible infants were those that required assisted ventilation and had a diagnosis of respiratory distress syndrome (RDS), sepsis or pneumonia, aspiration syndrome, idiopathic persistent pulmonary hypertension, or suspected pulmonary hypoplasia. Subjects were randomized to receive either NO or placebo. The dose of NO was initiated at 5 ppm and could be increased up to 10 ppm to achieve a complete response (>20 mm Hg increase in $PaO_2$); weaning occurred 10 to 14 hours after initiation of treatment. The primary endpoint was BPD (defined as treatment with oxygen at 36 weeks of gestation) or death (occurring before discharge or within 365 days among hospitalized infants).

Baseline characteristics were similar between the treatment groups. The mean birth weight and gestational age for the NO group (n=210; 840 ± 264 g; 26 ± 2 weeks) was not significantly different in the placebo group (n=210; 837 ± 260 g; 26 ± 2 weeks). NO subjects had a mean age of 26 ± 23 hours and OI of 23 ± 17 at randomization while placebo subjects were 28 ± 22 hours old with a mean OI of 22 ± 17.

The trial was terminated prematurely after the second planned interim analysis due to 67% of the enrolled infants having reached a study endpoint (death, discharge to home, or 365 days of age). The mean OI at randomization was 24.6 for the first stratum and 20.4 for the second stratum (due to modified criteria after the first interim analysis). Thirty minutes after initiation of treatment, treatment with NO was associated with significant improvements in $PaO_2$ and OI compared to placebo ($P$<0.001 for both). At follow-up, there was no significant difference between NO and placebo in the incidence of the primary outcome (80% vs 82%; RR 0.97, 95% CI 0.86 to 1.06; $P$=0.52). The rates of BPD for NO and placebo were 60% and 68%, respectively (RR 0.90, 95% CI 0.75 to 1.08; $P$=0.26); the rates of death were 52% and 44%, respectively (RR 1.16, 95% CI 0.96 to 1.39; $P$=0.11). Secondary outcomes were not significantly different between NO and placebo subjects in incidence of Grade 3-4 IVH or PVL (39% vs 32%, respectively; RR 1.25, 95% CI 0.95 to 1.66, $P$=0.11); days of oxygen use (84 ± 63 vs 91 ± 61 days, respectively, $P$=0.91); physiological BPD (50% vs 60%, respectively; RR 0.87, 95% CI 0.68 to 1.10, $P$=0.17); days in hospital (101 ± 47 vs 111 ± 48 days, respectively, P=0.65); days on mechanical ventilation (39 ± 45 vs 47 ± 53 days, respectively, $P$=0.56); air leak (35% vs 32%, respectively, RR 1.12, 95% CI 0.78 to 1.61, $P$=0.55); or threshold retinopathy of prematurity (30% vs 32%, respectively, RR 1.16, 95% CI 0.81 to 1.64, $P$=0.42).

In a post hoc analysis, it was observed that NO-treated infants with a birth weight >1,000 g had a significantly lower risk of the primary endpoint compared to placebo (50% vs 69%; RR 0.72, 95% CI 0.54 to 0.96; $P$=0.03); however, NO-treated infants weighing ≤1,000 g had higher mortality (RR 1.28, 95% CI 1.06 to 1.54; p=0.01) and a higher rate of severe intraventricular hemorrhage (IVH) or PVL (RR 1.40, 95% CI 1.03 to 1.88; P=0.03). Conventional ventilation was found to be significantly associated with a higher incidence of death ($P$=0.01). The incidence of severe IVH or PVL in the overall population was not significantly different between groups.

*Van Meurs, 2007 (Concurrent, pilot study of larger preterm neonates)*

In a separate but concurrent, small pilot study of 29 larger preterm neonates (<34 GA weeks; birth weight >1,500 g) with severe respiratory failure (OI ≥15 × 2), Van Meurs and colleagues evaluated the safety and efficacy of NO use.[10] Eligible subjects required mechanical ventilation with an underlying diagnosis of RDS, pneumonia and/or sepsis, idiopathic pulmonary hypertension, aspiration syndromes, or suspected pulmonary hypoplasia. All must have received ≥1 dose of surfactant 4 hours prior to meeting the OI requirements. (NOTE: Initial OI requirement of OI ≥15 × 2 between 30 minutes and 12 hours apart was adjusted to OI ≥10 × 2 between 30 minutes and 24 hours apart after the Data Safety and Monitoring Committee noted higher mortality than expected in both treatment groups at interim analysis. Analysis was then stratified based on the OI criterion). Subjects were excluded if they presented with congenital heart disease, thrombocytopenia, bleeding diathesis, or a decision to withhold full treatment was made). Subjects were randomized to receive either NO therapy (n=14; males, n=10) initiated at 5 ppm (with potential increase to 10 ppm if needed) or placebo (n=15; males, n=9) with treatment continued up to 14 days. Primary outcomes were death and/or BPD and secondary outcomes included the incidence of Grade 3-4 IVH or PVL; days on oxygen; duration of hospitalization and ventilation; incidence of air leak, and retinopathy of prematurity.

No statistically significant between-group differences were noted in baseline characteristics with the majority of subjects having a diagnosis of RDS (NO, n=11; placebo, n=11) and none having aspiration syndromes. Prior to discharge, 36% of the NO subjects (n=5) and 27% of the control subjects (n=4) died. At study end, no significant differences were reported in either primary and secondary outcomes (before or after adjustment for OI entry strata). Post-adjustment for OI, the adjusted relative risk for death and/or BPD was reported as RR 0.80, 95% CI 0.43 to 1.48, *P*=0.50; death alone as RR 1.26, 95% CI 0.47 to 3.41, *P*=0.65; and BPD alone as RR 0.40, 95% CI 0.47 to 3.41, *P*=0.21 . Though the difference was not significant, no incidence of Grade 3-4 IVH/PVL or air leak was reported in the NO group, while IVH/PVL and air leak were reported in two control subjects each. There was a trend toward less days on ventilation (*P*=0.08) and time in hospital (*P*=0.10) in the NO group (8.7 ± 5.4 days; 36.0 ± 21.8 days) in comparison with controls (16.8 ± 13.9 days; 68.5 ± 58.3 days). Methemoglobin levels remained at ≤4% and NO$_2$ levels at ≤3 ppm for all subjects in both groups. Protocol deviations of open-label NO use were noted in two control subjects and one subject who was characterized as an NO non-responder. Van Meurs and colleagues concluded that NO use in this pilot study did not result in significant decrease in death and/or the incidence of BPD before or after OI stratification adjustment but suggested that further studies with larger sample sizes were needed to confirm these findings.

**Schrieber and Colleagues Preterm Study**

***Schreiber* et *al, 2003 (10 ppm on day 1, followed by 5 ppm × 6 days: effect of NO on CLD/death)***

Schreiber and colleagues conducted a randomized, single-center, double-blind, placebo-controlled, 2 × 2 factorial design trial in preterm infants (n=207; GA <34 weeks, <72 hours old, and birth weight <2,000 g) with RDS requiring mechanical ventilation and surfactant therapy.[8] Inclusion criteria included: tracheal intubation and mechanical ventilation required as well as exogenous surfactant, while neonates with major congenital abnormalities or hydrops fetalis were excluded. Neonates were randomized to receive either NO (n=105) or placebo (n=102) and either intermittent mandatory ventilation (IMV) or high-frequency oscillatory ventilation (HFOV). Treatment with NO was initiated at a dose of 10 ppm by continuous inhalation for the first day (12 to 24 hours) followed by 5 ppm for 6 days. Ventilation strategy was determined by clinicians according to protocol and could be changed during the study if the clinician felt the subject's condition was critical enough to warrant an alternative. If this occurred, the study gas was discontinued. Subjects who died prior to discharge or by six months of age, whichever came later, were in the analysis.

The primary outcome measure was death or chronic lung disease (CLD) and secondary outcomes included duration of mechanical ventilation and hospitalization. Additional *post hoc* analyses were performed to evaluate the influence of ventilator strategy, birth weight, and initial lung disease severity the primary outcomes. Adverse events were also captured and assessed, including IVH, PVL, pulmonary interstitial emphysema, pneumothorax, and patent ductus arteriosus requiring indomethacin or surgery. Methemoglobin levels were measured daily with a recommendation to stop the study gas if the concentrations exceeded 5%.

At baseline, the median OI was 6.94 and the mean birth weights were 1,017 g (NO) and 949 g (placebo). The incidence of the primary outcome (death or CLD) was significantly lower in the NO group (48.4%) compared to placebo (63.7%), (RR 0.76, 95% CI 0.60 to 0.97; *P*=0.03) and did not vary significantly in either group based on birth weight (*P*=0.36). In *post hoc* analysis, NO significantly decreased the risk of the primary outcome vs placebo among infants whose baseline OI was below the median (RR 0.53, 95% CI 0.35 to 0.81) but the influence of ventilation strategy was found to be nonsignificantly associated (*P*=0.11). The incidence of pulmonary hemorrhage did not differ significantly between groups; however, treatment with NO was associated with a significant reduction in the incidence of severe IVH and PVL compared to placebo (RR 0.53, 95% CI 0.28 to 0.98; P=0.04). No significant between-group differences were noted in pneumothorax, pulmonary interstitial emphysema, symptomatic patent ductus arteriosus, necrotizing enterocolitis, sepsis, retinopathy of prematurity or hydrocephalus. Among the survivors, the median duration of mechanical ventilation was 16 days in the NO group compared to 28.5 days in the placebo group (*P*=0.19). Median hospital stay duration was 65 days in the NO group vs 76 days for those subjects receiving placebo (*P*=0.22). Schreiber and colleagues concluded that low-dose NO initiated early reduces the incidence of CLD and death in these subjects and may also reduce the risk of two complications often associated with prematurity, severe IVH and PVL. Further studies, sufficiently powered to detect a significant interaction, were suggested to reevaluate the impact of IMV compared to HFOV on NO therapy in this population.

**Franco-Belgian Collaborative NO Trial**

***The Franco-Belgium Collaborative NO Trial Group, 1999***

A randomized, controlled, multicenter study was conducted by the Franco-Belgium Collaborative NO Trial Group in preterm (GA <33 weeks) and near-term (GA ≥33 weeks) neonates (<7 days of age) with severe neonatal respiratory failure (RF) and persistent hypoxemia (despite mechanical ventilation and surfactant therapy) to compare the effects of NO and conventional or HFOV treatment.[19] Preterm infants (n=85) with an OI between 12.5 and 30 were included in this study. Subjects were randomized to either 10 ppm NO (n=40) or ventilation treatment (conventional mandatory or high-frequency oscillatory) without NO (n=45). Subjects were also stratified by type of pulmonary disease, gestational age, and clinical examination into three groups (RDS; idiopathic PPHN; or MAS). Subjects were excluded if OI was higher than the upper limits requiring NO (per French Drug Agency recommendations) or if they had fatal congenital abnormalities, hypoplastic left heart, cyanotic cardiac disease, congenital diaphragmatic hernia or other pulmonary hypoplasia, refractory septic shock, abnormal neurological status secondary to birth asphyxia, ICH, or PDA with severe left-to-right shunting. Baseline arterial blood gases and ventilation/oxygenation parameters were taken and treatment initiated. Measurements were repeated at 30 minutes and again at 1, 2, 6, 12, and 24 hours of treatment. Methemoglobin was measured at the 4-hour time point and daily thereafter.

If OI reached the upper limits based on GA, 20 ppm NO was administered to neonates in the control group and exogenous surfactant and/or switch of ventilation type was carried out in the NO group (per French Drug Agency recommendations). The primary endpoint was decrease in OI at 2 hours. Secondary endpoints included duration of mechanical ventilation, oxygen use, and time in NICU, as well as incidence of CLD, ICH, PVL, and death.

Reductions in OI were greater in the NO group compared to controls (median -5.4 vs -3.6); however, this difference did not reach statistical significance ($P$=0.11). No significant differences were observed in between preterm NO and control subjects who were alive and without neurological complication (NO, 40%; control, 38%); had ICH and PVL (NO, 32%; control,27%); or those who died (NO,27%, control, 35%). Preterm neonates who received NO spent fewer days on mechanical ventilation (12 days vs 16 days), oxygen supplementation (14 days vs 23 days), and in the NICU (26 days vs 33 days); however, none of these differences were statistically significant ($P \geq 0.29$). Trial limitations, as reported by the authors, included: 1) substantial variability by trial site in ventilator strategies, extubation and oxygen supplementation criteria; 2) higher baseline OI in the NO group suggesting higher disease severity; 3) lacking of blinding; 4) crossover from placebo to NO for ethical reasons; and 5) less than expected preterm enrollment rate. The authors concluded that even though early, low-dose NO shortened duration of ventilation and length of NICU stay in the near-term neonates, it was not significantly beneficial in the preterm cohort of this study.

| -Reference -Study Design & Objective -Subjects | Inclusion/Exclusion | Treatment | Outcomes | Results | Conclusion |
|---|---|---|---|---|---|
| *SVEDENKRANS ET AL, 2015*<br><br>Preterm (<32 weeks) infants were studied to attempt to quantify pulmonary hypertension by conducting an inhaled nitric oxide challenge and measuring right ventricular function in response to this treatment.<br><br>N=42; | GA<32 weeks; exclusion criteria not specified.<br><br>mean GA=27(23-30)weeks; mean BW 888(520-1444)g; mean PMA 34 (33-37)weeks. | NO challenge with 10 ppm for 15 min | Measurements of right ventricular function: heart rate (HR); peripheral oxyhemoglobin saturation ($SpO_2$); RV acceleration time, RV output, tissue Doppler derived myocardial performance index, S' and atricuspid annular plane systolic excursion. | No significant changes in HR or $SpO_2$ during the test. Those subjects that required >0.25 $FiO_2$ had an increase in mean SpO2 from 91.4 to 94.8% (p<0.05). All other RV parameters measured, resulted in no significant differences seen during or after NO treatment or between infants on supplemental oxygen and air. | The authors stated that the majority of the study subjects failed to demonstrate a measurable response to NO and therefore this may suggest that in this population of very preterm infants, pulmonary vascular resistance only changes minimally or not at all. Additional studies in subjects with more severe respiratory dysfunction need to be studies, before clear conclusions can be drawn. |

| -Reference -Study Design & Objective -Subjects | Inclusion/Exclusion | Treatment | Outcomes | Results | Conclusion |
|---|---|---|---|---|---|
| *AIKIO ET AL, 2012*[60]<br><br>Transient defect in nitric oxide generation after rupture of fetal membranes and responsiveness to inhaled nitric oxide in very preterm infants with hypoxic respiratory failure<br><br>N=17<br><br>(Acute respiratory effects of NO in 5 of these neonates were described earlier in Aikio, 2003) | **Inclusion**: Infants born at very low gestational age (VLGA ≤ 32 weeks) who required mechanical ventilation and developed hypoxic respiratory failure (HRF) within 12 hours after birth. They were further evaluated as having persistent pulmonary hypertension of the newborn (PPHN) defined as elevated pulmonary vascular resistance with echocardiographic evidence. **Exclusion**: severe congenital anomalies<br><br>Mean gestational age: 27.8 weeks(± 2.3); Mean birth weight:1131g (±647) | 20 ppm to start via endotracheal tube during mechanical ventilation, once supplemental oxygen needs were <50% NO was weaned from 10 to 5 to 2 to 1 ppm over 24-75 hours. 7 of 17 NO subjects received high-frequency oscillatory ventilation.<br><br>Matched cohorts, as noted below, responded to conventional therapy and were defined by antenatal complications and did not receive NO:<br><br>PPROM, n=17 Spontaneous birth without PPROM, n=17 Preeclampsia, n=17 | Interleukin (IL) – 12p70, tumor necrosis factor-α, IL-6, IL-8, IL-10, and IL-1β were analyzed, as well as nitrite + nitrate measurements were quantified. Oxygenation index (OI) and (A/a ratio) were measured during NO therapy. | NO therapy was started at a median age of 4 hours (range: 1.5 to 16.5 hours). OI ↓ and A/a ratio ↑; IL-1β and IL-8↑; shortly after NO therapy initiation. The OI of infants from prolonged premature rupture of membranes (PPROM) at, before or after 24 weeks gestation all responded favorably to NO treatment. Median duration of NO therapy – 35 hours (lower quartile 27.5 hours, upper quartile 49.5 hours). A comparison of the nitrite + nitrate in the airway specimens between the NO therapy group and the matched control groups, only showed a significantly higher concentration in the NO group compared to the preeclampsia group (*P*<0.5). Two infants died after NO therapy and stabilization (one from septicemia at 9 days; one from large intracerebral hemorrhage post-drainage of tension pneumothorax). | NO therapy induced remission of HRF, ↑ nitrite + nitrate and cytokine levels were maintained after discontinuation of NO. Limiting the excessive inflammatory response during the recovery period and defining the cause and prevention of deficient NO production, may help to prevent HRF and chronic lung disease in very preterm infants. |

| -Reference -Study Design & Objective -Subjects | Inclusion/Exclusion | Treatment | Outcomes | Results | Conclusion |
|---|---|---|---|---|---|
| *SHARMA ET AL, 2011*[61]<br><br>Retrospective analysis of preterm neonates admitted to a single center in order to study the efficacy of NO in management of severe hypoxemia due to PPHN in the 1st week of life<br><br>N=23 | **Inclusion**: ≤34 weeks gestational age (GA) and presented with severe respiratory failure (OI≥15); echocardiographically confirmed PPHN at or prior to 7 days of age that was treated with 10-20 ppm NO. **Exclusion:** Severe congenital abnormalities<br><br>Mean GA: 29.8 ± 2 weeks Mean birth weight: 1,456 ± 430 g | NO initiated at a median of 19 hours (range: 6 to 156 hours) and continued for a median of 63 hours (range: 22 to 240 hours). | Outcome was measured by oxygenation response to NO | Fourteen of 23 subjects (61%) experienced a ≥20 mm Hg increase in partial pressure of arterial oxygen (PaO₂) within one hour of NO treatment, while improvement in the remaining 19 of 23 was apparent by the 2nd hour and continued to improve up to the 24 hours post-NO initiation. In the four subjects listed as non-responders by the investigators, PaO₂ improved by the 8th hour but there was no significant reduction in OI until 12 hours after NO was initiated. Additional supportive measures used in the management of the 23 neonates included: surfactant (n=23), high-frequency jet ventilation with a median duration of 9 days (range: 1-73 days) (n=21), and inotropic support (n=23).<br><br>Pneumothorax was reported in 8 subjects. Overall mortality was 26%. Of the six subjects who died, five were considered responders and one was a non-responder. In these six subjects, death was attributed to respiratory failure (n=2), late-onset sepsis (n=2), pulmonary hemorrhage (n=1), and necrotizing enterocolitis (n=1). Of the survivors, seven developed bronchopulmonary dysplasia and three subjects had Grade 3 intraventricular hemorrhage (2 of 3 prior to NO). No stage 3 retinopathy of prematurity (ROP) was reported. | The authors concluded that NO may be effective in selected premature neonates in this population. |

| -Reference -Study Design & Objective -Subjects | Inclusion/Exclusion | Treatment | Outcomes | Results | Conclusion |
|---|---|---|---|---|---|
| *DEWHURST ET AL, 2010*[63] <br><br> The European Inhaled Nitric oxide Registry was established in 2006 to collect standardized information relating to NO therapy in children treated in European centers. This study was conducted to present data relating to the use of NO in newborn infants included in this registry. <br><br> N=112 <br><br> Preterm, n=45 Term/near term, n=67 | **Inclusion:** Newborn infants treated with NO between January 2006 and December 2007 from seven participating centers that were included in the European Inhaled Nitric Oxide Registry. **Exclusion:** Those infants that had congenital heart defects were not included in this analysis. | Median duration of NO therapy was 2 days (interquartile range 1-4 days). <br><br> Median starting dose (20 ppm; range 3.3-25ppm), median maintenance dose (10 ppm; range 0.7-25 ppm), median maximum dose (20 ppm; range 5-25 ppm). | The items that were collected by the centers included: patient demographics, underlying clinical condition including primary and secondary respiratory diagnoses, markers of respiratory disease severity, treatment related variables, acute response to treatment, concomitant therapies and clinical outcomes including mortality, need for ECMO support and development of CLD. The occurrence of potential adverse events related to the use of NO including methemoglobinemia and hemorrhagic complications were also included. | Of the 112 patients, 79% (89/112) were considered acute responders, as defined by a reduction in OI >15% within 30-60 min of starting NO. In those that were responders there was a significant difference in the median OI prior to NO initiation ($P$ = 0.092; median OI in responders 47 vs non-responders 23) than non-responders and was more marked in the preterm group; $FiO_2$ was significantly higher (1.0 vs 0.8, $P$=0.021); and the OI response in percent was significantly different, $P$<0.001. <br><br> In terms of adverse events: 2 babies developed pulmonary hemorrhage (1 each in preterm and term/nearterm), 2 developed rebound hypoxemia on stopping NO (1 each in preterm and term/near-term) and 1 baby developed pneumothorax / pneumomediastinum (term/near-term). <br><br> Survival data revealed 48% of preterm infants survived. | Survival in preterm babies or survival in term babies was not predictable by acute response to NO. Of the 17 preterm infants who survived, there were 71% (12/17) who demonstrated an acute response and 29% or 5/17 that did not ($P$=1.0). <br><br> This data may be used to help in the clinical decision making that is required to monitor the use of NO, identify adverse effects and generate research hypothesis. |

| -Reference -Study Design & Objective -Subjects | Inclusion/Exclusion | Treatment | Outcomes | Results | Conclusion |
|---|---|---|---|---|---|
| *O'DONNELL, FRANTA, & MILETIN, 2010*[62] <br><br> A retrospective case analysis of eight preterm subjects with PPROM between 2007 and 2009 to determine if NO improved survival, oxygenation and other outcomes. <br><br> N=8 | **Inclusion:** Neonates <32 weeks GA, with birth weights of <1,500 g, who had premature, prolonged rupture of membranes (PPROM) >1 week, were in respiratory failure refractory to surfactant, and who received NO. Respiratory failure define as OI >10. **Exclusion:** Not provided. | NO dose not specified. | Primary outcomes: OI response, survival to discharge, BPD at 28 days and 36 weeks' gestation, IVH ≥Grade 2, and PVL. <br><br> Secondary outcomes: NEC ≥Stage Bell 2, ROP ≥Grade 2, pulmonary interstitial emphysema, pneumothorax, early onset sepsis, and patent ductus arteriosus that required treatment. | Mean gestational age was 27 + 3 weeks (SD ± 1.6) and the mean birth weight was 1,061 g (SD ± 195). The mean duration of PPROM was 45 ± 29 days. Prior to initiation of NO, the mean OI was 27.3 ± 12.8. After one hour of NO therapy, the mean OI was 6.3 ± 4.5. BPD was diagnosed in all subjects, but all survived to discharge. At 36 weeks, 67% continued to have BPD and 50% had ≥Grade 2 IVH or PVL. | The authors stated that their survival and morbidity data were improved vs other reports where NO therapy was not used and they concluded that results supported the use of NO in preterm neonates with PPROM. Further studies with larger sample sizes were suggested. |

| -Reference -Study Design & Objective -Subjects | Inclusion/Exclusion | Treatment | Outcomes | Results | Conclusion |
|---|---|---|---|---|---|
| *HOPPER ET AL, 2009*[64]<br><br>Retrospective chart review: Persistent pulmonary hypertension in very low birth weight infants<br><br>N=39 | **Inclusion:** Premature (<34 weeks gestation), very low birth weight (VLBW) (<1500 g birth weight) infants with persistent pulmonary hypertension of the newborn (PPHN) confirmed by echocardiography, and oxygenation index (OI) >10. **Exclusion:** ≥34 weeks gestational age, structural cardiac defects, severe congenital anomalies, >10 days of life. | 8 subjects received NO (dosage not specified) | The incidence of 3-4 intraventricular hemorrhage, survival to discharge, gestational age, birth weight, OI, Apgars, surfactant use, pressors and postnatal steroids in sustained (>24 hours) PPHN subjects were compared to severe PPHN subjects. | Incidence of severe PPHN on Day 1 of life was 11.3%; sustained PPHN had incidence of 3.8%. There were no significant differences between subjects with severe PPHN compared with sustained PPHN in any of the outcomes evaluated. | PPHN can lead to high morbidity in the VLBW population and optimum treatment has yet to be determined. |
| *SU & CHEN, 2008*[66]<br><br>A randomized, non-blinded, placebo-controlled study to determine whether NO therapy improves oxygenation in preterm infants with severe respiratory failure<br><br>N=65<br>NO, n=32<br>Placebo, n=33 | **Inclusion:** ≤31 gestational wks; OI≥25; RDS; birth weight ≤1500 g. **Exclusion:** Uncorrectable bleeding disorders; severe ICH; severe congenital abnormality; lethal chromosomal abnormality | Group A- NO started at a dose of 5 ppm increased every 6 h by 1ppm up to 20 ppm via conventional mechanical ventilation. Group B- oxygen therapy only. Mean treatment duration 4.9±2.3 days. | Primary outcomes- mean OI 24 h after randomization. Secondary outcomes- mortality rate, CLD, intracranial hemorrhage (ICH), patent ductus arteriosus (PDA) and retinopathy of prematurity (ROP). | The OI values in the NO treated group were significantly lower (*P*<0.01), than in the control group 0.5, 3, 12 and 24h after NO therapy. On a *post hoc* analysis, there were no significant between-group differences (*P*>0.05) in the incidence of the secondary outcomes, including duration of mechanical ventilation, CLD, ICH, PDA, ROP, pneumothorax, pulmonary hemorrhage, NEC, sepsis or PVL. Mean duration of NO therapy was 4.9 ± 2.3 days for the NO group (n=32). | NO therapy leads to an improvement in oxygenation without short-term side effects in premature infants with severe RDS and respiratory failure. NO however, does not significantly reduce mortality rate or the incidences of CLD, ICH, PDA or ROP. |

| -Reference -Study Design & Objective -Subjects | Inclusion/Exclusion | Treatment | Outcomes | Results | Conclusion |
|---|---|---|---|---|---|
| *KHEMANI ET AL, 2007*[67]<br><br>A retrospective study in older infants (who formerly preterm) with BPD to evaluate clinical features and outcomes in the surfactant era.<br><br>N=42 | **Inclusion:** Premature infants (<32 weeks of gestation) with BPD who were diagnosed as having PAH ≥2 months after birth, between 1998 and 2006, at a median age of 4.8 months that required supplemental oxygen therapy beyond 30 days of age. **Exclusion:** known structural airway or lung anomalies, congenital anomalies of the pulmonary arteries or pulmonary veins, major systemic vessel –to– pulmonary artery collateral vessels, congenital heart disease, severe liver disease, or PPHN | Not defined | Survival, progression or improvement in severity of PAH, and reactivity to inhaled pulmonary vasodilators. Independent variables: gestational age at birth, birth weight, small birth weight for gestational age, duration of MV, use of high-frequency ventilation, tracheostomy, PDA with indomethacin or surgery, necrotizing enterocolitis, gastrostomy tube placement, Grade III or IV intraventricular hemorrhage, method of delivery, maternal hypertension, maternal age, multiple-gestation pregnancy, and oligohydramnios. These variables were also assessed as associated with severe PAH. | The median gestational age at birth was 26 weeks (range: 23-32 weeks) and the median birth weight was 701 g (range: 355-1320 g). Eleven patients were small for gestational age. 12 patients showed[66] PAP and PVRI improvement with 100% oxygen and 80 ppm NO but remained elevated. PVRI decreased to 7.9 ± 3.8 WU with 100% oxygen and to 6.4± 3.1 WU with the addition of NO. | Premature infants with BPD and severe PAH are at high risk of death, particularly during the first 6 months after diagnosis of PAH. These conclusions were not associated with or without the use of NO. |

| -Reference -Study Design & Objective -Subjects | Inclusion/Exclusion | Treatment | Outcomes | Results | Conclusion |
|---|---|---|---|---|---|
| KUMAR ET AL,2007[97] <br><br> A retrospective chart review (June 2000 to October 2005) to identify risk factors for PH in infants <37 weeks GA and evaluate their response to NO. <br><br> N=61 preterm infants (<37 weeks) | **Inclusion:** infants with echocardiographic evidence of PH in the first four weeks of life and the presence of tricuspid regurgitation and right to left or bidirectional shunt at the foramen ovale or ductus arteriosus on echocardiography. **Exclusion:** Infants with congential heart disease | Infants were initially managed with mechanical ventilation (MV) and oxygen. Those that did not respond to MV, were treated with NO (5 to 15ppm) on >90% $O_2$ and on a mean airway pressure of greater than 13 cms $H_2O$ by high frequency oscillatory ventilation. Postductal $PaO_2$ of 20 mm Hg or greater within 30 min of starting NO with no change in inspired $O_2$ concentration, defined the responders. | Prenatal characteristics studied included antenatal steroids, chorioamnionitis, oligohydramnios, PPROM and pulmonary hypoplasia. Postnatal characteristics studied included GA, birth weight, Apgar score at 1 and 5 min, sex, respiratory distress syndrome (RDS), PDA, sepsis, systemic hypotension, severe IVH, response to NO and mortality. | The only independent predictor of NO usage was PPROM. There were 37% or 23/61 preterm infants with PH that were treated with NO and in addition to PPROM the other characteristics that were associated with PHT in this population compared to those that didn't receive NO, were lower GA, lower birth weight, RDS, systemic hypotension and oligohydramnios. <br><br> The percentage of infants that received NO compared to the percentage of infants that responded to NO in each of the GA groups were the following: GA 23-28 weeks(54 received/16 responded); 29-31 weeks (60 received/83 responded); 32-34 weeks (37 received/83 responded); 35-36 weeks (21 received/100 responded). | Those infants treated with NO had both significantly higher $O_2$ requirements (93 ± 3% vs 60 ± 12%; P<0.001) and significantly higher OI (38 ± 4 vs 9.5 ± 5; P<0.001) compared to those that did not receive NO. The non-responders to NO group had significantly lower GA and birthweight (P<0.01) as well as significantly higher mortality (P<0.04), compared to the responder group. |

| -Reference -Study Design & Objective -Subjects | Inclusion/Exclusion | Treatment | Outcomes | Results | Conclusion |
|---|---|---|---|---|---|
| TANAKA ET AL, 2007[68] <br><br> An historical cohort study to determine whether NO improves neurodevelopme nt-al outcomes for infants with PPHN. <br><br> N=31 <br><br> NO, n=16 100% oxygen, n=15) | **Inclusion:** Cerebral palsy in preterm singleton infants (<34 gestational weeks) with hypoxemic respiratory failure caused by PPHN. All neonates had clinical and echocardiographic evidence of pulmonary hypertension **Exclusion:** Those with structural heart disease. | NO at 5ppm increased by 10 ppm at 30-minute intervals, with and upper limit of 30 ppm. If no significant acute response, infants were continued at 5 ppm for 12 hours; if still no satisfactory response, then they were weaned off NO therapy. Infants that showed improvement continued to receive NO at the minimal level found to be effective (attempts were made to decrease the concentration by reducing it by 5 ppm at 30-minute intervals). | Primary outcome: Incidence of cerebral palsy, The incidence of cerebral palsy was then adjusted for the following variables: maternal fever (≥ 38ºC) during delivery, birth weight, Apgar score at 5 minutes, high-0frequency oscillatory ventilation, and surfactant therapy. | NO-treated patients had incidence of cerebral palsy of 12.5% vs 46.7% on 100% oxygen. After adjustment for variables described in outcomes, NO was associated with a decreased risk of cerebral palsy in preterm infants with PPHN when compared to 100% oxygen therapy. | NO therapy decreased the risk of cerebral palsy in preterm infants with PPHN. |

| -Reference -Study Design & Objective -Subjects | Inclusion/Exclusion | Treatment | Outcomes | Results | Conclusion |
|---|---|---|---|---|---|
| DANI ET AL, 2006[70] <br><br> A single site, randomized study to test whether NO can decrease the incidence of BPD and death in preterm infants with severe RDS. <br><br> N=40 <br><br> NO: n=20 Without NO, n=20. | **Inclusion:** Preterm infants (<30 weeks gestation) and from 0-7 days of life with severe RDS. **Exclusion:** major congenital malformations, hydrops fatalis, platelet count less than 50,000/mm[3], and tendency to bleed as revealed by hematuria, blood in the endotracheal aspirate, gastric aspirate, or stools and oozing from puncture sites. | NO at 10 ppm for 4 hours followed by 6 ppm until extubation, for $FiO_2$ was less than 30% with MAP less than 8 cm $H_2O$. NO was weaned after 72 hours of therapy by decreasing by 2 ppm every three hours. If $PaO_2$ decreased by more than 15%, NO was resumed. All enrolled patients were given surfactant treatment (Curosurf®, Chiesi, Italy: 200 mg/Kg) and an additional dose of surfactant was given 12 hours later or earlier if it was needed. Additional doses were allowed (maximum of four doses) if patients presented a $FiO_2 ≥ 0.40$. | Primary endpoint: death incidence or BPD among surviving infants. Secondary endpoints: evaluation of a/$APO_2$, OI, and VI. Changes during NO therapy, the duration of oxygen treatment, NCPAP, and MV, the incidence of PDA, PH, intraventricular hemorrhage, PVL, ROP, NEC, sepsis, and the length of stay in the ICU and in hospital. | Results for a$APO_2$, OI, and VI in the NO treated groups were significantly lower when compared to the control group. There were less frequent incidences in the NO group when compared to the control group (50 vs 90%, P=0.016) with regard to death and BPD. There were no significant differences between groups with regard to the other secondary endpoints, including occurrence and duration of oxygen supplementation, use of nasal CPAP or mechanical ventilation. High-frequency ventilation was used in 10 NO subjects (50%) vs 11 controls (55%). | NO treatment was effective as independent factor in decreasing the incidence of the combined endpoint of BPD and death in preterm infants with severe RDS. The patients with a birth weight lower than 750 g represents a risk factor for the failure of acute effects of NO therapy in promoting oxygenation improvement. |

| -Reference -Study Design & Objective -Subjects | Inclusion/Exclusion | Treatment | Outcomes | Results | Conclusion |
|---|---|---|---|---|---|
| **LINDWALL ET AL, 2005**<br><br>A randomized, prospective, double-blind, cross-over pilot study to explore the acute NO effects of NO in spontaneously breathing preterm infants treated with nCPAP for moderate RDS.<br><br>N=15 | **Inclusion**: CPAP of 4 cmH$_2$O or higher, arterial to alveolar oxygen tension ratio between 0.13 and 0.22 and a diagnosis of RDS based on the clinical picture and a typical radiographic appearance. **Exclusion** : birth weight less than 1 kg, gestational age <27 weeks, congenital diaphragmatic hernia, intracranial hemorrhage or other signs of severe brain damage, VOC, air leak, meconium aspiration, Apgar score at 5 min of ≤4, umbilical cord pH <6.9, and infection. | Patients were exposed to 10 ppm NO or placebo (nitrogen) for 30 min to examine effects on gas exchange and vital signs. | Primary: changes in oxygenation measured as aAPO$_2$. Secondary: SPO$_2$, PaCO$_2$, respiratory rate, heart rate, and invasive arterial blood pressure | The only outcome measures that were significant when comparing NO to placebo were with aAPO$_2$ (P=0.006) and SPO$_2$ (P=0.012). The increase in aAPO$_2$ during NO treatment was more pronounced in infants with a gestational age below 34 weeks (n=9, 95% CI for aAPO$_2$ difference: 0.006-0.08) than in more mature infants (n=6, 95% CI for aAPO$_2$ difference:-0.002-0.06). | NO added to nasal CPAP results in a slight but statistically significant acute improvement in oxygenation in newborn preterm infants treated with nasal CPAP for moderate RDS. |

| -Reference -Study Design & Objective -Subjects | Inclusion/Exclusion | Treatment | Outcomes | Results | Conclusion |
|---|---|---|---|---|---|
| **ATHAVALE ET AL, 2004**[73]<br><br>A Nonrandomized, prospective, uncontrolled study to evaluate the acute effects of low-dose in preterm infants with BPD<br><br>N=13 | Inclusion: birth weight 500 to 1500 g, FiO$_2$ ≥ 0.25 in order to maintain arterial SPO$_2$ greater than 90% while on mechanical ventilation. Exclusion: corticosteroid administration within 72 hours of the study, thrombocytopenia or any bleeding diathesis, intracranial hemorrhage ≥ Grade III, congenital cardiopulmonary anomaly, clinically significant PDA and documented septicemia. | NO at 10 ppm for 2 hours via MV. Three multiple-breath N$_2$ washouts were performed at the end of the first and the second hour of NO administration (1 hour NO, 2 hour NO). At the end of 2 hour NO, the echocardiographic evaluation was repeated while breathing NO. This was followed by an initial weaning to 5 ppm, followed by step reductions of 1 ppm. | Primary: effects on lung mechanics, ventilation, distribution, oxygenation and cardiac function. These were measured by R$_L$, C$_L$, N$_2$ clearance (to assess ventilation distribution). Oxygenation was assess as mean SPO$_2$ and FiO$_2$ over baseline, 1 hour NO and 2 hour NO. Cardiac function was assessed by echocardiographic evaluation, LVEF and right ventricular shortening fraction at baseline and at the end of 2 hour NO. | There were no changes in R$_L$, C$_L$, or N$_2$, clearance. No changes in mean heart rate and pulmonary artery pressure and no subject experienced systemic hypotension during NO. Though there was a small but significant improvement in LVEF at the 2$^{nd}$ hour, no significant change was observed in RVEF. FiO$_2$ did not change significantly from baseline, though a slight reduction was noted during the 1$^{st}$ NO hour commensurate with a slight improvement in oxygen saturation. Hypoxemic episodes (S$_P$O$_2$<85%) did not differ in frequency from baseline to NO therapy, but the duration was significantly greater during the 2$^{nd}$ hour of NO. All subjects were weaned from NO successfully and required supplemental oxygen for ≤28 days during hospitalization. Post-NO head ultrasounds showed no changes from baseline and all subjects were discharged alive. | Low-dose NO had no acute effects on lung function and oxygenation in ventilated preterm infants with BPD. |

| -Reference -Study Design & Objective -Subjects | Inclusion/Exclusion | Treatment | Outcomes | Results | Conclusion |
|---|---|---|---|---|---|
| **UGA ET AL, 2004[72]**<br><br>A retrospective comparative study of very low-birthweight infants with pulmonary hypoplasia due to oligohydramnios who had or had not been treated with NO therapy to determine whether NO therapy improves the survival of these infants.<br><br>N=18<br><br>NO, n=8<br>Control, n=10 | **Inclusion:** Very low birth weight infants with pulmonary hypoplasia due to oligohydramnios diagnosed if all of the following were satisfied: 1) artificial surfactant treatment did not improve the respiratory distress; 2) prolonged rupture of membrane continued for more than 5 days with oligohydramnios; 3) sufficient arterial oxygenation did not occur even after giving 100% oxygen, and more than 8 cm $H_2O$ of mean airway pressure was needed to maintain arterial oxygenation. **Exclusions:** not specified | NO ranged from 40 to 30 ppm and was continued for more than 24h. The concentration was reduced during therapy according to respiratory improvement. | Survival, intraventricular hemorrhage and bronchopulmonary dysplasia, mean airway pressure, oxygenation index, and alveolar arterial oxygen. | There were significant ($P<0.05$) differences in survival for more than 7 and 28 days and in value of alveolar arterial oxygen between the NO treated group and the control group. However the arterial oxygenation of the NO treated group 2 h after the treatment and the control group at the corresponding time did not differ significantly. | NO improved the arterial oxygenation and significantly improved the survival rate. A controlled study in preterm infants with pulmonary hypoplasia due to oligohydramnios to determine whether NO therapy improves survival is necessary. |

| -Reference -Study Design & Objective -Subjects | Inclusion/Exclusion | Treatment | Outcomes | Results | Conclusion |
|---|---|---|---|---|---|
| **AIKIO ET AL, 2003[74]**<br><br>A prospective study to test whether NO is effective in small premature infant with intractable respiratory failure.<br><br>N=5 | **Inclusion:** birth weights < 1500 g (VLBW) and a progressive respiratory failure before the age of 5 h. refractory respiratory failure was defined as a failure to attain adequate arterial oxygen tension on two consecutive determinations, despite 100% oxygen ventilation using a mean airway pressure of at least 13 cm $H_2O$ and two doses of exogenous surfactant therapy (Curosurf, 100 mg kg$^{-1}$) **Exclusions:** lethal malformation, unevacuated pneumothorax, extreme prematurity (gestation <24 + 0wk), severe shock and severe bleeding disorder with disseminated coagulopathy as judged from low platelet count and typical findings in peripheral blood sample | Mechanically ventilated subjects were started at 20 ppm NO with dose titration to the lowest effective dose over a period of 3-4 days. Surfactant was given at least once. Met-Hb and $NO_2$ levels taken every 12 hours. Airway specimens tested for cytokines and surfactant proteins. Results were compared with 2 other with similar gestation; 1) those with histological chorioamnionitis but no clinical signs of systemic infection (n=5); and 2) those without evidence of chorioamnionitis or prenatal infection (n=5). | OI and arterial/alveolar ratios for oxygen tension (a/A) measured respiratory status. Inducible nitric oxide synthase (NOS2); surfactant protein A (SP-A); interleukin-1β (IL-1β); and interleukin-8 (IL-8) were collected from airway specimens to determine immune responsiveness of the immature lung over three time periods (0-24 hours; 36-72 hours; and 94-144 hours). | Subjects required ventilation for a mean of 12 days. No side effects except one transient case of methemoglobinemia. In all cases oxygen saturation increased, allowing a decrease in oxygen requirements within 10 min. Immunoreactive NOS2 in airway cells, and the levels of SP-A and IL-1β (interleukin-1) in the airway specimens were low soon after birth, but increased significantly by age 36-72 h. Vs infants without clinical signs of infection, these subjects had a lower NOS2 and IL-1β. Follow-up at corrected ages (full term to 24 months) showed no abnormal neurological and respiratory findings. | After surfactant therapy, infants that were VLBW with progressive respiratory failure and infection at birth that were treated with NO responded markedly. |

75

| -Reference -Study Design & Objective -Subjects | Inclusion/Exclusion | Treatment | Outcomes | Results | Conclusion |
|---|---|---|---|---|---|
| **CLARK PL ET AL, 2002**[75] <br><br> A prospective open label study to determine whether NO in very low birth weight infants with CLD might improve oxygenation without adverse effects. <br><br> N=33 <br><br> 21 males, mean gestational age was 25.3 weeks (23-29); mean age at enrollment was 19 days (9-29). | Inclusion: birth weight <1250 g; age<30days, but ≥ 10 days; need for assisted ventilation; need for $FIO_2$ ≥0.4 without fluctuations of >0.25 in the preceding 24 hours; and clinical course and radiographic findings compatible with CLD. Exclusion: initiation of systemic corticosteroid or inhaled β agonist therapy within the preceding 48 hrs; new diagnosis of sepsis within the preceding 48 hrs; thrombocytopenia; progressive intraventricular hemorrhage; lethal congenital anomaly; complex congenital heart disease. | NO initially at 20 ppm for 3 hours, then it was decided to continue if there was evidence of efficacy. After 36 hrs, dose was reduced to 15 ppm and then reduced further every 12 hrs by 2-3 ppm to a dose of 2 ppm as long as there was no worsening of oxygenation. Treatment was discontinued by 7 days if no evidence of dependency was detected. If dependency was seen, No was continued with repeated trials of discontinuation every 24-48 hrs. | Echocardiographic analysis, respiratory monitoring, tracheal aspirate analysis. | The significant changes regarding pulmonary gas exchange $FIO_2$ at 3 hours and at 72 hours compared to baseline values ($P$< 0.05); mean $FIO_2$ at 72 hours was also lower than that at 3 hours ($P$<0.05). The magnitude of reduction in $FIO_2$ correlated inversely with the baseline $FIO_2$ ($P$< 0.05); $r$= -0.72). Eleven of the 27 infants in whom echocardiography was performed, showed evidence of increased pulmonary vascular resistance. There no differences found in tracheal aspirate samples when comparing between baseline and during NO or after NO therapy. At 6 month postconceptional age, out of 25 available records, 10 infants continued treatment with supplemental oxygen by nasal cannula; 15 were breathing room air. | In infants with early chronic lung disease, NO improved oxygenation in most of them, without inducing changes in inflammatory markers or oxidative injury. |

| -Reference -Study Design & Objective -Subjects | Inclusion/Exclusion | Treatment | Outcomes | Results | Conclusion |
|---|---|---|---|---|---|
| **TRUOG ET AL, 2002**[76] <br><br> Study design not specified. Objective was to determine the effects, if any, of the interaction of NO with peptide endothelin-1 in CLD and PPHN. <br><br> N=54 <br><br> PPHN, n=21 CLD, n=33 | **PPHN-Inclusion**: mean 39.4 wks GA (36-72); mean birth weight 3470 g; PPHN confirmed by echocardiogram. **Exclusion**: not specified **CLD-Inclusion**: Birth weight <1250 g; age between 10 and 30 days, required assisted ventilation and an $FIO_2$≥ 0.4 to maintain $SPO_2$ of 88-94%. **Exclusion**: initiation of systemic corticosteroid or inhaled β agonist within 48h, sepsis within 48 h, thrombocytopenia, progressive, IVH lethal congenital anomaly and complex congenital heart disease. | NO started at 20 ppm with dose reduced in 204 ppm decrements after 48 h for 7 days. | Plasma levels at baseline and after 24 h of NO were measured for endothelin-1 (ET-1) in PPHN patients. In CLD patients, tracheal aspirate samples were collected and the fluid aspirated from airways was measured prior to ET-1 analysis and at 48 h after NO initiation and 48 h after NO discontinuation. | PPHN: when baseline values were compared to NO at 24 h, there was a significant inverse correlation between change in plasma ET-1 and change in arterial partial pressure of $O_2$. CLD: There were no significant correlations between any of the values in this patient group. | No therapy give in the short-term was associated with a decrease in plasma ET-1 levels in PPHN, but did not affect tracheal aspirate ET-1 in CLD. |

| -Reference -Study Design & Objective -Subjects | Inclusion/Exclusion | Treatment | Outcomes | Results | Conclusion |
|---|---|---|---|---|---|
| **DU ET AL, 2002**[77] <br><br> Retrospective to assess effects of NO as a rescue therapy for term and preterm neonates with HRF. <br><br> N=75 <br><br> Term, n=40 preterm, n=35 | **Inclusion**: term and preterm neonates with HRF requiring intratracheal intubation and MV, inspired $FIO_2$ >0.8 or MAP ≥10 cmH$_2$O, and transcutaneous pulse $SPO_2$ <85% or $PaO_2$ <50 mmHg; pre-ductal vs post-ductal transcutaneous $O_2$ saturation gradient of ≥ 10% or an echocardiographic evidence of PPHN (in 45 patients) including a right-to-left shunting through ductus or ovale foramen with either a tricuspid insufficiency jet with an estimated systolic pulmonary artery pressure ≥75% of systolic aortic pressure.[98] **Exclusion**: fatal congenital cardiac abnormalities, refractory shock, IVH ≥ Grade 2[98] | NO initial dose of 5-20 ppm via infant ventilator with Pulmonox™ NO delivery system. If improvement in oxygenation, dose was reduced by 3-6 ppm at 4 h and kept for a period from several hours to longer than 24 h. in very immature neonates, less than 10 ppm of NO was used at the beginning and during maintenance. | OI, TWOI, methemoglobin in blood during NO inhalation. Response assessment was also conducted in a sub-group analysis of term and preterm neonates according to their baseline pH<7.25 and ≥ 7.25. | For the **term** infants, percent decrease in OI was significant compared to baseline at both 3 h and 24h, TWOI was significantly reduced compared to preterm infants. For **preterm** infants, significant percent decreases in OI were only seen at 24 h of NO compared to baseline. In the sub-analysis significant decrease in OI over time was seen in those with pH ≥ 7.25 at 30 min, 3 and 24 h. In those with pH <7.25, significant decreases in OI could only be observed at 24 h ($P$<0.05 vs baseline). | In term and preterm neonates with HRF, NO improved oxygenation and its response was in favor of term infants or those with high blood pH values at study entry. |

76

| -Reference -Study Design & Objective -Subjects | Inclusion/Exclusion | Treatment | Outcomes | Results | Conclusion |
|---|---|---|---|---|---|
| KINSELLA ET AL, 1999[12] <br><br> Multicenter, double-blind, randomized, controlled to investigate whether low-dose NO would improve survival in premature neonates with HRF. <br><br> Early rescue treatment study[92] <br><br> N=80 <br><br> Low-dose NO, n=48 <br> Without NO, n=32 | Inclusion: delivery at gestational age 34 wks or less; age 7 days or younger; severe hypoxemia despite mechanical ventilation and surfactant treatment when indicated. Exclusion: fatal congenital anomalies or congenital heart disease (except atrial and ventricular septal defects). | NO – 5 ppm for 7 days via MV with standard ventilators or with high-frequency devices. A threshold of 15% or more increase in OI was used to warrant restarting study gas, if restarted periods without gas were kept to every 2 days for a maximum treatment duration of 14 days. | Primary outcome was survival to discharge. Secondary outcome- rate and severity of ICH, pulmonary hemorrhage, duration of ventilation, and CLD at 36 weeks' postconceptional age | No group had improved oxygenation after 60 min ($P$=0.03). Survival at discharge was 52% in the NO group and 47% in controls ($P$=0.65). Causes of death were similar in the two groups. . Days on ventilation were significantly less in the NO-treated group (median: 26; range: 3-69 days) compared to the controls (median: 37; range: 8-395 days) ($P$=0.046) <br><br> There were no differences in adverse events or outcomes. Methemoglobin levels were similar between the groups. No significant differences were noted between NO and control subjects in the incidence of intracranial hemorrhage Grades 1-4 (51% vs 50%, P=0.93); pulmonary hemorrhage (13% vs 9%); symptomatic patent ductus arteriosus (21% vs 19%); periventricular leukomalacia (8% vs 13%, $P$=0.62). One NO subject had retinopathy of prematurity requiring treatment compared with three in the control group ($P$=0.10). | Low-dose NO improved oxygenation but did not improve survival in severely hypoxemic premature neonates. Low-dose NO in the most critically ill premature neonates does not increase the risk of ICH and my decrease risk of chronic lung injury. |

| -Reference -Study Design & Objective -Subjects | Inclusion/Exclusion | Treatment | Outcomes | Results | Conclusion |
|---|---|---|---|---|---|
| YADAV & EMMERSON, 1999[99] <br><br> Retrospective analysis to evaluate the most appropriate subgroup of preterm subjects for NO use. <br><br> N=41 | Inclusion: mean birth weight (1.0kg (SD 0.46 kg) and gestational age of 27 weeks (SD: 2-6). Exclusion: no major congenital malformation. <br><br> Mean OI: 40 (17) | After maximal therapeutic intervention, NO initiated at 10 ppm and titrated by 5 ppm depending on response. Administered x 60 minutes. | Response, survival, incidence of intracranial hemorrhage. | Two groups of neonates identified: 1) responders, who had a decrease in OI of ≥10 within 60 minutes of NO initiation; 2) non-responders. 26 were responders and 15 were not. Gestational age, birth weight, clinical risk index scores, time of NO initiation, NO doses, and OI did not vary significantly between the groups. | Overall, 25 of 41 subjects (61%) died prior to discharge. There were more deaths in the non-responders vs responders (14 of 15 vs 11 of 26). A significant association was noted in multivariate analysis (survival as dependent variable; gestational age, birth weight, OI, and pH as independent variables) between NO response and survival at discharge ($P$=0.0). The retrospective design prevented investigation of any causal relation between response at 60 minutes and survival. Further studies were suggested to define the preterm subgroup who would benefit the most from NO. |

| -Reference -Study Design & Objective -Subjects | Inclusion/Exclusion | Treatment | Outcomes | Results | Conclusion |
|---|---|---|---|---|---|
| CHEUNG ET AL, 1998[79]<br><br>Retrospective chart review to determine outcomes of VLBW neonates treated with NO.<br><br>NO, n=24<br><br>Gestational age: 24-30 weeks; birth weights: 340-1, 460 g | **Inclusion:** birth weight ≤1,500 g, severe hypoxemic respiratory failure (OI 28-52), unresponsive to aggressive conventional treatment that had been treated with NO. **Exclusion:** birth weight >1,501 g or lack of treatment with NO. Infants with congenital abnormalities were also excluded. | If hypoxemia persisted at $FiO_2$ >0.90, NO was added at physician's discretion beginning at 20 ppm with attempts to lower the dose once every 2 hours by 5 ppm increments to determine minimal dose able to provide maximal results. In NO responders (increase in $PaO_2$ of ≥10 mm Hg), attempts to wean were made once every 24 hours by decreasing the dose in 5 ppm increments every 15-30 minutes. Arterial blood gases were drawn prior to NO therapy and repeated at dose changes. Cranial ultrasounds were done at baseline and within 24 hours of NO initiation whenever possible to be repeated at physician's discretion within 4 days of birth, 14 days post-birth, and prior to discharge. $FiO_2$, mean airway pressure, and OI were obtained within 1 hour prior to NO and repeated at 1, 4, 8, and 24 hours after NO was started. | Oxygenation and mortality were outcomes in main phase. Survivors <1,250 g birth weight had neurodevelopmental follow-up at 13-40 months adjusted age (Bayley Scales of Developmental Indices: MDI, PDI, blindness, sensorineural hearing loss, cerebral palsy, mortality) | **Main Phase:**<br>The chart review revealed that NO was initiated at 2-322 hours post-birth and continued for a mean duration of 20 hours (range: 3-130 hours). Oxygenation improved soon after NO was initiated with significant decreases from baseline in OI and increases in $PaO_2$ at 1 hour post-initiation and thereafter ($P<0.05$). $FiO_2$ and mean airway pressure gradually decreased and became significant at 4 and 24 hours post-initiation ($P<0.05$). No significant changes were noted in $PaCO_2$ or pH. No significant between-group differences were noted in baseline characteristics other than a modestly smaller birth weight in non-survivors ($P=0.08$). Six subjects received cranial ultrasounds prior to NO initiation, five of whom had normal results while one had Grade I IVH. Within 24 hours of starting NO, 17 had scans and one subject died prior to scanning. After 24 hours of NO, 5 subjects developed Grade III or IV IVH and were discontinued while Grade I or II was noted in 4 other subjects. At 48-72 hours after NO therapy, 3 of 12 subjects with normal ultrasounds at 24 hours developed Grade IV IVH and were subsequently discontinued on NO. Development or progression of IVH while receiving NO was suspected or documented in 15 of the 23 surviving subjects. Overall, 13 of 24 subjects (54%) died at some point during hospitalization (Grade IV IVH, n=10; intractable hypoxemia, n=2; and necrotizing enterocolitis, n=1). **Survivor Follow-up:** Of the 11 subjects who survived to discharge, one with spastic quadriplegia cerebral palsy died at 1 year chronologic age due to pneumonia. | Survivor Follow-up (continued): At 13-40 months adjusted age, 10 subjects underwent neurodevelopmental evaluation. Nine of ten were found to have a mean MDI of 81 ± 21 and mean PDI of 64 ± 22. Three of ten (30%) were developmentally normal, 2 (20%) had neurodevelopmental delay, and 5 (50%) were disabled. Of the survivors weighing ≤1,250 g at birth, 215 (96%) were evaluated at 12-36 months adjusted age and 29 (18%) were found to have neurodevelopmental disability (severe mental retardation, n=26 (12%); cerebral palsy, n=19 (9%); visual impairment, n=9 (4%); sensorineural hearing loss, n=4 (2%); and 2 or more disabilities, n=15 (7%). Four (40%) of the subjects had slow physical growth and 8 (80%) were diagnosed with bronchopulmonary dysplasia. Four of 10 survivors had recurrent wheezing episodes but did not require regular medications, while 1 child had recurrent pneumonia and 1 child required regular bronchodilators for chronic lung disease. Overall study mortality rate was high at 14 of 24 (58%).<br><br>Cheung and colleagues concluded that cranial ultrasonography should be recommended before, during, and after NO therapy. Further randomized, controlled studies were suggested to fully evaluate NO use in VLBW premature neonates due to the poor outcomes demonstrated in this study. |

| -Reference -Study Design & Objective -Subjects | Inclusion/Exclusion | Treatment | Outcomes | Results | Conclusion |
|---|---|---|---|---|---|
| LAUBSCHER ET AL, 1997[60]<br><br>A prospective study examining the response to three levels of NO used as rescue therapy in term and preterm infants.<br><br>N=30<br><br>Median gestational age 30 (range 24-42) weeks.; birth weight of 1396 g (range 548-4270) and postnatal age of 2 days (range 1-14). | **Inclusion:** Fully ventilated (rate >60 bpm) and required an inspired oxygen concentration ≥ 0.5, despite administration of surfactant replacement therapy (SRT) where relevant and had no evidence of a bleeding tendency or a low platelet count. If no improvement was seen or was deteriorating despite optimizing other therapies. **Exclusion:** ICH | NO via constant flow ventilator or high frequency oscillator at 10, 20, and 40 ppm each for 20 minutes. After each 20 min period, the NO was stopped for up to 2 min, with continuous oxygenation monitoring throughout. If a reduction in oxygenation occurred, NO was immediately restarted at the next level. | OI at three NO levels compared to baseline. | All three NO levels produced a significant reduction in OI. Overall maximum reduction in OI was a median change of 33% (range - 9%-90%); this didn't differ significantly between term and preterm. There was a significant response in the preterm infants at the 40 ppm dose when compared to the term infants ($P<0.01$). | Authors concluded that there was variability in the response to three levels of NO and that NO dosage should be individualized, and especially in very immature infants, levels up to 40 ppm should be considered. |

| -Reference -Study Design & Objective -Subjects | Inclusion/Exclusion | Treatment | Outcomes | Results | Conclusion |
|---|---|---|---|---|---|
| *SKIMMING ET AL, 1997*[81] A prospective, study to evaluate the effects of NO in preterm infants with RDS whose lungs were on MV and receiving supplemental oxygen therapy. N=23 NO 20ppm, n=12 NO 5ppm, n=11 | **Inclusion:** Less than 36 weeks gestation on supplemental oxygen therapy between ages 24 and 168 hours. Mean age: $28.0 \pm 0.6$ weeks, mean postnatal age during treatment was $49.8 \pm 8.1$ hours. Mean weight:1176 $\pm$ 130 gm. **Exclusion:** congenital cardiopulmonary anomaly, breathing without MV, undergoing ventilation by high-frequency modality, allowed to breathe less than 50% oxygen, hemodynamically unstable, treated without an arterial catheter positioned in either the descending aorta or a lower extremity artery. | All infants were treated with surfactant before receiving NO at least 2 hours before. NO of either 5 or 20 ppm, randomly assigned. Each treatment lasted at least 15 minutes. Following an equilibrium period, blood sampling and recording of hemodynamic variables, either 5 or 20 ppm NO was administered. The 15 minute equilibrium and data-gathering was then repeated. Then the NO was stopped and 15 minutes later the data was gathered again. | BP, HR, spontaneous respiratory rate (sRR), $PaCO_2$, $PaO_2$, $SaO_2$, systemically measured arterial oxyhemoglobin saturation. | Both concentrations of NO caused significant increases in systemically measured arterial oxyhemoglobin saturation, $PaO_2$, $SpO_2$, and a significant decrease in sRR. Eighteen of the infants who received nitric oxide were discharged from the hospital alive. | The authors conclude that NO can cause in increase in the blood oxygenation of premature infants and is relatively dose independent in the range of 5-20ppm. |

| -Reference -Study Design & Objective -Subjects | Inclusion/Exclusion | Treatment | Outcomes | Results | Conclusion |
|---|---|---|---|---|---|
| *SUBHEDAR, RYAN, SHAW, 1997*[82] An open, randomized controlled trial to determine whether treatment with NO and/or dexamethasone (DX) reduces the incidence of CLD and/or death in high risk preterm infants. Later treatment study, based on BPD risk[82] N=42 NO alone, n=10 DX alone, n=11 Both, n=10 Neither, n=11 | **Inclusion:** If at 96 hours of age 1) gestational age <32 weeks completed; 2) mechanically ventilated since birth for respiratory distress; 3) received surfactant therapy; 4) deemed to be at high risk of developing CLD. **Exclusion:** major congenital anomaly, structural cardiac defect or significant ductal shunting, culture positive sepsis, IVH with parenchymal involvement, pulmonary or gastrointestinal hemorrhage, disordered coagulation or thrombocytopenia. | Four treatment arms: NO alone starting at 20 ppm; NO plus DX (starting at 0.5 mg/kg/dose for 6 doses); Neither; and DX alone. Weaning for NO was according to response within the first 2 hours of treatment. The dose of DX was reduced if hypertension or hyperglycemia occurred and were resistant to treatment. | Primary: combined incidence of death before discharge and CLD. Secondary: time to successful extubation, duration of mechanical ventilation, length of hospital stay, and incidence of neonatal complications such as air leak, PDA requiring medical or surgical intervention, necrotizing enterocolitis, IVH, and sepsis. | No significant differences were seen between any of the treated groups when compared to the control groups with respect to incidence of death and CLD or with regard to any of the secondary outcome measures. | At 96 hours of age, neither treatment with NO nor DX prevented CLD or death. |

| -Reference -Study Design & Objective -Subjects | Inclusion/Exclusion | Treatment | Outcomes | Results | Conclusion |
|---|---|---|---|---|---|
| **SUBHEDAR & SHAW, 1997**[85]<br><br>As part of an open, randomized controlled trial,[82] this study evaluated changes in oxygenation, respiratory support, and pulmonary hemodynamics of NO in preterm infants.<br><br>N=42<br><br>NO, n=20<br>Control, n=22 | **Inclusion:** at 96 hours of age fulfilled criteria for CLD. **Exclusion:** Not defined. | All infants were given artificial surfactant (ALEC, Britannia Pharmaceuticals), those below 1 kg were treated with indomethacin, 0.1 mg/kg/dose at intervals of 12 hours, immediately after birth and for two further doses. NO doses were as described in the open, randomized controlled study above. | OI, FIO$_2$, echocardiographic examination, PAP, acceleration time to right ventricular time ration (AT:RVET), systolic PAP, right ventricular output. | Significant changes between the treated group and the control group were seen in MAP (higher in the control group until 30 minutes) $P$=0.04; OI reduction (greater in the treated group within 30 minutes) by 16.9%, $P$<0.01; early responders greater in the treated group 65 vs 18%, $P$<0.01; AT:RVET rapidly rose in treated group with a median rise of 0.04 within 30 minutes ($P$<0.01); both baseline median systolic PAP and in the first 30 minutes of the median change in systolic PAP (-4.6 range of -8.6 to -2.3 mmHg), were significantly different, compared to control, $P$=0.04 for (both baseline and first 30 minutes). | The authors conclude that as part of a controlled study, the treatment with NO rapidly improves oxygenation and lowers PAP in preterm infants. |
| **BENNETT ET AL, 2001**[84]<br><br>Reporting of the 30-month neurodevelopmental status of high-risk preterm infants originally studied in an open, randomized, controlled trial.<br><br>N=21<br><br>NO, n=10<br>Control, n=11 | **Inclusion:** <32 weeks gestation at 96 hours of age if met criteria for high risk of dying or developing CLD. **Exclusion:** periventricular hemorrhage and parenchymal involvement at trial entry | NO from 5-20 ppm for 72 h or until extubation. All infants were treated with artificial surfactant, MV and majority of them exposed to antenatal corticosteroids. Control infants received postnatal dexamethasone. | long-term mortality, neurodevelopmental delay (mild, moderate and severe mental developmental index (MDI) and psychomotor developmental index (PDI), cerebral palsy, sensorineural impairment and severe neurodisability. | No significant differences were found in long-term mortality, neurodevelopmental delay, severe neurodisability or cerebral palsy between treated and control infants. | NO did not have any significant effect on either survival or long-term neurodevelopmental status in high-risk preterm infants. |

| -Reference -Study Design & Objective -Subjects | Inclusion/Exclusion | Treatment | Outcomes | Results | Conclusion |
|---|---|---|---|---|---|
| **VAN MEURS ET AL, 1997**[100]<br><br>Study design not specified. Study was to determine whether premature infants with severe RDS would respond to NO with an improvement in oxygenation.<br><br>N=11<br><br>Mean gestational age 29.8 weeks, mean birth weight 1448 g. | **Inclusion:** Birth weight 750-2500 g; postnatal age 24-72 hours; more than 4 hours since final dose of surfactant; PaO$_2$ of 40-75 torr while on MV with FIO$_2$ ≥ 0.5 and MAP >10 cmH$_2$O. Clinical and radiographic evidence of severe RDS. **Exclusion:** vasodilator within 2 hours of study entry, significant congenital anomalies, including congenital heart disease, ICH >Grade1, platelet count >100,000 cmm; or a bleeding diathesis. | Four concentrations of NO treatment: 1, 5, 10, 20 with one placebo treatment at 0 ppm, each for 15 minutes followed by 15 min of no treatment. The lowest concentration that was found to give a positive response was the initiation dose and then if there was a ≥ 50% increase in the a/APO$_2$, NO was continued. If aAPO$_2$ ≥50% increase did not occur, higher doses were attempted up to a maximum of 20 ppm. If a ≥50% increase did not occur at any dosage, NO was discontinued. Weaning was attempted every 12 hours, with a 50% reduction in NO dosage. The maximum length of NO therapy was limited to 7 days. | Pre and post-treatment aAPO$_2$ ratios. | There were significant responses at all the concentration of NO ($P$<0.01), but none with the placebo dose. The responses to doses of 5, 10, and 20 ppm were significantly different from that of placebo by repeated measures ANOVA and the Tukey-Kramer post-hoc test ($P$<0.05).<br><br>An unexpected observation was the number of infants who eventually developed a severe ICH. Three infants had ICH noted on the first ultrasound scan after NO treatment and an additional four infants subsequently developed ICH. None of the infants had ICH at the beginning of the study. | The authors concluded that NO may be an effective method of improving oxygenation in infants with severe RDS. However, the incidence of ICH observed in this study needs to be carefully evaluated before considering routine use of NO in preterm infants. |

| -Reference -Study Design & Objective -Subjects | Inclusion/Exclusion | Treatment | Outcomes | Results | Conclusion |
|---|---|---|---|---|---|
| *PELIOWSKI ET AL, 1995*[83]<br><br>Observational study to evaluate the use of NO in premature infants unresponsive from conventional management, with prolonged rupture of membranes (PROM) and oligohydramnios. All infants had antepartum history of oligohydramnios and PROM for more than 3 days.<br><br>N=8<br><br>Birth weight between 520-1440 g and age between 24-31 weeks gestation. | **Inclusion:** Premature infants who were unresponsive to maximal therapy on oxygen via ventilation or high-frequency oscillation and surfactant therapy. MAP of ≥ 14 cm $H_2O$ and OI of 19 or more. **Exclusion:** unspecified. | Initially at dose of 20 ppm, followed by reduction by 5 ppm within 2 hours of administration based on tolerance. NO was continued until OI was <10.<br><br>Response was based on a $PaO_2$ ≥10 mmHg. Infants received the lowest dose of NO that showed improvement. Weaning attempts were made every 24 hours in 15 minute increments by 5 ppm and subsequently discontinued. If the $PaO_2$ fell by $PaO_2$ ≥ 10 mmHg, the higher dose was reinstated. If the OI fell to <10 during the weaning process, then NO was discontinued. | MAP, and OI (pre- and post-NO). | There were decreases in MAP and improvements in oxygenation demonstrated by decreases in OI in all infants, but none were significant.<br><br>Five of the eight infants survived. The causes of death were sepsis and IVH (infant 1); massive IVH with severe parenchymal involvement (infants 3 and 6).<br><br>NO was continued for an average of 47 hours with a range of 6 to 137 hours. | The authors conclude that this study suggests that NO can result in improvements in oxygenation, with the ability to reduce the MAP and thus decrease barotrauma. |

GA-gestational age; BW-birth weight; PMA-post-menstrual age; MV-mechanical ventilation; BPD- bronchopulmonary dysplasia; PAH-pulmonary arterial hypertension; PPHN- persistent pulmonary hypertension of the newborn; PAP- pulmonary artery pressure; PVR- pulmonary vascular resistance index; WU- Wood units; $aAPO_2$ – arterial-alveolar oxygen ratio; OI-oxygenation index; VI- ventilatory index; NCPAP- nasal continuous positive airway pressure; PH- pulmonary hypertension; PVL-periventricular leukomalacia; NEC- necrotizing enterocolitis; nCPAP- nasal continuous positive airway pressure; $R_L$- airway resistance; $C_L$ - lung compliance; LVEF-left ventricular ejection fraction; CLD- chronic lung disease; $SPO_2$ – oxygen saturation; IVH- intraventricular hemorrhage; MAP- mean airway pressure; $PaO_2$- partial pressure of arterial oxygen; TWOI- time weighted oxygenation index; SRT- surfactant replacement therapy.

## CLINICAL SAFETY

- Potential toxic effects associated with NO administration include methemoglobinemia, nitrogen dioxide ($NO_2$) formation, and rebound phenomenon.[101] Methemoglobin and $NO_2$ levels must be monitored during NO delivery.

- A number of studies have shown that inhaled nitric oxide may lead to an increase in left ventricular filling pressure and the occurrence of pulmonary edema in patients with left ventricular dysfunction; use caution when administering NO under those circumstances.[102,103]

- The post-marketing surveillance data notes that accidental exposure to NO in hospital staff has been associated with chest discomfort, dizziness, dry throat, dyspnea, and headache.[101]

Reference List

1. Hamilton BE, Martin JA, Ventura SJ. Births: Preliminary Data for 2009. http://www.cdc.gov/nchs/data/nvsr/nvsr59/nvsr59 03.pdf.Updated 2010. Accessed August 17,2011.

2. Mercier JC, Olivier P, Loron G, Fontaine R, Maury L, Baud O. Inhaled nitric oxide to prevent bronchopulmonary dysplasia in preterm neonates. Semin Fetal Neonatal Med. 2009;14(1):28-34.

3. Bhatt AJ, Pryhuber GS, Huyck H, Watkins RH, Metlay LA, Maniscalco WM. Disrupted pulmonary vasculature and decreased vascular endothelial growth factor, Flt-1, and TIE-2 in human infants dying with bronchopulmonary dysplasia. Am J Respir Crit Care Med. 2001 ;164(10):1971-1980.

4. Balasubramaniam V, Tang JR, Maxey A, Plopper CG, Abman SH. Mild hypoxia impairs alveolarization in the endothelial nitric oxide synthase-deficient mouse. Am J Physiol Lung Cell Mol Physiol. 2003;284(6):L964-L971.

5. Lin YJ, Markham NE, Balasubramaniam V, et al. Inhaled nitric oxide enhances distal lung growth after exposure to hyperoxia in neonatal rats. PediatrRes. 2005;58(1):22-29.

6. Tang JR, Markham NE, Lin YJ, et al. Inhaled nitric oxide attenuates pulmonary hypertension and improves lung growth in infant rats after neonatal treatment with a VEGF receptor inhibitor. Am J Physiol Lung Cell Mol Physiol. 2004;287(2):L344-L351.

7. McCurnin DC, Pierce RA, Chang LY, et al. Inhaled NO improves early pulmonary function and modifies lung growth and elastin deposition in a baboon model of neonatal chronic lung disease. Am J Physiol Lung Cell Mol Physiol. 2005;288(3):L450-L459.

8. Schreiber MD, Gin-Mestan K, Marks JD, Huo D, Lee G, Srisuparp P. Inhaled nitric oxide in premature infants with the respiratory distress syndrome. N Engl J Med. 2003;349(22):2099-2107.

9. Van Meurs KP, Rhine WD, Asselin JM, Durand DJ. Response of premature infants with severe respiratory failure to inhaled nitric oxide. Preemie NO Collaborative Group. Pediatr Pulmonol. 1997;24(5):319-323.

10. Van Meurs KP, Hintz SR, Ehrenkranz RA, et al. Inhaled nitric oxide in infants >1500 g and <34 weeks gestation with severe respiratory failure. J Perinatol. 2007;27(6):347-352.

11. Van Meurs KP, Wright LL, Ehrenkranz RA, et al. Inhaled nitric oxide for premature infants with severe respiratory failure. N Engl J Med. 2005;353(1):13-22.

12. Kinsella JP, Walsh WF, Bose CL, et al. Inhaled nitric oxide in premature neonates with severe hypoxaemic respiratory failure: a randomised controlled trial. Lancet. 1999;354(9184):1061-1065.

13. Ballard RA, Truog WE, Cnaan A, et al. Inhaled nitric oxide in preterm infants undergoing mechanical ventilation. N Engl J Med. 2006;355(4):343-353.

14. Ballard RA. Inhaled nitric oxide in preterm infants--correction. N Engl J Med. 2007;357(14):1444-1445.

15. Mercier JC, Hummler H, Durrmeyer X, et al. Inhaled nitric oxide for prevention of bronchopulmonary dysplasia in premature babies (EUNO): a randomised controlled trial. Lancet. 2010;376(9738):346-354.

16. Kinsella JP, Cutter GR, Walsh WF, et al. Early inhaled nitric oxide therapy in premature newborns with respiratory failure. N Engl J Med. 2006;355(4):1061-1065.

17. Field D, Elbourne D, Truesdale A, et al. Neonatal Ventilation With Inhaled Nitric Oxide Versus Ventilatory Support Without Inhaled Nitric Oxide for Preterm Infants With Severe Respiratory Failure: the INNOVO multicentre randomised controlled trial (ISRCTN 17821339). Pediatrics. 2005;115(4):926-936.

18. Hascoet JM, Fresson J, Claris O, et al. The safety and efficacy of nitric oxide therapy in premature infants. J Pediatr. 2005;146(3):318-323.

19. Mercier JC, The Franco-Belgium Collaborative NO,Trial Group. Early compared with delayed inhaled nitric oxide in moderately hypoxaemic neonates with respiratory failure: a randomised controlled trial. Lancet. 1999;354(9184):1066-1071.

20. Donohue PK, Gilmore MM, Cristofalo E, et al. Inhaled Nitric Oxide in Preterm Infants: A Systematic Review. Pediatrics. 2011.

21. Askie LM, Ballard RA, Cutter GR, et al. Inhaled Nitric Oxide in Preterm Infants: An Individual-Patient Data Meta-analysis of Randomized Trials. Pediatrics. 2011.

22. Barrington KJ, Finer N. Inhaled nitric oxide for respiratory failure in preterm infants. Cochrane Database Syst Rev. 2010;12:CD000509.

23. Hoehn T, Krause MF, Buhrer C. Meta-analysis of inhaled nitric oxide in premature infants: an update. Klin Padiatr.

2006;218(2):57-61.

24. Hoehn T, Krause MF, Buhrer C. Inhaled nitric oxide in premature infants--a meta-analysis. J Perinat Med. 2000;28(1):7-13.

25. Srisuparp P, Heitschmidt M, Schreiber MD. Inhaled nitric oxide therapy in premature infants with mild to moderate respiratory distress syndrome. J Med Assoc Thai. 2002;85 Suppl 2:S469-S478.

26. Watson RS, Clermont G, Kinsella JP, et al. Clinical and economic effects of iNO in premature newborns with respiratory failure at 1 year. Pediatrics. 2009;124(5):1333-1343.

27. Hibbs AM, Walsh-Sukys MC, Martin RJ, et al. One-year respiratory outcomes of preterm infants enrolled in the Nitric Oxide (to prevent) Chronic Lung Disease trial. J Pediatr. 2008;153(4):525-529.

28. Hoo AF, Beardsmore CS, Castle RA, et al. Respiratory function during infancy in survivors of the INNOVO trial. Pediatr Pulmonol. 2009;44(2):155-161.

29. Walsh-Sukys MC, Hibbs AM, Martin CR, et al. Two-year Neurodevelopmental Outcomes of Ventilated Preterm Infants Treated with Inhaled Nitric Oxide. J Pediatr. 2010;156(4):556-561.

30. Keller RL, Walsh-Sukys MC, Vittinghoff E, Palermo L, Ballard PL, Ballard RA.. Response to Inhaled Nitric Oxide (iNO) and Neurodevelopmental impairment (NDI) in NO CLD. Baltimore, MD:2009.

31. Kinsella JP, Cutter GR, Walsh WF, et al.. Outcomes of Premature Infants Enrolled in the Early Inhaled Nitric Oxide for the Prevention of Chronic Lung Disease Trial. Baltimore, MD:2009.

32. Hintz SR, Van Meurs KP, Perritt R, et al. Neurodevelopmental outcomes of premature infants with severe respiratory failure enrolled in a randomized controlled trial of inhaled nitric oxide. J Pediatr. 2007;151(1):16-22.

33. Mestan KK, Marks JD, Hecox K, Huo D, Schreiber MD. Neurodevelopmental outcomes of premature infants treated with inhaled nitric oxide. N Engl J Med. 2005;353(1):23-32.

34. Durrmeyer X, Hummler H, Sanchez-Luna M, et al. Two-Year Outcomes of a Randomized Controlled Trial of Inhaled Nitric Oxide in Premature Infants. Pediatrics. 2013.

35. Huddy CL, Bennett CC, Hardy P, et al. The INNOVO multicentre randomised controlled trial: Neonatal ventilation with Inhaled Nitric Oxide versus Ventilatory support without nitric oxide for severe respiratory failure in preterm infants: follow up at 4-5 years (ISRCTN 1782 1339). Arch Dis Child Fetal Neonatal Ed. 2008.

36. Patrianakos-Hoobler AI, Marks JD, Msall ME, Huo D, Schreiber MD. Safety and efficacy of inhaled nitric oxide treatment for premature infants with respiratory distress syndrome: follow-up evaluation at early school age. Acta Paediatr. 2011;100(4):524-528.

37. Hamon I, Gaga S, Espagne S, Merdariu D, Debruille C, Hascoet JM.. Clinical Outcome at 7 Years of Age in Former Premature Infants Treated at Birth with Inhaled Nitric Oxide (iNO). Baltimore, MD:2009.

38. Hamon I, Deforge H, Espagne S, Gaga S, Selton D, Hascoet JM.. Neurodevelopmental and Cognitive Outcomes at School Age of Premature Infants Treated at Birth with Inhaled Nitric Oxide (iNO). Baltimore, MD:2009.

39. Hascoet JM, Marchal F, Schweitzer C, Tankeu S, Hamon I. . Respiratory Outcome at 7 Years of Age in Former Sick Premature Infants Treated with Inhaled Nitric Oxide (iNO) at Birth. Baltimore, MD:2009.

40. Ballard PL, Truog WE, Merrill JD, et al. Plasma biomarkers of oxidative stress: relationship to lung disease and inhaled nitric oxide therapy in premature infants. Pediatrics. 2008;121(3):555-561.

41. Cooper S, Marks JD, Meisel M, White MK, Huo D, Schreiber MD. Birthweight distribution of severe IVH/PVL and death in premature infants treated with inhaled nitric oxide. Pediatr Res. 2011;70(4):AbsE-PAS20113835.358433.

42. Ambalavanan N, Van Meurs KP, Perritt R, et al. Predictors of death or bronchopulmonary dysplasia in preterm infants with respiratory failure. Journal of Perinatology: Official Journal of the California Perinatal Association. 2008;28(6):420-426.

43. Hintz S, Van Meurs KP, The NICHD Neonatal RN.. Reliability and Accuracy of Cranial Ultrasound in the NICHD Randomized Controlled Trial of Inhaled Nitrix Oxide for Premature Infants with Severe Respiratory Failure. San Francisco, CA:2006.

44. White M, Marks JD, Schreiber SA, Huo D, Msall ME and Schreiber MD. No Effect of Inhaled Nitric Oxide on IVH Extension in Premature Infants with Moderate RDS. 05/02, Poster E-PAS20102849.278.

45. Kaplan H, Lorch SA, Luan X, et al.. Role of Inhaled Nitric Oxide in Evolution of Brain Lesions in the Premature Infant. San Francisco, CA:2006.

46. Desandes R, Desandes E, Droulle P, Didier F, Longrois D, Hascoet JM. Inhaled nitric oxide improves oxygenation in very premature infants with low pulmonary blood flow. Acta Paediatr. 2004;93(1):66-69.

47. Truog WE, Ballard PL, Norberg M, et al. Inflammatory markers and mediators in tracheal fluid of premature infants treated with inhaled nitric oxide. Pediatrics. 2007;119(4):670-678.

48. Izhar FM, Rumilla K, Kim Y, Hershenson MB, Schreiber MD.. Inhaled Nitric Oxide Prevents the Increase in Tracheal Aspirate IL-8 Concentrations in Premature Newborn Infants with Respiratory Distress Syndrome. Baltimore, MD:2001.

49. Posencheg MA, Gow AJ, Truog WE, et al. Inhaled nitric oxide in premature infants: effect on tracheal aspirate and plasma nitric oxide metabolites. J Perinatol. 2010;30(4):275-280.

50. Ballard PL, Keller RL, Harmon C and Ballard RA. Urinary Nitric Oxide Metabolites in Preterm Infants with Lung Disease. 05/01, Poster E-PAS20101464.117.

51. Keller RL, Vittinghoff E, Palermo L, Ballard RA, Ballard PL, Truog W.. Response to Inhaled Nitric Oxide (iNO): Further analysis of the NO CLD Trial. Baltimore, MD:2009.

52. Ahluwalia J, Tooley J, Cheema I, et al. A dose response study of inhaled nitric oxide in hypoxic respiratory failure in preterm infants. Early Hum Dev. 2006;82(7):477-483.

53. Sokol GM, Van Meurs KP, The NICHD Neonatal RN.. Does Inhaled Nitric Oxide (iNO) Exhibit Dose Related Toxicity in the Premature Infant with Severe Respiratory Failure? San Francisco, CA:2006.

54. Chock VY, Van Meurs KP, Hintz SR, et al. Inhaled Nitric Oxide for Preterm Premature Rupture of Membranes, Oligohydramnios, and Pulmonary Hypoplasia. Am J Perinatol. 2009;26(4):317-322.

55. Di Fiore JM, Hibbs AM, Zadell AE, et al. The effect of inhaled nitric oxide on pulmonary function in preterm infants. J Perinatol. 2007;27(12):766-771.

56. Izhar FM, Rumilla K, Borg MJ, Kim Y, Hershenson MB, Schreiber MD.. Pulmonary Safety of Inhaled Nitric Oxide in Premature Newborn Infants with Respiratory Distress. Boston, MA:2000.

57. Truffert P, Llado-Paris J, Mercier JC, Dehan M, Breart G. Early inhaled nitric oxide in moderately hypoxemic preterm and term newborns with RDS: the RDS subgroup analysis of the Franco-Belgian iNO Randomized Trial. Eur J Pediatr. 2003;162(9):646-647.

58. Gordon PV, Aschner JL, The Preemie iNO Subcommittee on Behalf of the NICHD Neonatal,Research Network.. Can Inhaled Nitric Oxide (iNO) Alter the Natural History of Spontaneous Intestinal Perforations (SIP) in the ELBW Infant?: A Post Hoc Analysis of the NICHD Preemie iNO Study. San Francisco, CA:.

59. Ballard PL, Merrill JD, Truog WE, et al. Surfactant function and composition in premature infants treated with inhaled nitric oxide. Pediatrics. 2007;120(2):346-353.

60. Aikio O, Metsola J, Vuolteenaho R, Perhomaa M, Hallman M. Transient Defect in Nitric Oxide Generation after Rupture of Fetal Membranes and Responsiveness to Inhaled Nitric Oxide in Very Preterm Infants with Hypoxic Respiratory Failure. J Pediatr. 2012.

61. Sharma S, Palafoutas J and Ramasethu J. Inhaled nitric oxide (iNO) in management of pulmonary hypertension (PPHN) in preterm infants. Presented at: Pediatric Academic Societies and Asian Society for Pediatric Research Annual Meeting, April 30 to May 3, 2011, Denver, CO. Abstract E-PAS2011809. Accessed at: http://www.abstracts2view.com/pas/view.php?nu=PAS11L1 1791.

62. O'Donnell DC, Franta J and Miletin J. The use of Inhaled Nitric Oxide in Premature Infants with Premature Rupture of Membranes: A Case Series. Presented at: Neonatology, 2010, E-PAS2010563.

63. Dewhurst C, Ibrahim H, Gothberg S, Jonsson B, Subhedar N. Use of inhaled nitric oxide in the new born period: results from the European inhaled nitric oxide registry. Acta Paediatr. 2010.

64. Hopper AO, Ninnis JR, Mulla N, Peverini R, Terry M and Deming D. Persistent Pulmonary Hypertension in Very Low Birth Weight Infants: Role for Inhaled Nitric Oxide? 05/09, Abstract 3857.86.

65. Kumar VH, Hutchison AA, Lakshminrusimha S, Morin FC,III, Wynn RJ, Ryan RM. Characteristics of pulmonary hypertension in preterm neonates. J Perinatol. 2007;27(4):214-219.

66. Su PH, Chen JY. Inhaled nitric oxide in the management of preterm infants with severe respiratory failure. J Perinatal. 2008;28(2):112-116.

67. Khemani E, McElhinney DB, Rhein L, et al. Pulmonary artery hypertension in formerly premature infants with bronchopulmonary dysplasia: clinical features and outcomes in the surfactant era. Pediatrics. 2007;120(6):1260-1269.

68. Tanaka Y, Hayashi T, Kitajima H, Sumi K, Fujimura M. Inhaled nitric oxide therapy decreases the risk of cerebral palsy in preterm infants with persistent pulmonary hypertension of the newborn. Pediatrics. 2007;119(6):1159-1164.

69. Dani C, Bertini G. Inhaled nitric oxide for the treatment of preterm infants with respiratory distress syndrome. Neonatology. 2008;94(2):87-95.

70. Dani C, Bertini G, Pezzati M, Filippi L, Cecchi A, Rubaltelli FF. Inhaled nitric oxide in very preterm infants with severe respiratory distress syndrome. Acta Paediatr. 2006;95(9):1116-1123.

71. Lindwall R, Blennow M, Svensson M, et al. A pilot study of inhaled nitric oxide in preterm infants treated with nasal continuous positive airway pressure for respiratory distress syndrome. Intensive Care Med. 2005;31(7):959-964.

72. Uga N, Ishii T, Kawase Y, Arai H, Tada H. Nitric oxide inhalation therapy in very low-birthweight infants with hypoplastic lung due to oligohydramnios. Pediatr Int. 2004;46(1):10-14.

73. Athavale K, Claure N, D'Ugard C, Everett R, Swaminathan S, Bancalari E. Acute effects of inhaled nitric oxide on pulmonary and cardiac function in preterm infants with evolving bronchopulmonary dysplasia. J Perinatol. 2004;24(12):769-774.

74. Aikio O, Saarela T, Pokela ML, Hallman M. Nitric oxide treatment and acute pulmonary inflammatory response in very premature infants with intractable respiratory failure shortly after birth. Acta Paediatr. 2003;92(1):65-69.

75. Clark PL, Ekekezie II, Kaftan HA, Castor CA, Truog WE. Safety and efficacy of nitric oxide in chronic lung disease. Arch Dis Child Fetal Neonatal Ed. 2002;86(1):F41-F45.

76. Truog WE, Pallotto E, Clark P, et al. Interaction of endogenous endothelin-1 and inhaled nitric oxide in term and preterm infants. Clin Sci (Lond). 2002;103 Suppl 48:294S-297S.

77. Du LZ, Shi LP, Zhou BH, et al. Inhaled nitric oxide in preterm and term neonates with hypoxemic respiratory failure and persistent pulmonary hypertension. Acta Pharmacol Sin. 2002;23:69-73.

78. Banks BA, Seri I, Ischiropoulos H, Merrill J, Rychik J, Ballard RA. Changes in oxygenation with inhaled nitric oxide in severe bronchopulmonary dysplasia. Pediatrics. 1999;103(3):610-618.

79. Cheung PY, Peliowski A, Robertson CM. The outcome of very low birth weight neonates (</=1500 g) rescued by inhaled nitric oxide: neurodevelopment in early childhood. J Pediatr. 1998;133(6):735-739.

80. Laubscher B, Greenough A, Kavvadia V, Devane SP. Response to nitric oxide in term and preterm infants. Eur J Pediatr. 1997;156(8):639-642.

81. Skimming JW, Bender KA, Hutchison AA, Drummond WH. Nitric oxide inhalation in infants with respiratory distress syndrome. J Pediatr. 1997;130(2):225-230.

82. Subhedar NV, Ryan SW, Shaw NJ. Open randomised controlled trial of inhaled nitric oxide and early dexamethasone in high risk preterm infants. Arch Dis Child Fetal Neonatal Ed. 1997;77(3):F185-F190.

83. Peliowski A, Finer NN, Etches PC, Tierney AJ, Ryan CA. Inhaled nitric oxide for premature infants after prolonged rupture of the membranes. J Pediatr. 1995;126(3):450-453.

84. Bennett AJ, Shaw NJ, Gregg JE, Subhedar NV. Neurodevelopmental outcome in high-risk preterm infants treated with inhaled nitric oxide. Acta Paediatr. 2001 ;90(5):573-576.

85. Subhedar NV, Shaw NJ. Changes in oxygenation and pulmonary haemodynamics in preterm infants treated with inhaled nitric oxide. Arch Dis Child Fetal Neonatal Ed. 1997;77(3):F191-F197.

86. Cole FS, Alleyne C, Barks JD, et al. NIH Consensus Development Conference Statement: Inhaled Nitric-Oxide Therapy for Premature Infants. Pediatrics. 2011.

87. Abman SH, Hansmann G, Archer SL, et al. Pediatric Pulmonary Hypertension: Guidelines From the American Heart Association and American Thoracic Society. Circulation. 2015. doi: CIR.0000000000000329 [pii].

88. Truog WE, Nelin LD, Das A, et al. Inhaled nitric oxide usage in preterm infants in the NICHD neonatal research network: inter-site variation and propensity evaluation. J Perinatol. 2014.

89. Kumar P, Committee on Fetus and Newborn, American Academy of Pediatrics. Use of inhaled nitric oxide in preterm infants. Pediatrics. 2014;133(1):164-170.

90. Ballard PL, Gonzales LW, Godinez RI, et al. Surfactant composition and function in a primate model of infant chronic lung disease: effects of inhaled nitric oxide. Pediatr Res. 2006;59(1):157-162.

91. Schreiber MD, Marks JD, Ballard R, et al. Response to Kumar on behalf of the AAP Committee on Fetus and Newborn. Pediatrics [Replies to Use of Inhaled Nitric Oxide in Preterm Infants]. 2014;133(1):164-170.

92. Barrington KJ, Finer NN. Inhaled nitric oxide for respiratory failure in preterm infants. Cochrane Database Syst Rev. 2009(1):CD000509.

93. Hamon I, Fresson J, Nicolas MB, Buchweiller MC, Franck P, Hascoet JM. Early inhaled nitric oxide improves oxidative balance in very preterm infants. Pediatr Res. 2005;57(5):637-643.

94. Van Meurs KP, Stevenson DK, Ehrenkranz RA, et al.. Inhaled Nitric Oxide for Preterm Infants with Severe Respiratory Failure. San Francisco, CA:2004.

95. Van Meurs KP, Benitz W, Hintz S, Bender RH, Poole K, for the NICHD Neonatal,Research Network.. Does Oxygenation Index Correlate with Outcome in Premature Infants with Respiratory Failure? Washington, DC:2005.

96. Finer NN. Inhaled nitric oxide for preterm infants: a therapy in search of an indication? The search continues. J Pediatr. 2005;146(3):301-303.

97. Kumar A, Zarychanski R, Pinto R, et al. Critically III Patients With 2009 Influenza A(H1N1) Infection in Canada. JAMA. 2009;302(17):1872-1879.

98. Davidson D, Barefield ES, Kattwinkel J, et al. Inhaled nitric oxide for the early treatment of persistent pulmonary hypertension of the term newborn: a randomized, double-masked, placebo-controlled, dose-response, multicenter study. The I-NO/PPHN Study Group. Pediatrics. 1998;101(3):325-334.

99. Yadav M, Emmerson AJ. Inhaled nitric oxide in premature neonates. Lancet. 1999;354(9196):2162-2163.

100. Konduri GG, Vohr B, Robertson C, et al. Early inhaled nitric oxide therapy for term and near-term newborn infants with hypoxic respiratory failure: neurodevelopmental follow-up. J Pediatr. 2007;150(3):235-40, 240.

101. INOmax® (nitric oxide) for inhalation 100 and 800 ppm (parts per million) [package insert]. Hampton, NJ:INO Therapeutics;2013.

102. Loh E, Stamler JS, Hare JM, Loscalzo J, Colucci WS. Cardiovascular effects of inhaled nitric oxide in patients with left ventricular dysfunction. Circulation. 1994;90(6):2780-2785.

103. Barst RJ, Agnoletti G, Fraisse A, Baldassarre J, Wessel DL. Vasodilator Testing with Nitric Oxide and/or Oxygen in Pediatric Pulmonary Hypertension. Pediatr Cardiol. 2010.

**Claims**

1. A system for reducing the risk of right ventricular failure during left ventricular assist device (LVAD) use, the system comprising:

   a means for measuring one or more pulmonary hemodynamic parameters of a patient with an LVAD;
   a nitric oxide delivery device; and
   a control system (240) configured to:

   receive a first pulmonary hemodynamic value from the means for measuring one or more pulmonary hemodynamic parameters;
   prompt the nitric oxide delivery device to administer inhaled nitric oxide to the patient with the LVAD;
   receive a second pulmonary hemodynamic value from the means for measuring one or more pulmonary hemodynamic parameters; and
   adjust the inhaled nitric oxide based on a comparison of the second pulmonary hemodynamic value to a predetermined range, and
   wherein the pulmonary hemodynamic parameter is selected from left atrial pressure and pulmonary capillary wedge pressure.

2. The system of claim 1, wherein the system is configured to administer inhaled nitric oxide at a concentration of 5 to 80 ppm for at least 10 minutes.

3. The system of claim 1, wherein the control system is further configured to:
   place the patient on a heart transplant list if the increase in left atrial pressure and/or pulmonary capillary wedge pressure from the first pulmonary hemodynamic value to the second pulmonary hemodynamic value is less than 5 mm Hg.

4. The system of claim 1, wherein the system is configured to administer inhaled nitric oxide at a concentration of 5 to 80 ppm for a time period in the range from 5 to 30 minutes, the administering being performed between one and five times per day.

5. The system of claim 1, comprising means for measuring one or more additional pulmonary hemodynamic parameters, the one or more additional pulmonary hemodynamic parameters being selected from cardiac output (CO), mean pulmonary artery pressure (mPAP), transpulmonary gradient (TPG) and pulmonary vascular resistance (PVR).

6. The system of claim 1, wherein the means for measuring one or more pulmonary hemodynamic parameters is a right heart catheter.

**7.** The system of claim 1, wherein the system is configured to administer the inhaled nitric oxide for at least 12 hours a day for at least 20 days.

**8.** The system of claim 1, wherein the control system is further configured to provide an alert if the one or more pulmonary hemodynamic parameters are outside of the predetermined range.

**Patentansprüche**

**1.** System zum Reduzieren des Risikos eines rechtsventrikulären Versagens während der Verwendung eines linksventrikulären Unterstützungssystems (LVAD), wobei das System umfasst:

ein Mittel zum Messen eines oder mehrerer pulmonaler hämodynamischer Parameter eines Patienten mit einem LVAD;
eine Vorrichtung zur Abgabe von Stickstoffmonoxid; und
ein Steuersystem (240), das ausgebildet ist:

einen ersten pulmonalen hämodynamischen Wert von dem Mittel zum Messen eines oder mehrerer pulmonaler hämodynamischer Parameter zu empfangen;
die Vorrichtung zur Abgabe von Stickstoffmonoxid aufzufordern, dem Patienten mit dem LVAD inhalatives Stickstoffmonoxid zu verabreichen;
einen zweiten pulmonalen hämodynamischen Wert von dem Mittel zum Messen eines oder mehrerer pulmonaler hämodynamischer Parameter zu empfangen; und
das inhalative Stickstoffmonoxid auf der Grundlage eines Vergleichs des zweiten pulmonalen hämodynamischen Werts mit einem vorgegebenen Bereich anzupassen, und
wobei der pulmonale hämodynamische Parameter aus dem linken Vorhofdruck und dem Lungenkapillarenverschlussdruck ausgewählt wird.

**2.** System nach Anspruch 1, wobei das System ausgebildet ist, inhalatives Stickstoffmonoxid in einer Konzentration von 5 bis 80 ppm für mindestens 10 Minuten zu verabreichen.

**3.** System nach Anspruch 1, wobei das Steuersystem ferner ausgebildet ist:
den Patienten auf eine Herztransplantationsliste zu setzen, wenn der Anstieg des linken Vorhofdrucks und/oder des Lungenkapillarenverschlussdrucks vom ersten pulmonalen hämodynamischen Wert zum zweiten pulmonalen hämodynamischen Wert weniger als 5 mm Hg beträgt.

**4.** System nach Anspruch 1, wobei das System ausgebildet ist, inhalatives Stickstoffmonoxid in einer Konzentration von 5 bis 80 ppm über einen Zeitraum im Bereich von 5 bis 30 Minuten zu verabreichen, wobei die Verabreichung zwischen ein- und fünfmal pro Tag durchgeführt wird.

**5.** System nach Anspruch 1, umfassend Mittel zum Messen eines oder mehrerer zusätzlicher pulmonaler hämodynamischer Parameter, wobei der eine oder die mehreren zusätzlichen pulmonalen hämodynamischen Parameter aus Herzzeitvolumen (CO), mittlerem Pulmonalarteriendruck (mPAP), transpulmonalem Gradienten (TPG) und pulmonalem Gefäßwiderstand (PVR) ausgewählt sind.

**6.** System nach Anspruch 1, wobei das Mittel zum Messen eines oder mehrerer pulmonaler hämodynamischer Parameter ein Rechtsherzkatheter ist.

**7.** System nach Anspruch 1, wobei das System ausgebildet ist, das inhalatives Stickstoffmonoxid mindestens 12 Stunden pro Tag über mindestens 20 Tage zu verabreichen.

**8.** System nach Anspruch 1, wobei das Steuersystem ferner ausgebildet ist, einen Alarm auszugeben, wenn der eine oder die mehreren pulmonalen hämodynamischen Parameter außerhalb des vorgegebenen Bereichs liegen.

**Revendications**

1. Système pour réduire le risque de défaillance ventriculaire droite au cours de l'utilisation d'un dispositif d'assistance ventriculaire gauche (LVAD), le système comprenant :

   un moyen pour mesurer un ou plusieurs paramètres hémodynamiques pulmonaires d'un patient avec un LVAD ;
   un dispositif de distribution d'oxyde nitrique ; et
   un système de commande (240) configuré pour :

   recevoir une première valeur hémodynamique pulmonaire du moyen pour mesurer un ou plusieurs paramètres hémodynamiques pulmonaires ;
   inviter le dispositif de distribution d'oxyde nitrique à administrer de l'oxyde nitrique inhalé au patient avec le LVAD ;
   recevoir une deuxième valeur hémodynamique pulmonaire du moyen pour mesurer un ou plusieurs paramètres hémodynamiques pulmonaires ; et
   ajuster l'oxyde nitrique inhalé sur la base d'une comparaison de la deuxième valeur hémodynamique pulmonaire à une plage prédéterminée ; et
   dans lequel le paramètre hémodynamique pulmonaire est sélectionné parmi la pression atriale gauche et la pression capillaire pulmonaire bloquée.

2. Système selon la revendication 1, dans lequel le système est configuré pour administrer de l'oxyde nitrique inhalé à une concentration de 5 à 80 ppm pendant au moins 10 minutes.

3. Système selon la revendication 1, dans lequel le système de commande est en outre configuré pour :
   placer le patient sur une liste de transplantation cardiaque si l'augmentation de la pression atriale gauche et/ou de la pression capillaire pulmonaire bloquée de la première valeur hémodynamique pulmonaire à la deuxième valeur hémodynamique pulmonaire est inférieure à 5 mm Hg.

4. Système selon la revendication 1, dans lequel le système est configuré pour administrer de l'oxyde nitrique inhalé à une concentration de 5 à 80 ppm pendant une période de temps dans la plage de 5 à 30 minutes, l'administration étant réalisée entre une et cinq fois par jour.

5. Système selon la revendication 1, comprenant des moyens pour mesurer un ou plusieurs paramètres hémodynamiques pulmonaires supplémentaires, l'un ou plusieurs paramètres hémodynamiques pulmonaires supplémentaires étant sélectionnés parmi le débit cardiaque (CO), la pression artérielle pulmonaire moyenne (mPAP), le gradient transpulmonaire (TPG) et la résistance vasculaire pulmonaire (PVR).

6. Système selon la revendication 1, dans lequel le moyen pour mesurer un ou plusieurs paramètres hémodynamiques pulmonaires est un cathétérisme cardiaque droit.

7. Système selon la revendication 1, dans lequel le système est configuré pour administrer l'oxyde nitrique inhalé pendant au moins 12 heures par jour pendant au moins 20 jours.

8. Système selon la revendication 1, dans lequel le système de commande est en outre configuré pour fournir une alerte si l'un ou plusieurs paramètres hémodynamiques pulmonaires sont en dehors de la plage prédéterminée.

FIG. 1

EP 3 970 730 B1

FIG. 2

BATTERY
PACK

BATTERY
PACK

LVAD          INO Delivery System

FIG. 3

FIG. 4

EP 3 970 730 B1

EP 3 970 730 B1

| Possible Nitric Oxide Delivery System Output Parameters |
| :---: |
| Respiratory Rate<br>Current NO Delivery Rate<br>Adverse Event Status |

| Possible LVAD Output Parameters |
| :---: |
| Pump Power<br>Patient Heart Rate<br>Blood Flow (through Pump)<br>Pulsatility Index<br>LVAD Pump Speed<br>Adverse Event Status |

| Control System |
| :---: |
| Therapeutic Algorithms |

| Nitric Oxide Delivery System Input Parameters |
| :---: |
| Reset Nitric Oxide Delivery Rate |

| LVAD Input Parameters |
| :---: |
| Reset LVAD Pump Speed |

## FIG. 5

MAIN MENU

| MODE SELECTION | ALARM SETTINGS | CONFIGURATION SETTINGS |
|---|---|---|

| MODE 1: PH Resolution Likelihood (and Heart Transplant Viability) |
| MODE 2: LVAD Settings Optimization |
| MODE 3: Right Ventricular Function Optimization During LVAD Use |
| MODE 4: Left Ventricular Functional Assessment |
| MODE 5: Heart Exercise |
| MODE 6: NO System / LVAD System Integration Controller Setup |

FIG. 6

EP 3 970 730 B1

EP 3 970 730 B1

**MAIN MENU**

| MODE SELECTION | ALARM SETTINGS | CONFIGURATION SETTINGS |
|---|---|---|

| Nitric Oxide System Alarm Settings |
|---|
| LVAD Alarm Settings |

**FIG. 7**

MAIN MENU

| MODE SELECTION | ALARM SETTINGS | CONFIGURATION SETTINGS |
| --- | --- | --- |
| | | Nitric Oxide System Settings |
| | | LVAD System Settings |

FIG. 8

EP 3 970 730 B1

## SOFTWARE FLOW CHART

PH Resolution Likelihood
(and Heart Transplant Viability)
Menu Topic Selected in Main Menu

↓

PH Resolution SubMenu Displayed

↓

PRE-Nitric Oxide Administration
Hemodynamic Parameters
Entry Fields Populated by User
Including:
Mean Pulmonary Arterial Pressure
Pulmonary Capillary Wedge Pressure
Transpulmonary Gradient
Pulmonary Vascular Resistance

↓

"Continue" Button Appears Once
Acceptable Hemodynamic Parameter
or Parameters Combination Populated

↓

Clinician Prompted to Initiate Nitric Oxide
Administration at a Particular (PPM) Dose

↓

"Continue" Button to be Pressed
Upon Initiation of NO Therapy

↓

Therapy Duration Timing
Countdown Screen Appears

↓

POST-Nitric Oxide Administration
Hemodynamic Parameters
Entry Fields Populated by User
Inclusive of:
Mean Pulmonary Arterial Pressure
Pulmonary Capillary Wedge Pressure
Transpulmonary Gradient
Pulmonary Vascular Resistance

↓

PRE and POST Hemodynamic Parameter
Variation Displayed, Therapeutic
Recommendations Displayed

## FIG. 9

## SOFTWARE FLOW CHART

```
┌─────────────────────────────────────────┐
│          LVAD Settings Optimization        │
│        Menu Topic Selected in Main Menu    │
└─────────────────────────────────────────┘
                    │
                    ▼
┌─────────────────────────────────────────┐
│      LVAD Settings Optimization SubMenu    │
│                 Displayed                  │
└─────────────────────────────────────────┘
                    │
                    ▼
┌─────────────────────────────────────────┐
│       Perform Baseline Echocardiogram      │
│         Without Nitric Oxide Onboard       │
└─────────────────────────────────────────┘
                    │
                    ▼
┌─────────────────────────────────────────┐
│   Adjust Nitric Oxide Dose to a Reasonable │
│      Therapeutic Dose (e.g. 20 ppm)        │
└─────────────────────────────────────────┘
                    │
                    ▼
┌─────────────────────────────────────────┐
│          Adjust Pump Flow Upwards          │
│            by X (e g. 0.2) LPM             │
└─────────────────────────────────────────┘
                    │
                    ▼
┌─────────────────────────────────────────┐
│    After X (e.g. 10) minutes, Perform      │
│        Intermediate Echocardiogram         │
└─────────────────────────────────────────┘
                    │
                    ▼
         ╱───────────────────────╲
   No   ╱  Does Echocardiogram Show ╲
◄──────╱      Flattening of Septum?    ╲
        ╲                              ╱
         ╲───────────────────────╱
                    │ Yes
                    ▼
┌─────────────────────────────────────────┐
│      Record Nitric Oxide Dose and Pump     │
│      Speed at which Flattening Occurs      │
└─────────────────────────────────────────┘
                    │
                    ▼
         ╱───────────────────────╲
   No   ╱    Is Pump Speed NO Dose  ╲
◄──────╱  Combination an Acceptable Upper╲
        ╲           Limit?             ╱
         ╲───────────────────────╱
                    │ Yes
                    ▼
┌─────────────────────────────────────────┐
│    Record Pump Speed and Nitric Oxide Dose │
│          as "Upper Pump Speed Limit"       │
└─────────────────────────────────────────┘
                    │
                    ▼
                  ( A )
```

## FIG. 10

```
        (A)
         │
         ▼
┌─────────────────────────────┐
│  Ensure Nitric Oxide Dose   │
│  Remains at Previously      │
│  Defined Setting            │
└─────────────────────────────┘
         │
         ▼
┌─────────────────────────────┐
│  Adjust Pump Setting        │
│  Downward by X (e.g. 0.2)   │
│  LPM                        │
└─────────────────────────────┘
         │
         ▼
┌─────────────────────────────┐
│  After X (e.g. 10) minutes, │
│  Perform Intermediate       │
│  Echocardiogram             │
└─────────────────────────────┘
         │
         ▼
    Does Echocardiogram Show
    Opening of Aortic Valve?  ── No ──┐
         │ Yes
         ▼
┌─────────────────────────────┐
│  Record Nitric Oxide Dose   │
│  and Pump Speed at which    │
│  Opening Occurs             │
└─────────────────────────────┘
         │
         ▼
    Is Pump Speed NO Dose
    Combination an Acceptable  ── No ──┐
    Lower Limit?
         │ Yes
         ▼
┌─────────────────────────────┐
│  Record Pump Speed and      │
│  Nitric Oxide Dose as       │
│  "Lower Pump Speed Limit"   │
└─────────────────────────────┘
```

## FIG. 10 (Continued)

## SOFTWARE FLOW CHART

```
┌─────────────────────────────────────┐
│   Right Ventricular Function         │
│   Optimization During LVAD Use       │
│   Menu Topic Selected in Main Menu   │
└─────────────────────────────────────┘
                 │
                 ▼
┌─────────────────────────────────────┐
│  Right Ventricular Function Optimization │
│  During LVAD Use SubMenu Displayed   │
└─────────────────────────────────────┘
                 │
                 ▼
```

**INITIAL SETTINGS**

```
┌─────────────────────────────────────┐
│  Set, Enter (if Already Set) or Modify │
│  Concentration of Nitric Oxide Dose  │
└─────────────────────────────────────┘
                 │
                 ▼
┌─────────────────────────────────────┐
│  Set, Enter (if Already Set) or Modify │
│  Number of hours of NO Per Day       │
│  and Hour Range for Dosing           │
└─────────────────────────────────────┘
                 │
                 ▼
┌─────────────────────────────────────┐
│  Set, Enter (if Already Set) or Modify the │
│  Pump Functional Parameters (e.g. Pump │
│  Speed/Flow, Pulsatility Index, Pump Power) │
└─────────────────────────────────────┘
                 │
                 ▼
```

**CONFIRM HEMODYNAMIC PARAMETERS ACCEPTABLE**

```
┌─────────────────────────────────────┐
│  Physiologic Parameters Available are │
│  Displayed e.g. Heart Rate, Breath Rate │
└─────────────────────────────────────┘
                 │
                 ▼
        ╱────────────────────╲
       ╱  Are Measured Hemodynamic ╲───── No
       ╲  Parameters Acceptable?    ╱
        ╲────────────────────╱
                 │ Yes
```

Note: Hemodynamic Variables may be Obtained from any Number of Clinical Sources

**THERAPEUTIC GOALS**

```
┌─────────────────────────────────────┐
│     Set the LVAD Operational Limits  │
└─────────────────────────────────────┘
                 │
                 ▼
┌─────────────────────────────────────┐
│  Set the Nitric Oxide Delivery Operational Limits │
└─────────────────────────────────────┘
                 │
                 ▼
                ( A )
```

## FIG. 11

SET THE ALARM MODE { Set Alarm to Audible, Tactile, Visual, or Text Messaging

CONTROL SYSTEM SETTINGS {
- Select the Controller Input/Output Variables
- Select the Input/Output Transfer Function (from a Selectable List)
- Note: The Controller is Implemented as any Type of Linear or Nonlinear Controller which Accomplishes the Input/Outpul Transfer Function Specified by the User and With the Dynamic Performance Specified by the User.
- Select the Desired Controller Dynamic Performance (e.g. $T_{10}$-$T_{90}$ Time)

RETURN TO MAIN MENU { Implement the Settings and RETURN to Main Menu

## FIG. 11 (Continued)

SOFTWARE FLOW CHART

Left Ventricle Functional Assessment
Menu Topic Selected in Main Menu

↓

Left Ventricle Functional Assessment
SubMenu Displayed

↓

NO DOSE { Ensure Nitric Oxide Dose at Appropriate Level
(NO Dose Placed in Entry Field)

↓

Duration of Assessment Set

↓

PRE PUMP SETTING
REDUCTION HEMODYNAMIC
VALUES { Clinician Entry of Hemodynamic Values
(e.g. LAP, PCWP,CO etc) — Note: The Hemodynamics
Measurements can be taken from
a Number of Clinical Sources

↓

PUMP SETTING REDUCTION { Pump Speed Entry Field and Start Button
to Reduce or Stop Pump (and NO) at
Clinician Specified Settings

↓

POST PUMP SETTING
REDUCTION HEMODYNAMIC
VALUES { Clinician Entry of Hemodynamic Values
(e.g. LAP, PCWP,CO etc) — Note: The Hemodynamics
Measurements can be taken from
a Number of Clinical Sources

↓

( A )

FIG. 12

A

CLINICAL"RULES SET"
APPLIED FOR ASSESSMENT
OF LEFT VENTRICLE HEALTH

Use of Predefined Clinically Derived
"Rules Set" to Determine, Based on the
Difference Between Pre and Post Pump
Adjustment Hemodynamic Values, if the
Left Ventricle is Improving

Reset the LVAD and NO to Original
Settings and Return to Main Menu

FIG. 12 (Continued)

## SOFTWARE FLOW CHART

```
┌─────────────────────────────────────────┐
│           Heart Exercise                 │
│   Menu Topic Selected in Main Menu       │
└─────────────────────────────────────────┘
                    │
                    ▼
┌─────────────────────────────────────────┐
│           Heart Exercise                 │
│         SubMenu Displayed                │
└─────────────────────────────────────────┘
                    │
                    ▼
┌─────────────────────────────────────────┐
│  Current Pump Speed and NO Dose Displayed│    Note: Modified Alarm Thresholds
│  Clinician Entry Fields for Pump Speed and│   might also be Included in this
│  NO Dose Modulation Values, Therapy Duration│  Settings Screen
│  and Repetition Count Values, and Number of│
│           Times Per Day                  │
└─────────────────────────────────────────┘
                    │
                    ▼
┌─────────────────────────────────────────┐
│      Heart Exercise Initiation Button    │
└─────────────────────────────────────────┘
                    │
                    ▼
┌─────────────────────────────────────────┐
│    Run Heart Exercises with the Previously│
│  Set Repetition Value at the Number of Times│
│           Per Day Specified              │
└─────────────────────────────────────────┘
                    │
                    ▼
┌─────────────────────────────────────────┐
│      (Optional) Display LVAD and NO      │
│         System Parameters                │
└─────────────────────────────────────────┘
                    │
                    ▼
┌─────────────────────────────────────────┐
│         Return to Main Menu              │
└─────────────────────────────────────────┘
```

## FIG. 13

SOFTWARE FLOW CHART

| Global Controller Setup Menu Topic Selected in Main Menu |
| --- |

↓

| Global Controller Setup SubMenu Displayed |
| --- |

↓

Target Output Parameter Values {

| LVAD Pump Speed Target Value (and/or Operational Envelope) Specified NO System Dose Target Value (and/or Operational Envelope) Specified |
| --- |

↓

Input Controller Parameters As Specified by User {

| Check List of Input Parameters for Use in Controller Including: Respiratory Rate, Current NO Delivery Rate, LVAD Pump Power, Heart Rate, Current Pump Flow Setting, Pulsatility Index, or User Specified Hemodynamic Parameters |
| --- |

↓

Input/Output Transfer Function as Selected from Selectable List {

| Transfer Function Input vs. Output Mathematical Relationship Specified. (Lists of User Selectable Mathematical Relationships Made Available to User). Example: Output Parameter / Input Parameter = 1.2 + 0.02t |
| --- |

↓

( A )

FIG. 14

EP 3 970 730 B1

(A)

Input/Output Dynamic Relationship Specified by User

Dynamic Relationship Specified by User Example: Input Parameter Step Change Yields 90% Change in Output Parameter in X (User Specified) Minutes

Nitric Oxide Delivery Scheme

Set Nitric Oxide Delivery Scheme. The Scheme can Include Continuous NO Delivery or Pulsed NO Delivery with the Number of Inspiratory Pulses and Timing to be Set in this Screen

NOTE: Periodic Adjustments of Pulsing Scheme, if Selected, may be Warranted Pending Feedback from RHI or FMD Testing

Universality of Controller Operation Specified

Checklist (User specified) of Modes in which this Control System Behavior is Active

Return to Main Menu; Display Settings in Main Menu

FIG. 14 (Continued)

EP 3 970 730 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5396882 A
- US 7560076 B
- US 5558083 A
- US 7523752 B
- US 8573209 B
- US 8573210 B
- US 8770199 B
- US 20140283828

### Non-patent literature cited in the description

- **ENDO GEORGE J et al.** Nitric oxide inhalation prompts weaning from the right ventricular assist device: Evaluation under continuous-flow biventricular assistance. *JOURNAL OF THORACIC AND CARDIOVASCULAR SURGERY,* 10 February 2002, vol. 124 (4
- **HAMILTON BE ; MARTIN JA ; VENTURA SJ.** *Births: Preliminary Data,* 2009, http://www.cdc.gov/nchs/data/nvsr/nvsr59/nvsr59 03.pdf
- **MERCIER JC ; OLIVIER P ; LORON G ; FONTAINE R ; MAURY L ; BAUD O.** Inhaled nitric oxide to prevent bronchopulmonary dysplasia in preterm neonates. *Semin Fetal Neonatal Med,* 2009, vol. 14 (1), 28-34
- **BHATT AJ ; PRYHUBER GS ; HUYCK H ; WATKINS RH ; METLAY LA ; MANISCALCO WM.** Disrupted pulmonary vasculature and decreased vascular endothelial growth factor, Flt-1, and TIE-2 in human infants dying with bronchopulmonary dysplasia. *Am J Respir Crit Care Med,* 2001, vol. 164 (10), 1971-1980
- **BALASUBRAMANIAM V ; TANG JR ; MAXEY A ; PLOPPER CG ; ABMAN SH.** Mild hypoxia impairs alveolarization in the endothelial nitric oxide synthase-deficient mouse. *Am J Physiol Lung Cell Mol Physiol.,* 2003, vol. 284 (6), L964-L971
- **LIN YJ ; MARKHAM NE ; BALASUBRAMANIAM V et al.** Inhaled nitric oxide enhances distal lung growth after exposure to hyperoxia in neonatal rats. *PediatrRes.,* 2005, vol. 58 (1), 22-29
- **TANG JR ; MARKHAM NE ; LIN YJ et al.** Inhaled nitric oxide attenuates pulmonary hypertension and improves lung growth in infant rats after neonatal treatment with a VEGF receptor inhibitor. *Am J Physiol Lung Cell Mol Physiol.,* 2004, vol. 287 (2), L344-L351

- **MCCURNIN DC ; PIERCE RA ; CHANG LY et al.** Inhaled NO improves early pulmonary function and modifies lung growth and elastin deposition in a baboon model of neonatal chronic lung disease. *Am J Physiol Lung Cell Mol Physiol.,* 2005, vol. 288 (3), L450-L459
- **SCHREIBER MD ; GIN-MESTAN K ; MARKS JD ; HUO D ; LEE G ; SRISUPARP P.** Inhaled nitric oxide in premature infants with the respiratory distress syndrome. *N Engl J Med,* 2003, vol. 349 (22), 2099-2107
- **VAN MEURS KP ; RHINE WD ; ASSELIN JM ; DURAND DJ.** Response of premature infants with severe respiratory failure to inhaled nitric oxide. Preemie NO Collaborative Group. *Pediatr Pulmonol,* 1997, vol. 24 (5), 319-323
- **VAN MEURS KP ; HINTZ SR ; EHRENKRANZ RA et al.** Inhaled nitric oxide in infants >1500 g and <34 weeks gestation with severe respiratory failure. *J Perinatol,* 2007, vol. 27 (6), 347-352
- **VAN MEURS KP ; WRIGHT LL ; EHRENKRANZ RA et al.** Inhaled nitric oxide for premature infants with severe respiratory failure. *N Engl J Med,* 2005, vol. 353 (1), 13-22
- **KINSELLA JP ; WALSH WF ; BOSE CL et al.** Inhaled nitric oxide in premature neonates with severe hypoxaemic respiratory failure: a randomised controlled trial. *Lancet,* 1999, vol. 354 (9184), 1061-1065
- **BALLARD RA ; TRUOG WE ; CNAAN A et al.** Inhaled nitric oxide in preterm infants undergoing mechanical ventilation. *N Engl J Med,* 2006, vol. 355 (4), 343-353
- **BALLARD RA.** Inhaled nitric oxide in preterm infants--correction. *N Engl J Med,* 2007, vol. 357 (14), 1444-1445
- **MERCIER JC ; HUMMLER H ; DURRMEYER X et al.** Inhaled nitric oxide for prevention of bronchopulmonary dysplasia in premature babies (EUNO): a randomised controlled trial. *Lancet,* 2010, vol. 376 (9738), 346-354

- **KINSELLA JP ; CUTTER GR ; WALSH WF et al.** Early inhaled nitric oxide therapy in premature newborns with respiratory failure. *N Engl J Med,* 2006, vol. 355 (4), 1061-1065
- **FIELD D ; ELBOURNE D ; TRUESDALE A et al.** Neonatal Ventilation With Inhaled Nitric Oxide Versus Ventilatory Support Without Inhaled Nitric Oxide for Preterm Infants With Severe Respiratory Failure: the INNOVO multicentre randomised controlled trial (IS-RCTN 17821339). *Pediatrics,* 2005, vol. 115 (4), 926-936
- **HASCOET JM ; FRESSON J ; CLARIS O et al.** The safety and efficacy of nitric oxide therapy in premature infants. *J Pediatr,* 2005, vol. 146 (3), 318-323
- **MERCIER JC.** The Franco-Belgium Collaborative NO,Trial Group. Early compared with delayed inhaled nitric oxide in moderately hypoxaemic neonates with respiratory failure: a randomised controlled trial. *Lancet,* 1999, vol. 354 (9184), 1066-1071
- **DONOHUE PK ; GILMORE MM ; CRISTOFALO E et al.** Inhaled Nitric Oxide in Preterm Infants: A Systematic Review. *Pediatrics,* 2011
- **ASKIE LM ; BALLARD RA ; CUTTER GR et al.** Inhaled Nitric Oxide in Preterm Infants: An Individual-Patient Data Meta-analysis of Randomized Trials. *Pediatrics,* 2011
- **BARRINGTON KJ ; FINER N.** Inhaled nitric oxide for respiratory failure in preterm infants. *Cochrane Database Syst Rev,* 2010, vol. 12, CD000509
- **HOEHN T ; KRAUSE MF ; BUHRER C.** Meta-analysis of inhaled nitric oxide in premature infants: an update. *Klin Padiatr,* 2006, vol. 218 (2), 57-61
- **HOEHN T ; KRAUSE MF ; BUHRER C.** Inhaled nitric oxide in premature infants--a meta-analysis. *J Perinat Med,* 2000, vol. 28 (1), 7-13
- **SRISUPARP P ; HEITSCHMIDT M ; SCHREIBER MD.** Inhaled nitric oxide therapy in premature infants with mild to moderate respiratory distress syndrome. *J Med Assoc Thai,* 2002, vol. 85 (2), S469-S478
- **WATSON RS ; CLERMONT G ; KINSELLA JP et al.** Clinical and economic effects of iNO in premature newborns with respiratory failure at 1 year. *Pediatrics,* 2009, vol. 124 (5), 1333-1343
- **HIBBS AM ; WALSH-SUKYS MC ; MARTIN RJ et al.** One-year respiratory outcomes of preterm infants enrolled in the Nitric Oxide (to prevent) Chronic Lung Disease trial. *J Pediatr,* 2008, vol. 153 (4), 525-529
- **HOO AF ; BEARDSMORE CS ; CASTLE RA et al.** Respiratory function during infancy in survivors of the INNOVO trial. *Pediatr Pulmonol,* 2009, vol. 44 (2), 155-161
- **WALSH-SUKYS MC ; HIBBS AM ; MARTIN CR et al.** Two-year Neurodevelopmental Outcomes of Ventilated Preterm Infants Treated with Inhaled Nitric Oxide. *J Pediatr,* 2010, vol. 156 (4), 556-561
- **KELLER RL ; WALSH-SUKYS MC ; VITTINGHOFF E ; PALERMO L ; BALLARD PL ; BALLARD RA.** Response to Inhaled Nitric Oxide (iNO) and Neurodevelopmental impairment (NDI) in NO CLD. Baltimore, MD, 2009
- **KINSELLA JP ; CUTTER GR ; WALSH WF et al.** Outcomes of Premature Infants Enrolled in the Early Inhaled Nitric Oxide for the Prevention of Chronic Lung Disease Trial. Baltimore, MD, 2009
- **HINTZ SR ; VAN MEURS KP ; PERRITT R et al.** Neurodevelopmental outcomes of premature infants with severe respiratory failure enrolled in a randomized controlled trial of inhaled nitric oxide. *J Pediatr,* 2007, vol. 151 (1), 16-22
- **MESTAN KK ; MARKS JD ; HECOX K ; HUO D ; SCHREIBER MD.** Neurodevelopmental outcomes of premature infants treated with inhaled nitric oxide. *N Engl J Med,* 2005, vol. 353 (1), 23-32
- **DURRMEYER X ; HUMMLER H ; SANCHEZ-LUNA M et al.** Two-Year Outcomes of a Randomized Controlled Trial of Inhaled Nitric Oxide in Premature Infants. *Pediatrics,* 2013
- **HUDDY CL ; BENNETT CC ; HARDY P et al.** The INNOVO multicentre randomised controlled trial: Neonatal ventilation with Inhaled Nitric Oxide versus Ventilatory support without nitric oxide for severe respiratory failure in preterm infants: follow up at 4-5 years (ISRCTN 1782 1339). *Arch Dis Child Fetal Neonatal Ed,* 2008
- **PATRIANAKOS-HOOBLER AL ; MARKS JD ; MSALL ME ; HUO D ; SCHREIBER MD.** Safety and efficacy of inhaled nitric oxide treatment for premature infants with respiratory distress syndrome: follow-up evaluation at early school age. *Acta Paediatr,* 2011, vol. 100 (4), 524-528
- **HAMON I ; GAGA S ; ESPAGNE S ; MERDARIU D ; DEBRUILLE C ; HASCOET JM.** Clinical Outcome at 7 Years of Age in Former Premature Infants Treated at Birth with Inhaled Nitric Oxide (iNO). Baltimore, MD, 2009
- **HAMON I ; DEFORGE H ; ESPAGNE S ; GAGA S ; SELTON D ; HASCOET JM.** Neurodevelopmental and Cognitive Outcomes at School Age of Premature Infants Treated at Birth with Inhaled Nitric Oxide (iNO). Baltimore, MD, 2009
- **HASCOET JM ; MARCHAL F ; SCHWEITZER C ; TANKEU S ; HAMON I.** Respiratory Outcome at 7 Years of Age in Former Sick Premature Infants Treated with Inhaled Nitric Oxide (iNO) at Birth. Baltimore, MD, 2009
- **BALLARD PL ; TRUOG WE ; MERRILL JD et al.** Plasma biomarkers of oxidative stress: relationship to lung disease and inhaled nitric oxide therapy in premature infants. *Pediatrics,* 2008, vol. 121 (3), 555-561

- COOPER S ; MARKS JD ; MEISEL M ; WHITE MK ; HUO D ; SCHREIBER MD. Birthweight distribution of severe IVH/PVL and death in premature infants treated with inhaled nitric oxide. *Pediatr Res,* 2011, vol. 70 (4
- AMBALAVANAN N ; VAN MEURS KP ; PERRITT R et al. Predictors of death or bronchopulmonary dysplasia in preterm infants with respiratory failure. *Journal of Perinatology: Official Journal of the California Perinatal Association,* 2008, vol. 28 (6), 420-426
- HINTZ S ; VAN MEURS KP. *The NICHD Neonatal RN.. Reliability and Accuracy of Cranial Ultrasound in the NICHD Randomized Controlled Trial of Inhaled Nitrix Oxide for Premature Infants with Severe Respiratory Failure. San Francisco, CA,* 2006
- WHITE M ; MARKS JD ; SCHREIBER SA ; HUO D ; MSALL ME ; SCHREIBER MD. No Effect of Inhaled Nitric Oxide on IVH Extension in Premature Infants with Moderate RDS. *05/02, Poster E-PAS20102849.278*
- KAPLAN H ; LORCH SA ; LUAN X et al. *Role of Inhaled Nitric Oxide in Evolution of Brain Lesions in the Premature Infant. San Francisco, CA,* 2006
- DESANDES R ; DESANDES E ; DROULLE P ; DIDIER F ; LONGROIS D ; HASCOET JM. Inhaled nitric oxide improves oxygenation in very premature infants with low pulmonary blood flow. *Acta Paediatr,* 2004, vol. 93 (1), 66-69
- TRUOG WE ; BALLARD PL ; NORBERG M et al. Inflammatory markers and mediators in tracheal fluid of premature infants treated with inhaled nitric oxide. *Pediatrics,* 2007, vol. 119 (4), 670-678
- IZHAR FM ; RUMILLA K ; KIM Y ; HERSHENSON MB ; SCHREIBER MD. *Inhaled Nitric Oxide Prevents the Increase in Tracheal Aspirate IL-8 Concentrations in Premature Newborn Infants with Respiratory Distress Syndrome. Baltimore, MD,* 2001
- POSENCHEG MA ; GOW AJ ; TRUOG WE et al. Inhaled nitric oxide in premature infants: effect on tracheal aspirate and plasma nitric oxide metabolites. *J Perinatol,* 2010, vol. 30 (4), 275-280
- BALLARD PL ; KELLER RL ; HARMON C ; BALLARD RA. Urinary Nitric Oxide Metabolites in Preterm Infants with Lung Disease. *05/01, Poster E-PAS20101464.117*
- KELLER RL ; VITTINGHOFF E ; PALERMO L ; BALLARD RA ; BALLARD PL ; TRUOG W. *Response to Inhaled Nitric Oxide (iNO): Further analysis of the NO CLD Trial. Baltimore, MD,* 2009
- AHLUWALIA J ; TOOLEY J ; CHEEMA I et al. A dose response study of inhaled nitric oxide in hypoxic respiratory failure in preterm infants. *Early Hum Dev,* 2006, vol. 82 (7), 477-483
- SOKOL GM ; VAN MEURS KP. Does Inhaled Nitric Oxide (iNO) Exhibit Dose Related Toxicity in the Premature Infant with Severe Respiratory Failure? San Francisco, CA. *The NICHD Neonatal RN,* 2006
- CHOCK VY ; VAN MEURS KP ; HINTZ S et al. Inhaled Nitric Oxide for Preterm Premature Rupture of Membranes, Oligohydramnios, and Pulmonary Hypoplasia. *Am J Perinatol,* 2009, vol. 26 (4), 317-322
- DI FIORE JM ; HIBBS AM ; ZADELL AE et al. The effect of inhaled nitric oxide on pulmonary function in preterm infants. *J Perinatol,* 2007, vol. 27 (12), 766-771
- IZHAR FM ; RUMILLA K ; BORG MJ ; KIM Y ; HERSHENSON MB ; SCHREIBER MD. *Pulmonary Safety of Inhaled Nitric Oxide in Premature Newborn Infants with Respiratory Distress. Boston, MA,* 2000
- TRUFFERT P ; LLADO-PARIS J ; MERCIER JC ; DEHAN M ; BREART G. Early inhaled nitric oxide in moderately hypoxemic preterm and term newborns with RDS: the RDS subgroup analysis of the Franco-Belgian iNO Randomized Trial. *Eur J Pediatr,* 2003, vol. 162 (9), 646-647
- GORDON PV ; ASCHNER JL. The Preemie iNO Subcommittee on Behalf of the NICHD Neonatal,Research Network.. Can Inhaled Nitric Oxide (iNO) Alter the Natural History of Spontaneous Intestinal Perforations (SIP) in the ELBW Infant?. *A Post Hoc Analysis of the NICHD Preemie iNO Study. San Francisco, CA*
- BALLARD PL ; MERRILL JD ; TRUOG WE et al. Surfactant function and composition in premature infants treated with inhaled nitric oxide. *Pediatrics,* 2007, vol. 120 (2), 346-353
- AIKIO O ; METSOLA J ; VUOLTEENAHO R ; PERHOMAA M ; HALLMAN M. Transient Defect in Nitric Oxide Generation after Rupture of Fetal Membranes and Responsiveness to Inhaled Nitric Oxide in Very Preterm Infants with Hypoxic Respiratory Failure. *J Pediatr,* 2012
- SHARMA S ; PALAFOUTAS J ; RAMASETHU J. Inhaled nitric oxide (iNO) in management of pulmonary hypertension (PPHN) in preterm infants. *Pediatric Academic Societies and Asian Society for Pediatric Research Annual Meeting, April 30 to May 3, 2011, Denver, CO,* 30 April 2011, http://www.abstracts2view.com/pas/view.php?nu=PAS11L1 1791
- O'DONNELL DC ; FRANTA J ; MILETIN J. The use of Inhaled Nitric Oxide in Premature Infants with Premature Rupture of Membranes: A Case Series. *Neonatology,* 2010

- **DEWHURST C ; IBRAHIM H ; GOTHBERG S ; JONSSON B ; SUBHEDAR N.** Use of inhaled nitric oxide in the new born period: results from the European inhaled nitric oxide registry. *Acta Paediatr,* 2010
- **KUMAR VH ; HUTCHISON AA ; LAKSHMIN-RUSIMHA S ; MORIN FC,III ; WYNN RJ ; RYAN RM.** Characteristics of pulmonary hypertension in preterm neonates. *J Perinatol,* 2007, vol. 27 (4), 214-219
- **SU PH ; CHEN JY.** Inhaled nitric oxide in the management of preterm infants with severe respiratory failure. *J Perinatal,* 2008, vol. 28 (2), 112-116
- **KHEMANI E ; MCELHINNEY DB ; RHEIN L et al.** Pulmonary artery hypertension in formerly premature infants with bronchopulmonary dysplasia: clinical features and outcomes in the surfactant era. *Pediatrics,* 2007, vol. 120 (6), 1260-1269
- **TANAKA Y ; HAYASHI T ; KITAJIMA H ; SUMI K ; FUJIMURA M.** Inhaled nitric oxide therapy decreases the risk of cerebral palsy in preterm infants with persistent pulmonary hypertension of the newborn. *Pediatrics,* 2007, vol. 119 (6), 1159-1164
- **DANI C ; BERTINI G.** Inhaled nitric oxide for the treatment of preterm infants with respiratory distress syndrome. *Neonatology,* 2008, vol. 94 (2), 87-95
- **DANI C ; BERTINI G ; PEZZATI M ; FILIPPI L ; CECCHI A ; RUBALTELLI FF.** Inhaled nitric oxide in very preterm infants with severe respiratory distress syndrome. *Acta Paediatr,* 2006, vol. 95 (9), 1116-1123
- **LINDWALL R ; BLENNOW M ; SVENSSON M et al.** A pilot study of inhaled nitric oxide in preterm infants treated with nasal continuous positive airway pressure for respiratory distress syndrome. *Intensive Care Med,* 2005, vol. 31 (7), 959-964
- **UGA N ; ISHII T ; KAWASE Y ; ARAI H ; TADA H.** Nitric oxide inhalation therapy in very low-birthweight infants with hypoplastic lung due to oligohydramnios. *Pediatr Int,* 2004, vol. 46 (1), 10-14
- **ATHAVALE K ; CLAURE N ; D'UGARD C ; EVERETT R ; SWAMINATHAN S ; BANCALARI E.** Acute effects of inhaled nitric oxide on pulmonary and cardiac function in preterm infants with evolving bronchopulmonary dysplasia. *J Perinatol,* 2004, vol. 24 (12), 769-774
- **AIKIO O ; SAARELA T ; POKELA ML ; HALLMAN M.** Nitric oxide treatment and acute pulmonary inflammatory response in very premature infants with intractable respiratory failure shortly after birth. *Acta Paediatr,* 2003, vol. 92 (1), 65-69
- **CLARK PL ; EKEKEZIE II ; KAFTAN HA ; CASTOR CA ; TRUOG WE.** Safety and efficacy of nitric oxide in chronic lung disease. *Arch Dis Child Fetal Neonatal Ed,* 2002, vol. 86 (1), F41-F45
- **TRUOG WE ; PALLOTTO E ; CLARK P et al.** Interaction of endogenous endothelin-1 and inhaled nitric oxide in term and preterm infants. *Clin Sci (Lond),* 2002, vol. 103 (48), 294S-297S
- **DU LZ ; SHI LP ; ZHOU BH et al.** Inhaled nitric oxide in preterm and term neonates with hypoxemic respiratory failure and persistent pulmonary hypertension. *Acta Pharmacol Sin,* 2002, vol. 23, 69-73
- **BANKS BA ; SERI I ; ISCHIROPOULOS H ; MERRILL J ; RYCHIK J ; BALLARD RA.** Changes in oxygenation with inhaled nitric oxide in severe bronchopulmonary dysplasia. *Pediatrics,* 1999, vol. 103 (3), 610-618
- **CHEUNG PY ; PELIOWSKI A ; ROBERTSON CM.** The outcome of very low birth weight neonates (</=1500 g) rescued by inhaled nitric oxide: neurodevelopment in early childhood. *J Pediatr,* 1998, vol. 133 (6), 735-739
- **LAUBSCHER B ; GREENOUGH A ; KAVVADIA V ; DEVANE SP.** Response to nitric oxide in term and preterm infants. *Eur J Pediatr,* 1997, vol. 156 (8), 639-642
- **SKIMMING JW ; BENDER KA ; HUTCHISON AA ; DRUMMOND WH.** Nitric oxide inhalation in infants with respiratory distress syndrome. *J Pediatr,* 1997, vol. 130 (2), 225-230
- **SUBHEDAR NV ; RYAN SW ; SHAW NJ.** Open randomised controlled trial of inhaled nitric oxide and early dexamethasone in high risk preterm infants. *Arch Dis Child Fetal Neonatal Ed,* 1997, vol. 77 (3), F185-F190
- **PELIOWSKI A ; FINER NN ; ETCHES PC ; TIERNEY AJ ; RYAN CA.** Inhaled nitric oxide for premature infants after prolonged rupture of the membranes. *J Pediatr,* 1995, vol. 126 (3), 450-453
- **BENNETT AJ ; SHAW NJ ; GREGG JE ; SUBHEDAR NV.** Neurodevelopmental outcome in high-risk preterm infants treated with inhaled nitric oxide. *Acta Paediatr,* 2001, vol. 90 (5), 573-576
- **SUBHEDAR NV ; SHAW NJ.** Changes in oxygenation and pulmonary haemodynamics in preterm infants treated with inhaled nitric oxide. *Arch Dis Child Fetal Neonatal Ed,* 1997, vol. 77 (3), F191-F197
- **COLE FS ; ALLEYNE C ; BARKS JD et al.** NIH Consensus Development Conference Statement: Inhaled Nitric-Oxide Therapy for Premature Infants. *Pediatrics,* 2011
- **ABMAN SH ; HANSMANN G ; ARCHER SL et al.** Pediatric Pulmonary Hypertension: Guidelines From the American Heart Association and American Thoracic Society. *Circulation,* 2015
- **TRUOG WE ; NELIN LD ; DAS A et al.** Inhaled nitric oxide usage in preterm infants in the NICHD neonatal research network: inter-site variation and propensity evaluation. *J Perinatol,* 2014

- Use of inhaled nitric oxide in preterm infants. **KUMAR P.** Pediatrics. Committee on Fetus and Newborn, American Academy of Pediatrics, 2014, vol. 133, 164-170
- **BALLARD PL ; GONZALES LW ; GODINEZ RL et al.** Surfactant composition and function in a primate model of infant chronic lung disease: effects of inhaled nitric oxide. *Pediatr Res,* 2006, vol. 59 (1), 157-162
- **SCHREIBER MD ; MARKS JD ; BALLARD R et al.** Response to Kumar on behalf of the AAP Committee on Fetus and Newborn. *Pediatrics [Replies to Use of Inhaled Nitric Oxide in Preterm Infants,* 2014, vol. 133 (1), 164-170
- **BARRINGTON KJ ; FINER NN.** Inhaled nitric oxide for respiratory failure in preterm infants. *Cochrane Database Syst Rev,* 2009, vol. 1, CD000509
- **HAMON I ; FRESSON J ; NICOLAS MB ; BUCHWEILLER MC ; FRANCK P ; HASCOET JM.** Early inhaled nitric oxide improves oxidative balance in very preterm infants. *Pediatr Res,* 2005, vol. 57 (5), 637-643
- **VAN MEURS KP ; STEVENSON DK ; EHRENKRANZ RA et al.** *Inhaled Nitric Oxide for Preterm Infants with Severe Respiratory Failure. San Francisco, CA,* 2004
- **VAN MEURS KP ; BENITZ W ; HINTZ S ; BENDER RH ; POOLE K.** Does Oxygenation Index Correlate with Outcome in Premature Infants with Respiratory Failure? Washington, DC. NICHD Neonatal,Research Network, 2005

- **FINER NN.** Inhaled nitric oxide for preterm infants: a therapy in search of an indication? The search continues. *J Pediatr,* 2005, vol. 146 (3), 301-303
- **KUMAR A ; ZARYCHANSKI R ; PINTO R et al.** Critically Ill Patients With 2009 Influenza A(H1N1) Infection in Canada. *JAMA,* 2009, vol. 302 (17), 1872-1879
- **DAVIDSON D ; BAREFIELD ES ; KATTWINKEL J et al.** Inhaled nitric oxide for the early treatment of persistent pulmonary hypertension of the term newborn: a randomized, double-masked, placebo-controlled, dose-response, multicenter study. *Pediatrics,* 1998, vol. 101 (3), 325-334
- **YADAV M ; EMMERSON AJ.** Inhaled nitric oxide in premature neonates. *Lancet,* 1999, vol. 354 (9196), 2162-2163
- **KONDURI GG ; VOHR B ; ROBERTSON C et al.** Early inhaled nitric oxide therapy for term and near-term newborn infants with hypoxic respiratory failure: neurodevelopmental follow-up. *J Pediatr,* 2007, vol. 150 (3), 235-40
- **HAMPTON, NJ.** INOmax® (nitric oxide) for inhalation 100 and 800 ppm (parts per million) [package insert. *INO Therapeutics,* 2013
- **LOH E ; STAMLER JS ; HARE JM ; LOSCALZO J ; COLUCCI WS.** Cardiovascular effects of inhaled nitric oxide in patients with left ventricular dysfunction. *Circulation,* 1994, vol. 90 (6), 2780-2785
- **BARST RJ ; AGNOLETTI G ; FRAISSE A ; BALDASSARRE J ; WESSEL DL.** Vasodilator Testing with Nitric Oxide and/or Oxygen in Pediatric Pulmonary Hypertension. *Pediatr Cardiol,* 2010